# EUROPEAN PATENT APPLICATION

(11) **EP 4 538 261 A1**
(43) Date of publication of application: **16.04.2025**
(21) Application number: 23819180.3
(22) Date of filing: 07.06.2023
(51) Int. Cl.: C07D 257/04, C07D 401/14, C07D 401/12, C07D 401/10, C07D 405/14, C07D 405/10, C07D 405/12, A61K 31/41, A61P 29/00

(54) **N-TETRAZOLYL ARYL UREA DERIVATIVES, PREPARATION METHOD THEREFOR, AND USE THEREOF**

(30) Priority: 07.06.2022 CN 202210643732
(71) Applicant: Hangzhou Yirui Pharmaceutical Technology Co., Ltd., Hangzhou, Zhejiang 310018 (CN)
(72) Inventor: ZHOU, Xuefei, Hangzhou, Zhejiang 310018 (CN); WANG, Xiaolu, Hangzhou, Zhejiang 310018 (CN); CHENG, Gang, Hangzhou, Zhejiang 310018 (CN); YE, Qing, Hangzhou, Zhejiang 310018 (CN); SHEN, Tao, Hangzhou, Zhejiang 310018 (CN)
(74) Representative: Abel & Imray LLP
(86) International application number: PCT/CN2023/098927
(87) International publication number: WO 2023/237015

(57) **Abstract**

The present invention relates to the field of biological medicines. Disclosed are N-tetrazolyl aryl urea derivatives, a preparation method therefor, and the use thereof. The present invention has recently discovered the N-tetrazolyl aryl urea derivatives, and further found that the compounds have an antagonistic activity against bradykinin B1 receptor, so that the compounds can be used as novel bradykinin B1 receptor antagonists and can be further used for treating or preventing related diseases caused by abnormal expression of the bradykinin B1 receptor. In particular, the present invention has recently found that N-tetrazolyl aryl urea derivatives have prevention or treatment effects on corona virus disease 2019, pulmonary edema, diabetes complications, allergic conjunctivitis, etc.

## Description

### Field of the Invention

The present invention relates to the field of biological medicines, and in particular to an N-tetrazolyl aryl urea-based derivative as a bradykinin B1 receptor antagonist, a method for preparing the same, a pharmaceutical composition containing the same, and a use thereof as a medicament for treating and preventing diseases caused by abnormal expression of the bradykinin B1 receptor.

### Background of the Invention

Endogenous bradykinin B1 receptor agonist is produced by the activated kallikrein-kinin system, including bradykinin des-Arg9-BK and Lys-des-Arg9-BK. Bradykinin is a vasoactive peptide that is generated by the activation of prokallikrein via the action of kallikrein during inflammatory responses. Bradykinin may specifically bind to bradykinin receptors, thereby activating inflammatory signaling pathways and promoting a series of biological responses such as vasodilation, increased vascular permeability, non-vascular smooth muscle contraction, inflammatory responses, and pain. Currently, there are two known subtypes of bradykinin receptors, namely B1 and B2 receptors (B 1R and B2R). These two receptors have a structure of G protein-coupled receptor (GPCR), but they play many different biological functions in the human body (Rizzi A 1997; L. M. FREDRIK LEEB-LUNDBERG 2005).

Under physiological conditions, B1 receptor is hardly expressed in healthy tissues, and its expression is only induced by endogenous stimulating factors such as endotoxins, cytokines, and growth factors that are produced during tissue injury. For example, the expression of B1 receptor is found to be upregulated in inflammatory conditions of various diseases (such as asthma, pneumonia, arthritis, diabetes, endometriosis, ulcerative colitis, Crohn's disease, etc.) and in some neurological diseases (such as epilepsy, stroke, and multiple sclerosis, etc.). IL-1β may directly upregulate bradykinin B1 receptor (Phagoo SB 1999), whereas upon activation of bradykinin B2 receptor, it further induces upregulation of B1 receptor by activating NF-κB and thereby leading to the expression of IL-1β in fibroblasts (L. M. FREDRIK LEEB-LUNDBERG 2005). Upon upregulation, bradykinin B1 receptor is mainly expressed on macrophages, neutrophils, fibroblasts, neurons, smooth muscle cells, and vascular endothelium (Christopher I Fincham 2009). This is in distinguished from the B2 receptor, which is expressed at a persistently high level in healthy tissues such as vascular and non-vascular smooth muscle and cardiac tissue.

Activation of bradykinin B1 receptor produces pain, promotes inflammatory responses, increases vascular permeability, and promotes tissue fibrosis. In contrast to bradykinin B2 receptor, bradykinin B1 receptor does not undergo cellular internalization and desensitization under repeated stimulation by agonists, and activation of bradykinin B1 receptor triggers autoinduction, which may lead to the amplification and persistence of inflammatory or pain processes (Phagoo SB 1999; Westermann, D. 2009; Walsh, D.A. et al., 2006; Farkas S. et al., 2011).

One of action mechanisms of bradykinin B1 receptor is to induce gene expression and production of interleukins such as IL-6 and IL-8, promote production of PGE2 (prostaglandin 2) and thereby activate inflammation-related prostaglandin signaling pathways, phosphorylation and upregulation of TRPV1 (transient receptor potential vanilloid 1) receptors. Those receptors further trigger pain responses, enhance pain transduction, and induce neurogenic inflammation by releasing neuropeptides in inflamed tissues (Ch Golias 2007, R Hayashi 2000).

Bradykinin B1 receptor plays a key role in many diseases, especially chronic inflammatory lesions. Anti-inflammatory and analgesic effects have been shown by blocking bradykinin B1 receptor in animal models, supporting the potential pharmacological effects of the bradykinin B1 receptor antagonist (Campos, M.M. et al. 2006; Wang, PH. et al. 2009; Passos, G.F. et al. 2013; Gobeil, F. et al. 2014; Huart, A. 2015; Ferreira, J. et al. 2001; Ferreira, J. et al. 2005; Gougat, J.B. et al. 2004; Fox, A. et al. 2005). Given that bradykinin B1 receptor is not expressed in normal tissues, but are only expressed and upregulated in cases of tissue injury and lesions, thus the bradykinin B1 receptor inhibitor has a therapeutic safety.

Therefore, it is necessary to continuously develop more novel bradykinin B1 receptor antagonists.

### Summary of the Invention

The present invention provides an N-tetrazolyl aryl urea-based derivative, a method for preparing the same, and the use of the same. The present inventors have newly discovered a class of N-tetrazolyl aryl urea-based derivative, and found that such a compound has an antagonistic activity against bradykinin B1 receptor. Thus, the compound is useful as a novel bradykinin B1 receptor antagonist, and is further useful for treating or preventing related diseases caused by abnormal expression of the bradykinin B1 receptor.

The specific technical solutions of the present invention are as follows.

In one aspect, the present invention provides an N-tetrazolyl aryl urea-based derivative, which is a compound represented by Formula I, or a cis-trans isomer thereof, or a pharmaceutically acceptable salt or solvate thereof;

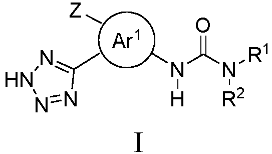

In Formula I, R¹ and R² together with the N atom attached thereto may form a 3- to 8-membered heterocyclic group containing at least 1 nitrogen atom. Optionally, the 3- to 8-membered heterocyclic group may be substituted by 1 to 3 of identical or different functional groups A, wherein the functional group A may be selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, alkylamino, dialkylamino, acetylamino and cycloamino.

Alternatively, R¹ and R² together with the N atom attached thereto may form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂. The 8- to 12-membered bicyclic heterocyclic group may comprise a spiro heterocyclic group, a bridged bicyclic heterocyclic group and a fused bicyclic heterocyclic group. Optionally, the 8- to 12-membered bicyclic heterocyclic group may be substituted by 1 to 3 of identical or different functional groups B, wherein the functional group B may be selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

Alternatively, each of R¹ and R² may be independently selected from the group consisting of the following groups:
(i) H, C₁-C₁₀ alkyl, C₂-C₁₀ alkenyl and C₂-C₁₀ alkynyl, optionally, C₁-C₁₀ alkyl, C₂-C₁₀ alkenyl, or C₂-C₁₀ alkynyl may be substituted by 1 to 3 of identical or different functional groups C, wherein the functional group C may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino; or
(ii) phenyl, 5- to 6-membered heteroaryl, C₅-C₇ cycloalkyl and 5- to 7-membered heterocyclic group, wherein the 5- to 6-membered heteroaryl or 5- to 7-membered heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, C₅-C₇ cycloalkyl or 5- to 7-membered heterocyclic group may be substituted by 1 to 3 of identical or different functional groups D, wherein the functional group D may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

In Formula I, Ar¹ may be selected from the group consisting of phenyl, 5-to 6-membered heteroaryl, C₅-C₇ cycloalkyl and C₉-C₁₀ dicycloalkyl, wherein the 5- to 6-membered heteroaryl contains 1 or 2 heteroatoms or heteroatom-containing groups independently selected from the group consisting of NH, N, O, S, SO and SO₂. Optionally, the phenyl, 5- to 6-membered heteroaryl, C₅-C₇ cycloalkyl or C₉-C₁₀ dicycloalkyl may be substituted by 1 to 3 of identical or different functional groups E, wherein the functional group E may be selected from the group consisting of hydroxyl, halogen, amino, cyano, nitro, C₁-C₆ alkyl, C₁-C₆- alkoxy, halogenated C₁-C₆ alkyl, halogenated C₁-C₆ alkoxy, alkylamino, dialkylamino, acetylamino, N-methyl-N-acetylamino and cycloamino.

In Formula I, Z may be selected from the group consisting of phenyl, 5- to 6-membered heteroaryl, C₅-C₇ cycloalkyl, 5- to 7-membered heterocyclic group, C₉-C₁₀ dicycloalkyl, 9- to 10-membered fused bicyclic heterocyclic group, 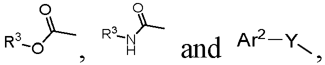 wherein the 5- to 6-membered heteroaryl, 5- to 7-membered heterocyclic group or 9- to 10-membered fused bicyclic heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, C₅-C₇ cycloalkyl, 5- to 7-membered heterocyclic group, C₉-C₁₀ dicycloalkyl or 9- to 10-membered fused bicyclic heterocyclic group may be substituted by 1 to 3 of identical or different functional groups F, wherein the functional group F may be selected from the group consisting of hydroxyl, halogen, amino, cyano, nitro, C₁-C₆ alkyl, C₃-C₇ cycloalkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino, or alternatively, the functional group F may form a 5- to 8-membered heterocyclic ring fused to a phenyl, a 5- to 6-membered heteroaryl, a C₅-C₇ cycloalkyl, a 5- to 7-membered heterocyclic group, a C₉-C₁₀ dicycloalkyl, or a 9- to 10-membered fused bicyclic heterocyclic group,

R³ may be selected from the group consisting of C₁-C₆ alkyl, C₃-C₇ cycloalkyl, C₃-C₇ cycloalkenyl, 5- to 7-membered heterocyclic group, phenyl and 5- to 6-membered heteroaryl. Optionally, the C₁-C₆ alkyl, C₃-C₇ cycloalkyl, C₃-C₇ cycloalkenyl, 5- to 7-membered heterocyclic group, phenyl or 5- to 6-membered heteroaryl may be substituted by 1 to 3 of identical or different functional groups G, wherein the functional group G may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

Y may be selected from the group consisting of O or NH.

Ar² may be selected from the group consisting of phenyl, 5- to 6-membered heteroaryl, C₁₀-C₂₀ fused cyclic group, C₁-C₆ alkyl, 5- to 7-membered cycloalkyl, 5- to 7-membered heterocyclic group, and C₁-C₆ alkoxy. The 5- to 6-membered heteroaryl or 5- to 7-membered heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, C₁₀-C₂₀ fused cyclic group, C₁-C₆ alkyl, 5- to 7-membered cycloalkyl, 5- to 7-membered heterocyclic group, or C₁-C₆ alkoxy may be substituted by 1 to 3 of identical or different functional groups H, wherein the functional group H may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

In another aspect, the present invention provides an N-tetrazolyl aryl urea-based derivative, which is a compound represented by Formula I, or a cis-trans isomer thereof, or a pharmaceutically acceptable salt or solvate thereof.

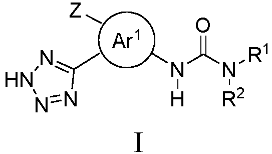

Wherein:
R¹ and R² together with the N atom attached thereto form a 3- to 8-membered heterocyclic group containing at least 1 nitrogen atom, wherein the above groups are substituted by 1 to 3 of identical or different functional groups A, and the functional group A is selected from the group consisting of hydroxyl, halogen, amino, cyano, nitro, C₁-C₃ alkyl, C₁-C₃ alkenyl, C₁-C₃ alkynyl, C₁-C₃ alkoxy, halogenated C₁-C₃ alkyl, C₁-C₃ fluoroalkoxy, alkylamino, dialkylamino, acetylamino or cycloamino; or
R¹ and R² together with the N atom attached thereto form an 8- to 12-membered bicyclic group containing at least 1 nitrogen atom and additionally containing 0, 1 or 2 heteroatoms or heteroatom-containing groups independently selected from the group consisting of NH, N, O, S, SO and SO₂, wherein the bicyclic group comprises a spiro cycloalkyl, a bridged bicycloalkyl and a fused bicycloalkyl, and the above groups are substituted by 1 to 3 of identical or different functional groups B, and the functional group B is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino; or
Each of R¹ and R² is independently selected from the group consisting of the following groups:
   (i) H, C₁-C₁₀ alkyl, C₂-C₁₀ alkenyl, C₂-C₁₀ alkynyl, wherein the above groups except for H are substituted by 1 to 3 of identical or different functional groups C, and the functional group C is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino; or
   (ii) phenyl, 5- to 6-membered heteroaryl, C₅-C₇ cycloalkyl, 5- to 7-membered heterocyclic group, wherein the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, heteroatom or heteroatom-containing group is substituted by 1 to 3 of identical or different functional groups D, and the functional group D is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino;
Ar¹ is selected from the group consisting of:
   phenyl, 5- to 6-membered heteroaryl, C₅-C₇ cycloalkyl, C₉-C₁₀ fused bicyclic group, wherein the 5- to 6-membered heteroaryl contains 1 or 2 heteroatoms or heteroatom-containing groups independently selected from the group consisting of NH, N, O, S, SO and SO₂, and the phenyl, heteroatom or heteroatom-containing group is substituted by 1 to 3 of identical or different functional groups E, and the functional group E is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino;
   Z is selected from the group consisting of the following groups:
      (i) phenyl, 5- to 6-membered heteroaryl, C₅-C₇ cycloalkyl, 5- to 7-membered heterocyclic group, C₉-C₁₀ fused bicyclic group, 9- to 10-membered fused heterobicyclic group, wherein the 5- to 6-membered heteroaryl, 5- to 7-membered heterocyclic group or 9- to 10-membered fused heterobicyclic group contains 1, 2 or 3 heteroatoms or heteroatom-containing groups independently selected from the group consisting of NH, N, O, S, SO and SO₂, and the phenyl, heteroatom or heteroatom-containing group is substituted by 1 to 3 of identical or different functional groups F, and the functional group F is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino and cycloamino, or alternatively, the functional groups F form a 5- to 8-membered heterocyclic ring; or
      (ii) 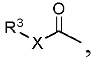 wherein X is selected from the group consisting of O or NH, and R³ is selected from the group consisting of C₁-C₆ alkyl, C₃-C₇ cycloalkyl, C₃-C₇ cycloalkenyl, 5- to 7-membered heterocyclic group, phenyl, 5- to 6-membered heteroaryl, and the above groups are substituted by 1 to 3 of identical or different functional groups G, and the functional group G is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
      (iii) 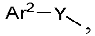 wherein Y is selected from the group consisting of O or NH, and Ar² is selected from the group consisting of phenyl, 5- to 6-membered heteroaryl, C₁₀-C₂₀ fused cyclic group, C₁-C₆ alkyl, 5- to 7-membered cycloalkyl, 5- to 7-membered heterocyclic group, C₁-C₆ alkoxy, wherein the above groups are substituted by 1 to 3 of identical or different functional groups H, and the functional group H is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂.

The present inventors have newly discovered that the above-mentioned N-tetrazolyl aryl urea-based derivative has an antagonistic activity against bradykinin B1 receptor. Thus, the compound is useful as a class of novel bradykinin B1 receptor antagonist, and is further useful for treating or preventing related diseases caused by abnormal expression of the bradykinin B1 receptor. Preferably, the above-mentioned N-tetrazolyl aryl urea-based derivative has good selectivity for B1R over bradykinin B2 receptor (B2R). As compared with many B1 antagonists reported so far, the above-mentioned N-tetrazolyl aryl urea-based derivative of the present invention is characterized by containing a structure of urea functional group. The present inventors have searched through scifinder and found that there is no report in the prior art that the N-tetrazolyl aryl urea-based derivative has an activity against B1.

In a third aspect, the present invention provides a method for preparing the above-mentioned N-tetrazolyl aryl urea-based derivative, comprising the steps as set forth below.

Method 1: for the compound represented by Formula I, wherein R¹ and R² are non-hydrogen groups, or R¹ and R² together with the N atom attached thereto form a heterocyclic group containing at least 1 nitrogen atom, the method for preparing is set forth below.

A 2-halogenated-5-nitroaromatic carbonitrile represented by Formula a is coupled with Z-B(OH)₂ boric acid represented by Formula b under an alkaline condition to obtain a compound represented by Formula c. The cyano group in a compound represented by Formula c is subjected to addition with sodium azide to obtain a tetrazolyl intermediate represented by Formula d. The tetrazolyl intermediate is protected by Trt- group to obtain an intermediate represented by Formula e. Then, the nitro group is reduced to obtain an amino compound represented by Formula f. The amino compound is converted to an isocyanate compound represented by Formula g. The isocyanate compound is reacted with an amine to produce a urea compound represented by Formula h. Finally, Trt- group is removed from the urea compound to obtain the target compound.

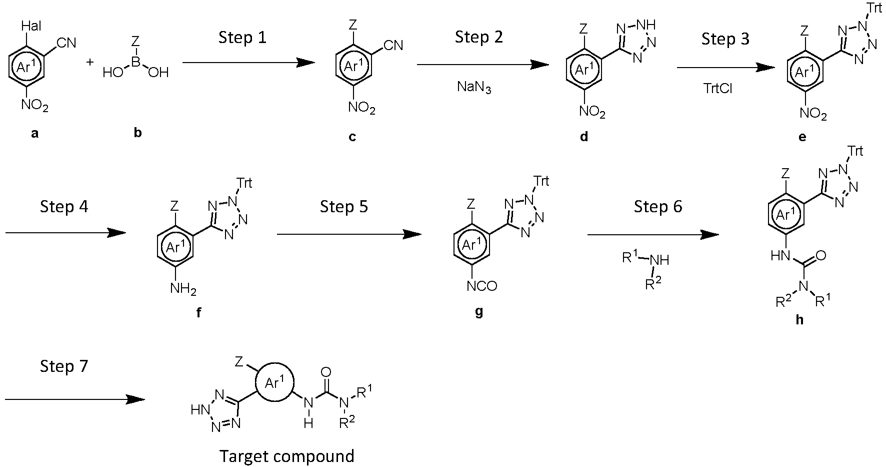

Compounds 1-14, 18, 59-62 in the Examples were prepared according to the above Method 1.

Method 2: for the compound represented by Formula I, wherein Z is substituted or unsubstituted phenoxy, the method for preparing is set forth below.

A 2-halogenated-5-nitroaromatic carbonitrile represented by Formula a is subjected to to a nucleophilic substitution reaction with a substituted phenol represented by Formula b2 under an alkaline condition, to obtain a phenol ether-based intermediate represented by Formula c2. The remaining steps are the same as Steps 2, 3, 4, 5, 6 and 7 as described in Method 1 to finally obtain the corresponding target compound.

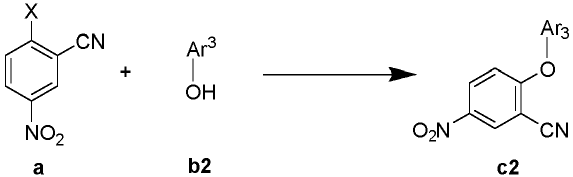

Compounds 63 and 64 in the Examples were prepared according to the above Method 2.

Method 3: for the compound represented by Formula I, wherein Z is 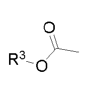 or 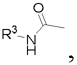 Steps 2, 3, 4, 5, 6 and 7 as described in Method 1 are performed except that the compound represented by Formula c in Method 1 is replace with 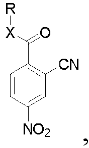 to finally obtain the target product.

Compounds 15 and 17 in the Examples were prepared according to the above Method 3.

Method 4: for the compound represented by Formula I, wherein one of R¹ or R² is a hydrogen atom, at first, Steps 1, 2, 3 and 4 in Method 1 were performed to obtain the amino intermediate represented by Formula f. Then, the amino intermediate is directly reacted with an isocyanate compound to produce a urea compound represented by Formula h1. Finally, the urea compound is subjected to Trt-deprotection to obtain the target compound.

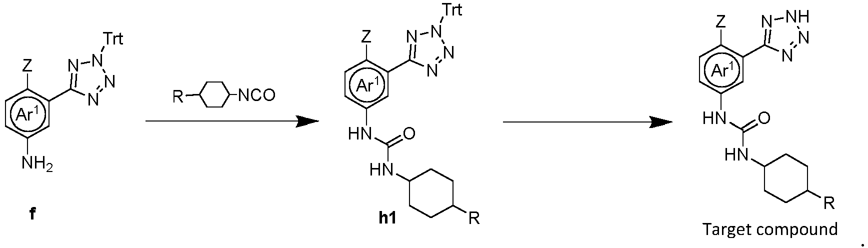

Compounds 58-62 and 66-71 in the Examples were prepared according to the above Method 4.

In a fourth aspect, the present invention provides a pharmaceutical composition comprising the above-mentioned N-tetrazolyl aryl urea-based derivative, a pharmaceutically acceptable carrier or excipient, and optionally another therapeutic agent.

In a fifth aspect, the present invention provides a use of the above-mentioned N-tetrazolyl aryl urea-based derivative in the preparation of a bradykinin B1 receptor antagonist.

In a sixth aspect, the present invention provides a use of the above-mentioned N-tetrazolyl aryl urea-based derivative as a bradykinin B1 receptor antagonist.

In a seventh aspect, the present invention provides a use of the above-mentioned N-tetrazolyl aryl urea-based derivative alone or in combination with other drugs in the preparation of a medicament for preventing or treating a disease mediated by the bradykinin B1 receptor.

In an eighth aspect, the present invention provides the above-mentioned N-tetrazolyl aryl urea-based derivative, alone or in combination with other drugs, for use of prevention or treatment of a disease mediated by the bradykinin B1 receptor.

In a ninth aspect, the present invention provides a method for preventing or treating a disease mediated by the bradykinin B1 receptor in a subject, comprising administering an effective amount of the above-mentioned N-tetrazolyl aryl urea-based derivative or the pharmaceutical composition to the subject.

The above-mentioned disease mediated by the bradykinin B1 receptor may include, but are not limited to, respiratory diseases, digestive diseases, cardiovascular diseases, urinary diseases, Dermatologic diseases, ophthalmic diseases, joint and bone diseases, central and peripheral nervous system diseases, infectious diseases, pain, diabetic complications, trauma and ophthalmic inflammatory diseases.

As non-limiting examples, the above-mentioned disease mediated by the bradykinin B1 receptor may be selected from the group consisting of acute pneumonia, pulmonary edema and acute respiratory distress syndrome caused by COVID-19, diabetic retinopathy, age-related macular degeneration, diabetic neuropathy, allergic conjunctivitis, chronic conjunctivitis and uveitis

As compared with the prior art, the beneficial effects of the present invention are as follows.
(1) The present inventors have newly discovered a class of N-tetrazolyl aryl urea-based derivative, which is a structurally novel selective B1R antagonist, and has been proven to have a high anti-target activity, a strong selectivity, especially a strong selectivity over B2R, a good stability *in vivo,* and have no inhibitory or inductive effects on various CYPs. Therefore, the compound may be further useful for treating or preventing related diseases caused by abnormal expression of the bradykinin B1 receptor.
(2) The present inventors have newly discovered that the N-tetrazolyl aryl urea-based derivative is effective in the prevention or treatment of diseases such as COVID-19, pulmonary edema, diabetes-related complications, and ophthalmic inflammatory diseases. The pharmacodynamic studies in the present invention reveals that the medicament containing such a compound can inhibit the production and secretion of various inflammatory factors by IMR-90 cells induced by bradykinin des-Arg9-BK. In a mouse model of LPS-induced acute lung injury and ARDS, the compound can alleviate the lung injury and pulmonary edema of the mouse, and improve the declining tendancy of blood oxygen partial pressure and oxygen saturation. In a rat model of diabetic retinopathy, such a compound can improve the retinal inflammatory response caused by diabetes, as well as reduce permeability and osmosis of retinal vascular and stasis number of retinal leukocytes. In a mouse model of allergic conjunctivitis, such a compound can significantly improve ocular clinical symptoms such as conjunctival edema, conjunctival congestion, eyelid congestion and edema, and tearing, improve inflammatory cell infiltration and conjunctival tissue, and significantly reduce degranulation of mast cells in allergic responses, and reduce the expression of allergy markers of IL-4 and IgE. Therefore, the compound or he pharmaceutical composition of the present invention is expected to be useful for treating or preventing pneumonia and pulmonary edema caused by COVID-19, so as to reduce the risk of developing acute respiratory distress syndrome and improve the survival rate of the patients. The compound or the pharmaceutical composition of the present invention is also expected to be useful for treating and preventing diabetic retinopathy and age-related macular degeneration. In addition, the compound or the pharmaceutical composition of the present invention is also expected to be useful for treating or preventing ophthalmic inflammatory diseases such as allergic conjunctivitis, chronic conjunctivitis and uveitis.

### Description of the Figures

Figure 1 is a comparison diagram of the vascular permeability data of the rat model in different treatment groups in Test Example 3; in which ## indicates P<0.01 as compared with the negative control group; * indicates P<0.05 as compared with the model group; and ** indicates P<0.01 as compared with the model group;
Figure 2 is a comparison diagram of the IL-6 expression level (ELISA) data for the samples of serum, lung tissue, and bronchoalveolar lavage fluid of rats in different treatment groups in Test Example 3;
Figure 3 is a comparison diagram of the TNF-α expression level (ELISA) data for the samples of serum, lung tissue, and bronchoalveolar lavage fluid of rats in different treatment groups in Test Example 3;
Figure 4 is a comparison diagram of the intraocular distribution concentration (HPLC) data for different compounds administered to rats by eye drops in Test Example 4;
Figure 5 is a statistical diagram of the retinal vascular permeability density values for rats in different treatment groups in Test Example 4; in which ##P< 0.01, #P<0.05, as compared with the negative control group; and **P<0.01, *P <0.05, as compared with the model group;
Figure 6 is a retinal permeability staining graph (200X) for rats in different treatment groups in Test Example 4; in which the white arrow indicates permeation;
Figure 7 is a statistical diagram of retinal leukocyte counts for rats in different treatment groups in Test Example 4;
Figure 8 is a fluorescent staining graph (200X) of retinal vascular leukocytes for rats in different treatment groups in Test Example 4;
Figure 9 is a comparison diagram of expression level data of inflammation-related factors detected by qRT-PCR in different treatment groups in Test Example 4; in which ##P<0.01, #P<0.05, as compared with the negative control group; **P<0.01, *P<0.05, as compared with the model group;
Figure 10 is a comparison diagram of the observation results of symptoms of ocular allergic responses in different treatment groups in the mouse model of allergic conjunctivitis in Test Example 5; in which *P<0.05, ****P<0.0001, as compared with the normal control group; #P<0.05, ##P<0.01, ^{####}P<0.0001, as compared with the model group; ^{◆}P<0.05, ^{◆◆◆◆}P<0.0001, as compared with 0.1% dexamethasone;
Figure 11 is the results of hematoxylin-eosin staining (HE staining) in different treatment groups in Test Example 5;
Figure 12 is the results of toluidine blue staining (for the detection of mast cells) in different treatment groups in Test Example 5;
Figure 13 is a comparison diagram of the inflammatory cell count results in different treatment groups in Test Example 5; in which, *P<0.05, * * * *P<0.0001, as compared with the normal control group; #P<0.05, ##P<0.01, ^{####}P<0.0001, as compared with the model group;
Figure 14 is a comparison diagram of mast cell degranulation in different treatment groups in Test Example 5;
Figure 15 is a comparison diagram of IL-4 tissue expression levels in different treatment groups in Test Example 5;
Figure 16 is a comparison diagram of IgE tissue expression levels in different treatment groups in Test Example 5; in which *P<0.05, ****P<0.0001, as compared with the normal control group; #P<0.05, ##P<0.01, ^{####}P<0.0001, as compared with the model group.

### Detailed Description of the Invention

### Terminology

Unless defined otherwise, all technical and scientific terms used herein have the same meaning as is commonly understood by one of skill in the art to which the claimed subject matter belongs.

Unless otherwise indicated, conventional methods of mass spectroscopy, NMR, HPLC, protein chemistry, biochemistry, recombinant DNA techniques and pharmacology, within the skill of the art are employed in the disclosure. Unless specific definitions are provided, the nomenclature employed in connection with, and the laboratory procedures and techniques of, analytical chemistry, synthetic organic chemistry, and medicinal and pharmaceutical chemistry described herein are those known in the art. The foregoing techniques and procedures can be generally performed by conventional methods well known in the art and as described in various general and more specific references.

The term "alkyl" refers to an aliphatic hydrocarbon group, which may be a branched or linear alkyl group. Depending on the structure, an alkyl group may be a monoradical or a diradical (i.e., an alkylene group).

The term "alkoxy" refers to a -O-alkyl group, where alkyl is as defined herein.

The term "cycloalkyl" refers to a monocyclic or polycyclic group that contains only carbon and hydrogen. Cycloalkyl groups include groups having from 3 to 12 ring atoms. Depending on the structure, a cycloalkyl group may be a monoradical or a diradical (e.g., a cycloalkylene group).

The term "dicycloalkyl" refers to a group having two rings and containing only carbon and hydrogen.

The term "heteroaryl" refers to an aromatic ring in which one or more of the atoms constituting the ring are a heteroatom selected from the group consisting of nitrogen, oxygen and sulfur.

The term "heterocyclic group" as used herein refers to a non-aromatic ring in which one or more of the atoms constituting the ring are a heteroatom selected from the group consisting of nitrogen, oxygen and sulfur. Heterocycloalkyl ring may be a monocyclic or polycyclic group formed by three, four, five, six, seven, eight, nine, or more than nine atoms.

The term "bicyclic heterocyclic group" as used herein refers to a heterocyclic group having two rings, in which one ring contains at least one nitrogen atom and the other ring contains 0, 1 or 2 heteroatoms selected from the group consisting of nitrogen, oxygen and sulfur. As non-limiting examples, the bicyclic heterocyclic group may be azaoxaspirodecyl or octahydrofuranopyridyl (e.g., a 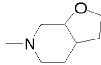 group).

The term "spiro heterocyclic group" refers to a heterocyclic group having two rings sharing one carbon atom, and one or more of the atoms constituting the ring are a heteroatom selected from the group consisting of nitrogen, oxygen and sulfur. The term "bridged bicyclic heterocyclic group" refers to a heterocyclic group having two rings sharing two non-adjacent carbon atoms, and one or more of the atoms constituting the ring are a heteroatom selected from the group consisting of nitrogen, oxygen and sulfur. The term "fused bicyclic heterocyclic group" refers to a heterocyclic group having two rings sharing two adjacent carbon atoms, and one or more of the atoms constituting the ring are a heteroatom selected from the group consisting of nitrogen, oxygen and sulfur.

The term "cis-trans isomer" refers to a diastereomer in which the spatial arrangement of the groups is different due to the presence of cyclohexyl in the compound represented by formula Ib.

The term "solvate" refers to a solvent compound formed when a compound is dissolved in a solvent and the solvent molecules are combined with the compound, thereby changing the original state of the compound.

The term "optionally" means that the subsequently described event(s) may occur or may not occur, and includes both event(s), which occur, and events that do not occur. The term "optionally substituted" or "substituted" means that the referenced group may be substituted with one or more additional group(s).

The present invention will be further described below in conjunction with the embodiments.

### Overall embodiment

In one aspect, the present invention provides an N-tetrazolyl aryl urea-based derivative, which is a compound represented by Formula I, or a cis-trans isomer thereof, or a pharmaceutically acceptable salt or solvate thereof;

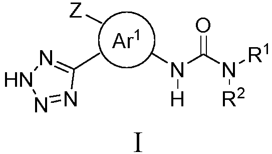

In Formula I, R¹ and R² together with the N atom attached thereto may form a 3- to 8-membered heterocyclic group containing at least 1 nitrogen atom. Optionally, the 3- to 8-membered heterocyclic group may be substituted by 1 to 3 of identical or different functional groups A, wherein the functional group A may be selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, alkylamino, dialkylamino, acetylamino and cycloamino.

Alternatively, R¹ and R² together with the N atom attached thereto may form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂. The 8- to 12-membered bicyclic heterocyclic group may comprise a spiro heterocyclic group, a bridged bicyclic heterocyclic group and a fused bicyclic heterocyclic group. Optionally, the 8- to 12-membered bicyclic heterocyclic group may be substituted by 1 to 3 of identical or different functional groups B, wherein the functional group B may be selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

Alternatively, each of R¹ and R² may be independently selected from the group consisting of the following groups:
(i) H, C₁-C₁₀ alkyl, C₂-C₁₀ alkenyl and C₂-C₁₀ alkynyl, optionally, C₁-C₁₀ alkyl, C₂-C₁₀ alkenyl, or C₂-C₁₀ alkynyl may be substituted by 1 to 3 of identical or different functional groups C, wherein the functional group C may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino; or
(ii) phenyl, 5- to 6-membered heteroaryl, C₅-C₇ cycloalkyl and 5- to 7-membered heterocyclic group, wherein the 5- to 6-membered heteroaryl or 5- to 7-membered heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, C₅-C₇ cycloalkyl or 5- to 7-membered heterocyclic group may be substituted by 1 to 3 of identical or different functional groups D, and the functional group D may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

In Formula I, Ar¹ may be selected from the group consisting of phenyl, 5-to 6-membered heteroaryl, C₅-C₇ cycloalkyl and C₉-C₁₀ dicycloalkyl, wherein the 5- to 6-membered heteroaryl contains 1 or 2 heteroatoms or heteroatom-containing groups independently selected from the group consisting of NH, N, O, S, SO and SO₂. Optionally, the phenyl, 5- to 6-membered heteroaryl, C₅-C₇ cycloalkyl or C₉-C₁₀ dicycloalkyl may be substituted by 1 to 3 of identical or different functional groups E, wherein the functional group E may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

In Formula I, Z may be selected from the group consisting of phenyl, 5- to 6-membered heteroaryl, C₅-C₇ cycloalkyl, 5- to 7-membered heterocyclic group, C₉-C₁₀ dicycloalkyl, 9- to 10-membered fused bicyclic heterocyclic group, 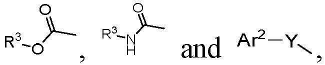 wherein the 5- to 6-membered heteroaryl, 5- to 7-membered heterocyclic group or 9- to 10-membered fused bicyclic heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, C₅-C₇ cycloalkyl, 5- to 7-membered heterocyclic group, C₉-C₁₀ dicycloalkyl or 9- to 10-membered fused bicyclic heterocyclic group may be substituted by 1 to 3 of identical or different functional groups F, wherein the functional group F may be selected from the group consisting of hydroxyl, halogen, amino, cyano, nitro, C₁-C₆ alkyl, C₃-C₇ cycloalkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino, or alternatively, the functional group F may form a 5- to 8-membered heterocyclic ring fused to a phenyl, a 5- to 6-membered heteroaryl, a C₅-C₇ cycloalkyl, a 5- to 7-membered heterocyclic group, a C₉-C₁₀ dicycloalkyl, or a 9- to 10-membered fused bicyclic heterocyclic group,

R³ may be selected from the group consisting of C₁-C₆ alkyl, C₃-C₇ cycloalkyl, C₃-C₇ cycloalkenyl, 5- to 7-membered heterocyclic group, phenyl and 5- to 6-membered heteroaryl. Optionally, the C₁-C₆ alkyl, C₃-C₇ cycloalkyl, C₃-C₇ cycloalkenyl, 5- to 7-membered heterocyclic group, phenyl or 5- to 6-membered heteroaryl may be substituted by 1 to 3 of identical or different functional groups G, wherein the functional group G may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

Y may be selected from the group consisting of O or NH.

Ar² may be selected from the group consisting of phenyl, 5- to 6-membered heteroaryl, C₁₀-C₂₀ fused cyclic group, C₁-C₆ alkyl, 5- to 7-membered cycloalkyl, 5- to 7-membered heterocyclic group, and C₁-C₆ alkoxy. The 5- to 6-membered heteroaryl or 5- to 7-membered heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, C₁₀-C₂₀ fused cyclic group, C₁-C₆ alkyl, 5- to 7-membered cycloalkyl, 5- to 7-membered heterocyclic group, or C₁-C₆ alkoxy may be substituted by 1 to 3 of identical or different functional groups H, wherein the functional group H may be selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

In another aspect, the present invention provides an N-tetrazolyl aryl urea-based derivative, which is a compound represented by Formula I, or a cis-trans isomer thereof, or a pharmaceutically acceptable salt or solvate thereof.

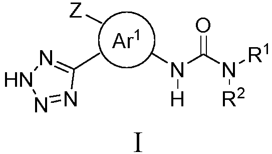

Wherein:
R¹ and R² together with the N atom attached thereto form a 3- to 8-membered heterocyclic group containing at least 1 nitrogen atom, wherein the above groups are substituted by 1 to 3 of identical or different functional groups A, and the functional group A is selected from the group consisting of hydroxyl, halogen, amino, cyano, nitro, C₁-C₃ alkyl, C₁-C₃ alkenyl, C₁-C₃ alkynyl, C₁-C₃ alkoxy, halogenated C₁-C₃ alkyl, C₁-C₃ fluoroalkoxy, alkylamino, dialkylamino, acetylamino or cycloamino; or
R¹ and R² together with the N atom attached thereto form an 8- to 12-membered bicyclic group containing at least 1 nitrogen atom and additionally containing 0, 1 or 2 heteroatoms or heteroatom-containing groups independently selected from the group consisting of NH, N, O, S, SO and SO₂, wherein the bicyclic group comprises a spiro cycloalkyl, a bridged bicycloalkyl and a fused bicycloalkyl, and the above groups are substituted by 1 to 3 of identical or different functional groups B, and the functional group B is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino; or
Each of R¹ and R² is independently selected from the group consisting of the following groups:
   (i) H, C₁-C₁₀ alkyl, C₂-C₁₀ alkenyl, C₂-C₁₀ alkynyl, wherein the above groups except for H are substituted by 1 to 3 of identical or different functional groups C, and the functional group C is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino; or
   (ii) phenyl, 5- to 6-membered heteroaryl, C₅-C₇ cycloalkyl, 5- to 7-membered heterocyclic group, wherein the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, heteroatom or heteroatom-containing group is substituted by 1 to 3 of identical or different functional groups D, and the functional group D is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino;
Ar¹ is selected from the group consisting of:
   phenyl, 5- to 6-membered heteroaryl, C₅-C₇ cycloalkyl, C₉-C₁₀ fused bicyclic group, wherein the 5- to 6-membered heteroaryl contains 1 or 2 heteroatoms or heteroatom-containing groups independently selected from the group consisting of NH, N, O, S, SO and SO₂, and the phenyl, heteroatom or heteroatom-containing group is substituted by 1 to 3 of identical or different functional groups E, and the functional group E is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino;
   Z is selected from the group consisting of the following groups:
      (i) phenyl, 5- to 6-membered heteroaryl, C₅-C₇ cycloalkyl, 5- to 7-membered heterocyclic group, C₉-C₁₀ fused bicyclic group, 9- to 10-membered fused heterobicyclic group, wherein the 5- to 6-membered heteroaryl, 5- to 7-membered heterocyclic group or 9- to 10-membered fused heterobicyclic group contains 1, 2 or 3 heteroatoms or heteroatom-containing groups independently selected from the group consisting of NH, N, O, S, SO and SO₂, and the phenyl, heteroatom or heteroatom-containing group is substituted by 1 to 3 of identical or different functional groups F, and the functional group F is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino and cycloamino, or alternatively, the functional groups F form a 5- to 8-membered heterocyclic ring; or
      (ii) 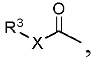 wherein X is selected from the group consisting of O or NH, and R³ is selected from the group consisting of C₁-C₆ alkyl, C₃-C₇ cycloalkyl, C₃-C₇ cycloalkenyl, 5- to 7-membered heterocyclic group, phenyl, 5- to 6-membered heteroaryl, and the above groups are substituted by 1 to 3 of identical or different functional groups G, and the functional group G is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
      (iii) 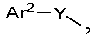 wherein Y is selected from the group consisting of O or NH, and Ar² is selected from the group consisting of phenyl, 5- to 6-membered heteroaryl, C₁₀-C₂₀ fused cyclic group, C₁-C₆ alkyl, 5- to 7-membered cycloalkyl, 5- to 7-membered heterocyclic group, C₁-C₆ alkoxy, wherein the above groups are substituted by 1 to 3 of identical or different functional groups H, and the functional group H is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂.

In a preferred embodiment, in the N-tetrazolyl aryl urea-based derivative represented by Formula I of any of the above aspects, R¹ and R² together with the N atom attached thereto may form a 3- to 8-membered heterocyclic group containing at least 1 nitrogen atom. Optionally, the 3- to 8-membered heterocyclic group may be substituted by 1 to 3 of identical or different functional groups A, wherein the functional group A is selected from the group consisting of C₁-C₆ alkyl, C₁-C₆ alkoxy, halogenated C₁-C₆ alkyl and halogenated C₁-C₆ alkoxy; or
R¹ and R² together with the N atom attached thereto may form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂. The 8- to 12-membered bicyclic heterocyclic group may comprise spiro cycloalkyl, bridged bicycloalkyl and fused bicycloalkyl. Optionally, the 8- to 12-membered bicyclic heterocyclic group may be substituted by 1 to 3 of identical or different functional groups B, wherein the functional group B may be selected from the group consisting of C₁-C₆ alkyl, C₁-C₆ alkoxy, halogenated C₁-C₆ alkyl and halogenated C₁-C₆ alkoxy; or
each of R¹ and R² is independently selected from the group consisting of the following groups:
   (i) H and C₁-C₆ alkyl, wherein C₁-C₆ alkyl may be optionally substituted by 1 to 3 of identical or different functional groups C, wherein the functional group C may be selected from the group consisting of C₁-C₆ alkoxy and halogenated C₁-C₆ alkoxy;
   (ii) phenyl and C₅-C₇ cycloalkyl, the phenyl or C₅-C₇ cycloalkyl may be optionally substituted by 1 to 3 of identical or different functional groups D, wherein the functional group D may be selected from the group consisting of C₁-C₆ alkyl, C₁-C₆ alkoxy, halogenated C₁-C₆ alkyl and halogenated C₁-C₆ alkoxy.

Ar¹ may be selected from the group consisting of phenyl and 5- to 6-membered heteroaryl, wherein the 5- to 6-membered heteroaryl may contain 1 or 2 heteroatoms or heteroatom-containing groups independently selected from the group consisting of NH, N, O, S, SO and SO₂. Optionally, the phenyl or 5-to 6-membered heteroaryl may be substituted by 1 to 3 of identical or different functional groups E, wherein the functional group E may be selected from the group consisting of halogen, C₁-C₆ alkyl, C₁-C₆ alkoxy, halogenated C₁-C₆ alkyl and halogenated C₁-C₆ alkoxy.

Z may be selected from the group consisting of phenyl, 5- to 6-membered heteroaryl, 5- to 7-membered heterocyclic group, 9- to 10-membered fused bicyclic heterocyclic group, 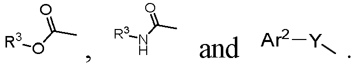 The 5- to 6-membered heteroaryl, 5- to 7-membered heterocyclic group, or 9- to 10-membered fused bicyclic heterocyclic group may contain 1, 2 or 3 heteroatoms or heteroatom-containing groups independently selected from the group consisting of NH, N, O, S, SO and SO₂. Optionally, the phenyl, 5- to 6-membered heteroaryl, 5- to 7-membered heterocyclic group or 9- to 10-membered fused bicyclic heterocyclic group may be substituted by 1 to 3 of identical or different functional groups F, wherein the functional group F may be selected from the group consisting of halogen, C₁-C₆ alkyl, C₃-C₇ cycloalkyl, C₁-C₆ alkoxy, halogenated C₁-C₆ alkyl and halogenated C₁-C₆ alkoxy, or the functional group F may form a 5- to 8-membered heterocyclic ring fused to a phenyl;
R³ may be selected from the group consisting of C₁-C₆ alkyl, C₃-C₇ cycloalkyl, 5- to 7-membered heterocyclic group and 5- to 6-membered heteroaryl. Optionally, the C₁-C₆ alkyl, C₃-C₇ cycloalkyl or 5- to 6-membered heteroaryl may be substituted by 1 to 3 of identical or different functional groups G, wherein the functional group G may be selected from the group consisting of C₁-C₆ alkyl, C₁-C₆ alkoxy, halogenated C₁-C₆ alkyl and halogenated C₁-C₆ alkoxy;
Y may be O;
Ar² may be selected from the group consisting of phenyl, 5- to 6-membered heteroaryl and 5- to 7-membered heterocyclic group. The 5- to 6-membered heteroaryl or 5- to 7-membered heterocyclic group may contain 1, 2 or 3 heteroatoms or heteroatom-containing groups independently selected from the group consisting of NH, N, O, S, SO and SO₂. Optionally, the phenyl, 5- to 6-membered heteroaryl or 5- to 7-membered heterocyclic group may be substituted by 1 to 3 of identical or different functional groups H, wherein the functional group H may be selected from the group consisting of C₁-C₆ alkyl, C₁-C₆ alkoxy, halogenated C₁-C₆ alkyl and halogenated C₁-C₆ alkoxy.

In a preferred embodiment, in the N-tetrazolyl aryl urea-based derivative represented by Formula I of any of the above aspects, R¹ and R² together with the N atom attached thereto may form a 3- to 8-membered heterocyclic group containing at least 1 nitrogen atom. Optionally, the 3- to 8-membered heterocyclic group may be substituted by 1 to 3 of identical or different functional groups A, wherein the functional group A may be selected from the group consisting of C₁-C₆ alkyl and halogenated C₁-C₆ alkyl; or
R¹ and R² together with the N atom attached thereto may form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂. The 8- to 12-membered bicyclic heterocyclic group comprises spiro cycloalkyl and fused bicycloalkyl; or
R¹ may be selected from the group consisting of H and C₁-C₆ alkyl. R² may be selected from the group consisting of phenyl and C₅-C₇ cycloalkyl. Optionally, the phenyl or C₅-C₇ cycloalkyl may be substituted by 1 to 3 of identical or different functional groups D, wherein the functional group D is selected from the group consisting of C₁-C₆ alkyl and halogenated C₁-C₆ alkyl.
Ar¹ may be selected from the group consisting of phenyl and 5- to 6-membered heteroaryl, wherein the 5- to 6-membered heteroaryl contains 1 or 2 heteroatoms or heteroatom-containing groups independently selected from the group consisting of NH, N, O, S, SO and SO₂. Optionally, the phenyl may be substituted by 1 to 3 of identical or different halogens;
Z may be selected from the group consisting of phenyl, 5- to 6-membered heteroaryl, 5- to 7-membered heterocyclic group, 9- to 10-membered fused bicyclic heterocyclic group, 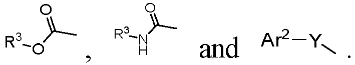 The 5- to 6-membered heteroaryl, 5- to 7-membered heterocyclic group, or 9- to 10-membered fused bicyclic heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl, 5- to 7-membered heterocyclic group or 9- to 10-membered fused bicyclic heterocyclic group may be substituted by 1 to 3 of identical or different functional groups F, wherein the functional group F may be selected from the group consisting of halogen, C₁-C₆ alkyl, C₃-C₇ cycloalkyl, C₁-C₆ alkoxy and halogenated C₁-C₆ alkyl, or the functional group F may form a 5- to 8-membered heterocyclic ring fused to a phenyl;
R³ may be selected from the group consisting of C₁-C₆ alkyl, C₃-C₇ cycloalkyl, 5- to 7-membered heterocycloalkyl and 5- to 6-membered heteroaryl. Optionally, the C₃-C₇ cycloalkyl, 5- to 7-membered heterocycloalkyl or 5- to 6-membered heteroaryl may be substituted by 1 to 3 of identical or different functional groups G, wherein the functional group G may be selected from the group consisting of C₁-C₆ alkyl;
Ar² is selected from the group consisting of phenyl, 5- to 6-membered heteroaryl and 5- to 7-membered heterocyclic group, the 5- to 6-membered heteroaryl or 5- to 7-membered heterocyclic group contains 1, 2 or 3 heteroatoms or heteroatom-containing groups independently selected from the group consisting of NH, N, O, S, SO and SO₂. Optionally, the phenyl, 5- to 6-membered heteroaryl or 5- to 7-membered heterocyclic group may be substituted by 1 to 3 of identical or different substituents selected from the group consisting of C₁-C₆ alkyl and C₁-C₆ alkoxy.

In a preferred embodiment, in the N-tetrazolyl aryl urea-based derivative represented by Formula I of any of the above aspects, R¹ and R² together with the N atom attached thereto may form a piperidyl. Optionally, the piperidyl may be substituted by substituent(s) selected from the group consisting of methyl, tert-butoxy and trifluoromethyl; or
R¹ and R² together with the N atom attached thereto may form an azaoxaspirodecyl or an octahydrofuranopyridyl; or
R¹ may be selected from the group consisting of H and methyl; and R² may be selected from the group consisting of phenyl and cyclohexyl. Optionally, the phenyl may be substituted by trifluoromethyl. Optionally, the cyclohexyl may be substituted by methyl or tert-butyl.
Ar¹ may be selected from the group consisting of phenyl and pyridyl. Optionally, the phenyl may be substituted by a fluorine atom.
Z may be selected from the group consisting of phenyl, phenoxy, pyridyl, indazolyl, imidazolyl, tetrahydropyranyl, tetrahydropyranyloxy, dihydropyranyl, 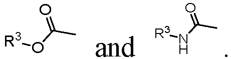 Optionally, the phenyl, pyridyl, indazolyl, imidazolyl, tetrahydropyranyl or dihydropyranyl may be substituted by 1 to 3 of identical or different functional groups F, wherein the functional group F may be selected from the group consisting of methyl, tert-butyl, methoxy, ethoxy, isopropoxy, cyclobutyl, -Cl, difluoromethyl and trifluoromethyl, or the functional group F may form a dioxolane ring fused to a phenyl. Optionally, the phenoxy may be substituted by 1 to 2 methoxy groups,
R³ may be selected from the group consisting of ethyl, cyclopentyl, cyclohexyl, tetrahydrofuranyl, and pyridyl optionally substituted by methyl.

In a preferred embodiment, the N-tetrazolyl aryl urea-based derivative represented by Formula I of any of the above aspects may be a compound represented by Formula Ia, Ib or Ic, or a cis-trans isomer thereof, or a pharmaceutically acceptable salt thereof;

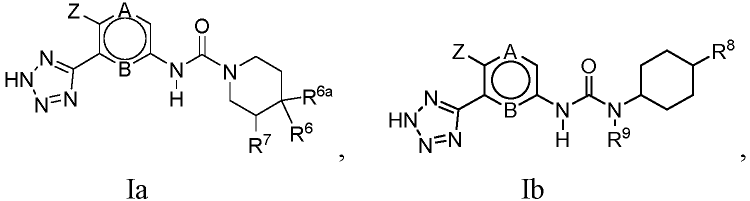

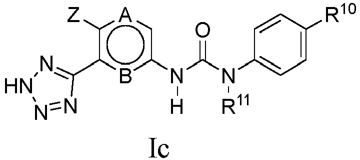

In the compound represented by Formula Ia:
each of A and B may be independently a carbon atom or a nitrogen atom;
Z may be selected from the group consisting of phenyl, pyridyl, indazolyl, imidazolyl, tetrahydropyranyl, dihydropyranyl, 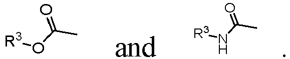 Optionally, the phenyl, pyridyl, indazolyl, imidazolyl, tetrahydropyranyl or dihydropyranyl may be substituted by 1 to 3 of identical or different functional groups F, wherein the functional group F may be selected from the group consisting of methyl, tert-butyl, methoxy, ethoxy, isopropoxy, cyclobutyl, -Cl, difluoromethyl and trifluoromethyl, or the functional group F may form a dioxolane ring fused to a phenyl. R³ may be selected from the group consisting of ethyl, cyclopentyl, cyclohexyl, tetrahydrofuranyl, and pyridyl optionally substituted by methyl.
each of R⁷, R^{6a} and R⁶ may be independently selected from the group consisting of H, methyl, tert-butoxy and trifluoromethyl; or
R⁷ is a hydrogen atom, R⁶ and R^{6a} together with the carbon atoms attached thereto may form an oxolane ring; or
R^{6a} is a hydrogen atom, R⁶ and R⁷ together with the carbon atoms attached thereto may form an oxolane ring.

In the compound represented by Formula Ib:
each of A and B may be independently a carbon atom or a nitrogen atom.
Z may be selected from the group consisting of phenyl, phenoxy, pyridyl, dihydropyranyl, tetrahydropyranyl and tetrahydropyranyloxy. Optionally, the phenyl or phenoxy may be substituted by 1 to 2 methoxy groups, or optionally fused to a dioxolane ring. Optionally, the pyridyl may be substituted by an ethoxy;
R⁸ may be tert-butyl or methyl.
R⁹ may be H or methyl.

In the compound represented by Formula Ic:
each of A and B may be independently a carbon atom or a nitrogen atom;
Z may be selected from the group consisting of phenyl, phenoxy, pyridyl, dihydropyranyl, tetrahydropyranyl and tetrahydropyranyloxy. Optionally, the phenyl or phenoxy may be substituted by 1 to 2 methoxy groups, or optionally may fused to a dioxolane ring. Optionally, the pyridyl may be substituted by an ethoxy;
R¹⁰ may be trifluoromethyl;
R¹¹ may be H.

Preferably, in the N-tetrazolyl aryl urea-based derivative represented by Formula I of any of the above aspects, Ar¹ is selected from the group consisting of the following phenyl, pyridyl, and pyrazinyl groups: 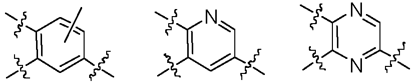 wherein, the above groups are substituted by 1 to 3 of identical or different functional groups I, and the functional group I is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino.

Preferably, the N-tetrazolyl aryl urea-based derivative of any of the above aspects is a compound represented by Formula Ia or Ib, or a cis-trans isomer thereof, or a pharmaceutically acceptable salt thereof.

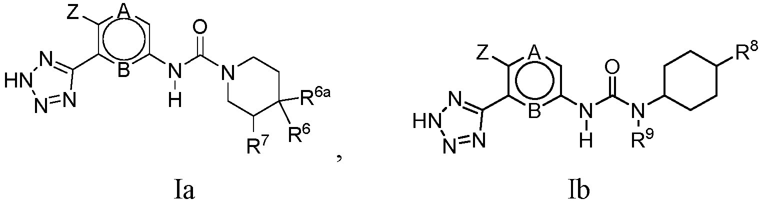

In the compound represented by Formula Ia:
each of A and B is independently a carbon atom or a nitrogen atom;
Z is selected from the group consisting of the following groups:
   (i) phenyl, pyridyl, pyrazolyl, imidazolyl, indazolyl, wherein the above groups are substituted by 1 to 3 of identical or different functional groups J, and the functional group J is selected from the group consisting of hydroxyl, halogen, amino, cyano, nitro, C₁-C₆ alkyl, C₁-C₆ cycloalkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino, or alternatively, the functional group J form a 5-membered heterocyclic ring; or
   (ii) 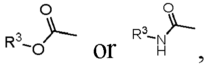 wherein R³ is selected from the group consisting of C₁-C₆ alkyl, C₁-C₆ cycloalkyl, C₃-C₇ cycloalkyl, C₃-C₇ aryl, 5- to 7-membered heterocyclic group, phenyl, 5- to 6-membered heteroaryl, wherein the above groups are substituted by 1 to 3 of identical or different functional groups K, and the functional group K is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
   (iii) a phenoxy group, wherein the group is substituted by 1 to 3 of identical or different functional groups L, and the functional group L is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
   (iv) a 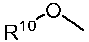 group, wherein R¹⁰ is selected from the group consisting of C₁-C₆ alkyl, 5- to 7-membered cycloalkyl, 5- to 6-membered heteroaryl, 5- to 7-membered heterocyclic group, wherein the above groups are substituted by 1 to 3 of identical or different functional groups M, and the functional group M is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂;
R^{6a} is a hydrogen atom, and each of R⁶ and R⁷ is independently selected from the group consisting of C₁-C₆ alkyl, C₁-C₆ cycloalkyl, C₃-C₁₀ cycloalkyl, wherein the above groups are substituted by 1 to 3 of identical or different functional groups N, and the functional group N is selected from the group consisting of hydroxyl, halogen, amino, cyano, nitro; or
R^{6a} is a hydrogen atom, and R⁶ and R⁷ together with the two carbon atoms attached thereto form a 4- to 8-membered cyclic group, or a 4- to 8-membered bicyclic heterocyclic group containing 1 heteroatom or heteroatom-containing group independently selected from the group consisting of NH, N, O, S, SO and SO₂, wherein the above groups are substituted by 1 to 3 of identical or different functional groups O, and the functional group O is selected from the group consisting of hydroxyl, halogen, amino, cyano, nitro; or
R⁷ is a hydrogen atom, and R⁶ and R^{6a} together with the two carbon atoms attached thereto form a 4- to 8-membered cyclic group, or a 4- to 8-membered heterocyclic group containing 1 heteroatom or heteroatom-containing group independently selected from the group consisting of NH, N, O, S, SO and SO₂, wherein the above groups are substituted by 1 to 3 of identical or different functional groups P, and the functional group P is selected from the group consisting of hydroxyl, halogen, amino, cyano, nitro;

In the compound represented by Formula Ib:
each of A and B is independently a carbon atom or a nitrogen atom;
Z is selected from the group consisting of the following groups:
   (i) phenyl, pyridyl, pyrazolyl, imidazolyl, indazolyl, wherein the above groups are substituted by 1 to 3 of identical or different functional groups Q, and the functional group Q is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino; or
   (ii) 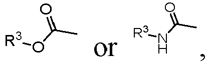 wherein R³ is selected from the group consisting of C₁-C₆ alkyl, C₁-C₆ cycloalkyl, C₃-C₁₀ cycloalkyl, C₃-C₁₀ aryl, wherein the above groups are substituted by 1 to 3 of identical or different functional groups R, and the functional group R is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino; or
   (iii) a phenoxy group, wherein the group is substituted by 1 to 3 of identical or different functional groups S, and the functional group S is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino; or
   (iv) a 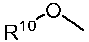 group, wherein R¹⁰ is selected from the group consisting of C₁-C₆ alkyl, 5- to 7-membered cycloalkyl, 5- to 6-membered heteroaryl, 5- to 7-membered heterocyclic group, wherein the above groups are substituted by 1 to 3 of identical or different functional groups T, and the functional group T is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂;
R⁸ is selected from the group consisting of C₁-C₆ alkyl, C₁-C₆ cycloalkyl, C₃-C₁₀ aryl, wherein the above groups are substituted by 1 to 3 of identical or different functional groups U, and the functional group U is selected from the group consisting of hydroxyl, halogen, amino, cyano, nitro;
R⁹ is selected from the group consisting of C₁-C₆ alkyl, C₁-C₆ cycloalkyl, C₃-C₁₀ aryl, wherein the above groups are substituted by 1 to 3 of identical or different functional groups V, and the functional group V is selected from the group consisting of hydroxyl, halogen, amino, cyano, and nitro.

Further preferably, the N-tetrazolyl aryl urea-based derivative may be selected from the group consisting of the following compounds, or a cis-trans isomer thereof, or a pharmaceutically acceptable salt or solvate thereof;
4-(tert-butyl)-N-(3',4'-dimethoxy-2-(2H-tetrazol-5-yl)-[1,1'-biphenyl]-4-yl)piperidine-1-carboxamide,
N-(3',4'-dimethoxy-2-(2H-tetrazol-5-yl)-1,1'-biphenyl]-4-yl)-4-(trifluoromethyl)piperidine-1-carboxamide,
N-(3',4'-dimethoxy-2-(2H-tetrazol-5-yl)-[1,1'-biphenyl]-4-yl)-1-oxy-8-azaspiro[4.5]decane-1-carboxamide,
N-(3',4'-dimethoxy-2-(2H-tetrazol-5-yl)-[1,1'-biphenyl]-4-yl)hexahydrofuro[2,3-c]pyridine-6(2H)-carboxamide,
N-(4-(6-ethoxypyrid-3-yl)-3-(2H-tetrazol-5-yl)phenyl)-4-methylpiperidine-1-carboxamide,
4-(tert-butyl)-N-(4-(6-ethoxypyrid-3-yl)-3-(2H-tetrazol-5-yl)phenyl)piperidine-1-carboxamide,
**4-(tert-butyl)-N-(6'-ethoxy-3-(2H-tetrazol-5-yl)-[2,3'-bipyridyl]-5-**yl)piperidine-1-carboxamide,
4-(tert-butyl)-N-(4-(6-ethoxypyrid-3-yl)-3-fluoro-5-(2H-tetrazol-5-yl)phenyl)piperidine-1-carboxamide,
4-(tert-butyl)-N-(4-(1-cyclobutyl-1H-pyrazol-4-yl)-3-(2H-tetrazol-5-yl)phenyl)piperidine-1-carboxamide,
4-(tert-butyl)-N-(4-(1-methyl-1H-indazol-5-yl)-3-(2H-tetrazol-5-yl)phenyl)piperidine-1-carboxamide,
4-(tert-butyl)-N-(2-fluoro-3',4'-dimethoxy-6-(2H-tetrazol-5-yl)-[1,1'-biphenyl]-4-yl)piperidine-1-carboxamide,
N-(3',4'-dimethoxy-2-(2H-tetrazol-5-yl)-[1,1'-biphenyl]-4-yl)-4,4-dimethylpiperidine-1-carboxamide,
4-(tert-butyl)-N-(4'-methoxy-3'-methyl-2-(2H-tetrazol-5-yl)-[1,1'-biphenyl]-4-yl)piperidine-1-carboxamide,
4-(tert-butyl)-N-(3'-chloro-4'-methoxy-2-(2H-tetrazol-5-yl)-[1,1'-biphenyl]-4-yl)piperidine-1-carboxamide,
cyclohexyl 4-(4-(tert-butyl)piperidine-1-formamido)-2-(2H-tetrazol-5-yl)benzoate,
cyclohexyl 4-(4-(tert-butyl)piperidine-1-formamido)-2-fluoro-6-(2H-tetrazol-5-yl)benzoate,
ethyl 4-(4-(tert-butyl)piperidine-1-formamido)-2-fluoro-6-(2H-tetrazol-5-yl)benzoate,
4-(tert-butyl)-N-(3-fluoro-5-(2H-tetrazol-5-yl)-4-(6-(trifluoromethyl)pyrid-3-yl)phenyl)piperidine-1-carboxamide,
4-(tert-butyl)-N-(3-fluoro-5-(2H-tetrazol-5-yl)-4-(6-(trifluoromethyl)pyrid-3-yl)phenyl)piperidine-1-carboxamide,
N-(2-fluoro-3',4'-methoxy-6-(2H-tetrazol-5-yl)-[1,1'-biphenyl]-4-yl)-4-methylpiperidine-1-carboxamide,
4-methyl-N-(4-(1-methyl-1H-indazol-5-yl)-3-(2H-tetrazol-5-yl)phenyl)piperidine-1-carboxamide,
4-methyl-N-(4-(1-methyl-1H-indazol-5-yl)-3-(2H-tetrazol-5-yl)phenyl)piperidine-1-carboxamide,
cyclohexyl 2-fluoro-4-(4-methylpiperidine-1-formamido)-6-(2H-tetrazol-5-yl)benzoate,
ethyl 2-fluoro-4-(4-methylpiperidine-1-formamido)-6-(2H-tetrazol-5-yl)benzoate,
N-(4-(6-ethoxypyrid-3-yl)-3-fluoro-5-(2H-tetrazol-5-yl)phenyl)-4-(trifluoromethyl)piperidine-1-carboxamide,
N-(2-fluoro-3',4'-methoxy-6-(2H-tetrazol-5-yl)-[1,1'-biphenyl]-4-yl)-4-(trifluoromethyl)piperidine-1-carboxamide,
N-(4-(1-methyl-1H-indazol-5-yl)-3-(2H-tetrazol-5-yl)phenyl)-4-(trifluoromethyl)piperidine-1-carboxamide,
N-(3'-chloro-4'-methoxy-2-(2H-tetrazol-5-yl)-[1,1'-biphenyl]-4-yl)-4-(trifluoromethyl)piperidine-1-carboxamide,
cyclohexyl 2-fluoro-6-(2H-tetrazol-5-yl)-4-(4-(trifluoromethyl)piperidine-1-formamido)benzoate,
ethyl 2-fluoro-6-(2H-tetrazol-5-yl)-4-(4-(trifluoromethyl)piperidine-1-formamido)benzoate,
N-(4-(6-ethoxypyrid-3-yl)-3-fluoro-5-(2H-tetrazol-5-yl)phenyl)-4-methylpiperidine-1-carboxamide,
4-(tert-butyl)-N-(3-fluoro-4-(1-methyl-1H-indazol-5-yl)-5-(2H-tetrazol-5-yl)phenyl)piperidine-1-carboxamide,
4-(tert-butyl)-N-(3'-chloro-2-fluoro-4'-methoxy-6-(2H-tetrazol-5-yl)-[1,1'-biphenyl]-4-yl)piperidine-1-carboxamide,
N-(3-fluoro-4-(1-methyl-1H-indazol-5-yl)-5-(2H-tetrazol-5-yl)phenyl)-4-(trifluoromethyl)piperidine-1-carboxamide,
4-(tert-butyl)-N-(6-(3,4-dimethoxyphenyl)-5-(2H-tetrazol-5-yl)pyrid-3-yl)piperidine-1-carboxamide,
4-(tert-butyl)-N-(6-(1-methyl-1H-indazol-5-yl)-5-(2H-tetrazol-5-yl)pyrid-3-yl)piperidine-1-carboxamide,
ethyl 5-(4-(tert-butyl)piperidine-1-formamido)-3-(2H-tetrazol-5-yl)pyridylcarboxylate,
cyclohexyl 5-(4-(tert-butyl)piperidine-1-formamido)-3-(2H-tetrazol-5-yl)pyridylcarboxylate,
N-(4-(benzo[d][1,3]dioxolan-5-yl)-3-(2H-tetrazol-5-yl)phenyl)-4-(tert-butyl)piperidine-1-carboxamide,
4-(tert-butyl)-N-(4-(tetrahydro-2H-pyran-4-yl)-3-(2H-tetrazol-5-yl)phenyl)piperidine-1-carboxamide,
4-(tert-butyl)-N-(4-(3,4-dihydro-2H-pyran-4-yl)-3-(2H-tetrazol-5-yl)phenyl)piperidine-1-carboxamide,
4-(tert-butyl)-N-(4-(6-(difluoromethyl)pyrid-3-yl)-3-(2H-tetrazol-5-yl)phenyl)piperidine-1-carboxamide,
cyclopentyl 4-(4-(tert-butyl)piperidine-1-formamido)-2-(2H-tetrazol-5-yl)benzoate,
tetrahydrofuran-3-yl 4-(4-(tert-butyl)piperidine-1-formamido)-2-(2H-tetrazol-5-yl)benzoate,
ethyl 4-(4-(tert-butyl)piperidine-1-formamido)-2-(2H-tetrazol-5-yl)benzoate,
4-(tert-butyl)-N-(4-(cyclopentylaminoformyl)-3-(2H-tetrazol-5-yl)phenyl)piperidine-1-carboxamide,
4-(tert-butyl)-N-(4-((5-methylpyridin-2-yl)aminoformyl)-3-(2H-tetrazol-5-yl)phenyl)piperidine-1-carboxamide,
4-(tert-butyl)-N-(4-(5-methoxypyrid-3-yl)-3-(2H-tetrazol-5-yl)phenyl)piperidine-1-carboxamide,
4-(tert-butyl)-N-(4-(6-isopropoxypyrid-3-yl)-3-(2H-tetrazol-5-yl)phenyl)piperidine-1-carboxamide,
4-(tert-butyl)-N-(4-(5-methoxypyrid-3-yl)-3-(2H-tetrazol-5-yl)phenyl)piperidine-1-carboxamide,
4-(tert-butyl)-N-(4-(6-methylpyrid-3-yl)-3-(2H-tetrazol-5-yl)phenyl)piperidine-1-carboxamide,
4-(tert-butyl)-N-(4-(2-methylpyridin-4-yl)-3-(2H-tetrazol-5-yl)phenyl)piperidine-1-carboxamide,
4-(tert-butyl)-N-(4-(cyclopentylaminoformyl)-3-fluoro-5-(2H-tetrazol-5-yl)phenyl)piperidine-1-carboxamide,
4-(tert-butyl)-N-(3-fluoro-4-((5-methylpyridin-2-yl)aminoformyl)-5-(2H-tetrazol-5-yl)phenyl)piperidine-1-carboxamide,
4-(tert-butyl)-N-(3-fluoro-4-(6-methylpyrid-3-yl)-5-(2H-tetrazol-5-yl)phenyl)piperidine-1-carboxamide,
4-(tert-butyl)-N-(3-fluoro-4-(6-isopropoxypyrid-3-yl)-5-(2H-tetrazol-5-yl)phenyl)piperidine-1-carboxamide,
4-(tert-butyl)-N-(4-(4-(tert-butyl)-1H-imidazol-1-yl)-3-fluoro-5-(2H-tetrazol-5-yl)phenyl)piperidine-1-carboxamide,
4-(tert-butyl)-N-(4-(4-(tert-butyl)-1H-imidazol-1-yl)-3-(2H-tetrazol-5-yl)phenyl)piperidine-1-carboxamide,
1-(4-(tert-butyl)cyclohexyl)-3-(3',4'-dimethoxy-2-(2H-tetrazol-5-yl)-[ 1,1'-biphenyl]-4-yl)urea,
1-(3',4'-dimethoxy-2-(2H-tetrazol-5-yl)-[1,1'-biphenyl]-4-yl)-3-(4-(trifluoromethyl)phenyl)urea,
1-(3',4'-dimethoxy-2-(2H-tetrazol-5-yl)-[1,1'-biphenyl]-4-yl)-3-((cis)-4-methylcyclohexyl)urea,
1-(3',4'-dimethoxy-2-(2H-tetrazol-5-yl)-[1,1'-biphenyl]-4-yl)-3-((trans)-4-methylcyclohexyl)urea,
1-(4-(3,4-dimethoxyphenoxy)-3-(2H-tetrazol-5-yl)phenyl)-3-((cis)-4-methylcyclohexyl)urea,
1-(4-(3,4-dimethoxyphenoxy)-3-(2H-tetrazol-5-yl)phenyl)-3-((trans)-4-methylcyclohexyl)urea,
1-((trans)-4-(tert-butyl)cyclohexyl)-3-(3',4'-dimethoxy-2-(2H-tetrazol-5-yl)-[1,1'-biphenyl]-4-yl)-1-methylurea,
1-(4-(3,6-dihydro-2H-pyran-4-yl)-3-(2H-tetrazol-5-yl)phenyl)-3-((trans)-4-methylcyclohexyl)urea,
1-((cis)-4-methylcyclohexyl)-3-(4-(tetrahydro-2H-pyran-4-yl)-3-(2H-tetrazol-5-yl)phenyl)urea,
1-((trans)-4-methylcyclohexyl)-3-(4-(tetrahydro-2H-pyran-4-yl)-3-(2H-tetrazol-5-yl)phenyl)urea,
1-(4-(benzo[d][1,3]dioxolan-5-yl)-3-(2H-tetrazol-5-yl)phenyl)-3-((trans)-4-methylcyclohexyl)urea,
1-(4-(benzo[d][1,3]dioxolan-5-yl)-3-(2H-tetrazol-5-yl)phenyl)-3-((cis)-4-(tert-butyl)cyclohexyl)urea,
1-(4-(6-ethoxypyrid-3-yl)-3-(2H-tetrazol-5-yl)phenyl)-3-((trans)-4-methylcyclohexyl)urea,
1-(cis)-4-methylcyclohexyl-3-(4-(tetrahydropyran-4-yl)oxy)-3-(2H-tetrazol-5-yl)phenyl)urea,
1-(trans)-4-methylcyclohexyl-3-(4-tetrahydropyran-4-yl)oxy)-3-(2H-tetrazol-5-yl)phenyl)urea.

In a third aspect, the present invention provides a method for preparing the above-mentioned N-tetrazolyl aryl urea-based derivative.

Method 1: in the compound represented by Formula I, wherein R¹ and R² are non-hydrogen groups, or R¹ and R² together with the N atom attached thereto form a heterocyclic group containing at least 1 nitrogen atom, the method for preparing of the present invention may comprise the steps of:
coupling 2-halogenated-5-nitroaromatic carbonitrile represented by Formula a with Z-B(OH)₂ boric acid represented by Formula b under an alkaline condition to obtain a compound represented by Formula c; subjecting the cyano group in the compound represented by Formula c to addition with sodium azide to obtain a tetrazolyl intermediate represented by Formula d, performing Trt- protection to obtain an intermediate represented by Formula e, and then reducing the nitro group to obtain an amino compound represented by Formula f, and then converting the amino compound to an isocyanate compound represented by Formula g, reacting the isocyanate compound with an amine to produce a urea compound represented by Formula h, and finally subjecting Trt-deprotection to obtain the target compound;

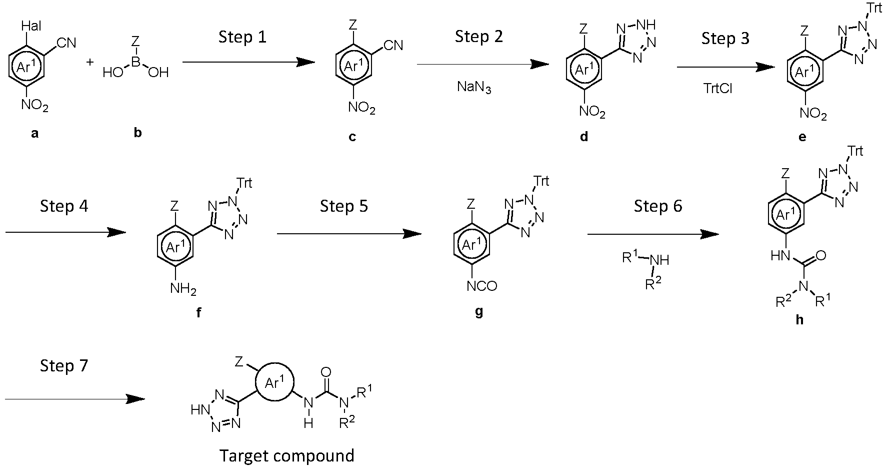

Compounds 1-14, 18 and 59-62 in the Examples were prepared according to the above Method 1.

Method 2: in the compound represented by Formula I, wherein Z is substituted or unsubstituted phenoxy, the method for preparing of the present invention may comprise the steps of:
subjecting the 2-halogenated-5-nitroaromatic carbonitrile represented by Formula a to a nucleophilic substitution reaction with a substituted phenol represented by Formula b2 under an alkaline condition to obtain a phenol ether- based intermediate represented by Formula c2; and performing Steps 2, 3, 4, 5, 6 and 7 as described in Method 1 to finally obtain the corresponding target compound;

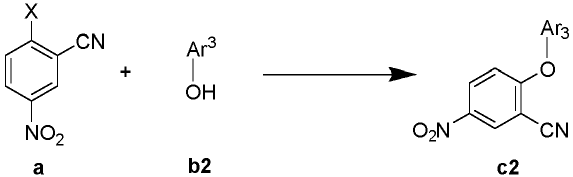

Compounds 63 and 64 in the Examples were prepared according to the above Method 2.

Method 3: in the compound represented by Formula I, wherein Z is 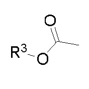 or 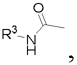 the method for preparing of the present invention may comprise the steps of:
performing Steps 2, 3, 4, 5, 6 and 7 as described in Method 1 except that the compound represented by Formula c in Method 1 is replace with with 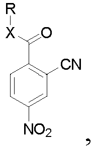 to finally obtain the target product;
Compounds 15 and 17 in the Examples were prepared according to the above Method 3.

Method 4: in the compound represented by Formula I, wherein one of R¹ or R² is a hydrogen atom, the method for preparing of the present invention may comprise the steps of:
at first, performing Steps 1, 2, 3 and 4 in Method 1 to obtain the amino intermediate represented by Formula f, and then reacting the amino intermediate directly with an isocyanate compound to produce a urea compound represented by Formula h1, and finally subjecting Trt-deprotection to obtain the target compound;

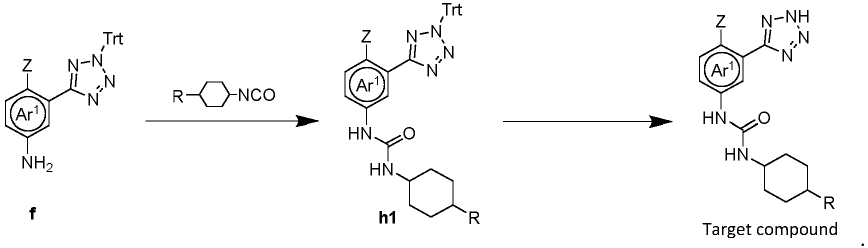

Compounds 58-62 and 66-71 in the Examples were prepared according to the above Method 4.

In a fourth aspect, the present invention provides a pharmaceutical composition, which may comprise the above-mentioned N-tetrazolyl aryl urea-based derivative, a pharmaceutically acceptable carrier or excipient, and optionally other therapeutic agents.

The pharmaceutical composition of the present invention may contain at least one active ingredient and one or more pharmaceutically acceptable carriers or excipients; the active ingredient may include at least one of the compound represented by Formula I, a cis-trans isomer thereof, and a pharmaceutically acceptable salt or solvate thereof.

Preferably, the pharmaceutically acceptable salt may be an alkali metal salt, an alkaline earth metal salt, or an ammonium salt.

Further preferably, the alkali metal salt may be a sodium salt, a potassium salt, or a lithium salt; the alkaline earth metal salt may be a calcium salt or a magnesium salt.

More preferably, the pharmaceutically acceptable salt may be a sodium salt or a potassium salt.

Preferably, the carrier or excipient may include one or more of conventional diluents, fillers, adhesives, wetting agents, disintegrants, absorption promoters, surfactants, adsorption carriers, lubricants, flavoring agents and sweeteners in the pharmaceutical field.

Preferably, the pharmaceutical composition of the present invention may be in the form of tablets, capsules, patches, emulsions, suspensions, gels, powders, granules, oral preparations, eye drops or injections. Preferably, the pharmaceutical composition of the present invention may be in the form of injections, oral preparations or eye drops. The pharmaceutical compositions of the above forms may be prepared according to conventional methods in the pharmaceutical field.

Typically, the pharmaceutical composition of the present invention may be formulated according to techniques known in the art. The pharmaceutical composition may be in any form suitable for oral, parenteral, topical, intranasal, intrabronchial, sublingual, ocular, aural, rectal, intravaginal or transdermal administration. If the composition is intended for parenteral administration, it may be formulated for intravenous, intramuscular, intraperitoneal, subcutaneous administration, or delivered directly to a target organ or tissue by injection, infusion or other delivery methods. In the present invention, the route of administration is preferably injection, oral administration or ocular administration.

In a fifth aspect, the present invention provides a use of the above-mentioned N-tetrazolyl aryl urea-based derivative in the preparation of a bradykinin B1 receptor antagonist.

In a sixth aspect, the present invention provides a use of the above-mentioned N-tetrazolyl aryl urea-based derivative as a bradykinin B1 receptor antagonist.

Preferably, the bradykinin B1 receptor antagonist or medicament comprises at least one active ingredient and one or more pharmaceutically acceptable carriers or excipients; the active ingredient is at least one of the compound represented by Formula I, a cis-trans isomer thereof, and a pharmaceutically acceptable salt or solvate thereof.

In a seventh aspect, the present invention provides a use of the above-mentioned N-tetrazolyl aryl urea-based derivative alone or in combination with other drugs in the preparation of a medicament for preventing or treating a disease mediated by the bradykinin B1 receptor.

In an eighth aspect, the present invention provides the above-mentioned N-tetrazolyl aryl urea-based derivative, alone or in combination with other drugs, for use in the preventment or treatment of a disease mediated by the bradykinin B1 receptor.

In a ninth aspect, the present invention provides a method for preventing or treating a disease mediated by the bradykinin B1 receptor in a subject, comprising administering an effective amount of the above-mentioned N-tetrazolyl aryl urea-based derivative or the pharmaceutical composition to the subject.

The amount of the N-tetrazolyl aryl urea-based derivative used in the present invention may also depend on the condition of the subject to whom the N-tetrazolyl aryl urea-based derivative is administered, the dosage form, etc. Specifically, the dosage administered may depend on the patient's condition, weight, age, gender, etc., and cannot be generalized. The dosage administered may be determined empirically by a qualified physician.

The above-mentioned disease mediated by the bradykinin B1 receptor may include, but are not limited to, respiratory diseases, digestive diseases, cardiovascular diseases, urinary diseases, Dermatologic diseases, ophthalmic diseases, joint and bone diseases, central and peripheral nervous system diseases, infectious diseases, pain, diabetic complications, trauma and ophthalmic inflammatory diseases.

Preferably, the disease or a syndrome, condition or symptom thereof is related to an inflammatory response caused by an infectious disease, including pneumonia, pulmonary edema and acute respiratory distress syndrome caused by COVID-19 infection, pneumonia and type I hypersensitivity caused by respiratory syncytial virus, bacterial sepsis and shock caused by sepsis; or complications caused by diabetes, including retinal edema and lesions, macular degeneration, neuralgia, diabetic hand and foot ulcers caused by diabetes; or ophthalmic inflammatory diseases, including: allergic conjunctivitis, chronic conjunctivitis and uveitis.

As non-limiting examples, the above-mentioned disease mediated by the bradykinin B1 receptor may be selected from the group consisting of acute pneumonia, pulmonary edema and acute respiratory distress syndrome caused by COVID-19, diabetic retinopathy, age-related macular degeneration, diabetic neuropathy, allergic conjunctivitis, chronic conjunctivitis and uveitis.

The above-mentioned N-tetrazolyl aryl urea-based derivative of the present invention may be prepared, studied and used as a therapeutic or preventive meidicament alone or in combination in a wide range of disease fields. Based on its action mechanism of inhibiting inflammatory responses and pain, the compound of the present invention is expected to be mainly used for the following indications in the clinic, including but not limited to the preventive and therapeutic uses in these listed diseases:
Respiratory diseases: any known and unknown respiratory and lung inflammation caused by viral infection, such as pneumonia, pulmonary edema and acute respiratory distress syndrome caused by COVID-19, any inflammatory events related to airway diseases, such as chronic obstructive pulmonary disease, asthma, emphysema, respiratory distress syndrome, bronchitis, pneumonia, cough, lung injury, pulmonary fibrosis, allergic rhinitis, vascular rhinitis and angioedema, etc.
Digestive diseases: inflammatory bowel diseases including Crohn's disease and ulcerative colitis, irritable bowel syndrome and pancreatitis, etc.
Cardiovascular diseases: including congestive heart failure, myocarditis (including infectious and non-infectious myocarditis), pericarditis and Takayasu arteritis, etc.
Urinary diseases: nephritis, cystitis (interstitial cystitis), painful bladder syndrome and overactive bladder.
Dermatologic diseases: including pruritus, inflammatory Dermatologic diseases, psoriasis, atopic dermatitis (eczema), neurodermatitis and herpes zoster, etc.
Ophthalmological diseases: allergic conjunctivitis, chronic conjunctivitis, uveitis, diabetic retinopathy (DR), age-related macular degeneration (AMD), and pathological choroidal or retinal vascularization and inflammation from any mechanism, retinal vein occlusion, ocular trauma, surgically induced edema, cystic macular edema, ocular ischemia, retinopathy of prematurity, hypertensive retinopathy, and glaucoma, etc.
Joint and bone diseases: including rheumatoid arthritis, gout, osteoarthritis and ankylosing spondylitis.
Central and peripheral nervous system diseases: neurodegenerative diseases, including Parkinson's disease, Alzheimer's diseases, amyotrophic lateral sclerosis (ALS), epilepsy, migraine, stroke, closed head trauma and multiple sclerosis.
Infectious diseases: including HIV infection and tuberculosis, COVID-19 virus, respiratory syncytial virus, herpes zoster virus, Hantavirus, rhinovirus, bacterial infectious sepsis and shock caused by sepsis, periodontitis, parasitic infections such as malaria and schistosomiasis.
Pain: including visceral pains, such as pains related to pancreatitis, interstitial cystitis, bladder pain syndrome, renal colic or prostatitis, chronic pelvic pain, or pain related to infiltrative endometriosis; neuropathic pains, such as diabetic and cancer pains, herpes zoster neuralgia, trigeminal neuralgia, postoperative pain syndrome, bone and joint pains (osteoarthritis), spinal pain, toothache, chronic pelvic pain, or inflammatory pains from different sources (including, but not limited to, pains associated with osteoarthritis, rheumatoid arthritis, rheumatic diseases, tenosynovitis, gout, ankylosing spondylitis and bursitis).
Complications of diabetes: diabetic hand and foot ulcers, diabetic nephropathy, diabetic retinopathy, etc.
Trauma: edema, including cerebral edema, burns, sunburn, sprains or fractures;
Ophthalmic inflammatory diseases: including allergic conjunctivitis, chronic conjunctivitis and uveitis.
Others: including primary peritonitis, secondary peritonitis, septic shock, sepsis, etc.

In addition, the compound of the present invention has been verified for its primary pharmacological and pharmacodynamic effects via animal tests. It can be useful primarily for acute pneumonia, pulmonary edema and acute respiratory distress syndrome caused by COVID-19, diabetic retinopathy, age-related macular degeneration, diabetic indications such as diabetic neuropathy, ophthalmic inflammatory diseases such as allergic conjunctivitis, chronic conjunctivitis, uveitis and the like. The main action mechanisam as revealed will be described as follows.

Action mechanism of bradykinin B1 receptor antagonist in pneumonia caused by COVID-19 is set forth below.

As confirmed by recent literatures, the spike protein of COVID-19 internalizes angiotensin-converting enzyme 2 (ACE2) by binding to it, thereby reducing the expression level and function of ACE2, causing disfunction of the renin-angiotensin system (RAS system). Loss in the efficacy of retroegulation of the bradykinin system continuously amplifies and strengthens the proinflammatory response of the bradykinin system, and further causes increased vascular permeability and pulmonary edema. As verified by the aminal tests, upon injection of the spike protein of SARS or COVID-19 (without infection ability), the level and function of ACE2 are downregulated, while lung injury is further aggravated to cause pulmonary edema. It is also reported that use of recombinant ACE2 protein (hrsACE2) can effectively block the spike protein on the surface of COVID-19, thereby maintaining the normal level of ACE2 on the surface of lung cells and inhibiting the viral invasion, and is useful as an emergency prevention method against infection after exposure to COVID-19. These research results suggest that the level of ACE2 on the surface of lung cells is negatively correlated with the degree of pulmonary edema.

At the same time, ACE2 is also an important signal-regulated enzyme of the cross-talk between the RAS system and the kallikrein-kinin system (KKS system). In the KKS system, ACE2 can degrade and inactivate endogenous bradykinin des-Arg9-BK and Lys-des-Arg9-BK, which are agonists specific for bradykinin receptor 1 (B1R), and are closely related to persistent inflammatory responses. According to the molecular mechanism of the KKS system, it can be deduced that upon binding to ACE2 and invasion into alveolar epithelial cells of COVID-19 to cause local injury of the lung tissue, bradykinin (BK) and pancreatic kinin (Lys-BK) (agonists specific for bradykinin receptor-2 (B2R)) are first released to activate B2R which participates in the acute inflammatory response. Then, those two B2R-specific bradykinins are hydrolyzed by ACE1 into B1R-specific bradykinin, which participates in upregulation and activation of B1R (while downregulating B2R), causing the release of a large number of inflammatory factors, an increase in capillary permeability and vascular leakage. At the same time, the inflammatory factors in turn upregulate the BIR level in a way of positive feedback, and further aggravat the inflammatory response, thereby causing pulmonary edema. Due to the dysfunction of ACE2 caused by COVID-19 infection, the hydrolysis and inactivation of B1R-specific bradykinin are lost, causing B1R to be in a state of continuous activation, thereby aggravating pulmonary edema, and ultimately causing acute respiratory distress syndrome and endangering life.

Based on the molecular mechanism of the above-mentioned KKS system, a research group has clinically used the B2R peptide-based antagonist Icatibant (trade name: Firazyr) to treat pulmonary edema in COVID-19 patients (9 patients in the drug group and 18 patients in the placebo group). The results reveal that Icatibant can significantly improve the hypoxia condition caused by pulmonary edema in COVID-19 patients. It has been found in a case-control study of 30 patients with severe COVID-19 that as compared with the standard treatment group of pneumonia caused by COVID-19, Icatibant can significantly improve lung CT scores and increase eosinophils in the blood, although it does not shorten the recovery time in the clinic. From the above limited clinical research data, although a B2R antagonist can partially improve pulmonary edema caused by COVID-19, the efficacy is not significant. This may be related to the biological characteristics of B2R. Although B2R is also involved in inflammatory response, it is limited to the early stage of inflammation, because B2R will be quickly desensitized and internalized after repeated activation by bradykinin, which causes loss in its function. In addition, B2R is expressed in normal tissues, and will bring about side effects related to cardiovascular system after being inhibited. Therefore, B2R has great limitations as a target for the treatment of pneumonia caused by COVID-19. On the contrary, upon upregulation of B1R in inflammatory tissues, neither desensitization nor internalization is caused by its repeated stimulation. Thus, B1R may continuously participate in the process of inflammatory response. Moreover, B1R is only expressed in inflammatory tissues, and is not expressed in normal tissues. Thus, the side effects on normal tissues can be avoided upon its administration. Based on the above-summarized molecular mechanism of the KKS system and the clinical trial results of Icatibant, we assume that B1R antagonist will have a better therapeutic effect on pneumonia and pulmonary edema caused by COVID-19, especially in severe cases. In addition, its administration in early stage can potentially prevent excessive inflammatory response caused by COVID-19 infection and avoid the occurrence of pulmonary edema, reduce the incidence of severe illness, and have stronger specificity and safety. Unfortunately, currently, there are no medicines of B1R antagonist which is clinically useful clinic in the domestic or abroad.

It has been demonstrated in the pharmacodynamics study on the compound as a B1R antagonist that the present compound can inhibit production and secretion of various inflammatory factors by IMR-90 cells induced by bradykinin des-Arg9-BK, and inhibit inflammatory pulmonary edema in rats induced by des-Arg9-BK and IL-1β. In a animal model of LPS-induced acute lung injury and ARDS, the present compound can alleviate the lung injury and pulmonary edema in mice, and improve the declining tendancy of blood oxygen partial pressure and oxygen saturation.

Therefore, the pharmaceutical preparation containing the compound of the present invention is expected to be useful for treating and preventing pneumonia and pulmonary edema caused by COVID-19, reduce the risk of developing acute respiratory distress syndrome, and improve the survival rate of patients.

Action mechanism of bradykinin B1 receptor antagonist in diabetes-related complications is set forth below.

Diabetic retinopathy (DR) is the most common ocular microvascular complication in diabetic patients and the major cause of blindness in adults. With an increase in the number of diabetic patients and extension of survival time, DR has become the primary cause of severe visual impairment in the population aged from 20 to 79 in China, and is one of the main diseases causing blindness in the middle-aged and elderly population. According to a research report on the epidemiological survey of Chinese residents from 1990 to 2020, the incidence of diabetic retinopathy in the normal population and diabetic patients was 1.7% and 22.4%, respectively (Deng Yuxuan 2020).

At present, the treatment of DR is limited to the late stage of diabetic oculopathy, at this time the retinal blood vessels and optic nerves have been irreversibly injuried. In addition to drug treatments for controlling the blood glucose level, controlling the blood pressure, and lowering blood lipids level, as well as laser therapy and surgical treatments, the treatment methods are limited to intraocular injections of anti-vascular endothelial growth factor (VEGF) monoclonal antibodies or glucocorticoids. Those treatment methods are invasive, with poor compliance of the patients. Moreover, those treatments can only prevent the progression of DR, but can hardly restore the lost vision. Taking intraocular injection of vascular endothelial growth inhibitor as an example, in addition to a poor clinical improvement or drug resistance in part of the patients, long-term repeated intraocular injections may cause serious adverse effects including endophthalmitis, vitreous hemorrhage, retinal detachment, traumatic cataracts, and increased intraocular pressure. Therefore, clinically there is a need for more effective treatment and prevention medicament to intervene in the early stage of DR. The compound or preparation used as the B1R antagonist in the present invention is administered by intraocular injection or eye drops alone or in combination with other drugs to treat and prevent the fundus inflammation in the early stage of DR and the retinal change in the mid- to-late stage of DR.

The treatment of DR by the B1R antagonist is via the mechanism that the expression and upregulation of B1 receptor is triggerd under the pathological conditions caused by proinflammatory cytokines and hyperglycemia-related oxidative stress. Studies have shown that B1 receptor activates iNOS by activating the Gαi/ERK/MAPK signaling pathway, thereby causing a series of inflammatory responses in the early stage of DR. Therefore, the activation of B1 receptor as a signal from upstream may promote the expression of iNOS, and meanwhile continuously aggravate the inflammatory response, enhance the vascular leakage and proliferation by self-induction and positive feedback amplification, and finally injure the retinal nerves. It has been reported in the literatures inhibition of B1 receptor can reduce the oxidative stress response, reduce the vascular permeability, control inflammatory responses such as reducing the leukocyte infiltration and reverse the expression levels of inflammatory mediators such as B1R, iNOS, IL-1b, COX-2, VEGF-A, VEGF-R2, and HIP-1a, thereby reducing changes in vascular structure, retinal edema, and other irreversible injury. Therefore, B1 receptor inhibitors, as a drug target for the treatment and prevention of DR, are expected to fill a significant gap in this therapeutic area.

### Preparation Example 1

### Synthesis of 4-(tert-butyl)-N-(3',4'-dimethoxy-2-(2H-tetrazol-5-yl)-[1,1'-biphenyl]-4-yl)piperidine-1-carboxamide (Compound 1):

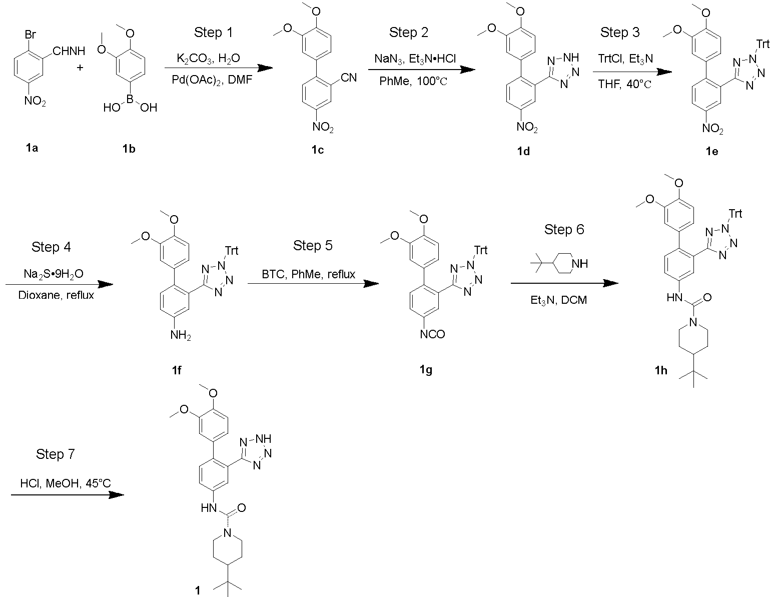

Step 1: Synthesis of 3',4'-dimethoxy-4-nitro-[1,1'-biphenyl]-2-carbonitrile (1c): 2.04 g (9.00 mmol) of 2-bromo-5-nitrobenzonitrile, 1.97 g (10.80 mmol) of 3,4-dimethoxyphenylboronic acid, 2.48 g (18.00 mmol) of potassium carbonate, 0.01 g (0.045 mmol) of palladium acetate, 100 mL of N,N-dimethylformamide and 20 mL of water were added to a reaction flask, and the mixture was stirred at the room temperature for 6 h under nitrogen protection. After the reaction was completed, 500 mL of water was added. The resultant mixture was adjusted to be acidic with dilute hydrochloric acid, and then was extracted with dichloromethane. The organic phase was washed with water for 3 times, dried over anhydrous, filtered, and concentrated under reduced pressure. The residue was recrystallized from ethyl acetate to obtain 2.08 g of 1c. Yield: 81%.

Step 2: Synthesis of 5-(3',4'-dimethoxy-4-nitro-[1,1'-biphenyl]-2-yl)-2H-tetrazole (1d): 1.71 g (6.00 mmol) of 3',4'-dimethoxy-4-nitro-[1,1'-biphenyl]-2-carbonitrile and 90 mL of toluene were added to a reaction flask, and then 1.17 g (18.00 mmol) of sodium azide and 2.89 g (21.00 mmol) of triethylammonium chloride were added in sequence. The reaction was carried out at 90°C for 15 h. After the reaction was completed, the mixture was adjusted to have a pH of 2 by adding dilute hydrochloric acid, and then was extracted with ethyl acetate. The organic layer was dried over anhydrous sodium sulfate, filtered, and concentrated under reduced pressure. The residue was recrystallized from ethyl acetate to obtain 1.58 g of 1d. Yield: 80%.

Step 3: Synthesis of 5-(3',4'-dimethoxy-4-nitro-[1,1'-biphenyl] -2-yl)-2-trityl-2H-tetrazole (1e): 5.06 g (15.47 mmol) of 5-(3',4'-dimethoxy-4-nitro-[1,1'-biphenyl]-2-yl)-2H-tetrazole, 4.53 g (16.24 mmol) of triphenylchloromethane, 1.64 g (16.24 mmol) of triethylamine and 225 mL of tetrahydrofuran were added to a reaction flask. The reaction was carried out at 40°C for 3 h. After the reaction was completed, the mixture was diluted with water, extracted with ethyl acetate, washed with saturated brine, dried over anhydrous sodium sulfate, filtered, and concentrated under reduced pressure. The residue was recrystallized from ethyl acetate to obtain 5.0 g of 1e. Yield: 57%.

Step 4: Synthesis of 3',4'-dimethoxy-2-(2-trityl-2H-tetrazol-5-yl)-[1,1'-biphenyl]-4-amine (1f): 1.16 g (2.04 mmol) of 1e, 45 mL of 1,4-dioxane and 2.94 g (12.24 mmol) of sodium sulfide nonahydrate were added to a reaction flask. The reaction was carried out under reflux for 4 h. After the reaction was completed, the mixture was diluted with water, extracted with ethyl acetate, washed with saturated brine, dried over anhydrous sodium sulfate, filtered, and concentrated under reduced pressure. The residue was recrystallized from ethyl acetate to obtain 0.88 g of 1f. Yield: 80%.

Step 5: Synthesis of 5-(4-isocyanato-3',4'-dimethoxy-[1,1'-biphenyl]-2-yl)-2-trityl-2H-tetrazole (1g): 0.30 g (0.56 mmol) of 1f, 0.08 g (0.28 mmol) of triphosgene and 30 mL of anhydrous toluene were added to a reaction flask. The reaction was carried out under reflux for 4 h. After the reaction was completed, the reaction solution was concentrated under reduced pressure. The residue was directly used for the next step.

Step 6: Synthesis of 4-(tert-butyl)-N-(3',4'-dimethoxy-2-(2-trityl-2H-tetrazol-5-yl)-[1,1'-biphenyl]-4-yl)piperidine-1-carboxamide (2a): 0.099 g (0.56 mmol) of 4-tert-butylpiperidine hydrochloride, 0.06 g (0.56 mmol) of triethylamine and 20 mL of anhydrous DCM were added to a reaction flask. The mixture was cooled to 0°C, and then 0.32 g (0.56 mmol) of 1g in 15 mL of anhydrous DCM was added. After the addition was completed, the mixture was cooled to to the room temperature, and the reaction was carried out for 1 h. After the reaction was completed, the reaction solution was concentrated under reduced pressure, and the residue was purified by silica gel column chromatography (DCM:EA:MeOH=120:1:1, v/v) to obtain 0.23 g of 2a. Yield through the two steps: 58%.

Step 7: Synthesis of 1-(4-(tert-butyl)cyclohexyl)-3-(3',4'-dimethoxy-2-(2H-tetrazol-5-yl)-[1,1'-biphenyl]-4-yl)urea (1): 0.12 g (0.16 mmol) of 2a and 12 mL of methanol were added to a reaction flask. Then concentrated hydrochloric acid was added dropwise until the pH was adjusted to 1-2. The reaction was carried out at 45°C for 1 h. After the reaction was completed, the mixture was diluted with water, extracted with ethyl acetate, dried over anhydrous sodium sulfate, filtered, concentrated under reduced pressure. The residue was recrystallized from ethyl acetate to obtain 0.03 g of l. Yield: 30%.

ESI-MS: m/z = 465 [M+H]⁺.¹H NMR (500 MHz, DMSO-*d₆*) δ 16.12 (s, 1H), 8.77 (s, 1H), 7.82 - 7.70 (m, 2H), 7.46 (d, *J =* 8.7 Hz, 1H), 6.87 (d, *J=* 8.1 Hz, 1H), 6.61 - 6.54 (m, 2H), 4.27 - 4.20 (m, 2H), 3.74 (s, 3H), 3.59 (s, 3H), 2.77 - 2.65 (m, 2H), 1.69 (d, *J =* 12.3 Hz, 2H), 1.26 - 1.17 (m, 1H), 1.17 - 1.05 (m, 2H), 0.86 (s, 9H).

### Preparation Example 2

### Synthesis of N-(3',4'-dimethoxy-2-(2H-tetrazol-5-yl)-[1,1'-biphenyl]-4-yl)-4-(trifluoromethyl)piperidine-1-carboxamide (Compound 2):

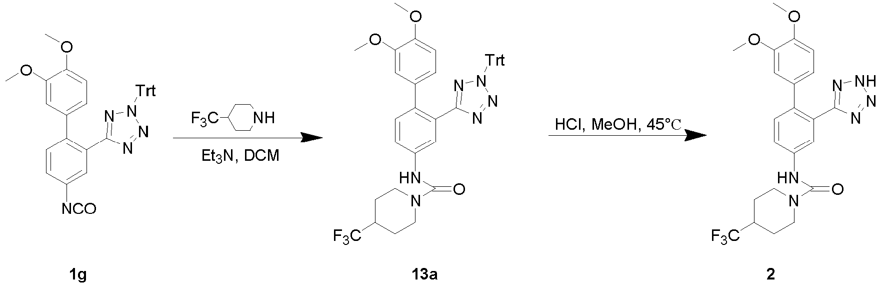

Step 1: Synthesis of N-(3',4'-dimethoxy-2-(2-trityl-2H-tetrazol-5-yl)-[1,1'-biphenyl]-4-yl)-4-(trifluoromethyl)piperidine-1-carboxamide (13a): the synthesis was performed according to Step 6 of Example 1, except that 4-tert-butylcyclohexylamine was replaced with 4-(trifluoromethyl)piperidine. Yield through the two steps: 39%.

Step 2: Synthesis of N-(3',4'-dimethoxy-2-(2H-tetrazol-5-yl)-[1,1'-biphenyl]-4-yl)-4-(trifluoromethyl)piperidine-1-carboxamide (2): the synthesis was performed according to Step 7 of Example 1, except that 1h was replaced with 13a. Yield: 49%.

ESI-MS: m/z =477 [M+H]⁺.¹H NMR (500 MHz, DMSO-*d₆*) δ 16.10 (s, 1H), 8.88 (s, 1H), 7.83 - 7.67 (m, 2H), 7.53 - 7.37 (m, 1H), 6.88 (d, *J=* 8.1 Hz, 1H), 6.67 - 6.49 (m, 2H), 4.25 (d, *J* = 13.4 Hz, 2H), 3.74 (s, 3H), 3.59 (s, 3H), 2.93 - 2.79 (m, 2H), 2.71 - 2.55 (m, 1H), 1.91 - 1.81 (m, 2H), 1.50 - 1.28 (m, 2H).

### Preparation Example 3

### Synthesis of N-(3',4'-dimethoxy-2-(2H-tetrazol-5-yl)-[1,1'-biphenyl]-4-yl)-1-oxy-8-azaspiro[4.5]decane-1-carboxamide (Compound 3):

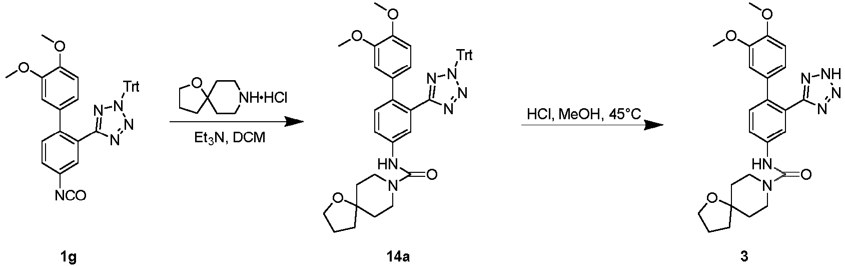

Step 1: Synthesis of N-(3',4'-dimethoxy-2-(2-trityl-2H-tetrazol-5-yl)-[1,1'-biphenyl]-4-yl)-1-oxa-8-azaspiro[4.5]decane-8-carboxamide (14a): 0.09 g (0.56 mmol) of 1-oxazole-8-azaspiro[4.5]decane hydrochloride, 0.12 g (1.23 mmol) of triethylamine and 20 mL of anhydrous DCM were added to a reaction flask. The mixture was cooled to 0°C. Then, 0.32 g (0.56 mmol) of 1g in 15 mL of anhydrous DCM solution was added. After the addition was completed, the mixture was warmed to the room temperature, and the reaction was carried out for 1 h. After the reaction was completed, the reaction solution was concentrated under reduced pressure, and the residue was purified by silica gel column chromatography (DCM:EA=10:1, v/v) to obtain 0.14 g of 14a. Yield through the two steps: 35%.

Step 2: Synthesis of N-(3',4'-dimethoxy-2-(2H-tetrazol-5-yl)-[1,1'-biphenyl]-4-yl)-1-oxy-8-azaspiro[4.5]decane-1-carboxamide (3): the synthesis was performed according to Step 7 of Example 1, except that 1h was replaced with 14a. Yield: 63%.

ESI-MS: m/z =465[M+H]⁺¹H NMR (500 MHz, DMSO-*d₆*) δ 16.14 (s, 1H), 8.81 (s, 1H), 7.76 (d, *J =* 7.5 Hz, 2H), 7.49 - 7.42 (m, 1H), 6.87 *(d, J =* 8.2 Hz, 1H), 6.61 - 6.54 (m, 2H), 3.76 (t*, J =* 6.7 Hz, 2H), 3.74 (s, 3H), 3.67 - 3.60 (m, 2H), 3.59 (s, 3H), 3.41 - 3.34 (m, 2H), 1.92 - 1.85 (m, 2H), 1.72 - 1.67 (m, 2H), 1.55 (t, *J =* 5.6 Hz, 4H).

### Preparation Example 4

### Synthesis of N-(3',4'-dimethoxy-2-(2H-tetrazol-5-yl)-[1,1'-biphenyl]-4-yl)hexahydrofuro[2,3-c]pyridine-6(2H)-carboxamide (Compound 4):

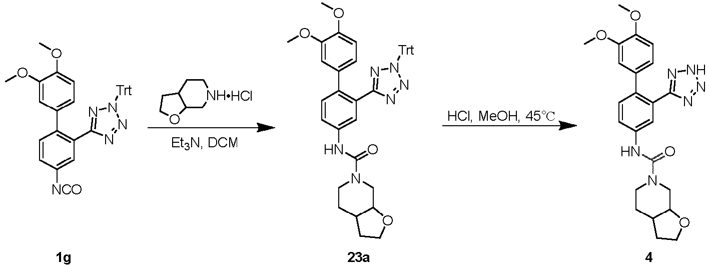

Step 1: Synthesis of N-(3',4'-dimethoxy-2-(2-trityl-2H-tetrazol-5-yl)-[1,1'-biphenyl]-4-yl)hexahydrofuro[2,3-c]pyridine-6(2H)-carboxamide (Compound 23a): the synthesis was performed according to Step 6 of Example 1, except that 1-oxazole-8-azaspiro[4.5]decane hydrochloride was replaced with octahydrofuran[2,3-c]pyridine hydrochloride. Yield: 88%.

Step 2: Synthesis of N-(3',4'-dimethoxy-2-(2H-tetrazol-5-yl)-[1,1'-biphenyl]-4-yl)hexahydrofuro[2,3-c]pyridine-6(2H)-carboxamide (Compound 4): the synthesis was performed according to Step 7 of Example 1, except that 1h was replaced with 23a. Yield: 54%.

ESI-MS: m/z =451[M+H]⁺¹H NMR (500 MHz, DMSO-*d₆*) δ 16.15 (s, 1H), 8.88 (s, 1H), 7.82 - 7.71 (m, 2H), 7.50 - 7.41 (m, 1H), 6.87 (d, *J =* 8.1 Hz, 1H), 6.57 (d, *J =* 8.9 Hz, 2H), 4.51 - 4.39 (m, 1H), 4.36 - 4.27 (m, 1H), 3.86 - 3.78 (m, 2H), 3.73 (s, 3H), 3.58 (s, 3H), 3.15 - 3.08 (m, 1H), 2.82 - 2.72 (m, 1H), 2.67 (t, *J =* 12.0 Hz, 1H), 2.10 - 1.93 (m, 2H), 1.65 - 1.51 (m, 1H), 1.48 - 1.27 (m, 2H).

### Preparation Example 5

### Synthesis of N-(4-(6-ethoxypyrid-3-yl)-3-(2H-tetrazol-5-yl)phenyl)-4-methylpiperidine-1-carboxamide (Compound 5):

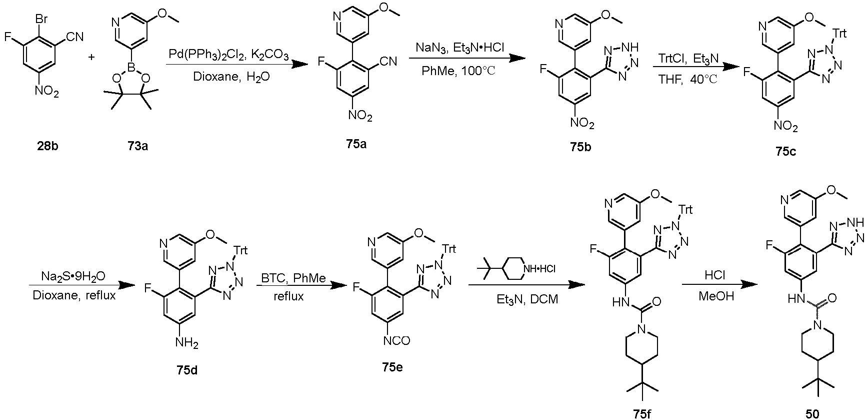

Step 1: Synthesis of 4-methylpiperidine-1-carbonyl chloride (25c): 0.99 g (10.00 mmol) of 25a, 1.24 g (4.30 mmol) of 25b and 40 mL of anhydrous DCM were added to a reaction flask. 1.12 g (8.80 mmol) of DIEPA was added dropwise at 0°C. After the addition was completed, the mixture was warmed to the room temperature, and the reaction was carried out for 1 h. After the reaction was completed, the mixture was diluted with water, and extracted with dichloromethane. The organic layer was dried over anhydrous sodium sulfate, filtered, and concentrated under reduced pressure. The residue was directly used for the next step.

Step 2: Synthesis of 2-(6-ethoxypyrid-3-yl)-5-nitrobenzonitrile (24b): the synthesis was performed according to Step 1 of Example 1, except that lb was replaced with 24a. Yield: 81%.

Step 3: Synthesis of 2-ethoxy-5-(4-nitro-2-(2H-tetrazol-5-yl)phenyl)pyridine (24c): the synthesis was performed according to Step 2 of Example 1, except that 1c was replaced with 24b. Yield: 58%.

Step 4: Synthesis of 2-ethoxy-5-(4-nitro-2-(2-trityl-2H-tetrazol-5-yl)phenyl)pyridine (24d): the synthesis was performed according to Step 3 of Example 1, except that 1d was replaced with 24c. Yield: 75%.

Step 5: Synthesis of 4-(6-ethoxypyrid-3-yl)-3-(2-trityl-2H-tetrazol-5-yl)phenylamine (24e): the synthesis was performed according to Step 4 of Example 1, except that 1e was replaced with 24d. Yield: 87%.

Step 6: Synthesis of N-(4-(6-ethoxypyrid-3-yl)-3-(2-trityl-2H-tetrazol-5-yl)phenyl)-4-methylpiperidine-1-carboxamide (25d): 0.22 g (0.42 mmol) of 24e, 0.11 g (0.68 mmol) of 25c and 8 mL of anhydrous pyridine were added to a reaction flask. Then, 0.20 g (0.04 mmol) of DIPEA was added dropwise. Then, the mixture was warmed to 65°C, and the reaction was carried out for 10 h. After the reaction was completed, the mixture was diluted with water, adjusted to have a pH of 6-7 by adding dilute hydrochloric acid, and extracted with ethyl acetate. The organic layer was dried over anhydrous sodium sulfate, filtered, concentrated under pressure. The residue was purified by silica gel column chromatography (DCM: EA= 10:1, v/v) to obtain 0.10 g of 25d. Yield: 37%.

Step 7: Synthesis of N-(4'-ethoxy-2-(2H-tetrazol-5-yl)-[1,1'-biphenyl] -4-yl)-4-methylpiperidine-1-carboxamide (5): the synthesis was performed according to Step 7 of Example 1, except that 1h was replaced with 25d. Yield: 62%.

ESI-MS: m/z =408[M+H]⁺.¹H NMR (500 MHz, DMSO-*d*₆) δ 16.31 (s, 1H), 8.79 (s, 1H), 7.88 (d, *J =* 2.6 Hz, 1H), 7.86 (d, *J =* 2.3 Hz, 1H), 7.76 (dd, *J* = 8.5, 2.3 Hz, 1H), 7.42 (d, *J =* 8.5 Hz, 1H), 7.29 (dd, *J* = 8.6, 2.6 Hz, 1H), 6.69 (d,*J* = 8.6 Hz, 1H), 4.28 (q, *J =* 7.1 Hz, 2H), 4.15 - 4.06 (m, 2H), 2.84 - 2.73 (m, 2H), 1.69 - 1.61 (m, 2H), 1.61 - 1.53 (m, 1H), 1.31 (t, *J* = 7.0 Hz, 3H), 1.07 - 0.99 (m, 2H), 0.93 (d, *J =* 6.5 Hz, 3H).

### Preparation Example 6

### Synthesis of 4-(tert-butyl)-N-(4-(6-ethoxypyrid-3-yl)-3-(2H-tetrazol-5-yl)phenyl)piperidine-1-carboxamide (Compound 6):

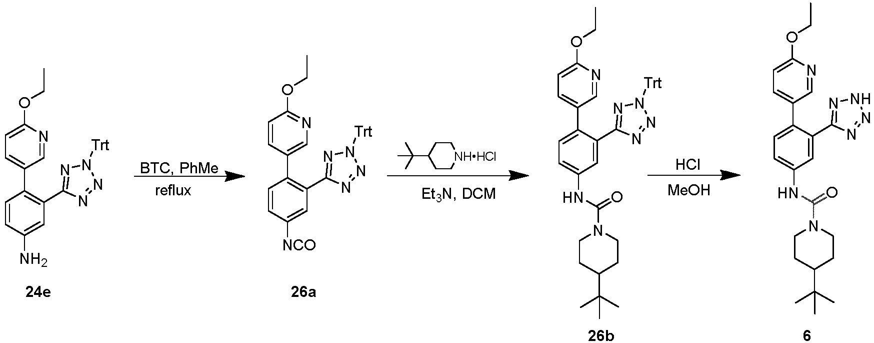

Step 1: Synthesis of 2-ethoxy-5-(4-isocyanato-2-(2-trityl-2H-tetrazol-5-yl)phenyl)pyridine (26a): the synthesis was performed according to Step 4 of Example 1, except that 1f was replaced with 24e, and the obtained residue was directly used for the next step.

Step 2: Synthesis of 4-(tert-butyl)-N-(4-(6-ethoxypyrid-3-yl)-3-(2-trityl-2H-tetrazol-5-yl)phenyl)piperidine-1-carboxamide (26b): the synthesis was performed according to Step 6 of Example 1, except that 1g was replaced with 26a, and 4-tert-butylcyclohexylamine was replaced with 4-tert-butylpiperidine hydrochloride. Yield through the two steps: 37%.

Step 3: Synthesis of 4-(tert-butyl)-N-(4-(6-ethoxypyrid-3-yl)-3-(2H-tetrazol-5-yl)phenyl)piperidine-1-carboxamide (6): the synthesis was performed according to Step 7 of Example 1, except that 1h was replaced with 26b. Yield: 42%.

ESI-MS: m/z = 450[M+H]⁺. ¹H NMR (400 MHz, DMSO-*d*₆) δ 16.33 (s, 1H), δ 8.84 (s, 1H), 7.89 (d, *J* = 2.6 Hz, 1H), 7.86 (d, *J* = 2.3 Hz, 1H), 7.77 (dd, *J =* 8.5, 2.3 Hz, 1H), 7.43 (d, *J* = 8.5 Hz, 1H), 7.29 (dd, *J* = 8.6, 2.6 Hz, 1H), 6.70 (d, *J* = 8.6 Hz, 1H), 4.33 - 4.19 (m, 4H), 2.71 (t, *J* = 11.8 Hz, 2H), 1.69 (d,*J* = 11.5 Hz, 2H), 1.31 (t, *J =* 7.0 Hz, 3H), 1.19 - 1.15 (m, 1H), 1.14 - 1.08 (m, 2H), 0.86 (s, 9H).

### Preparation Example 7

### Synthesis of 4-(tert-butyl)-N-(6'-ethoxy-3-(2H-tetrazol-5-yl)-[2,3'-bipyridyl]-5-yl)piperidine-1-carboxamide (Compound 7):

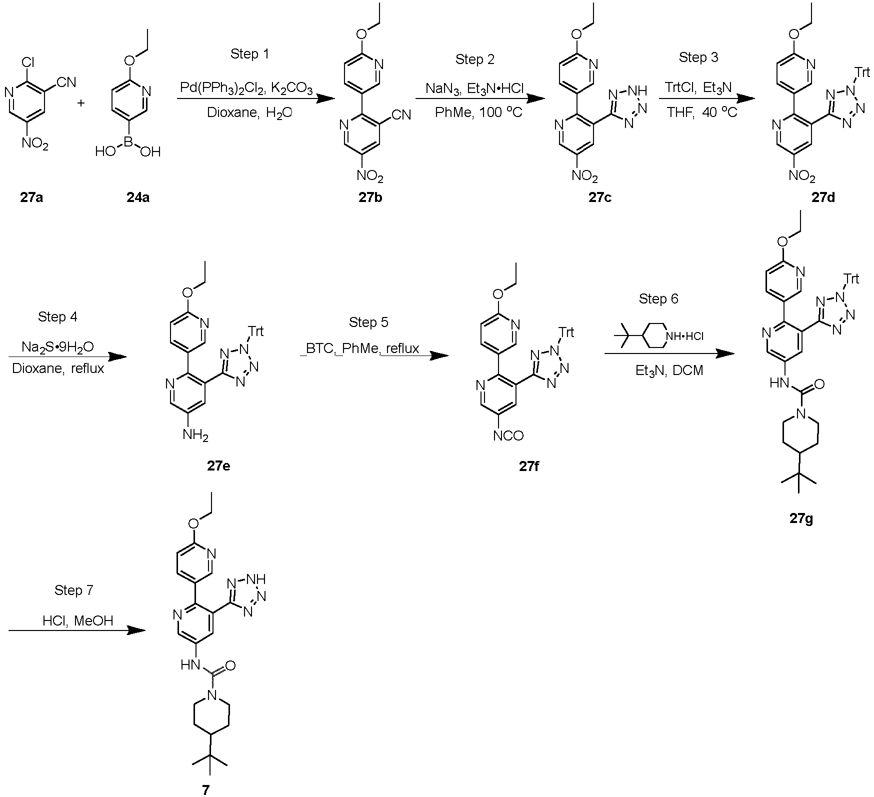

Step 1: Synthesis of 6'-ethoxy-5-nitro-[2,3'-bipyridyl]-3-carbonitrile (27b): 820 mg (4.46 mmol) of 27a, 900 mg (5.39 mmol) of 24a, 157 mg (0.22 mmol) of Pd(PPh₃)₂Cl₂, 1.80 g (13.04 mmol) of potassium carbonate, 20 mL of 1,4-dioxane and 10 mL of water were added to a reaction flask. The reaction was carried out for 3 h under reflux under nitrogen protection. After the reaction was completed, the mixture was cooled to the room temperature, diluted with water, and extracted with ethyl acetate. The organic layers were combined, washed with saturated brine, dried over anhydrous sodium sulfate, filtered, and concentrated under reduced pressure. The residue was purified by silica gel column chromatography (PE:EA= 4:1, v/v) to obtain 0.55 g of 27b. Yield: 46%.

Step 2: Synthesis of 6'-ethoxy-5-nitro-3-(2H-tetrazol-5-yl)-2,3'-bipyridyl (27c): the synthesis was performed according to Step 2 of Example 1, except that 1c was replaced with 27b. Yield: 93%.

Step 3: Synthesis of 6'-ethoxy-5-nitro-3-(2-trityl-2H-tetrazol-5-yl)-2,3'-bipyridyl (27d): the synthesis was performed according to Step 3 of Example 1, except that 1d was replaced with 27c. Yield: 89%.

Step 4: Synthesis of 6'-ethoxy-3-(2-trityl-2H-tetrazol-5-yl)-[2,3'-bipyridyl]-5-amine (27e): the synthesis was performed according to Step 4 of Example 1, except that 1e was replaced with 27d. Yield: 81%.

Step 5: Synthesis of 6'-ethoxy-5-isocyanato-3-(2-trityl-2H-tetrazol-5-yl)-2,3'-bipyridyl (27f): the synthesis was performed according to Step 5 of Example 1, except that 1f was replaced with 27e, and the residue was directly used for the next step.

Step 6: Synthesis of 4-(tert-butyl)-N-(6'-ethoxy-3-(2-trityl-2H-tetrazol-5-yl)-[2,3'-bipyridyl]-5-yl) piperidine-1-carboxamide (27g): the synthesis was performed according to Step 6 of Example 1, except that 1g was replaced with 27f, and 4-tert-butylcyclohexylamine was replaced with 4-tert-butylpiperidine hydrochloride. Yield through the two steps: 48%.

Step 7: Synthesis of 4-(tert-butyl)-N-(6'-ethoxy-3-(2H-tetrazol-5-yl)-[2,3'-bipyridyl]-5-yl)piperidine-1-carboxamide (7): the synthesis was performed according to Step 7 of Example 1, except that 1h was replaced with 27g. Yield: 41%.

ESI-MS: m/z = 451 [M+H]⁺. ¹H NMR (500 MHz, DMSO-*d*₆) δ 16.21 (s, 1H), δ 8.81 - 8.76 (m, 2H), 8.06 (d, *J* = 2.5 Hz, 1H), 8.00 (d, *J =* 2.4 Hz, 1H), 7.55 (dd, *J =* 8.6, 2.5 Hz, 1H), 6.62 (d, *J =* 8.6 Hz, 1H), 4.32 - 4.21 (m, 4H), 2.72 (t, *J =* 11.8 Hz, 2H), 1.69 *(d, J =* 11.9 Hz, 2H), 1.31 *(t, J =* 7.0 Hz, 3H), 1.20 - 1.10 (m, 3H), 0.86 (s, 9H).

### Preparation Example 8

### Synthesis of 4-(tert-butyl)-N-(4-(6-ethoxypyrid-3-yl)-3-fluoro-5-(2H-tetrazol-5-yl)phenyl)piperidine-1-carboxamide (Compound 8):

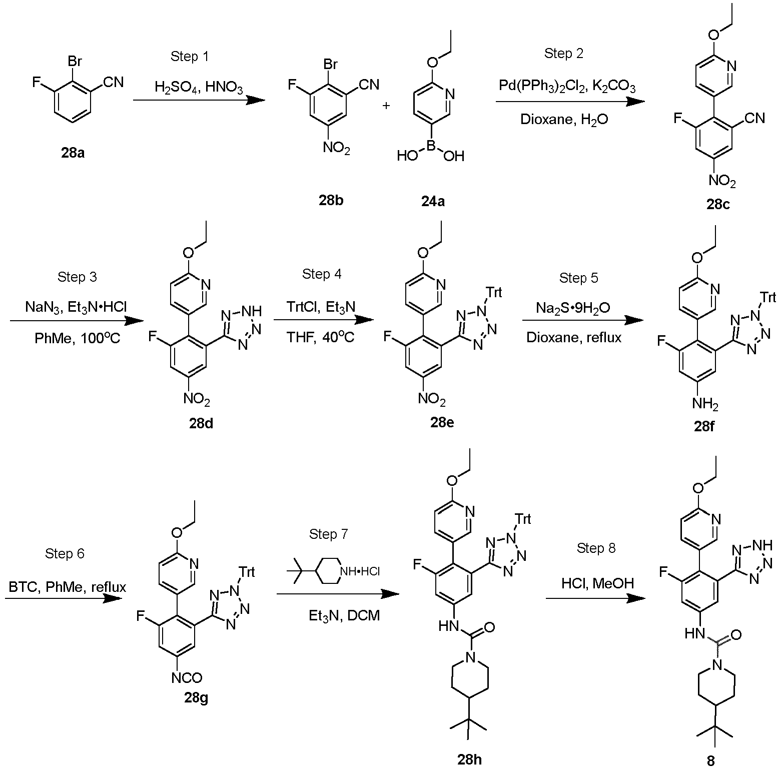

Step 1: Synthesis of 2-bromo-3-fluoro-5-nitrobenzonitrile (28b): 1.00 g (5 mmol) of 28a was added to a reaction flask. Then, 2 mL of concentrated sulfuric acid was added. Once the mixture was cooled to 0-5°C, 0.48 mL (7.5 mmol) of concentrated nitric acid was added dropwise. After the addition was completed, the mixture was kept warm, and the reaction was carried out for 1 h. Then, the reaction mixture was warmed to the room temperature, and the reaction was further carried out for 2 h. After the reaction was completed, the reaction solution was poured into ice water. The resulatant mixture was extracted with ethyl acetate. The organic layers were combined, washed with saturated brine, dried over anhydrous sodium sulfate, filtered, and concentrated under reduced pressure. The residue was purified by silica gel column chromatography (PE:EA=4:1, v/v) to obtain 328 mg of 28b. Yield: 31%.

Step 2: Synthesis of 2-(6-ethoxypyrid-3-yl)-3-fluoro-5-nitrobenzonitrile (28c): the synthesis was performed according to Step 1 of Example 1, except that 27a was replaced with 28b. Yield: 88%.

Step 3: Synthesis of 2-ethoxy-5-(2-fluoro-4-nitro-6-(2H-tetrazol-5-yl)phenyl)pyridine (28d): the synthesis was performed according to Step 2 of Example 1, except that 1c was replaced with 28c. Yield: 83%.

Step 4: Synthesis of 2-ethoxy-5-(2-fluoro-4-nitro-6-(2-trityl-2H-tetrazol-5-yl)phenyl)pyridine (28e): the synthesis was performed according to Step 3 of Example 1, except that 1d was replaced with 28d. Yield: 89%.

Step 5: Synthesis of 4-(6-ethoxypyrid-3-yl)-3-fluoro-5-(2-trityl-2H-tetrazol-5-yl)phenylamine (28f): the synthesis was performed according to Step 4 of Example 1, except that 1e was replaced with 28e. Yield: 79%.

Step 6: Synthesis of 2-ethoxy-5-(2-fluoro-4-isocyanato-6-(2-trityl-2H-tetrazol-5-yl)phenyl)pyridine (28g): the synthesis was performed according to Step 5 of Example 1, except that 1f was repelaced with 28f, and the residue was directly used for the next step.

Step 7: Synthesis of 4-(tert-butyl)-N-(4-(6-ethoxypyrid-3-yl)-3-fluoro-5-(2-trityl-2H-tetrazol-5-yl)phenyl)piperidine-1-carboxamide (28h): the synthesis was performed according to Step 6 of Example 1, except that 1g was replaced with 28g, and 4-tert-butylcyclohexylamine was replaced with 4-tert-butylpiperidine hydrochloride. Yield through the two steps: 46%.

Step 8: Synthesis of 4-(tert-butyl)-N-(4-(6-ethoxypyrid-3-yl)-3-fluoro-5-(2H-tetrazol-5-yl)phenyl)piperidine-1-carboxamide (8): the synthesis was performed according to Step 7 of Example 1, except that 1h was replaced with 28h. Yield: 64%.

ESI-MS: m/z = 468 [M+H]⁺. ¹H NMR (400 MHz, DMSO-*d*₆) δ 16.13 (s, 1H), δ 9.05 (s, 1H), 7.91 - 7.84 (m, 1H), 7.81 - 7.66 (m, 2H), 7.39 (d, *J* = 7.8 Hz, 1H), 6.76 (d, *J* = 8.5 Hz, 1H), 4.33 - 4.17 (m, 4H), 2.73 *(t, J =* 12.6 Hz, 2H), 1.70 (d, *J* = 13.3 Hz, 2H), 1.31 (t, *J* = 7.0 Hz, 3H), 1.21 - 1.17 (m, 1H), 1.16 - 1.08 (m, 2H), 0.86 (s, 9H).

### Preparation Example 9

### Synthesis of 4-(tert-butyl)-N-(4-(1-cyclobutyl-1H-pyrazol-4-yl)-3-(2H-tetrazol-5-yl)phenyl)piperidine-1-carboxamide (Compound 9):

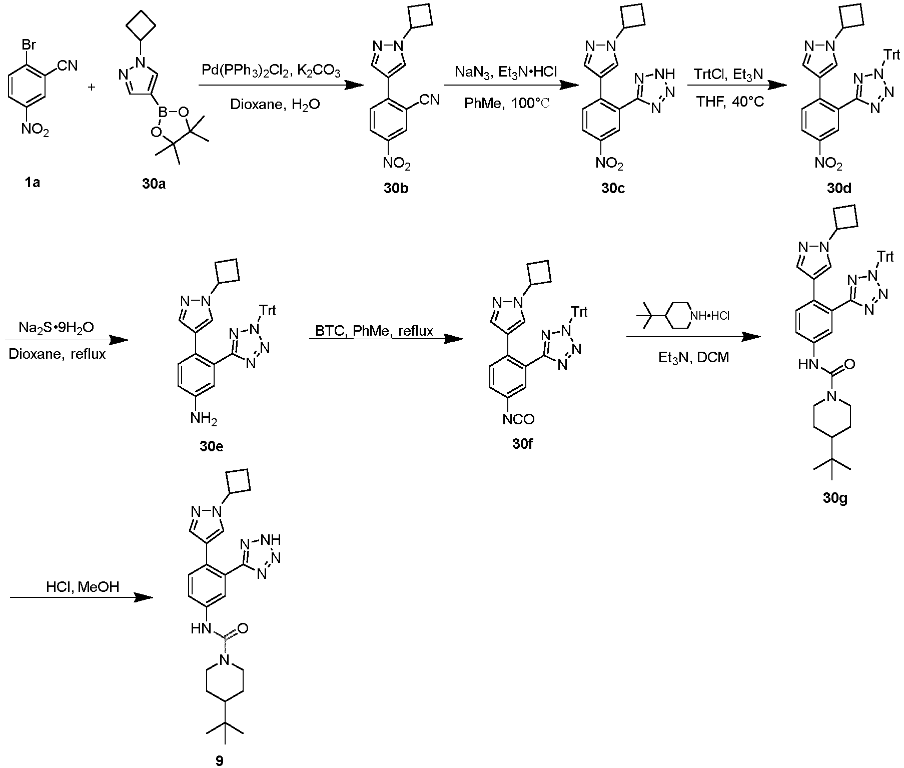

Step 1: Synthesis of 2-(1-cyclobutyl-1H-pyrazol-4-yl)-5-nitrobenzonitrile (30b): the synthesis method of 30b was similar to that of 27b, except that 27a was replaced with 1a, and 24a was replaced with 30a. Yield: 73%.

Step 2: Synthesis of 5-(2-(1-cyclobutyl-1H-pyrazol-4-yl)-5-nitrophenyl)-2H-tetrazole (30c): the synthesis method of 30c was similar to that of 1d, except that 1c was replaced with 30b. Yield: 78%.

Step 3: Synthesis of 5-(2-(1-cyclobutyl-1H-pyrazol-4-yl)-5-nitrophenyl)-2-trityl-2H-tetrazole (30d): the synthesis method of 30d was similar to that of 1e, except that 1d was replaced with 30c. Yield: 89%.

Step 4: Synthesis of 4-(1-cyclobutyl-1H-pyrazol-4-yl)-3-(2-trityl-2H-tetrazol-5-yl)phenylamine (30e): the synthesis method of 30e was similar to that of 1f, except that 1e was replaced with 30d. Yield: 79%.

Step 5: Synthesis of 5-(2-(1-cyclobutyl-1H-pyrazol-4-yl)-5-isocyanatophenyl)-2-trityl-2H-tetrazole (30f): the synthesis method of 30f was similar to that of 1g, except that 1f was replaced with 30e, and the residue was directly used for the next step.

Step 6: Synthesis of 4-(tert-butyl)-N-(4-(1-cyclobutyl-1H-pyrazol-4-yl)-3-(2-trityl-2H-tetrazol-5-yl)phenyl)piperidine-1-carboxamide (30g): the synthesis method of 30g was similar to that of 1h, except that 1g was replaced with 30f, and 4-tert-butylcyclohexylamine was replaced with 4-tert-butylpiperidine hydrochloride. Yield through the two steps: 38%.

Step 7: Synthesis of 4-(tert-butyl)-N-(4-(1-cyclobutyl-1H-pyrazol-4-yl)-3-(2H-tetrazol-5-yl)phenyl)piperidine-1-carboxamide (9): the synthesis method of 9 was similar to that of 1, except that 1h was replaced with 30g. Yield: 39%.

ESI-MS: m/z = 449 [M+H]⁺. ¹H NMR (400 MHz, DMSO-*d*₆) δ 16.08 (s, 1H), δ 8.75 (s, 1H), 7.76 - 7.65 (m, 2H), 7.59 (s, 1H), 7.51 (d, *J* = 8.5 Hz, 1H), 6.98 (s, 1H), 4.82 - 4.68 (m, 1H), 4.28 - 4.15 (m, 2H), 2.75 - 2.64 (m, 2H), 2.43 - 2.28 (m, 4H), 1.81 - 1.71 (m, 2H), 1.70 - 1.63 (m, 2H), 1.20 - 1.16 (m, 1H), 1.13 - 1.04 (m, 2H), 0.85 (s, 9H).

### Preparation Example 10

### Synthesis of 4-(tert-butyl)-N-(4-(1-methyl-1H-indazol-5-yl)-3-(2H-tetrazol-5-yl)phenyl)piperidine-1-carboxamide (Compound 10):

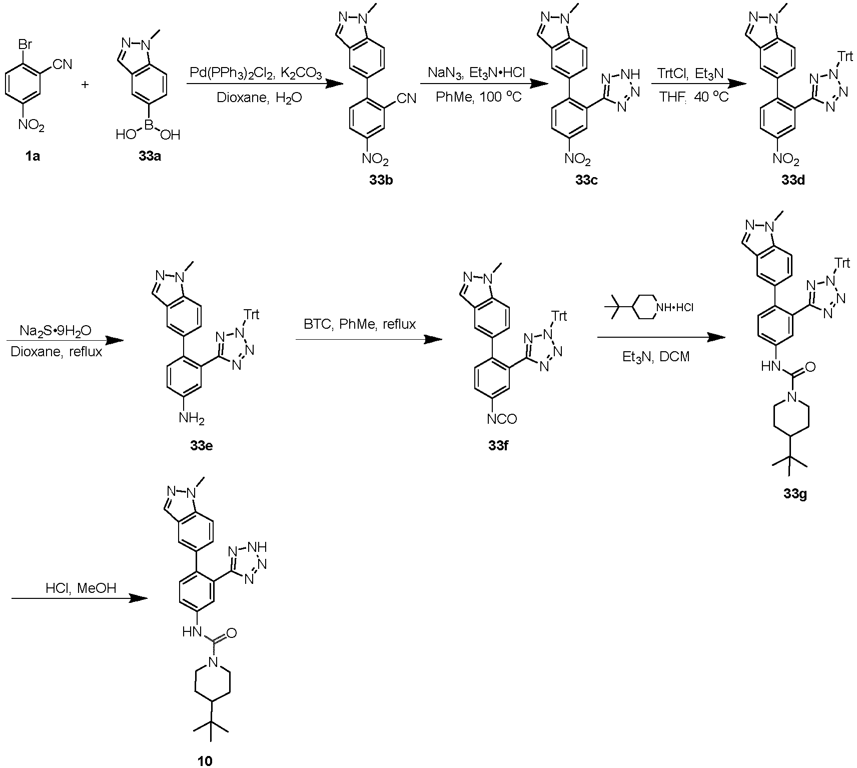

Step 1: Synthesis of 2-(1-methyl-1H-indazol-5-yl)-5-nitrobenzonitrile (33b): the synthesis method of 33b was similar to that of 27b, except that 27a was replaced with 27a, and 24a was replaced with 33a. Yield: 89%.

Step 2: Synthesis of 1-methyl-5-(4-nitro-2-(2H-tetrazol-5-yl)phenyl)-1H-indazole (33c): the synthesis method of 33c was similar to that of 1d, except that 1c was replaced with 33b. Yield: 91%.

Step 3: Synthesis of 1-methyl-5-(4-nitro-2-(2-trityl-2H-tetrazol-5-yl)phenyl)-1H-indazole (33d): the synthesis method of 33d was similar to that of 1e, except that 1d was replaced with 33c. Yield: 87%.

Step 4: Synthesis of 4-(1-methyl-1H-indazol-5-yl)-3-(2-trityl-2H-tetrazol-5-yl)phenylamine (33e): the synthesis method of 33e was similar to that of 1f, except that 1e was replaced with 33d. Yield: 89%.

Step 5: Synthesis of 5-(4-isocyanato-2-(2-trityl-2H-tetrazol-5-yl)phenyl)-1-methyl-1H-indazole (33f): the synthesis method of 33f was similar to that of 1g, except that 1f was replaced with 33e, and the residue was directly used for the next step.

Step 6: Synthesis of 4-(tert-butyl)-N-(4-(1-methyl-1H-indazol-5-yl)-3-(2-trityl-2H-tetrazol-5-yl)phenyl)piperidine-1-carboxamide (33g): the synthesis method of 33g was similar to that of 1h, except that 1g was replaced with 33f, and 4-tert-butylcyclohexylamine was replaced with 4-tert-butylpiperidine hydrochloride. Yield through the two steps: 40%.

Step 7: Synthesis of 4-(tert-butyl)-N-(4-(1-methyl-1H-indazol-5-yl)-3-(2H-tetrazol-5-yl)phenyl)piperidine-1-carboxamide (10): the synthesis method of 10 was similar to that of 1, except that 1h was replaced with 33g. Yield: 63%.

ESI-MS: m/z = 459 [M+H]⁺. ¹H NMR (600 MHz, DMSO-*d*₆) δ 16.02 (s, 1H), 8.80 (s, 1H), 8.01 (s, 1H), 7.85 (d, *J=* 2.3 Hz, 1H), 7.79 (dd, *J=* 8.5, 2.3 Hz, 1H), 7.52 - 7.46 (m, 3H), 6.97 (dd, *J =* 8.5, 1.7 Hz, 1H), 4.28 - 4.22 (m, 2H), 4.03 (s, 3H), 2.76 - 2.68 (m, 2H), 1.72 - 1.66 (m, 2H), 1.25 - 1.19 (m, 1H), 1.17 - 1.08 (m, 2H), 0.86 (s, 9H).

### Preparation Example 11

### Synthesis of 4-(tert-butyl)-N-(2-fluoro-3',4'-dimethoxy-6-(2H-tetrazol-5-yl)-[1,1'-biphenyl]-4-yl)piperidine-1-carboxamide (Compound 11):

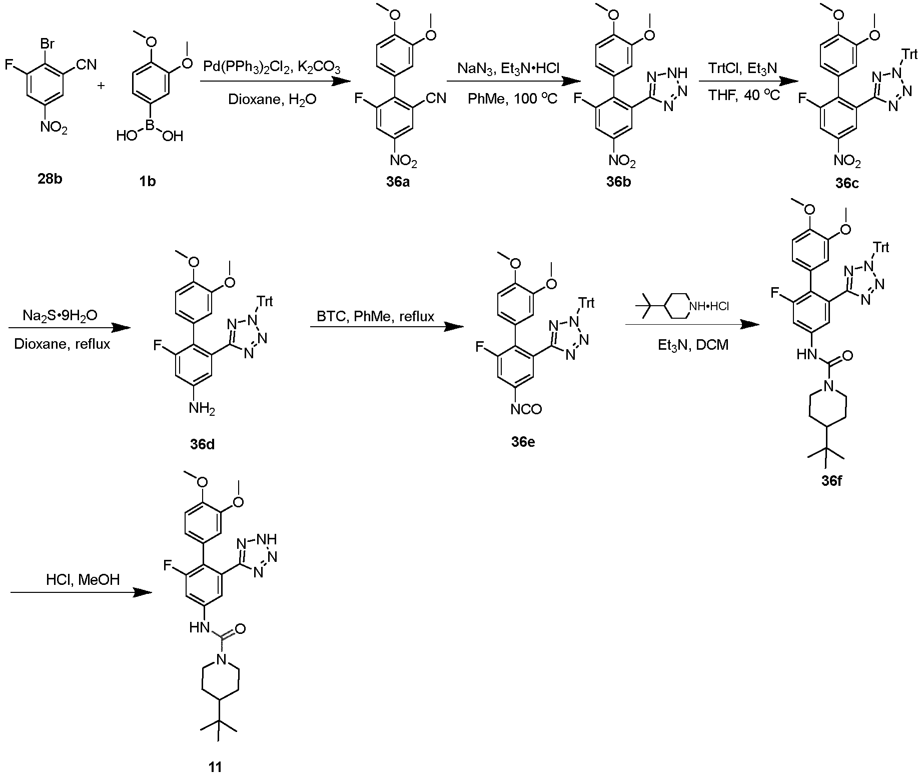

Step 1: Synthesis of 6-fluoro-3',4'-dimethoxy-4-nitro-[1,1'-biphenyl]-2-carbonitrile (36a): the synthesis method of 36a was similar to that of 27b, except that 27a was replaced with 28b, and 27a was replaced with 1b. Yield: 92%.

Step 2: Synthesis of 5-(6-fluoro-3',4'-dimethoxy-4-nitro-[1,1'-biphenyl] -2-yl)-2H-tetrazole (36b): the synthesis method of 36b was similar to that of 1d, except that 1c was replaced with 36a. Yield: 89%.

Step 3: Synthesis of 5-(6-fluoro-3',4'-dimethoxy-4-nitro-[1,1'-biphenyl] -2-yl)-2-trityl-2H-tetrazole (36c): the synthesis method of 36c was similar to that of 1e, except that 1d was replaced with 36b. Yield: 94%.

Step 4: Synthesis of 2-fluoro-3',4'-dimethoxy-6-(2-trityl-2H-tetrazol-5-yl)-[1,1'-biphenyl]-4-amine (36d): the synthesis method of 36d was similar to that of 1f, except that 1e was replaced with 36c. Yield: 89%.

Step 5: Synthesis of 5-(6-fluoro-4-isocyanato-3',4'-dimethoxy-[1,1'-biphenyl]-2-yl)-2-trityl-2H-tetrazole (36e): the synthesis method of 36e was similar to that of 1g, except that 1f was replaced with 36d, and the residue was directly used for the next step.

Step 6: Synthesis of 4-(tert-butyl)-N-(2-fluoro-3',4'-dimethoxy-6-(2-trityl-2H-tetrazol-5-yl)-[1,1'-biphenyl]-4-yl)piperidine-1-carboxamide (36f): the synthesis method of 36f was similar to that of 1h, except that 1g was replaced with 34e, and 4-tert-butylcyclohexylamine was replaced with 4-tert-butylpiperidine hydrochloride. Yield through the two steps: 41%.

Step 7: Synthesis of 4-(tert-butyl)-N-(2-fluoro-3',4'-dimethoxy-6-(2H-tetrazol-5-yl)-[1,1'-biphenyl]-4-yl)piperidine-1-carboxamide (11): the synthesis method of 11 was similar to that of 1, except that 1h was replaced with 36f. Yield: 62%.

ESI-MS: m/z = 483 [M+H]⁺. ¹H NMR (400 MHz, DMSO-*d*₆) δ 16.13 (s, 1H), δ 8.99 (s, 1H), 7.76 (dd, *J* = 12.8, 2.2 Hz, 1H), 7.62 (s, 1H), 6.94 - 6.82 (m, 1H), 6.68 - 6.52 (m, 2H), 4.23 (d, *J =* 13.0 Hz, 2H), 3.74 (s, 3H), 3.59 (s, 3H), 2.85 - 2.64 (m, 2H), 1.69 (d, *J =* 12.2 Hz, 2H), 1.23 - 1.17 (m, 1H), 1.17 - 1.08 (m, 2H), 0.86 (s, 9H).

### Preparation Example 12

### Synthesis of N-(3',4'-dimethoxy-2-(2H-tetrazol-5-yl)-[1,1'-biphenyl]-4-yl)-4,4-dimethylpiperidine-1-carboxamide (Compound 12):

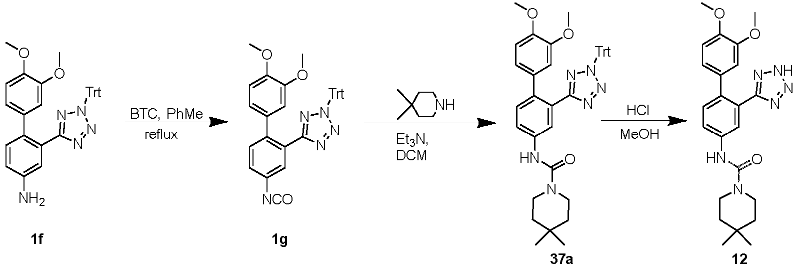

Step 1: Synthesis of N-(3',4'-dimethoxy-2-(2-trityl-2H-tetrazol-5-yl)-[1,1'-biphenyl]-4-yl)-4,4-dimethylpiperidine-1-carboxamide (37a): the synthesis was performed according to Step 6 of Example 1, except that 1f was replaced with 1g, 4-tert-butylcyclohexylamine was replaced with dimethylpyridine. Yield through the two steps: 40%.

Step 2: Synthesis of N-(3',4'-dimethoxy-2-(2H-tetrazol-5-yl)-[1,1'-biphenyl]-4-yl)-4,4-dimethylpiperidine-1-carboxamide (12): the synthesis was performed according to Step 7 of Example 1, except that 1h was replaced with 37a. Yield: 72%.

ESI-MS: m/z = 437 [M+H]⁺. ¹H NMR (600 MHz, DMSO-*d*₆) δ 16.05 (s, 1H), 8.74 (s, 1H), 7.77 (d, *J* = 8.1 Hz, 2H), 7.46 (d, *J* = 8.3 Hz, 1H), 6.87 *(d, J=* 8.2 Hz, 1H), 6.60 - 6.55 (m, 2H), 3.74 (s, 3H), 3.59 (s, 3H), 3.48 - 3.44 (m, 4H), 1.34 - 1.29 (m, 4H), 0.97 (s, 6H).

### Preparation Example 13

### Synthesis of 4-(tert-butyl)-N-(4'-methoxy-3'-methyl-2-(2H-tetrazol-5-yl)-[1,1'-biphenyl]-4-yl)piperidine-1-carboxamide (Compound 13):

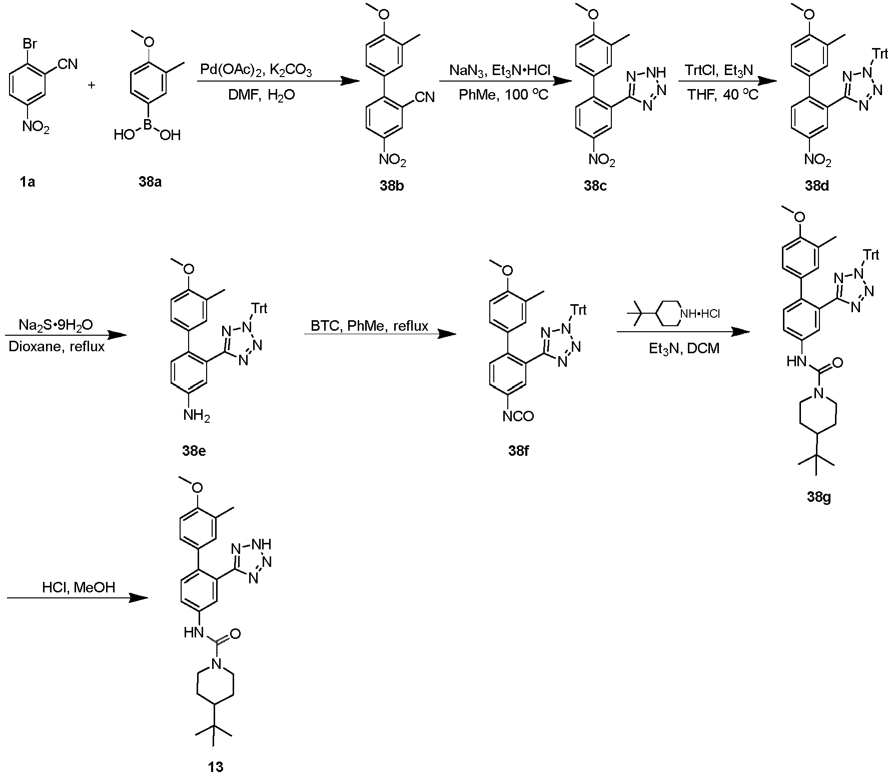

Step 1: Synthesis of 4'-methoxy-3'-methyl-4-nitro-[1,1'-biphenyl]-2-carbonitrile (38b): 2.00 g (8.81 mmol) of 1a, 1.75 g (10.57 mmol) of 38a, 20 mg (0.088 mmol) of Pd(OAc)₂, 2.43 g (17.62 mmol) of potassium carbonate in 12 mL aqueous solution and 60 mL of DMF were added to a reaction flask. The reaction was carried out at the room temperature for 2 h under nitrogen protection. After the reaction was completed, the mixture was diluted with EA, and then washed with saturated brine. The organic phase was dried over anhydrous sodium sulfate, filtered, concentrated. The residue was purified by silica gel column chromatography (PE:EA=3:1, v/v) to obtain 1.89 g of 38b. Yield: 80%.

Step 2: Synthesis of 5-(4'-methoxy-3'-methyl-4-nitro-[1,1'-biphenyl]-2-yl)-2H-tetrazole (38c): the synthesis method of 38c was similar to that of 1d, except that 1c was replaced with 38b. Yield: 87%.

Step 3: Synthesis of 5-(4'-methoxy-3'-methyl-4-nitro-[1,1'-biphenyl]-2-yl)-2-trityl-2H-tetrazole (38d): the synthesis method of 38d was similar to that of 1e, except that 1c was replaced with 38c. Yield: 84%.

Step 4: Synthesis of 4'-methoxy-3'-methyl-2-(2-trityl-2H-tetrazol-5-yl)-[1,1'-biphenyl]-4-amine (38e): the synthesis method of 38e was similar to that of 1f, except that 1e was replaced with 38d. Yield: 87%.

Step 5: Synthesis of 5-(4-isocyanato-4'-methoxy-3'-methyl-[1,1'-biphenyl]-2-yl)-2-trityl-2H-tetrazole (38f): the synthesis method of 38f was similar to that of 1g, except that 1f was replaced with 38e, and the residue was directly used for the next step.

Step 6: Synthesis of 4-(tert-butyl)-N-(4'-methoxy-3'-methyl-2-(2-trityl-2H-tetrazol-5-yl)-[1,1'-biphenyl]-4-yl)piperidine-1-carboxamide (38g): the synthesis method of 38g was similar to that of 1h, except that 1g was replaced with 36f, and 4-tert-butylcyclohexylamine was replaced with 4-tert-butylpiperidine hydrochloride. Yield through the two steps: 45%.

Step 7: Synthesis of 4-(tert-butyl)-N-(4'-methoxy-3'-methyl-2-(2H-tetrazol-5-yl)-[1,1'-biphenyl]-4-yl)piperidine-1-carboxamide (13): the synthesis method of 13 was similar to that of 1, except that 1h was replaced with 38g. Yield: 55%.

ESI-MS: m/z = 449 [M+H]⁺. ¹H NMR (600 MHz, DMSO-*d*₆) δ 16.11 (s, 1H), 8.76 (s, 1H), 7.79 - 7.73 (m, 2H), 7.39 (d, *J* = 8.4 Hz, 1H), 6.90 (d, *J =* 2.3 Hz, 1H), 6.82 (d, *J =* 8.5 Hz, 1H), 6.75 (dd, *J =* 8.4, 2.4 Hz, 1H), 4.28 - 4.20 (m, 2H), 3.76 (s, 3H), 2.74 - 2.67 (m, 2H), 2.08 (s, 3H), 1.72 - 1.64 (m, 2H), 1.25 - 1.18 (m, 1H), 1.16 - 1.06 (m, 2H), 0.86 (s, 9H).

### Preparation Example 14

### Synthesis of 4-(tert-butyl)-N-(3'-chloro-4'-methoxy-2-(2H-tetrazol-5-yl)-[1,1'-biphenyl]-4-yl)piperidine-1-carboxamide (Compound 14):

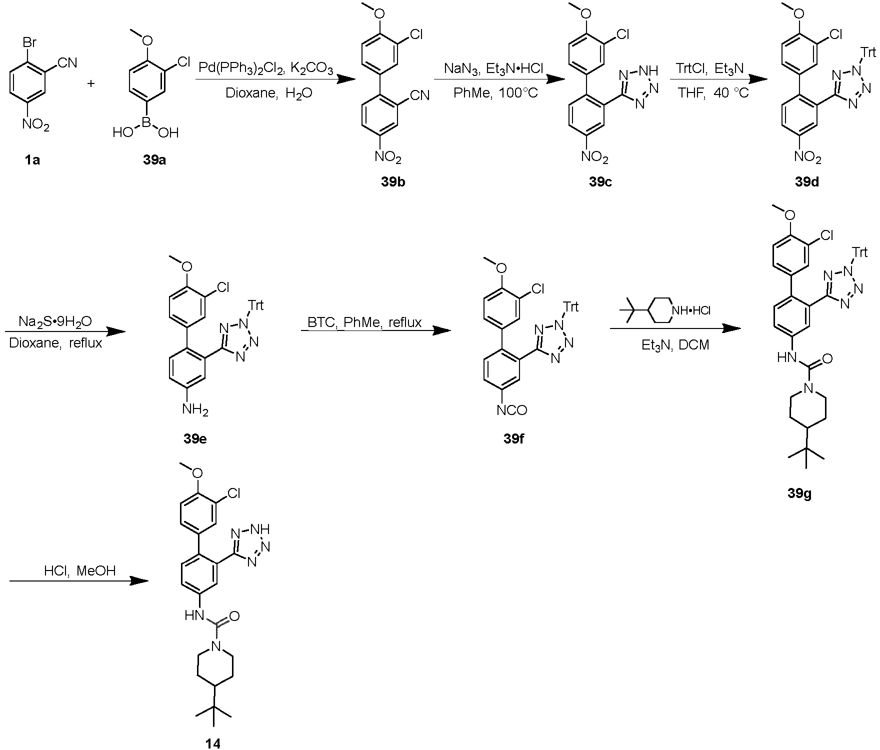

Step 1: Synthesis of 3'-chloro-4'-methoxy-4-nitro-[1,1'-biphenyl]-2-carbonitrile (39b): the synthesis was performed according to Step 2 of Example 1, except that 27a was replaced with 1a, and 24a was replaced with 29a. Yield: 73%.

Step 2: Synthesis of 5-(3'-chloro-4'-methoxy-4-nitro-[1,1'-biphenyl]-2-yl)-2H-tetrazole (39c): the synthesis was performed according to Step 2 of Example 1, except that 1c was replaced with 39b. Yield: 90%.

Step 3: Synthesis of 5-(3'-chloro-4'-methoxy-4-nitro-[1,1'-biphenyl]-2-yl)-2-trityl-2H-tetrazole (39d): the synthesis was performed according to Step 3 of Example 1, except that 1d was replaced with 39c. Yield: 87%.

Step 4: Synthesis of 3'-chloro-4'-methoxy-2-(2-trityl-2H-tetrazol-5-yl)-[1,1'-biphenyl]-4-amine (39e): the synthesis was performed according to Step 4 of Example 1, except that 1e was replaced with 39d. Yield: 71%.

Step 5: Synthesis of 5-(3'-chloro-4-isocyanato-4'-methoxy-[1,1'-biphenyl]-2-yl)-2-trityl-2H-tetrazole (39f): the synthesis was performed according to Step 5 of Example 1, except that 1f was replaced with 39e, and the residue was directly used for the next step.

Step 6: Synthesis of 4-(tert-butyl)-N-(3'-chloro-4'-methoxy-2-(2-trityl-2H-tetrazol-5-yl)-[1,1'-biphenyl]-4-yl)piperidine-1-carboxamide (39g): the synthesis was performed according to Step 6 of Example 1, except that 1g was replaced with 39f, and 4-tert-butylcyclohexylamine was replaced with 4-tert-butylpiperidine hydrochloride. Yield through the two steps: 52%.

Step 7: Synthesis of 4-(tert-butyl)-N-(3'-chloro-4'-methoxy-2-(2H-tetrazol-5-yl)-[1,1'-biphenyl]-4-yl)piperidine-1-carboxamide (14): the synthesis was performed according to Step 7 of Example 1, except that 1h was replaced with 39g. Yield: 63%.

ESI-MS: m/z = 470 [M+H]⁺. ¹H NMR (600 MHz, DMSO-*d*₆) δ 16.01 (s, 1H), δ 8.80 (s, 1H), 7.83 (d, *J=* 2.3 Hz, 1H), 7.77 (dd, *J* = 8.6, 2.3 Hz, 1H), 7.43 (d, *J* = 8.5 Hz, 1H), 7.15 (d, *J = 2.2* Hz, 1H), 7.05 (d, *J* = 8.6 Hz, 1H), 6.89 (dd, *J=* 8.5, 2.3 Hz, 1H), 4.27 - 4.20 (m, 2H), 3.84 (s, 3H), 2.75 - 2.67 (m, 2H), 1.71 - 1.65 (m, 2H), 1.25 - 1.18 (m, 1H), 1.17 - 1.07 (m, 2H), 0.86 (s, 9H).

### Preparation Example 15

### Synthesis of cyclohexyl 4-(4-(tert-butyl)piperidine-1-formamido)-2-(2H-tetrazol-5-yl)benzoate (Compound 15):

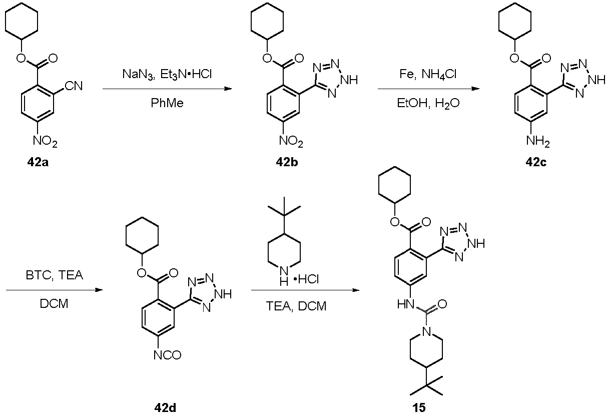

Step 1: Synthesis of cyclohexyl 4-nitro-2-(2H-tetrazol-5-yl)benzoate (42b): the synthesis was performed according to Step 2 of Example 1, except that 1c was replaced with 42a. Yield: 86%.

Step 2: Synthesis of cyclohexyl 4-amino-2-(2H-tetrazol-5-yl)benzoate (42c): 482 mg (1.52 mmol) of 42b, 593 mg (10.64 mmol) of iron powder and 244 mg (4.56 mmol) of ammonium chloride, 9 mL of ethanol and 3 mL of water were added to a reaction flask. The reaction was carried out under reflux for 0.5 h. After the reaction was completed, the mixture was filtered while hot. The filter cake was washed with ethyl acetate. The filtrate was combined, diluted with water, and extracted with ethyl acetate. The organic layers were combined, washed with saturated brine, dried over anhydrous sodium sulfate, filtered, and concentrated under reduced pressure. The residue was purified by silica gel column chromatography to obtain 42c. Yield: 81%.

Step 3: Synthesis of cyclohexyl 4-isocyanato-2-(2H-tetrazol-5-yl)benzoate (42d): 368 mg (1.28 mmol) of 42c and 5 mL of anhydrous dichloromethane were added to a reaction flask. The mixture was cooled to 0°C. Then, a solution of 191 mg (0.64 mmol) of triphosgene in 3 mL of anhydrous dichloromethane was slowly added dropwise, and then 143 mg (1.41 mmol) of triethylamine was slowly added. Then the mixture was warmed to the room temperature, and the reaction was carried out for 1 h. After the reaction was completed, the mixture was concentrated under reduced pressure to obtain 42d, which was directly used for the next step.

Step 4: Synthesis of cyclohexyl 4-(4-(tert-butyl)piperidine-1-formamido)-2-(2H-tetrazol-5-yl)benzoate (15): 228 mg (1.28 mmol) of 4-(tert-butyl)piperidine hydrochloride, 10 mL of anhydrous dichloromethane and 260 mg (2.57 mmol) of triethylamine were added to the reaction flask. The mixture was cooled to 0°C. A solution of 42d in 10 mL of anhydrous dichloromethane was slowly added dropwise. After the addition was completed, the mixture was warmed to the room temperature, and the reaction was carried out for 1 h. After the reaction was completed, the reaction solution was concentrated under reduced pressure and purified by silica gel column chromatography (DCM:MeOH:TEA = 30:2:1, v/v) to obtain 226 mg of 15. Yield through the two steps: 39%.

ESI-MS: m/z = 401 [M+H]⁺. ¹H NMR (500 MHz, DMSO-*d*₆) δ 16.49 (s, 1H), 9.09 (s, 1H), 7.95 (d, *J* = 8.7 Hz, 1H), 7.85 (dd, *J* = 8.7, 2.3 Hz, 1H), 7.80 (d, *J* = 2.2 Hz, 1H), 4.23 (d, *J* = 13.1 Hz, 2H), 4.09 (q, *J=* 7.1 Hz, 2H), 2.72 (t, *J* = 12.1 Hz, 2H), 1.68 (d, *J=* 11.4 Hz, 2H), 1.26 - 1.17 (m, 1H), 1.15 - 1.08 (m, 2H), 1.06 (t, *J* = 7.1 Hz, 3H), 0.85 (s, 9H).

### Preparation Example 16

### Synthesis of ethyl 4-(4-(tert-butyl)piperidine-1-formamido)-2-(2H-tetrazol-5-yl)benzoate (Compound 16):

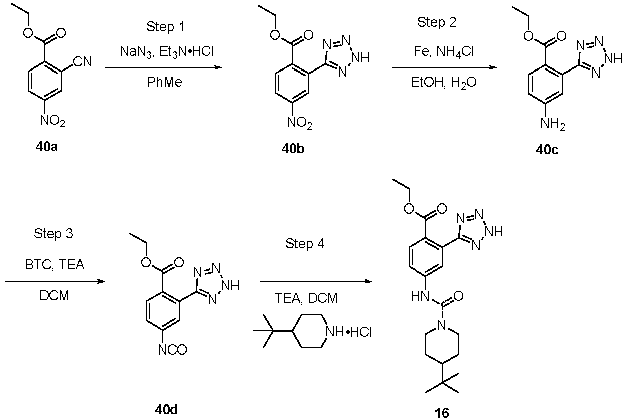

Step 1: Synthesis of ethyl 4-nitro-2-(2H-tetrazol-5-yl)benzoate (40b): the synthesis was performed according to Step 2 of Example 1, except that 1c was replaced with 40a. Yield: 92%.

Step 2: Synthesis of ethyl 4-amino-2-(2H-tetrazol-5-yl)benzoate (40c): 400 mg (1.52 mmol) of 40b, 593 mg (10.64 mmol) of iron powder and 244 mg (4.56 mmol) of ammonium chloride, 9 mL of ethanol and 3 mL of water were added to a reaction flask. The reaction was carried out under reflux for 0.5 h. After the reaction was completed, the mixture was filtered while hot. The filter cake was washed with ethyl acetate. The filtrate was combined, diluted with water, and extracted with ethyl acetate. The organic layer were combined, washed with saturated brine, dried over anhydrous sodium sulfate, filtered, and concentrated under reduced pressure. The residue was purified by silica gel column chromatography to obtain 40c. Yield: 85%.

Step 3: Synthesis of ethyl 4-isocyanato-2-(2H-tetrazol-5-yl)benzoate (40d): 300 mg (1.28 mmol) of 40c and 5 mL of anhydrous dichloromethane were added to a reaction flask. The mixture was cooled to 0°C. Then, a solution of 191 mg (0.64 mmol) of triphosgene in 3 mL of anhydrous dichloromethane was slowly added dropwise, and then 143 mg (1.41 mmol) of triethylamine was slowly added. Then the mixture was warmed to the room temperature, and the reaction was carried out for 1 h. After the reaction was completed, the reaction mixture was concentrated under reduced pressure to obtain 40d, which was directly used for the next step.

Step 4: Synthesis of ethyl 4-(4-(tert-butyl)piperidine-1-formamido)-2-(2H-tetrazol-5-yl)benzoate (17): 228 mg (1.28 mmol) of 4-(tert-butyl)piperidine hydrochloride, 10 mL of anhydrous dichloromethane and 260 mg (2.57 mmol) of triethylamine were added to a reaction flas. The mixture was cooled to 0°C, and then a solution of 40d in 10 mL of anhydrous dichloromethane was slowly added dropwise. After the addition was completed, the mixture was warmed to the room temperature, and the reaction was carried out for 1 h. After the reaction was completed, the reaction solution was concentrated under reduced pressure and purified by silica gel column chromatography (DCM:MeOH:TEA = 30:2:1, v/v) to obtain 170 mg of 16. Yield through the two steps: 33%.

ESI-MS: m/z = 401 [M+H]⁺. ¹H NMR (500 MHz, DMSO-*d*₆) δ 16.49 (s, 1H), 9.09 (s, 1H), 7.95 (d, *J* = 8.7 Hz, 1H), 7.85 (dd, *J* = 8.7, 2.3 Hz, 1H), 7.80 (d, *J* = 2.2 Hz, 1H), 4.23 (d, *J* = 13.1 Hz, 2H), 4.09 (q, *J=* 7.1 Hz, 2H), 2.72 (t, *J* = 12.1 Hz, 2H), 1.68 (d, *J=* 11.4 Hz, 2H), 1.26 - 1.17 (m, 1H), 1.15 - 1.08 (m, 2H), 1.06 (t, *J* = 7.1 Hz, 3H), 0.85 (s, 9H).

### Preparation Example 17

### Synthesis of cyclohexyl 4-(4-(tert-butyl)piperidine-1-formamido)-2-fluoro-6-(2H-tetrazol-5-yl)benzoate (Compound 17):

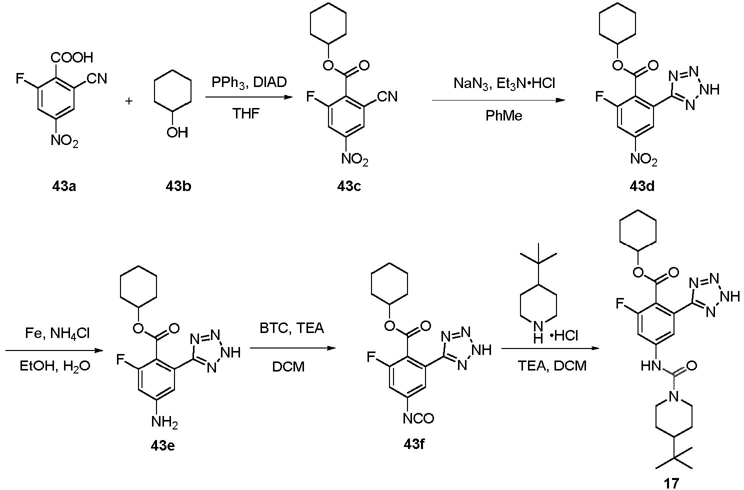

Step 1: Synthesis of cyclohexyl 2-cyano-6-fluoro-4-nitrobenzoate (43c): 1.00 g (10.00 mmol) of cyclohexanol, 2.52 g (12.00 mmol) of 2-cyano-6-fluoro-4-nitrobenzoic acid and 3.15 g (12.00 mmol) of triphenylphosphine were dissolved in 20 mL of anhydrous tetrahydrofuran. The solution was cooled to 0°C, and then 2.43 g (12.00 mmol) of diisopropyl azodicarboxylate was added. After the addition was completed, the mixture was warmed to the room temperature, and the reaction was carried out for 5 h. After the reaction was completed, the mixture was concentrated under reduced pressure, and the residue was purified by silica gel column chromatography (PE:EA=2:1, v/v) to obtain 2.42 g of 43c. Yield: 83%.

Step 2: Synthesis of cyclohexyl 2-fluoro-4-nitro-6-(2H-tetrazol-5-yl)benzoate (43d): the synthesis was performed according to Step 1 of Example 16, except that 1c was replaced with 43c. Yield: 85%.

Step 3: Synthesis of cyclohexyl 4-amino-2-fluoro-6-(2H-tetrazol-5-yl)benzoate (43e): the synthesis was performed according to Step 2 of Example 16, except that 40b was replaced with 43d. Yield: 79%.

Step 4: Synthesis of cyclohexyl 2-fluoro-4-isocyanato-6-(2H-tetrazol-5-yl)benzoate (43f): the synthesis was performed according to Step 3 of Example 16, except that 40c was replaced with 43e.

Step 5: Synthesis of cyclohexyl 4-(4-(tert-butyl)piperidine-1-formamido)-2-fluoro-6-(2H-tetrazol-5-yl)benzoate(17): the synthesis was performed according to Step 4 of Example 16, except that 40d was replaced with 43f. Yield through the two steps: 36%.

ESI-MS: m/z = 474 [M+H]⁺. ¹H NMR (500 MHz, DMSO-*d*₆) δ 16.31 (s, 1H), 8.61 (s, 1H), 8.10 (d, *J* = 1.6 Hz, 1H), 7.51 (dd, *J* = 8.1, 1.5 Hz, 1H), 4.73 - 4.61 (m, 1H), 4.25 *(d, J =* 13.1 Hz, 2H), 2.75 *(t, J =* 12.5 Hz, 2H), 1.74 (d, *J =* 12.2 Hz, 4H), 1.63 - 1.53 (m, 2H), 1.49 - 1.38 (m, 1H), 1.34 - 1.26 (m, 2H), 1.24 - 1.09 (m, 6H), 0.86 (s, 9H).

### Preparation Example 18

### Synthesis of 4-(tert-butyl)-N-(3-fluoro-5-(2H-tetrazol-5-yl)-4-(6-(trifluoromethyl)pyrid-3-yl)phenyl)piperidine-1-carboxamide (Compound 18):

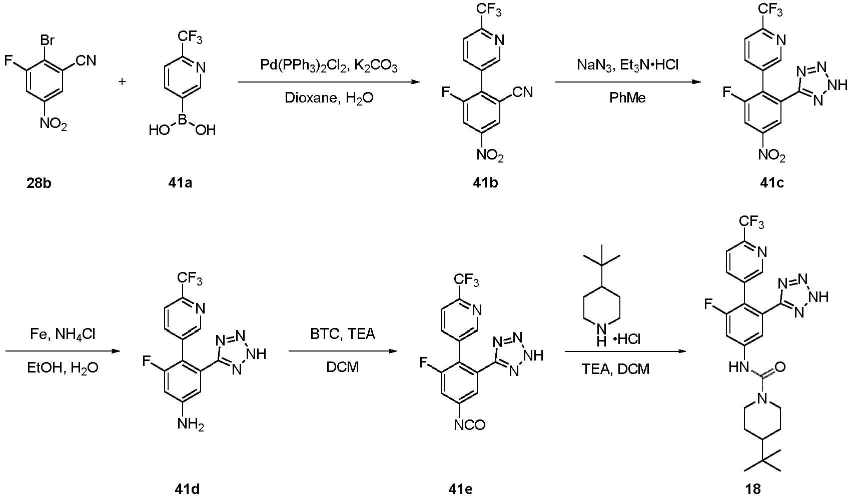

Step 1: Synthesis of 3-fluoro-5-nitro-2-(6-(trifluoromethyl)pyrid-3-yl)benzonitrile (41b): 900 mg (3.67 mmol) of 28b, 840 mg (5.39 mmol) of 2-trifluoromethyl-5-pyridineboronic acid, 129 mg (0.22 mmol) of Pd(PPh₃)₂Cl₂ and 1.52 g (13.04 mmol) of potassium carbonate, 16 mL of 1,4-dioxane and 8 mL of water were added to a reaction flask. The reaction was carried out under reflux for 2 h under nitrogen protection. After the reaction was completed, the mixture was cooled to the room temperature, diluted with water, extracted with ethyl acetate. The organic layers were combined, washed with saturated brine, dried over anhydrous sodium sulfate, filtered, and concentrated under reduced pressure. The residue was purified by silica gel column chromatography (PE:EA = 5:1, v/v) to obtain 650 mg of 41b. Yield: 57%.

Step 2: Synthesis of 5-(2-fluoro-4-nitro-6-(2H-tetrazol-5-yl)phenyl)-2-(trifluoromethyl)pyridine (41c): the synthesis was performed according to Step 1 of Example 16, except that 1c was replaced with 41b. Yield: 85%.

Step 3: Synthesis of 3-fluoro-5-(2H-tetrazol-5-yl)-4-(6-(trifluoromethyl)pyrid-3-yl)phenylamine (41d): the synthesis was performed according to Step 2 of Example 16, except that 40b was replaced with 41c. Yield: 90%.

Step 4: Synthesis of 5-(2-fluoro-4-isocyanato-6-(2H-tetrazol-5-yl)phenyl)-2-(trifluoromethyl)pyridine (41e): the synthesis was performed according to Step 3 of Example 16, except that 40c was replaced with 41d, and the residue was directly used for the next step.

Step 5: Synthesis of 4-(tert-butyl)-N-(3-fluoro-5-(2H-tetrazol-5-yl)-4-(6-(trifluoromethyl)pyrid-3-yl)phenyl)piperidine-1-carboxamide (18): the synthesis was performed according to Step 4 of Example 16, except that 40d was replaced with 41e. Yield through the two steps: 35%.

ESI-MS: m/z = 492 [M+H]⁺.¹H NMR (500 MHz, DMSO-*d*₆) δ 16.83 (s, 1H), 9.13 (s, 1H), 8.55 (s, 1H), 7.93 - 7.85 (m, 3H), 7.83 (dd, *J =* 12.8, 2.1 Hz, 1H), 4.25 (d, *J =* 13.1 Hz, 2H), 2.75 (t, *J =* 12.1 Hz, 2H), 1.71 (d, *J =* 11.8 Hz, 2H), 1.28 - 1.18 (m, 1H), 1.18 - 1.07 (m, 2H), 0.86 (s, 9H).

### Preparation Example 19

### Synthesis of N-(3',4'-dimethoxy-2-(2H-tetrazol-5-yl)-[1,1'-biphenyl]-4-yl)-4-methylpiperidine-1-sulfamide (Compound 19):

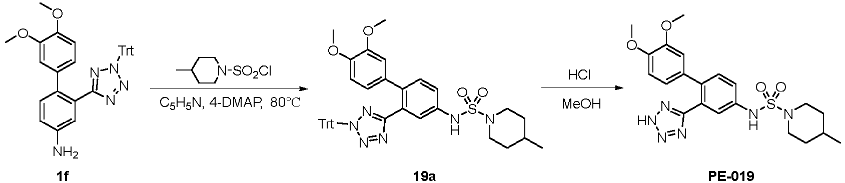

Step 1: Synthesis of N-(3',4'-dimethoxy-2-(2-trityl-2H-tetrazol-5-yl)-[1,1'-biphenyl]-4-yl)-4-methylpiperidine-1-sulfamide (19a): 53 mg (0.1 mmol) of 1f, 12 mg (0.1 mmol) of 4-DMAP and 2 mL of pyridine were added to a reaction flask, and then 20 mg (0.1 mmol) of 4-methylpiperidine-1-sulfonyl chloride was added. After the addition was completed, the mixture was warmed to 80 °C, and the reaction was carried out for 10 h. After the reaction was completed, the mixture was diluted with DCM, washed with dilute hydrochloric acid. The organic phase was dried over anhydrous sodium sulfate, filtered, and concentrated under reduced pressure. The residue was purified by silica gel column chromatography (PE: EA = 2: 1, v/v) to obtain 55 mg of 19a. Yield: 78%.

Step 2: Synthesis of N-(3',4'-dimethoxy-2-(2H-tetrazol-5-yl)-[1,1'-biphenyl]-4-yl)-4-methylpiperidine-1-sulfamide (19): the synthesis was performed according to Step 7 of Example 1, except that 1h was replaced with 19a. Yield: 55%.

ESI-MS: m/z =459[M+H]⁺.¹H NMR (500 MHz, Chloroform-*d*) δ 11.43 (s, 1H), 7.98 (d, *J* = 2.4 Hz, 1H), 7.49 (dd, *J* = 8.4, 2.5 Hz, 1H), 7.40 (d, *J* = 8.4 Hz, 1H), 6.96 (d, *J* = 8.2 Hz, 1H), 6.89 (s, 1H), 6.83 (dd, *J =* 8.2, 2.1 Hz, 1H), 6.71 (d, *J =* 2.1 Hz, 1H), 3.95 (s, 3H), 3.85 (d, *J =* 12.2 Hz, 2H), 3.80 (s, 3H), 2.92 - 2.80 (m, 2H), 1.77 - 1.68 (m, 2H), 1.54 - 1.46 (m, 1H), 1.28 - 1.25 (m, 2H), 0.95 (d, *J=* 6.5 Hz, 3H).

### Preparation Example 20

### Synthesis of N-(2-fluoro-3',4'-dimethoxy-6-(2H-tetrazol-5-yl)-[1,1'-biphenyl]-4-yl)-4-methylpiperidine-1-carboxamide (Compound 20):

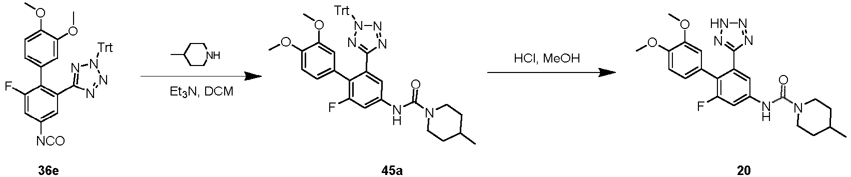

Step 1: Synthesis of N-(2-fluoro-3',4'-dimethoxy-6-(2-triphenyl-2H-tetrazol-5-yl)-[1,1'-biphenyl]-4-yl)-4-methylpiperidine-1-carboxamide (45a): the synthesis was performed according to Step 6 of Example 1, except that 1g was replaced with 36e, and 4-tert-butylcyclohexylamine was replaced with 4-methylpiperidine. Yield: 45%.

Step 2: Synthesis of N-(2-fluoro-3',4'-dimethoxy-6-(2H-tetrazol-5-yl)-[1,1'-biphenyl]-4-yl)-4-methylpiperidine-1-carboxamide (20): the synthesis was performed according to Step 7 of Example 1, except that 1h was replaced with 45a. Yield: 60%.

ESI-MS: m/z = 441 [M+H]⁺.¹H NMR (500 MHz, DMSO-*d*₆) δ 16.16 (s, 1H), 8.77 (s, 1H), 8.14 (d, *J* = 1.6 Hz, 1H), 7.71 (dd, *J* = 8.0, 1.4 Hz, 1H), 7.43 (dt, *J* = 7.5, 1.7 Hz, 1H), 7.16 (t, *J* = 1.8 Hz, 1H), 6.99 (d, *J* = 7.5 Hz, 1H), 4.24 - 4.18 (m, 2H), 3.71 (s, 3H), 3.57 (s, 3H), 2.75 - 2.63 (m, 2H), 1.68 (d, *J* = 12.3 Hz, 2H), 1.26 - 1.17 (m, 1H), 1.17 - 1.05 (m, 2H), 0.93 *(d, J=* 6.5 Hz, 3H).

### Preparation Example 21

### Synthesis of 4-methyl-N-(4-(1-methyl-1H-indazol-5-yl)-3-(2H-tetrazol-5-yl)phenyl)piperidine-1-carboxamide (Compound 21):

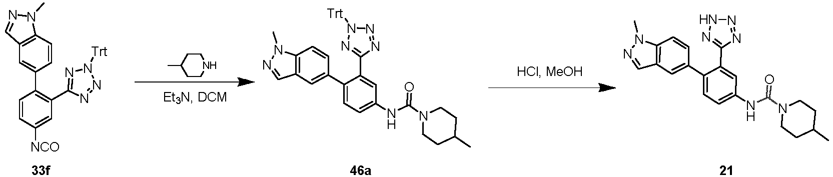

Step 1: Synthesis of 4-methyl-N-(4-(1-methyl-1H-indazol-5-yl)-3-(2-trityl-2H-tetrazol-5-yl)phenyl)piperidine-1-carboxamide (46a): the synthesis was performed according to Step 6 of Example 1, except that 1g was replaced with 33f, and 4-tert-butylcyclohexylamine was replaced with 4-methylpiperidine. Yield: 43%.

Step 2: Synthesis of 4-methyl-N-(4-(1-methyl-1H-indazol-5-yl)-3-(2H-tetrazol-5-yl)phenyl)piperidine-1-carboxamide (21): the synthesis was performed according to Step 7 of Example 1, except that 1h was replaced with 46a. Yield: 58%.

ESI-MS: m/z = 417 [M+H]⁺. ¹H NMR (600 MHz, DMSO-*d*₆) δ 16.02 (s, 1H), 8.80 (s, 1H), 8.01 (s, 1H), 7.85 (d, *J =* 2.3 Hz, 1H), 7.79 (dd, *J =* 8.5, 2.3 Hz, 1H), 7.52 - 7.46 (m, 3H), 6.97 (dd, *J =* 8.5, 1.7 Hz, 1H), 4.28 - 4.22 (m, 2H), 4.03 (s, 3H), 2.76 - 2.68 (m, 2H), 1.70 - 1.64 (m, 2H), 1.23 - 1.17 (m, 1H), 1.15 - 1.06 (m, 2H), 0.94 (d, *J =* 6.5 Hz, 3H).

### Preparation Example 22

### Synthesis of N-(3'-chloro-4'-methoxy-2-(2H-tetrazol-5-yl)-[1,1'-biphenyl]-4-yl)-4-methylpiperidine-1-carboxamide (Compound 22):

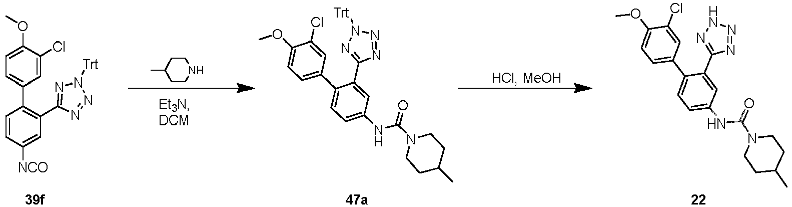

Step 1: Synthesis of N-(3'-chloro-4'-methoxy-2-(2-trityl-2H-tetrazol-5-yl)-[1,1'-biphenyl]-4-yl)-4-methylpiperidine-1-carboxamide (47a): the synthesis was performed according to Step 6 of Example 1, except that 1g was replaced with 39f, and 4-tert-butylcyclohexylamine was replaced with 4-methylpiperidine. Yield: 45%.

Step 2: Synthesis of N-(3'-chloro-4'-methoxy-2-(2H-tetrazol-5-yl)-[1,1'-biphenyl]-4-yl)-4-methylpiperidine-1-carboxamide (22): the synthesis was performed according to Step 7 of Example 1, except that 1h was replaced with 47a. Yield: 62%.

ESI-MS: m/z = 428 [M+H]⁺. ¹H NMR (600 MHz, DMSO-*d*₆) δ 16.01 (s, 1H), δ 8.80 (s, 1H), 7.83 (d, *J =* 2.3 Hz, 1H), 7.77 (dd, *J* = 8.6, 2.3 Hz, 1H), 7.43 (d, *J* = 8.5 Hz, 1H), 7.15 (d, *J* = 2.2 Hz, 1H), 7.05 (d, *J* = 8.6 Hz, 1H), 6.89 (dd, *J =* 8.5, 2.3 Hz, 1H), 4.27 - 4.20 (m, 2H), 3.84 (s, 3H), 2.75 - 2.67 (m, 2H), 1.71 - 1.65 (m, 2H), 1.25 - 1.18 (m, 1H), 1.17 - 1.07 (m, 2H), 0.96 (d, *J =* 6.1 Hz, 3H).

### Preparation Example 23

### Synthesis of cyclohexyl 2-fluoro-4-(4-methylpiperidine-1-formamido)-6-(2H-tetrazol-5-yl)benzoate (Compound 23):

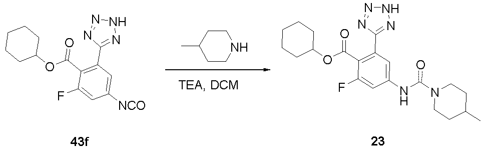

The synthesis was performed according to Step 6 of Example 1, except that 1g was replaced with 43f, and 4-tert-butylcyclohexylamine was replaced with 4-methylpiperidine. Yield: 47%.

ESI-MS: m/z = 474 [M+H]⁺. ¹H NMR (500 MHz, DMSO-*d*₆) δ 8.61 (s, 1H), 8.10 (d, *J* = 1.6 Hz, 1H), 7.51 (dd, *J* = 8.1, 1.5 Hz, 1H), 4.73 - 4.61 (m, 1H), 4.25 (d, *J* = 13.1 Hz, 2H), 2.75 (t, *J* = 12.5 Hz, 2H), 1.74 (d, *J* = 12.2 Hz, 4H), 1.63 - 1.53 (m, 2H), 1.49 - 1.38 (m, 1H), 1.34 - 1.26 (m, 2H), 1.24 - 1.09 (m, 6H), 0.94 (d, *J =* 6.1 Hz, 3H).

### Preparation Example 24

### Synthesis of ethyl 4-(4-methylpiperidine-1-formamido)-2-(2H-tetrazol-5-yl)benzoate (Compound 24):

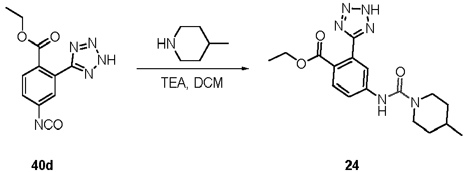

The synthesis was performed according to Step 6 of Example 1, except that 1g was replaced with 40d, and 4-tert-butylcyclohexylamine was replaced with 4-methylpiperidine. Yield: 44%.

ESI-MS: m/z = 359 [M+H]⁺. ¹H NMR (500 MHz, DMSO-*d*₆) δ 16.51 (s, 1H), 9.01 (s, 1H), 7.93 (d, *J* = 8.3 Hz, 1H), 7.81 (dd, *J* = 8.9, 2.3 Hz, 1H), 7.76 (d, *J* = 2.8 Hz, 1H), 4.24 (d, *J* = 13.1 Hz, 2H), 4.11 (q, *J* = 7.1 Hz, 2H), 2.75 (t,*J* = 12.1 Hz, 2H), 1.69 (d, *J =* 11.4 Hz, 2H), 1.24 - 1.15 (m, 1H), 1.14 - 1.06 (m, 2H), 1.02 (t, *J =* 7.1 Hz, 3H), 0.96 (d, *J* = 6.2 Hz, 3H).

### Preparation Example 25

### Synthesis of N-(4-(6-ethoxypyrid-3-yl)-3-fluoro-5-(2H-tetrazol-5-yl)phenyl)-4-(trifluoromethyl)piperidine-1-carboxamide (Compound 25):

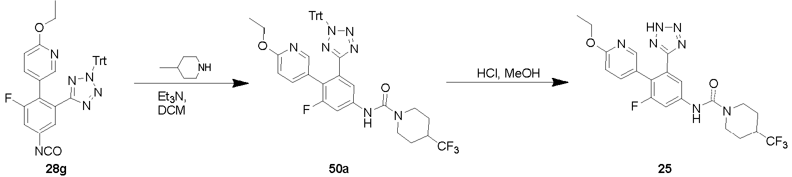

Step 1: Synthesis of N-(4-(6-ethoxypyrid-3-yl)-3-fluoro-5-(2-trimethyl - 2H-tetrazol-5-yl)phenyl)-4-(trifluoromethyl)piperidine-1-carboxamide (50a): the synthesis was performed according to Step 6 of Example 1, except that 1g was replaced with 28g, and 4-tert-butylcyclohexylamine was replaced with 4-methylpiperidine. Yield: 45%.

Step 2: Synthesis of N-(4-(6-ethoxypyrid-3-yl)-3-fluoro-5-(2H-tetrazol-5-yl)phenyl)-4-(trifluoromethyl)piperidine-1-carboxamide (PE-050): the synthesis was performed according to Step 7 of Example 1, except that 1h was replaced with 50a. Yield: 62%.

ESI-MS: m/z = 480 [M+H]⁺. ¹H NMR (400 MHz, DMSO-*d*₆) δ 16.10 (s, 1H), δ 9.09 (s, 1H), 7.95 - 7.88 (m, 1H), 7.84 - 7.69 (m, 2H), 7.41 (d, *J* = 7.9 Hz, 1H), 6.80 (d, *J* = 8.1 Hz, 1H), 4.39 - 4.21 (m, 4H), 2.79 (t, *J* = 12.6 Hz, 2H), 1.78 (d, *J* = 13.3 Hz, 2H), 1.36 (t, *J* = 7.0 Hz, 3H), 1.25 - 1.19 (m, 1H), 1.19 - 1.09 (m, 2H).

### Preparation Example 26

### Synthesis of N-(2-fluoro-3',4'-dimethoxy-6-(2H-tetrazol-5-yl)-[1,1'-biphenyl]-4-yl)-4-(trifluoromethyl)piperidine-1-carboxamide (Compound 26):

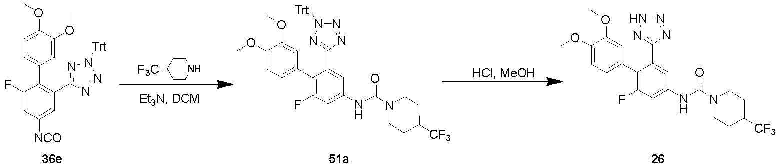

Step 1: Synthesis of N-(2-fluoro-3',4'-dimethoxy-6-(2-triphenyl-2H-tetrazol-5-yl)-[1,1'-biphenyl]-4-yl)-4-(trifluoromethyl)piperidine-1-carboxamide (51a): the synthesis was performed according to Step 6 of Example 1, except that 1g was replaced with 36e, and 4-tert-butylcyclohexylamine was replaced with 4-trifluoromethylpiperidine. Yield: 47%.

Step 2: Synthesis of N-(2-fluoro-3',4'-dimethoxy-6-(2H-tetrazol-5-yl)-[1,1'-biphenyl]-4-yl)-4-(trifluoromethyl)piperidine-1-carboxamide (26): the synthesis was performed according to Step 7 of Example 1, except that 1h was replaced with 51a. Yield: 59%.

ESI-MS: m/z = 495 [M+H]⁺. ¹H NMR (400 MHz, DMSO-*d*₆) δ 16.18 (s, 1H), δ 8.92 (s, 1H), 7.79 (dd, *J* = 12.8, 2.2 Hz, 1H), 7.61 (s, 1H), 6.98 - 6.83 (m, 1H), 6.69 - 6.58 (m, 2H), 4.24 (d, *J* = 13.0 Hz, 2H), 3.72 (s, 3H), 3.60 (s, 3H), 2.86 - 2.65 (m, 2H), 2.61 - 2.50 (m, 1H), 1.71 (d, *J=* 12.2 Hz, 2H), 1.17 - 1.08 (m, 2H).

### Preparation Example 27

### Synthesis of N-(4-(1-methyl-1H-indazol-5-yl)-3-(2H-tetrazol-5-yl)phenyl)-4-(trifluoromethyl)piperidine-1-carboxamide (Compound 27):

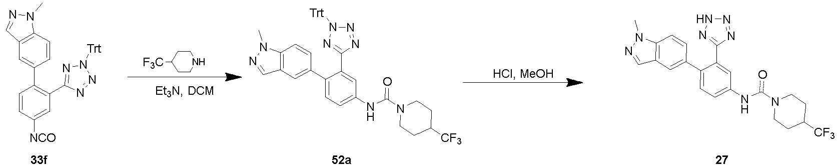

Step 1: Synthesis of N-(4-(1-methyl-1H-indazol-5-yl)-3-(2-trityl-2H-tetrazol-5-yl)phenyl)-4-(trifluoromethyl)piperidine-1-carboxamide (52a): the synthesis was performed according to Step 6 of Example 1, except that 1g was replaced with 33f, and 4-tert-butylcyclohexylamine was replaced with 4-trifluoromethylpiperidine. Yield: 47%.

Step 2: Synthesis of N-(4-(1-methyl-1H-indazol-5-yl)-3-(2H-tetrazol-5-yl)phenyl)-4-(trifluoromethyl)piperidine-1-carboxamide (Compound 27): the synthesis was performed according to Step 7 of Example 1, except that 1h was replaced with 52a. Yield: 59%.

ESI-MS: m/z = 471 [M+H]⁺. ¹H NMR (600 MHz, DMSO-*d*₆) δ 16.02 (s, 1H), 8.80 (s, 1H), 8.01 (s, 1H), 7.85 (d, *J =* 2.3 Hz, 1H), 7.79 (dd, *J =* 8.5, 2.3 Hz, 1H), 7.52 - 7.46 (m, 3H), 6.97 (dd, *J =* 8.5, 1.7 Hz, 1H), 4.28 - 4.22 (m, 2H), 4.03 (s, 3H), 2.79 - 2.73 (m, 2H), 2.70 - 2.53 (m, 1H), 1.70 - 1.64 (m, 2H), 1.15 - 1.06 (m, 2H).

### Preparation Example 28

### Synthesis of N-(3'-chloro-4'-methoxy-2-(2H-tetrazol-5-yl)-[1,1'-biphenyl]-4-yl)-4-(trifluoromethyl)piperidine-1-carboxamide (Compound 28):

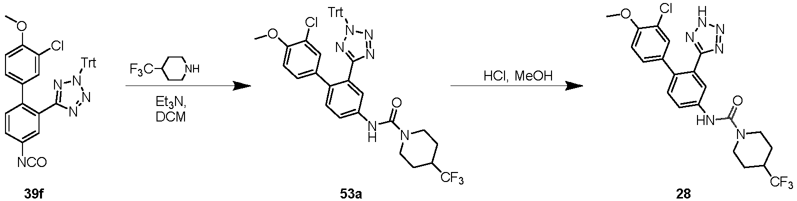

Step 1: Synthesis of N-(3'-chloro-4'-methoxy-2-(2-trityl-2H-tetrazol-5-yl)-[1,1'-biphenyl]-4-yl)-4-(trifluoromethyl)piperidine-1-carboxamide (53a): the synthesis was performed according to Step 6 of Example 1, except that 1g was replaced with 39f, and 4-tert-butylcyclohexylamine was replaced with 4-trifluoromethylpiperidine. Yield: 44%.

Step 2: Synthesis of N-(3'-chloro-4'-methoxy-2-(2H-tetrazol-5-yl)-[1,1'-biphenyl]-4-yl)-4-(trifluoromethyl)piperidine-1-carboxamide (28): the synthesis was performed according to Step 7 of Example 1, except that 1h was replaced with 53a. Yield: 59%.

ESI-MS: m/z = 481 [M+H]⁺. ¹H NMR (600 MHz, DMSO-*d*₆) δ 16.05 (s, 1H), δ 8.87 (s, 1H), 7.82 (d, *J =* 2.1 Hz, 1H), 7.79 (dd, *J* = 8.3, 2.3 Hz, 1H), 7.46 (d, *J* = 8.8 Hz, 1H), 7.19 (d, *J* = 2.4 Hz, 1H), 7.08 (d, *J* = 8.9 Hz, 1H), 6.90 (dd, *J =* 8.1, 2.4 Hz, 1H), 4.25 - 4.21 (m, 2H), 3.87 (s, 3H), 2.78 - 2.66 (m, 2H), 2.71 - 2.55 (m, 1H), 1.75 - 1.69 (m, 2H), 1.18 - 1.09 (m, 2H).

### Preparation Example 29

### Synthesis of cyclohexyl 2-fluoro-6-(2H-tetrazol-5-yl)-4-(4-(trifluoromethyl)piperidine-1-formamido)benzoate (Compound 29):

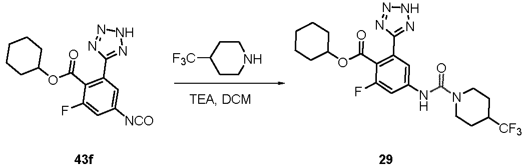

The synthesis was performed according to Step 6 of Example 1, except that 1g was replaced with 43f, and 4-tert-butylcyclohexylamine was replaced with 4-trifluoromethylpiperidine. Yield: 45%.

ESI-MS: m/z = 485 [M+H]⁺. ¹H NMR (500 MHz, DMSO-*d*₆) δ 16.36 (s, 1H), 8.90 (s, 1H), 8.08 (d, *J* = 1.9 Hz, 1H), 7.57 (dd, *J =* 8.1, 1.5 Hz, 1H), 4.73 - 4.61 (m, 1H), 4.25 (d, *J* = 13.1 Hz, 2H), 2.75 (t, *J* = 12.5 Hz, 2H), 2.71 - 2.55 (m, 1H), 1.74 (d, *J* = 12.2 Hz, 4H), 1.63 - 1.53 (m, 2H), 1.34 - 1.26 (m, 2H), 1.23 - 1.09 (m, 6H).

### Preparation Example 30

### Synthesis of ethyl 2-fluoro-6-(2H-tetrazol-5-yl)-4-(4-(trifluoromethyl)piperidine-1-formamido)benzoate (Compound 30):

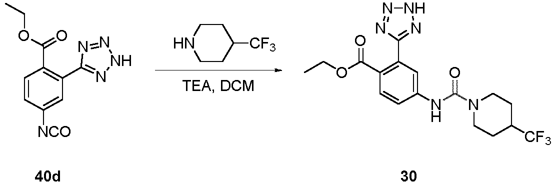

The synthesis was performed according to Step 6 of Example 1, except that 1g was replaced with 40d, and 4-tert-butylcyclohexylamine was replaced with 4-trifluoromethylpiperidine. Yield: 47%.

ESI-MS: m/z = 413 [M+H]⁺. ¹H NMR (500 MHz, DMSO-*d*₆) δ 16.40 (s, 1H), 9.03 (s, 1H), 7.96 (d, *J* = 8.7 Hz, 1H), 7.87 (dd, *J* = 8.7, 2.3 Hz, 1H), 7.83 (d, *J* = 2.2 Hz, 1H), 4.26 (d, *J* = 13.1 Hz, 2H), 4.12 (q, *J =* 7.1 Hz, 2H), 2.75 *(t, J* = 12.0 Hz, 2H), 2.71 - 2.55 (m, 1H), 1.68 (d, *J* = 11.3 Hz, 2H), 1.17 - 1.09 (m, 2H), 1.06 (t, *J* = 7.1 Hz, 3H).

### Preparation Example 31

### Synthesis of N-(4-(6-ethoxypyrid-3-yl)-3-fluoro-5-(2H-tetrazol-5-yl)phenyl)-4-methylpiperidine-1-carboxamide (Compound 31):

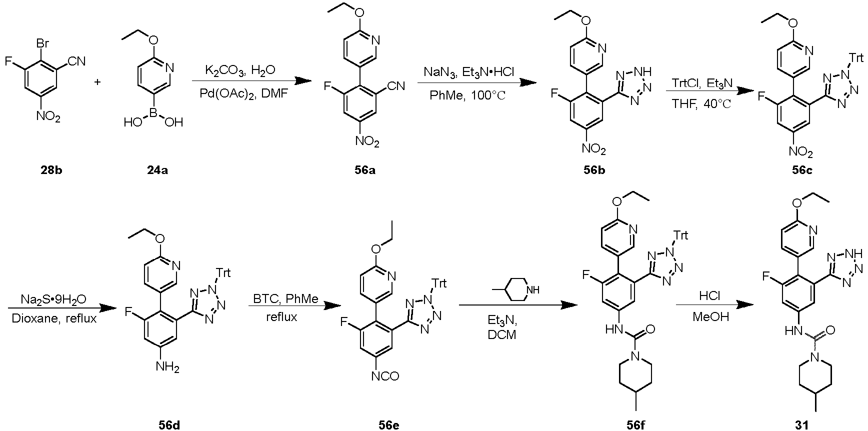

Step 1: Synthesis of 2-(6-ethoxypyrid-3-yl)-3-fluoro-5-nitrobenzonitrile (56a): the synthesis was performed according to Step 1 of Example 1, except that 1a was replaced with 28b, and 1b was replaced with 24a. Yield: 85%.

Step 2: Synthesis of 2-ethoxy-5-(2-fluoro-4-nitro-6-(2H-tetrazol-5-yl)phenyl)pyridine (56b): the synthesis was performed according to Step 2 of Example 1, except that 1c was replaced with 56a. Yield: 90%.

Step 3: Synthesis of 2-ethoxy-5-(2-fluoro-4-nitro-6-(2-trityl-2H-tetrazol-5-yl)phenyl)pyridine (56c): the synthesis was performed according to Step 3 of Example 1, except that 1d was replaced with 56b. Yield: 89%.

Step 4: Synthesis of 4-(6-ethoxypyrid-3-yl)-3-fluoro-5-(2-triphenyl-2H-tetrazol-5-yl)phenylamine (56d): the synthesis was performed according to Step 4 of Example 1, except that 1e was replaced with 56c. Yield: 88%.

Step 5: Synthesis of 2-ethoxy-5-(2-fluoro-4-isocyanato-6-(2-triphenyl-2H-tetrazol-5-yl)phenyl)pyridine (56e): the synthesis was performed according to Step 5 of Example 1, except that 1f was replaced with 56d.

Step 6: Synthesis of N-(4-(6-ethoxypyrid-3-yl)-3-fluoro-5-(2-triphenyl-2H-tetrazol-5-yl)phenyl)-4-methylpiperidine-1-carboxamide (56f): the synthesis was performed according to Step 6 of Example 1, except that 1g was replaced with 56e, and 4-tert-butylcyclohexylamine was replaced with 4-methylpiperidine. Yield through the two steps: 43%.

Step 7: Synthesis of N-(4-(6-ethoxypyrid-3-yl)-3-fluoro-5-(2H-tetrazol-5-yl)phenyl)-4-methylpiperidine-1-carboxamide (31): the synthesis was performed according to Step 7 of Example 1, except that 1h was replaced with 56f. Yield: 67%.

ESI-MS: m/z = 426[M+H]⁺. ¹H NMR (400 MHz, DMSO-*d*₆) δ 16.34 (s, 1H), δ 8.84 (s, 1H), 7.86 (d, *J* = 2.3 Hz, 1H), 7.77 (dd, *J* = 8.5, 2.3 Hz, 1H), 7.43 (d, *J =* 8.5 Hz, 1H), 7.29 (dd, *J =* 8.6, 2.6 Hz, 1H), 6.70 (d, *J* = 8.6 Hz, 1H), 4.31 - 4.15 (m, 4H), 2.73 (t, *J* = 11.8 Hz, 2H), 1.67 (d, *J* = 11.5 Hz, 2H), 1.36 (t, *J* = 7.4 Hz, 3H), 1.21 - 1.17 (m, 1H), 1.14 - 1.09 (m, 2H), 0.97 *(d, J =* 6.5 Hz, 3H).

### Preparation Example 32

### Synthesis of 4-(tert-butyl)-N-(3-fluoro-4-(1-methyl-1H-indazol-5-yl)-5-(2H-tetrazol-5-yl)phenyl)piperidine-1-carboxamide (Compound 32):

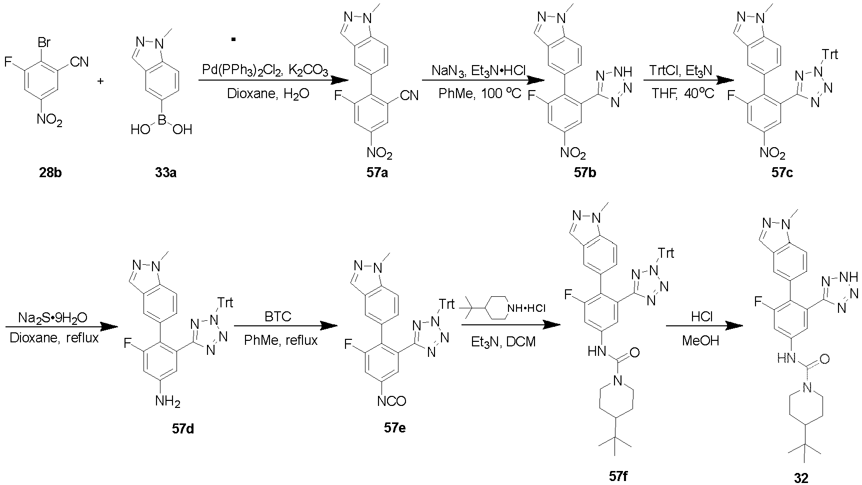

Step 1: Synthesis of 3-fluoro-2-(1-methyl-1H-indazol-5-yl)-5-nitrobenzonitrile (57a): the synthesis was performed according to Step 1 of Example 1, except that 27a was replaced with 28b, and 24a was replaced with 33a. Yield: 91%.

Step 2: Synthesis of 5-(2-fluoro-4-nitro-6-(2H-tetrazol-5-yl)phenyl)-1-methyl-1H-indazole (57b): the synthesis was performed according to Step 2 of Example 1, except that 1c was replaced with 57a. Yield: 90%.

Step 3: Synthesis of 5-(2-fluoro-4-nitro-6-(2-triphenyl-2H-tetrazol-5-yl)phenyl)-1-methyl-1H-indazole (57c): the synthesis was performed according to Step 3 of Example 1, except that 1d was replaced with 57b. Yield: 93%.

Step 4: Synthesis of 3-fluoro-4-(1-methyl-1H-indazol-5-yl)-5-(2-triphenyl-2H-tetrazol-5-yl)phenylamine (57d): the synthesis was performed according to Step 4 of Example 1, except that 1e was replaced with 57c. Yield: 86%.

Step 5: Synthesis of 5-(2-fluoro-4-isocyanato-6-(2-triphenyl-2H-tetrazol-5-yl)phenyl)-1-methyl-1H-indazole (57e): the synthesis was performed according to Step 5 of Example 1, except that 1f was replaced with 57d.

Step 6: Synthesis of 4-(tert-butyl)-N-(3-fluoro-4-(1-methyl-1H-indazol-5-yl)-5-(2-triphenyl-2H-tetrazol-5-yl)phenyl)piperidine-1-carboxamide (57f): the synthesis was performed according to Step 6 of Example 1, except that 1g was replaced with 57e, and 4-tert-butylcyclohexylamine was replaced with 4-tert-butylpiperidine hydrochloride. Yield through the two steps: 42%.

Step 7: Synthesis of 4-(tert-butyl)-N-(3-fluoro-4-(1-methyl-1H-indazol-5-yl)-5-(2H-tetrazol-5-yl)phenyl)piperidine-1-carboxamide (32): the synthesis was performed according to Step 7 of Example 1, except that 1h was replaced with 57f. Yield: 71%.

ESI-MS: m/z = 478 [M+H]⁺. ¹H NMR (600 MHz, DMSO-*d*₆) δ 16.12 (s, 1H), 8.80 (s, 1H), 7.85 (d, *J =* 2.3 Hz, 1H), 7.79 (dd, *J =* 8.5, 2.3 Hz, 1H), 7.52 - 7.46 (m, 3H), 6.97 (dd, *J =* 8.6, 1.5 Hz, 1H), 4.28 - 4.22 (m, 2H), 4.05 (s, 3H), 2.78 - 2.69 (m, 2H), 1.73 - 1.68 (m, 2H), 1.27 - 1.21 (m, 1H), 1.19 - 1.08 (m, 2H), 0.86 (s, 9H).

### Preparation Example 33

### Synthesis of 4-(tert-butyl)-N-(3'-chloro-2-fluoro-4'-methoxy-6-(2H-tetrazol-5-yl)-[1,1'-biphenyl]-4-yl)piperidine-1-carboxamide (Compound 33):

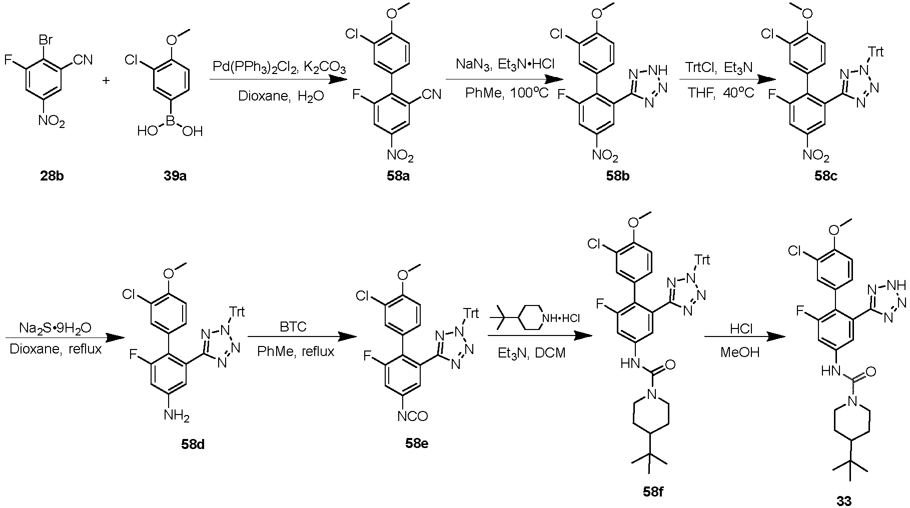

Step 1: Synthesis of 3'-chloro-6-fluoro-4'-methoxy-4-nitro-[1,1'-biphenyl]-2-carbonitrile (58a): the synthesis was performed according to Step 1 of Example 7, except that 27a was replaced with 28b, and 24a was replaced with 39a. Yield: 81%.

Step 2: Synthesis of 5-(3'-chloro-6-fluoro-4'-methoxy-4-nitro-[1,1'-biphenyl]-2-yl)-2H-tetrazole (58b): the synthesis was performed according to Step 2 of Example 1, except that 1c was replaced with 58a. Yield: 88%.

Step 3: Synthesis of 5-(3'-chloro-6-fluoro-4'-methoxy-4-nitro-[1,1'-biphenyl]-2-yl)-2-trityl-2H-tetrazole (58c): the synthesis was performed according to Step 3 of Example 1, except that 1d was replaced with 58b. Yield: 91%.

Step 4: Synthesis of 3'-chloro-2-fluoro-4'-methoxy-6-(2-trityl-2H-tetrazol-5-yl)-[1,1'-biphenyl]-4-amine (58d): the synthesis was performed according to Step 4 of Example 1, except that 1e was replaced with 58c. Yield: 87%.

Step 5: Synthesis of 5-(3'-chloro-6-fluoro-4-isocyanato-4'-methoxy-[1,1'-biphenyl]-2-yl)-2-trityl-2H-tetrazole (58e): the synthesis was performed according to Step 5 of Example 1, except that 1f was replaced with 58d.

Step 6: Synthesis of 4-(tert-butyl)-N-(3'-chloro-2-fluoro-4'-methoxy-6-(2-trityl-2H-tetrazol-5-yl)-[1,1'-biphenyl]-4-yl)piperidine-1-carboxamide (58f): the synthesis was performed according to Step 6 of Example 1, except that 1g was replaced with 58e, and 4-tert-butylcyclohexylamine was replaced with 4-tert-butylpiperidine hydrochloride. Yield through the two steps: 46%.

Step 7: Synthesis of 4-(tert-butyl)-N-(3'-chloro-2-fluoro-4'-methoxy-6-(2H-tetrazol-5-yl)-[1,1'-biphenyl]-4-yl) piperidine-1-carboxamide (33): the synthesis was performed according to Step 7 of Example 1, except that 1h was replaced with 58f. Yield: 69%.

ESI-MS: m/z = 488 [M+H]⁺. ¹H NMR (600 MHz, DMSO-*d*₆) δ 16.12 (s, 1H), δ 8.80 (s, 1H), 7.81 (dd, *J* = 8.6, 2.3 Hz, 1H), 7.47 (d, *J* = 8.5 Hz, 1H), 7.19 (d, *J* = 2.2 Hz, 1H), 7.03 *(d, J* = 8.6 Hz, 1H), 6.86 (dd, *J* = 8.5, 2.3 Hz, 1H), 4.25 - 4.20 (m, 2H), 3.81 (s, 3H), 2.74 - 2.66 (m, 2H), 1.73 - 1.67 (m, 2H), 1.24 - 1.18 (m, 1H), 1.16 - 1.08 (m, 2H), 0.85 (s, 9H).

### Preparation Example 34

### Synthesis of N-(3-fluoro-4-(1-methyl-1H-indazol-5-yl)-5-(2H-tetrazol-5-yl)phenyl)-4-(trifluoromethyl)piperidine-1-carboxamide (Compound 34):

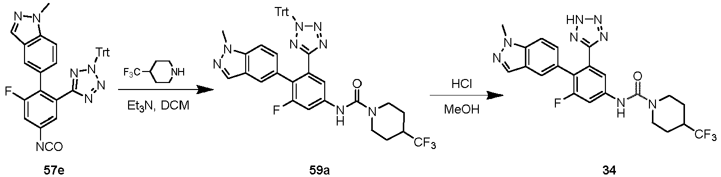

Step 1: Synthesis of N-(3-fluoro-4-(1-methyl-1H-indazol-5-yl)-5-(2-triphenyl-2H-tetrazol-5-yl)phenyl)-4-(trifluoromethyl)piperidine-1-carboxamide (59a): the synthesis was performed according to Step 6 of Example 1, except that 1g was replaced with 57e, and 4-tert-butylcyclohexylamine was replaced with 4-trifluoromethylpiperidine. Yield: 48%.

Step 2: Synthesis of N-(3-fluoro-4-(1-methyl-1H-indazol-5-yl)-5-(2H-tetrazol-5-yl)phenyl)-4-(trifluoromethyl)piperidine-1-carboxamide (34): the synthesis was performed according to Step 7 of Example 1, except that 1h was replaced with 59a. Yield: 72%.

ESI-MS: m/z = 478 [M+H]⁺. ¹H NMR (600 MHz, DMSO-*d*₆) δ 16.15 (s, 1H), 8.89 (s, 1H), 7.84 (d, *J=* 2.3 Hz, 1H), 7.75 (dd, *J=* 8.5, 2.3 Hz, 1H), 7.56 - 7.47 (m, 3H), 6.98 (dd, *J =* 8.6, 1.5 Hz, 1H), 4.28 - 4.22 (m, 2H), 4.05 (s, 3H), 2.78 - 2.69 (m, 2H), 2.67 - 2.55 (m, 1H), 1.73 - 1.68 (m, 2H), 1.19 - 1.08 (m, 2H).

### Preparation Example 35

### Synthesis of 4-(tert-butyl)-N-(6-(3,4-dimethoxyphenyl)-5-(2H-tetrazol-5-yl)pyrid-3-yl)piperidine-1-carboxamide (Compound 35):

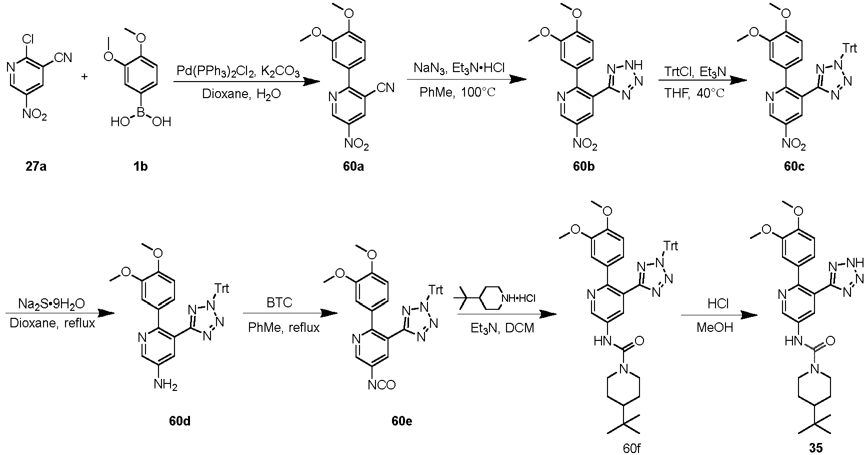

Step 1: Synthesis of 2-(3,4-dimethoxyphenyl)-5-nitronicotinonitrile (60a): the synthesis was performed according to Step 1 of Example 1, except that 1b was replaced with 24a. Yield: 56%.

Step 2: Synthesis of 2-(3,4-dimethoxyphenyl)-5-nitro-3-(2H-tetrazol-5-yl)pyridine (60b): the synthesis was performed according to Step 2 of Example 1, except that 1c was replaced with 60a. Yield: 91%.

Step 3: Synthesis of 2-(3,4-dimethoxyphenyl)-5-nitro-3-(2-trityl-2H-tetrazol-5-yl)pyridine (60c): the synthesis was performed according to Step 3 of Example 1, except that 1d was replaced with 60b. Yield: 89%.

Step 4: Synthesis of 6-(3,4-dimethoxyphenyl)-5-(2-trityl-2H-tetrazol-5-yl)pyridin-3-amine (60d): the synthesis was performed according to Step 4 of Example 1, except that 1e was replaced with 60c. Yield: 85%.

Step 5: Synthesis of 2-(3,4-dimethoxyphenyl)-5-isocyanato-3-(2-trityl-2H-tetrazol-5-yl)pyridine (60e): the synthesis was performed according to Step 5 of Example 1, except that 1f was replaced with 60d.

Step 6: Synthesis of 4-(tert-butyl)-N-(6-(3,4-dimethoxyphenyl)-5-(2-trityl-2H-tetrazol-5-yl)pyrid-3-yl)cyclohexane-1-carboxamide (60f): the synthesis was performed according to Step 6 of Example 1, except that 1g was replaced with 60e, and 4-tert-butylcyclohexylamine was replaced with 4-tert-butylpiperidine hydrochloride. Yield through the two steps: 44%.

Step 7: Synthesis of 4-(tert-butyl)-N-(6-(3,4-dimethoxyphenyl)-5-(2H-tetrazol-5-yl)pyrid-3-yl)piperidine-1-carboxamide (35): the synthesis was performed according to Step 7 of Example 1, except that 1h was replaced with 60f. Yield: 71%.

ESI-MS: m/z =467[M+H]⁺.¹H NMR (500 MHz, DMSO-*d*₆) δ 16.15 (s, 1H), 8.88 (s, 1H), 7.83 - 7.67 (m, 2H), 7.53 - 7.37 (m, 1H), 6.88 (d, *J =* 8.1 Hz, 1H), 6.67 - 6.49 (m, 1H), 4.27 - 4.20 (m, 2H), 2.75 - 2.67 (m, 2H), 1.71 - 1.65 (m, 2H), 1.25 - 1.18 (m, 1H), 1.17 - 1.07 (m, 2H), 0.85 (s, 9H).

### Preparation Example 36

### Synthesis of 4-(tert-butyl)-N-(6-(1-methyl-1H-indazol-5-yl)-5-(2H-tetrazol-5-yl)pyrid-3-yl)piperidine-1-carboxamide (Compound 36):

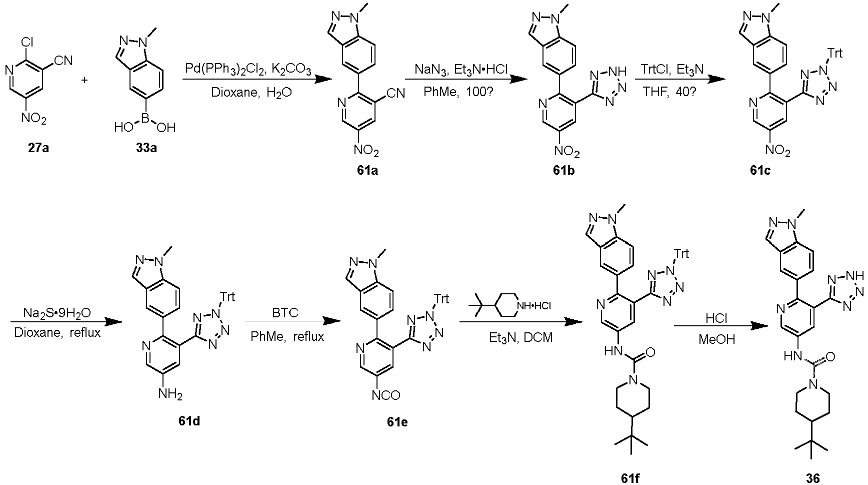

Step 1: Synthesis of 2-(1-methyl-1H-indazol-5-yl)-5-nitronicotinonitrile (61a): the synthesis was performed according to Step 1 of Example 1, except that 24a was replaced with 33a. Yield: 61%.

Step 2: Synthesis of 1-methyl-5-(5-nitro-3-(2H-tetrazol-5-yl)pyridin-2-yl)-1H-indazole (61b): the synthesis was performed according to Step 2 of Example 1, except that 1c was replaced with 61a. Yield: 89%.

Step 3: Synthesis of 1-methyl-5-(5-nitro-3-(2-triphenyl-2H-tetrazol-5-yl)pyridin-2-yl)-1H-indazole (61c): the synthesis was performed according to Step 3 of Example 1, except that 1d was replaced with 61b. Yield: 88%.

Step 4: Synthesis of 6-(1-methyl-1H-indazol-5-yl)-5-(2-trityl-2H-tetrazol-5-yl)pyridin-3-amine (61d): the synthesis was performed according to Step 4 of Example 1, except that 1e was replaced with 61c. Yield: 85%.

Step 5: Synthesis of 5-(5-isocyanato-3-(2-triphenyl-2H-tetrazol-5-yl)pyridin-2-yl)-1-methyl-1H-indazole (61e): the synthesis was performed according to Step 5 of Example 1, except that 1f was replaced with 61d.

Step 6: Synthesis of 4-(tert-butyl)-N-(6-(1-methyl-1H-indazol-5-yl)-5-(2-trityl-2H-tetrazol-5-yl)pyrid-3-yl)piperidine-1-carboxamide (61f): the synthesis was performed according to Step 6 of Example 1, except that 1g was replaced with 61e, and 4-tert-butylcyclohexylamine was replaced with 4-tert-butylpiperidine hydrochloride. Yield through the two steps: 46%.

Step 7: Synthesis of 4-(tert-butyl)-N-(6-(1-methyl-1H-indazol-5-yl)-5-(2H-tetrazol-5-yl)pyrid-3-yl)piperidine-1-carboxamide (36): the synthesis was performed according to Step 7 of Example 1, except that 1h was replaced with 61f. Yield: 69%.

ESI-MS: m/z = 461 [M+H]⁺. ¹H NMR (600 MHz, DMSO-*d*₆) δ 16.12 (s, 1H), 8.99 (s, 1H), 8.01 (s, 1H), 7.79 (dd, *J =* 8.5, 2.3 Hz, 1H), 7.52 - 7.46 (m, 3H), 6.97 (dd, *J=* 8.5, 1.7 Hz, 1H), 4.28 - 4.22 (m, 2H), 4.07 (s, 3H), 2.76 - 2.68 (m, 2H), 1.79 - 1.68 (m, 2H), 1.27 - 1.16 (m, 1H), 1.18 - 1.08 (m, 2H), 0.85 (s, 9H).

### Preparation Example 37

### Synthesis of ethyl 5-(4-(tert-butyl)piperidine-1-formamido)-3-(2H-tetrazol-5-yl)pyridylcarboxylate (Compound 37):

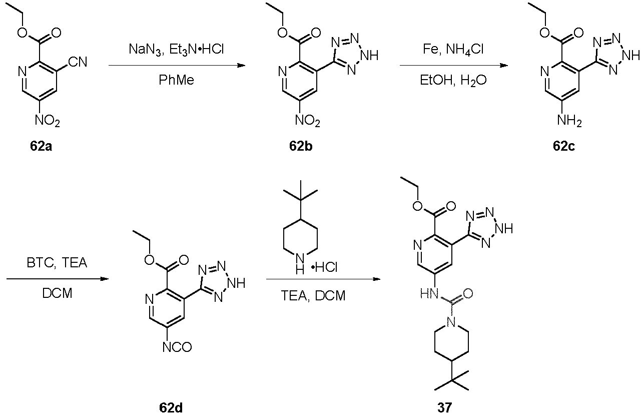

Step 1: Synthesis of ethyl 5-nitro-3-(2H-tetrazol-5-yl)pyridylcarboxylate (62b): the synthesis was performed according to Step 2 of Example 1, except that 1c was replaced with 62a. Yield: 83%.

Step 2: Synthesis of ethyl 5-amino-3-(2H-tetrazol-5-yl)pyridylcarboxylate (62c): the synthesis was performed according to Step 2 of Example 16, except that 40b was replaced with 62b. Yield: 81%.

Step 3: Synthesis of ethyl 5-isocyanato-3-(2H-tetrazol-5-yl)pyridylcarboxylate (62d): the synthesis was performed according to Step 3 of Example 16, except that 40c was replaced with 62c.

Step 4: Synthesis of ethyl 5-(4-(tert-butyl)piperidine-1-formamido)-3-(2H-tetrazol-5-yl)pyridylcarboxylate (37): the synthesis was performed according to Step 6 of Example 1, except that 1g was replaced with 62d, and 4-tert-butylcyclohexylamine was replaced with 4-tert-butylpiperidine hydrochloride. Yield through the two steps: 40%.

ESI-MS: m/z = 402 [M+H]⁺. ¹H NMR (500 MHz, DMSO-*d*₆) δ 16.31 (s, 1H), 9.03 (s, 1H), 7.95 (d, *J* = 8.7 Hz, 1H), 7.80 (d, *J* = 2.2 Hz, 1H), 4.21 (d,*J* = 13.1 Hz, 2H), 4.04 (q, *J* = 7.1 Hz, 2H), 2.73 (t, *J* = 12.1 Hz, 2H), 1.65 (d,*J* = 11.4 Hz, 2H), 1.29 - 1.18 (m, 1H), 1.16 - 1.07 (m, 2H), 1.04 (t, *J* = 7.1 Hz, 3H), 0.86 (s, 9H).

### Preparation Example 38

### Synthesis of cyclohexyl 5-(4-(tert-butyl)piperidine-1-formamido)-3-(2H-tetrazol-5-yl)pyridylcarboxylate (Compound 38):

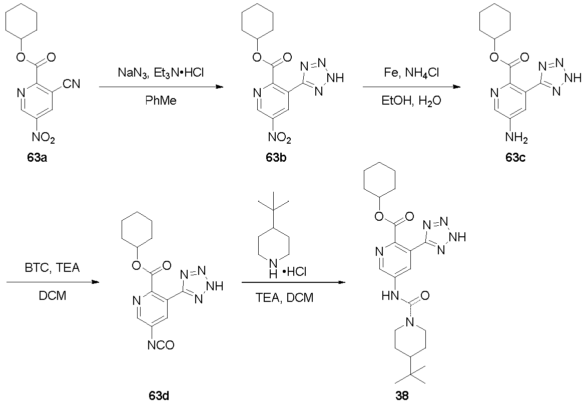

Step 1: Synthesis of intermediate 63b: the synthesis was performed according to Step 1 of Example 16, except that 1c was replaced with 63a. Yield: 81%.

Step 2: Synthesis of (63c): the synthesis was performed according to Step 2 of Example 16, except that 40b was replaced with 63b. Yield: 85%.

Step 3: Synthesis of (63d): the synthesis was performed according to Step 3 of Example 16, except that 40c was replaced with 63c.

Step 4: Synthesis of cyclohexyl 5-(4-(tert-butyl)piperidine-1-formamido)-3-(2H-tetrazol-5-yl)pyridylcarboxylate (38): the synthesis was performed according to Step 7 of Example 1, except that 1g was replaced with 63c, and 4-tert-butylcyclohexylamine was replaced with 4-tert-butylpiperidine hydrochloride. Yield through the two steps: 43%.

ESI-MS: m/z = 457 [M+H]⁺.¹H NMR (500 MHz, DMSO-*d*₆) δ 16.01 (s, 1H), 8.37 (d, *J* = 1.5 Hz, 1H), 8.14 (d, *J* = 7.5 Hz, 1H), 7.74 (dd, *J =* 7.5, 1.7 Hz, 1H), 4.76 - 4.66 (m, 1H), 4.25 (d, *J =* 12.1 Hz, 2H), 2.76 (t, *J =* 11.4 Hz, 2H), 1.68 (d, *J* = 12.2 Hz, 4H), 1.60 - 1.49 (m, 2H), 1.47 - 1.37 (m, 1H), 1.33 - 1.23 (m, 2H), 1.22 - 1.05 (m, 6H), 0.86 (s, 9H).

### Preparation Example 39

### Synthesis of N-(4-(benzo[d][1,3]dioxolan-5-yl)-3-(2H-tetrazol-5-yl)phenyl)-4-(tert-butyl)piperidine-1-carboxamide (Compound 39):

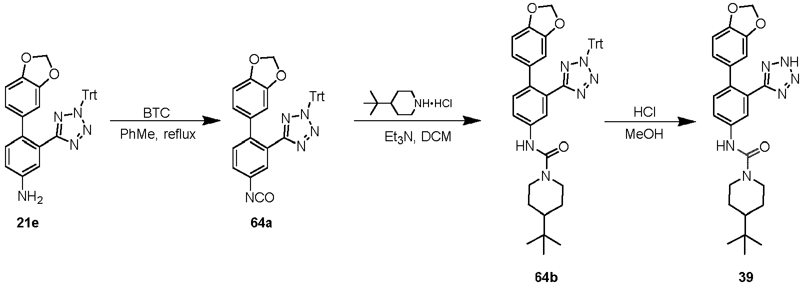

Step 1: Synthesis of 5-(2-(benzo[d][1,3] dioxin-5-yl)-5-isocyanatophenyl)-2-triphenyl-2H-tetrazole (64a): the synthesis was performed according to Step 5 of Example 1, except that 1f was replaced with 21e.

Step 2: Synthesis of N-(4-(benzo[d][1,3]dioxolan-5-yl)-3-(2-triphenyl-2H-tetrazol-5-yl)phenyl)-4-(tert-butyl)piperidine-1-carboxamide (64b): the synthesis was performed according to Step 6 of Example 1, except that 1g was replaced with 64a, and 4-tert-butylcyclohexylamine was replaced with 4-tert-butylpiperidine hydrochloride. Yield through the two steps: 49%.

Step 3: Synthesis of N-(4-(benzo[d][1,3]dioxolan-5-yl)-3-(2H-tetrazol-5-yl)phenyl)-4-(tert-butyl)piperidine-1-carboxamide (39): the synthesis was performed according to Step 7 of Example 1, except that 1h was replaced with 64b. Yield: 69%.

ESI-MS: m/z =450[M+H]⁺.¹H NMR (500 MHz, Chloroform-*d*) δ 16.23 (s, 1H), 8.62 (s, 1H), 7.72 (d, *J* = 2.4 Hz, 1H), 7.56 (dd, *J* = 8.4, 2.3 Hz, 1H), 7.37 (d, *J =* 8.5 Hz, 1H), 6.80 (d, *J =* 8.0 Hz, 1H), 6.62 (d, *J* = 1.7 Hz, 1H), 6.48 - 6.37 (m, 1H), 6.14 (d, *J* = 7.8 Hz, 1H), 6.01 (s, 2H), 4.27 - 4.20 (m, 2H), 2.75 - 2.67 (m, 2H), 1.71 - 1.65 (m, 2H), 1.25 - 1.18 (m, 1H), 1.17 - 1.07 (m, 2H), 0.86 (s, 9H).

### Preparation Example 40

### Synthesis of 4-(tert-butyl)-N-(4-(tetrahydro-2H-pyran-4-yl)-3-(2H-tetrazol-5-yl)phenyl)piperidine-1-carboxamide (Compound 40):

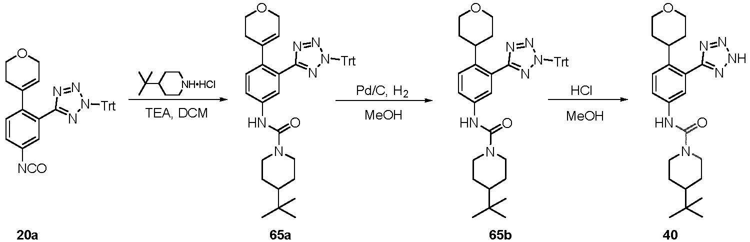

Step 1: Synthesis of 4-(tert-butyl)-N-(4-(3,6-dihydro-2H-pyran-4-yl)-3-(2-trityl-2H-tetrazol-5-yl)phenyl)cyclohexane-1-carboxamide (65a): the synthesis was performed according to Step 6 of Example 1, except that 1g was replaced with 20a, and 4-tert-butylcyclohexylamine was replaced with 4-tert-butylpiperidine hydrochloride. Yield: 45%.

Step 2: Synthesis of 4-(tert-butyl)-N-(4-(tetrahydro-2H-pyran-4-yl)-3-(2-trityl-2H-tetrazol-5-yl)phenyl)cyclohexane-1-carboxamide (65b): 0.12 g (0.19 mmol) of 65a was dissolved in 3 mL of methanol. The reaction was carried out at the room temperature for 5 h under 1 MPa of hydrogen pressure. After the reaction was completed, Pd/C was removed off by filtration. The filtrate was concentrated under reduced pressure, and the residue was purified by silica gel column chromatography to obtain 65b. Yield: 83%.

Step 3: Synthesis of 4-(tert-butyl)-N-(4-(tetrahydro-2H-pyran-4-yl)-3-(2H-tetrazol-5-yl)phenyl)piperidine-1-carboxamide (40): the synthesis was performed according to Step 7 of Example 1, except that 1h was replaced with 65b. Yield: 65%.

ESI-MS: m/z =414 [M+H]⁺.¹H NMR (600 MHz, DMSO-*d*₆) δ 16.51 (s, 1H), δ 8.45 (s, 1H), 7.64 (s, 1H), 7.47 (dd, *J =* 8.6, 2.4 Hz, 1H), 7.41 (d, *J* = 8.6 Hz, 1H), 4.27 - 4.20 (m, 2H), 3.94 - 3.85 (m, 2H), 3.31 - 3.25 (m, 2H), 3.10 - 2.94 (m, 1H), 2.75 - 2.67 (m, 2H), 1.90 - 1.81 (m, 2H), 1.71 - 1.65 (m, 2H), 1.25 - 1.18 (m, 1H), 1.17 - 1.07 (m, 2H), 0.86 (s, 9H).

### Preparation Example 41

### Synthesis of 4-(tert-butyl)-N-(4-(3,4-dihydro-2H-pyran-4-yl)-3-(2H-tetrazol-5-yl)phenyl)piperidine-1-carboxamide (41):

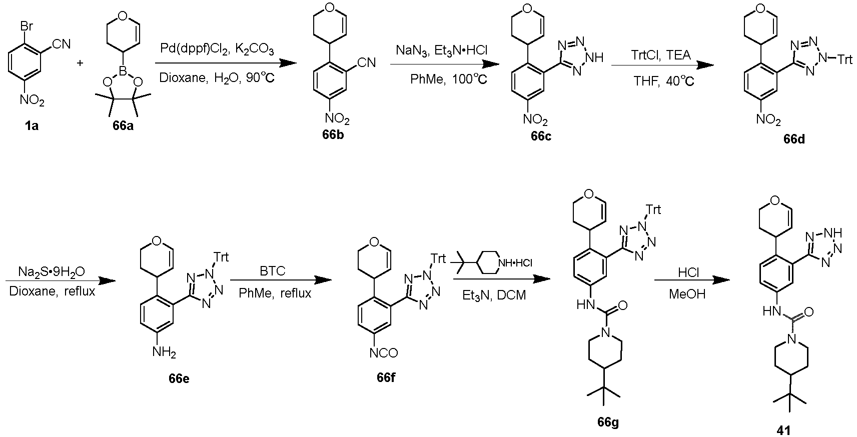

Step 1: Synthesis of 2-(3,4-dihydro-2H-pyran-4-yl)-5-nitrobenzonitrile (66b): the synthesis was performed according to Step 1 of Example 18, except that 28b was replaced with 1a, and 41a was replaced with 66a. Yield: 81%.

Step 2: Synthesis of 5-(2-(3,4-dihydro-2H-pyran-4-yl)-5-nitrophenyl)-2H-tetrazole (66c): the synthesis was performed according to Step 2 of Example 1, except that 1c was replaced with 67b. Yield: 83%.

Step 3: Synthesis of 5-(2-(3,4-dihydro-2H-pyran-4-yl)-5-nitrophenyl)-2-trityl-2H-tetrazole (66d): the synthesis was performed according to Step 3 of Example 1, except that 1d was replaced with 66c. Yield: 85%.

Step 4: Synthesis of 4-(3,4-dihydro-2H-pyran-4-yl)-3-(2-trityl-2H-tetrazol-5-yl)phenylamine (66e): the synthesis was performed according to Step 4 of Example 1, except that 1e was replaced with 66d. Yield: 87%.

Step 5: Synthesis of 5-(2-(3,4-dihydro-2H-pyran-4-yl)-5-isocyanatophenyl)-2-triphenyl-2H-tetrazole (66f): the synthesis was performed according to Step 5 of Example 1, except that 1f was replaced with 66e.

Step 6: Synthesis of 4-(tert-butyl)-N-(4-(3,4-dihydro-2H-pyran-4-yl)-3-(2-trityl-2H-tetrazol-5-yl)phenyl)piperidine-1-carboxamide (66g): the synthesis was performed according to Step 6 of Example 1, except that 1g was replaced with 66f, and 4-tert-butylcyclohexylamine was replaced with 4-tert-butylpiperidine hydrochloride. Yield through the two steps: 45%.

Step 7: Synthesis of 4-(tert-butyl)-N-(4-(3,4-dihydro-2H-pyran-4-yl)-3-(2H-tetrazol-5-yl)phenyl)piperidine-1-carboxamide (41): the synthesis was performed according to Step 7 of Example 1, except that 1h was replaced with 66g. Yield: 63%. ESI-MS: m/z =412 [M+H]⁺. ¹H NMR (500 MHz, DMSO-*d*₆) δ 16.31 (s, 1H), 8.58 (s, 1H), 8.26 (d, *J* = 1.4 Hz, 1H), 7.64 - 7.56 (m, 2H), 6.40 (dd, *J* = 11.0, 1.8 Hz, 1H), 5.68 (dd, *J* = 10.8, 6.2 Hz, 1H), 4.27 - 4.20 (m, 2H), 3.31 - 3.25 (m, 2H), 3.10 - 2.94 (m, 1H), 2.75 - 2.67 (m, 2H), 1.90 - 1.81 (m, 2H), 1.71 - 1.65 (m, 2H), 1.25 - 1.18 (m, 1H), 1.17 - 1.07 (m, 2H), 0.86 (s, 9H).

### Preparation Example 42

### Synthesis of 4-(tert-butyl)-N-(4-(6-(difluoromethyl)pyrid-3-yl)-3-(2H-tetrazol-5-yl)phenyl)piperidine-1-carboxamide (Compound 42):

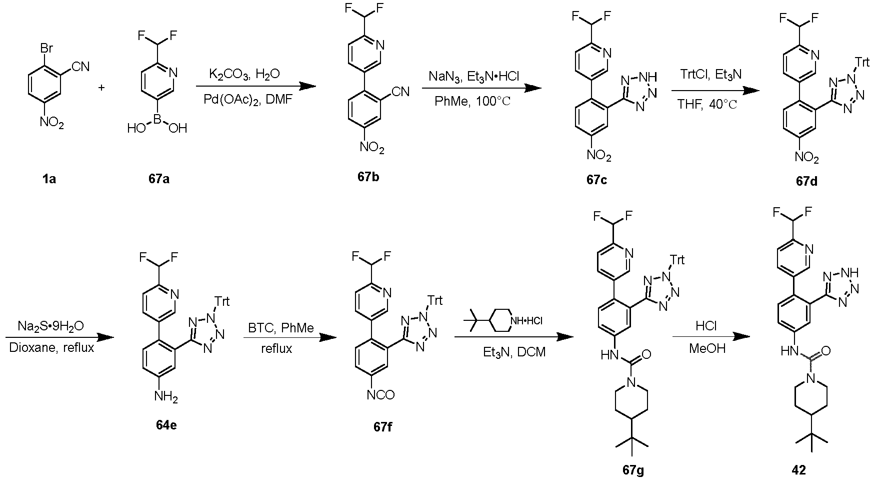

Step 1: Synthesis of 2-(6-(difluoromethyl)pyrid-3-yl)-5-nitrobenzonitrile (67b): the synthesis was performed according to Step 1 of Example 1, except that 1b was replaced with 67a. Yield: 81%.

Step 2: Synthesis of 2-(difluoromethyl)-5-(4-nitro-2-(2H-tetrazol-5-yl)phenyl)pyridine (67c): the synthesis was performed according to Step 2 of Example 1, except that 1c was replaced with 67b. Yield: 89%.

Step 3: Synthesis of 2-(difluoromethyl)-5-(4-nitro-2-(2-trityl-2H-tetrazol-5-yl)phenyl)pyridine (67d): the synthesis was performed according to Step 3 of Example 1, except that 1d was replaced with 67c. Yield: 86%.

Step 4: Synthesis of 4-(6-(difluoromethyl)pyrid-3-yl)-3-(2-trityl-2H-tetrazol-5-yl)phenylamine (67e): the synthesis was performed according to Step 4 of Example 1, except that 1e was replaced with 67d. Yield: 85%.

Step 5: Synthesis of 2-(difluoromethyl)-5-(4-isocyanato-2-(2-trityl-2H-tetrazol-5-yl)phenyl)pyridine (67f): the synthesis was performed according to Step 5 of Example 1, except that 1f was replaced with 67e.

Step 6: Synthesis of 4-(tert-butyl)-N-(4-(6-(difluoromethyl)pyrid-3-yl)-3-(2-trityl-2H-tetrazol-5-yl)phenyl)piperidine-1-carboxamide (67g): the synthesis was performed according to Step 6 of Example 1, except that 1g was replaced with 67f, and 4-tert-butylcyclohexylamine was replaced with 4-tert-butylpiperidine hydrochloride. Yield through the two steps: 43%.

Step 7: Synthesis of 4-(tert-butyl)-N-(4-(6-(difluoromethyl)pyrid-3-yl)-3-(2H-tetrazol-5-yl)phenyl)piperidine-1-carboxamide (42): the synthesis was performed according to Step 7 of Example 1, except that 1h was replaced with 67g. Yield: 65%.

ESI-MS: m/z =457 [M+H]⁺.¹H NMR (500 MHz, DMSO-*d*₆) δ 16.08 (s, 1H), 8.66 (d, *J=* 1.5 Hz, 1H), 8.56 (s, 1H), 8.44 (d, *J=* 1.5 Hz, 1H), 7.90 (d, *J=* 7.5 Hz, 1H), 7.77 (dd, *J* = 7.5, 0.7 Hz, 1H), 7.72 (dd, *J* = 7.5, 1.5 Hz, 1H), 7.55 (dd, *J =* 7.5, 1.5 Hz, 1H), 4.27 - 4.20 (m, 2H), 2.75 - 2.67 (m, 2H), 1.71 - 1.65 (m, 2H), 1.25 - 1.18 (m, 1H), 1.17 - 1.07 (m, 2H), 0.85 (s, 9H).

### Preparation Example 43

### Synthesis of cyclopentyl 4-(4-(tert-butyl)piperidine-1-formamido)-2-(2H-tetrazol-5-yl)benzoate (Compound 43):

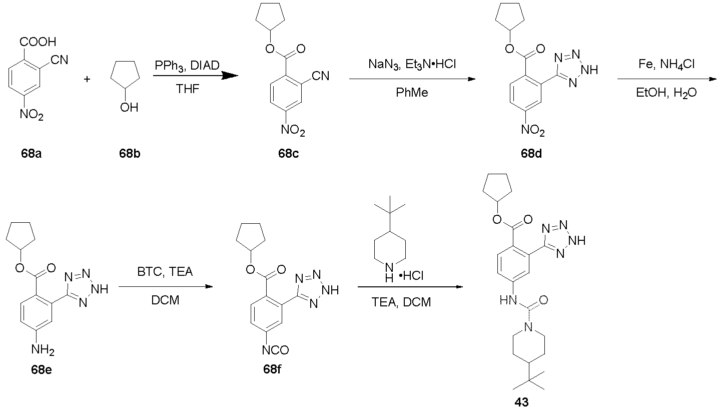

Step 1: Synthesis of cyclopentyl 2-cyano-4-nitrobenzoate (68c): the synthesis was performed according to Step 1 of Example 17, except that 43a was replaced with 68a, and 43b was replaced with 68b. Yield: 71%.

Step 2: Synthesis of cyclopentyl 4-nitro-2-(2H-tetrazol-5-yl)benzoate (68d): the synthesis was performed according to Step 2 of Example 1, except that 1c was replaced with 68c. Yield: 85%.

Step 3: Synthesis of cyclopentyl 4-amino-2-(2H-tetrazol-5-yl)benzoate (68e): the synthesis was performed according to Step 2 of Example 16, except that 40b was replaced with 68d. Yield: 89%.

Step 4: Synthesis of cyclopentyl 4-isocyanato-2-(2H-tetrazol-5-yl)benzoate (68f): the synthesis was performed according to Step 3 of Example 16, except that 40c was replaced with 43e.

Step 5: Synthesis of cyclopentyl 4-(4-(tert-butyl)piperidine-1-formamido)-2-(2H-tetrazol-5-yl)benzoate (43): the synthesis was performed according to Step 6 of Example 1, except that 1g was replaced with 68f, and 4-tert-butylcyclohexylamine was replaced with 4-tert-butylpiperidine hydrochloride. Yield through the two steps: 45%.

ESI-MS: m/z = 442 [M+H]⁺.¹H NMR (500 MHz, DMSO-*d*₆) δ 16.31 (s, 1H), 9.08 (s, 1H), 7.96 (d, *J* = 8.7 Hz, 1H), 7.88 (dd, *J* = 8.6, 2.3 Hz, 1H), 7.74 (d, *J* = 2.3 Hz, 1H), 4.78 - 4.66 (m, 1H), 4.24 (d, *J* = 13.1 Hz, 2H), 2.76 (t,*J* = 12.5 Hz, 2H), 1.69 (d, *J* = 12.2 Hz, 4H), 1.60 - 1.49 (m, 2H), 1.47 - 1.37 (m, 1H), 1.33 - 1.23 (m, 2H), 1.22 - 1.05 (m, 4H), 0.85 (s, 9H).

### Preparation Example 44

### Synthesis of tetrahydrofuran-3-yl 4-(4-(tert-butyl)piperidine-1-formamido)-2-(2H-tetrazol-5-yl)benzoate (Compound 44):

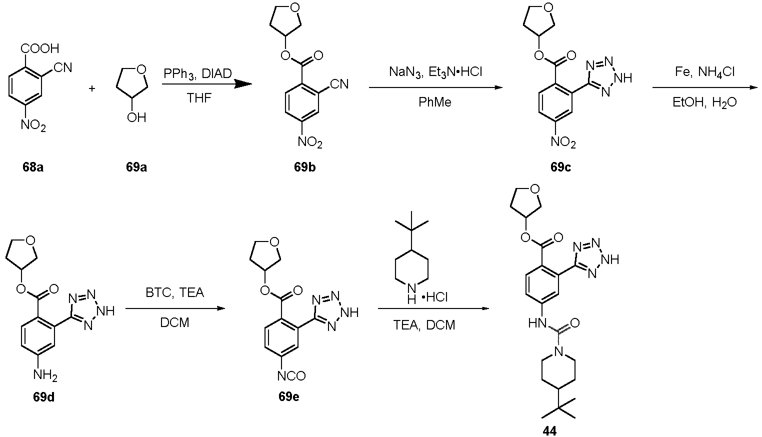

Step 1: Synthesis of tetrahydrofuran-3-yl 2-cyano-4-nitrobenzoate (69b): the synthesis was performed according to Step 1 of Example 17, except that 43a was replaced with 68a, and 43b was replaced with 69a. Yield: 74%.

Step 2: Synthesis of tetrahydrofuran-3-yl 4-nitro-2-(2H-tetrazol-5-yl)benzoate (69c): the synthesis was performed according to Step 2 of Example 1, except that 1c was replaced with 69b. Yield: 89%.

Step 3: Synthesis of tetrahydrofuran-3-yl 4-amino-2-(2H-tetrazol-5-yl)benzoate (69d): the synthesis was performed according to Step 2 of Example 16, except that 40b was replaced with 69c. Yield: 84%.

Step 4: Synthesis of tetrahydrofuran-3-yl 4-isocyanato-2-(2H-tetrazol-5-yl)benzoate (69e): the synthesis was performed according to Step 3 of Example 16, except that 40c was replaced with 69d.

Step 5: Synthesis of tetrahydrofuran-3-yl 4-(4-(tert-butyl)piperidine-1-formamido)-2-(2H-tetrazol-5-yl)benzoate (44): the synthesis was performed according to Step 6 of Example 1, except that 1g was replaced with 69e, and 4-tert-butylcyclohexylamine was replaced with 4-tert-butylpiperidine hydrochloride. Yield through the two steps: 43%.

ESI-MS: m/z = 444 [M+H]⁺.¹H NMR (500 MHz, DMSO-*d*₆) δ 16.31 (s, 1H), 8.64 (s, 1H), 8.37 (d, *J* = 1.5 Hz, 1H), 8.12 (d, *J* = 7.5 Hz, 1H), 7.75 (dd, *J* = 7.5, 1.6 Hz, 1H), 5.39 - 5.30 (m, 1H), 4.27 - 4.20 (m, 2H), 4.08 - 4.00 (m, 2H), 3.80 (td, *J* = 7.2, 1.9 Hz, 2H), 2.75 - 2.67 (m, 2H), 2.31 - 2.22(m, 2H), 1.71 - 1.65 (m, 2H), 1.25 - 1.18 (m, 1H), 1.17 - 1.07 (m, 2H), 0.86 (s, 9H).

### Preparation Example 45

### Synthesis of ethyl 4-(4-(tert-butyl)piperidine-1-formamido)-2-(2H-tetrazol-5-yl)benzoate (Compound 45):

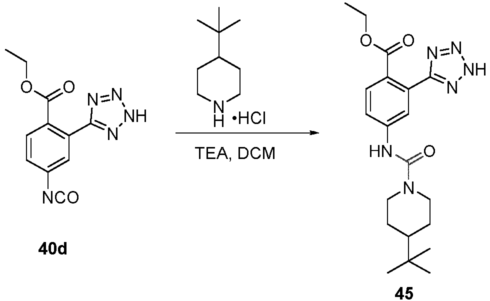

The synthesis was performed according to Step 6 of Example 1, except that 1g was replaced with 40d. Yield: 44%. ESI-MS: m/z = 359 [M+H]⁺. ¹H NMR (500 MHz, DMSO-*d*₆) δ 16.51 (s, 1H), 9.01 (s, 1H), 7.93 (d, *J* = 8.3 Hz, 1H), 7.81 (dd, *J* = 8.9, 2.3 Hz, 1H), 7.76 (d, *J =* 2.8 Hz, 1H), 4.24 (d, *J* = 13.1 Hz, 2H), 4.23 (d, *J* = 13.1 Hz, 2H), 4.09 (q, *J* = 7.1 Hz, 2H), 2.72 (t, *J =* 12.1 Hz, 2H), 1.68 (d, *J* = 11.4 Hz, 2H), 1.26 - 1.17 (m, 1H), 1.15 - 1.08 (m, 2H), 1.06 (t, *J* = 7.1 Hz, 3H), 0.85 (s, 9H).

### Preparation Example 46

### Synthesis of 4-(tert-butyl)-N-(4-(cyclopentylaminoformyl)-3-(2H-tetrazol-5-yl)phenyl)piperidine-1-carboxamide (Compound 46):

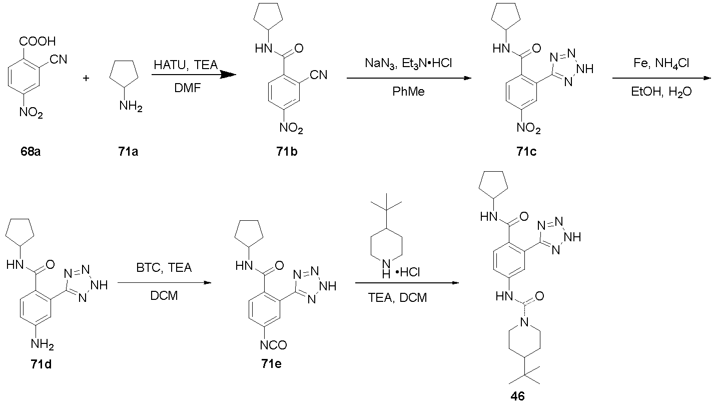

Step 1: Synthesis of 2-cyano-N-cyclopentyl-4-nitrobenzamide (71b): 0.50 g (2.60 mmol) and 0.22 g (2.60 mmol) of cyclopentylamine were dissolved in 10 mL of anhydrous DMF, and then 0.39 g (3.90 mmol) of triethylamine and 1.04 g (2.73 mmol) of HATU were added. The reaction was carried out at the room temperature for 1 h. After the reaction was completed, the mixture was diluted with water, and extracted with ethyl acetate. The organic layers were combined, washed with saturated brine, dried over anhydrous sodium sulfate, filtered, and concentrated under reduced pressure. The residue was purified by silica gel column chromatography (PE: EA = 3: 1, v/v) to obtain 0.50 g of 71b. Yield: 74%.

Step 2: Synthesis of N-cyclopentyl-4-nitro-2-(2H-tetrazol-5-yl)benzamide (71c): the synthesis was performed according to Step 2 of Example 1, except that 1c was replaced with 71b. Yield: 83%.

Step 3: Synthesis of 4-amino-N-cyclopentyl-2-(2H-tetrazol-5-yl)benzamide (71d): the synthesis was performed according to Step 2 of Example 16, except that 40b was replaced with 71c. Yield: 84%.

Step 4: Synthesis of N-cyclopentyl-4-isocyanato-2-(2H-tetrazol-5-yl)benzamide (71e): the synthesis was performed according to Step 3 of Example 16, except that 40c was replaced with 71d.

Step 5: Synthesis of 4-(tert-butyl)-N-(4-(cyclopentylaminoformyl)-3-(2H-tetrazol-5-yl)phenyl)piperidine-1-carboxamide (46): the synthesis was performed according to Step 6 of Example 1, except that 1g was replaced with 71e, and 4-tert-butylcyclohexylamine was replaced with 4-tert-butylpiperidine hydrochloride. Yield through the two steps: 43%.

ESI-MS: m/z = 441 [M+H]⁺.¹H NMR (500 MHz, DMSO-*d*₆) δ 16.01 (s, 1H), 8.65 (s, 1H), 8.37 (d, *J* = 1.5 Hz, 1H), 8.32 (d, *J* = 9.5 Hz, 1H), 8.14 (d, *J =* 7.5 Hz, 1H), 7.74 (dd, *J* = 7.5, 1.7 Hz, 1H), 4.76 - 4.65 (m, 1H), 4.22 (d,*J* = 13.1 Hz, 2H), 2.71 (t, *J* = 12.5 Hz, 2H), 1.68 (d, *J =* 12.2 Hz, 4H), 1.60 - 1.49 (m, 2H), 1.47 - 1.37 (m, 1H), 1.33 - 1.23 (m, 2H), 1.22 - 1.05 (m, 4H), 0.85 (s, 9H).

### Preparation Example 47

### Synthesis of 4-(tert-butyl)-N-(4-((5-methylpyridin-2-yl)aminoformyl)-3-(2H-tetrazol-5-yl)phenyl)piperidine-1-carboxamide (Compound 47):

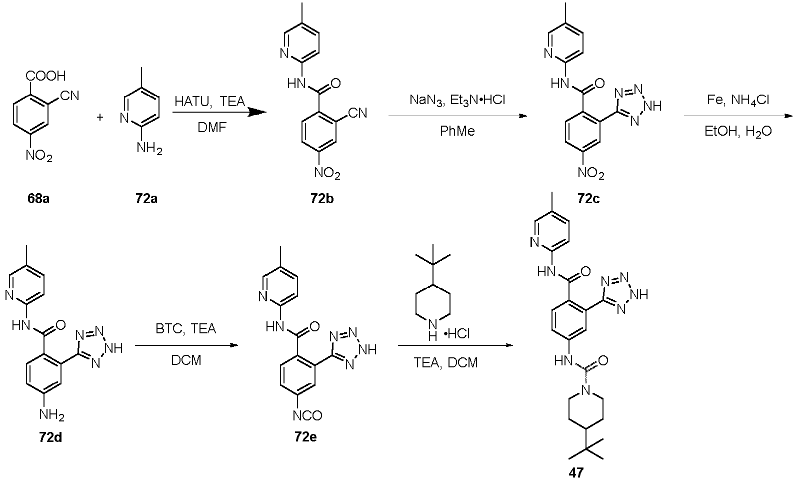

Step 1: Synthesis of 2-cyano-N-(5-methylpyridin-2-yl)-4-nitrobenzamide (72b): the synthesis was performed according to Step 1 of Example 47, except that 71a was replaced with 72a. Yield: 80%.

Step 2: Synthesis of N-(5-methylpyridin-2-yl)-4-nitro-2-(2H-tetrazol-5-yl)benzamide (72c): the synthesis was performed according to Step 2 of Example 1, except that 1c was replaced with 72b. Yield: 83%.

Step 3: Synthesis of 4-amino-N-(5-methylpyridin-2-yl)-2-(2H-tetrazol-5-yl)benzamide (72d): the synthesis was performed according to Step 2 of Example 16, except that 40b was replaced with 72c. Yield: 86%.

Step 4: Synthesis of 4-isocyanato-N-(5-methylpyridin-2-yl)-2-(2H-tetrazol-5-yl)benzamide (72e): the synthesis was performed according to Step 3 of Example 16, except that 40c was replaced with 72d.

Step 5: Synthesis of 4-(tert-butyl)-N-(4-((5-methylpyridin-2-yl)aminoformyl)-3-(2H-tetrazol-5-yl)phenyl)piperidine-1-carboxamide (47): the synthesis was performed according to Step 6 of Example 1, except that 1g was replaced with 72e, and 4-tert-butylcyclohexylamine was replaced with 4-tert-butylpiperidine hydrochloride. Yield through the two steps: 46%.

ESI-MS: m/z = 441 [M+H]⁺.¹H NMR (500 MHz, DMSO-*d*₆) δ 16.01 (s, 1H), 8.64 (s, 1H), 8.35 (d, *J* = 1.5 Hz, 1H), 8.16 (d, *J* = 7.5 Hz, 1H), 8.07 (dd, *J* = 1.5, 0.7 Hz, 1H), 7.75 - 7.67 (m, 2H), 7.65 - 7.59 (m, 1H), 4.27 - 4.20 (m, 2H), 2.75 - 2.67 (m, 2H), 2.56 (s, 1H), 1.71 - 1.65 (m, 2H), 1.25 - 1.18 (m, 1H), 1.17 - 1.07 (m, 2H), 0.86 (s, 9H).

### Preparation Example 48

### Synthesis of 4-(tert-butyl)-N-(4-(5-methoxypyrid-3-yl)-3-(2H-tetrazol-5-yl)phenyl)piperidine-1-carboxamide (Compound 48):

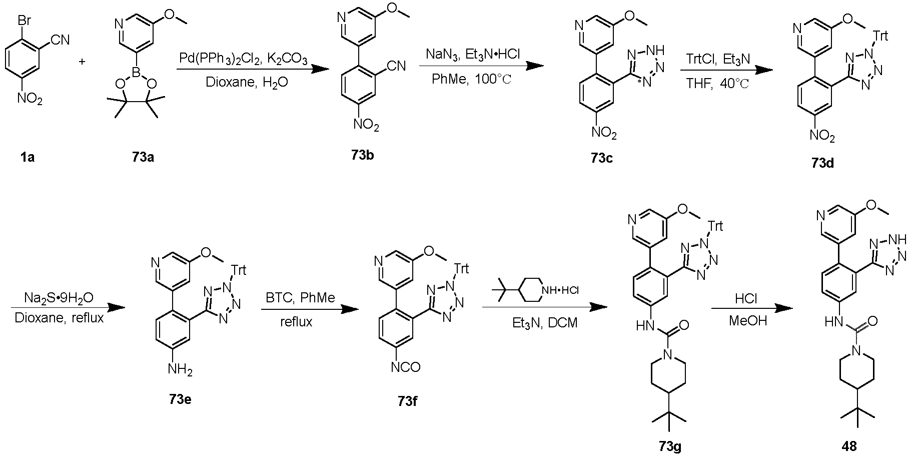

Step 1: Synthesis of 2-(5-methoxypyrid-3-yl)-5-nitrobenzonitrile (73b): the synthesis was performed according to Step 1 of Example 1, and 1b was replaced with 73a. Yield: 83%.

Step 2: Synthesis of 3-methoxy-5-(4-nitro-2-(2H-tetrazol-5-yl)phenyl)pyridine (73c): the synthesis was performed according to Step 2 of Example 1, except that 1c was replaced with 73b. Yield: 87%.

Step 3: Synthesis of 3-methoxy-5-(4-nitro-2-(2-trityl-2H-tetrazol-5-yl)phenyl)pyridine (73d): the synthesis was performed according to Step 3 of Example 1, except that 1d was replaced with 73c. Yield: 86%.

Step 4: Synthesis of 4-(5-methoxypyrid-3-yl)-3-(2-trityl-2H-tetrazol-5-yl)phenylamine (73e): the synthesis was performed according to Step 4 of Example 1, except that 1e was replaced with 73d. Yield: 79%.

Step 5: Synthesis of 3-(4-isocyanato-2-(2-trityl-2H-tetrazol-5-yl)phenyl)-5-methoxypyridine (73f): the synthesis was performed according to Step 5 of Example 1, except that 1f was replaced with 73e.

Step 6: Synthesis of 4-(tert-butyl)-N-(4-(5-methoxypyrid-3-yl)-3-(2-trityl-2H-tetrazol-5-yl)phenyl)piperidine-1-carboxamide (73g): the synthesis was performed according to Step 6 of Example 1, except that 1g was replaced with 73f, and 4-tert-butylcyclohexylamine was replaced with 4-tert-butylpiperidine hydrochloride. Yield through the two steps: 41%.

Step 7: Synthesis of 4-(tert-butyl)-N-(4-(5-methoxypyrid-3-yl)-3-(2H-tetrazol-5-yl)phenyl)piperidine-1-carboxamide (48): the synthesis was performed according to Step 7 of Example 1, except that 1h was replaced with 73g. Yield: 66%.

ESI-MS: m/z = 437 [M+H]⁺. ¹H NMR (400 MHz, DMSO-*d*₆) δ 16.33 (s, 1H), δ 8.84 (s, 1H), 7.93 (d, *J* = 2.9 Hz, 1H), 7.85 (d, *J* = 2.7 Hz, 1H), 7.78 (dd, *J =* 8.1, 2.3 Hz, 1H), 7.43 (d, *J* = 8.5 Hz, 1H), 7.29 (dd, *J* = 8.6, 2.6 Hz, 1H), 6.70 (d, *J* = 8.6 Hz, 1H), 4.33 - 4.19 (m, 5H), 2.75 (t, *J* = 11.8 Hz, 2H), 1.59 (d, *J* = 11.5 Hz, 2H), 1.24 - 1.17 (m, 1H), 1.16 - 1.09 (m, 2H), 0.86 (s, 9H).

### Preparation Example 49

### Synthesis of 4-(tert-butyl)-N-(4-(6-isopropoxypyrid-3-yl)-3-(2H-tetrazol-5-yl)phenyl)piperidine-1-carboxamide (Compound 49):

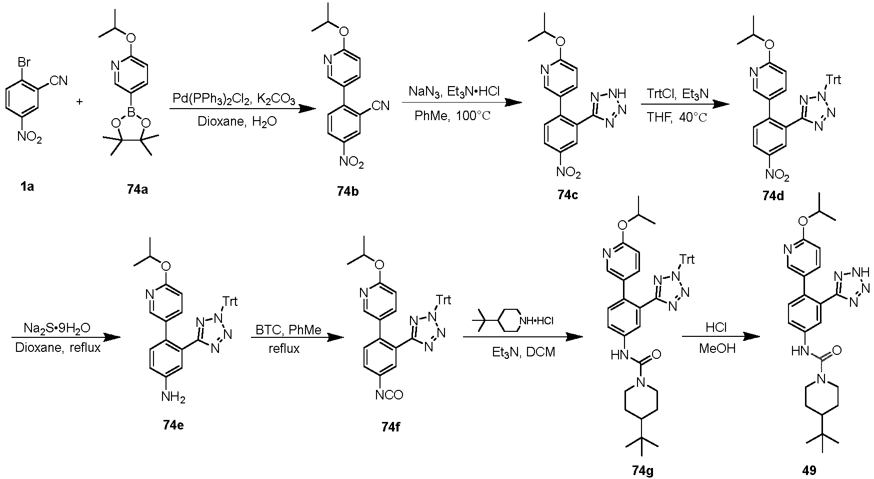

Step 1: Synthesis of 2-(6-isopropoxypyrid-3-yl)-5-nitrobenzonitrile (74b): the synthesis was performed according to Step 1 of Example 1, and 24a was replaced with 74a. Yield: 86%.

Step 2: Synthesis of 2-isopropoxy-5-(4-nitro-2-(2H-tetrazol-5-yl)phenyl)pyridine (74c): the synthesis was performed according to Step 2 of Example 1, except that 1c was replaced with 74b. Yield: 91%.

Step 3: Synthesis of 2-isopropoxy-5-(4-nitro-2-(2-trityl-2H-tetrazol-5-yl)phenyl)pyridine (74d): the synthesis was performed according to Step 3 of Example 1, except that 1d was replaced with 74c. Yield: 85%.

Step 4: Synthesis of 4-(6-isopropoxypyrid-3-yl)-3-(2-trityl-2H-tetrazol-5-yl)phenylamine (74e): the synthesis was performed according to Step 4 of Example 1, except that 1e was replaced with 74d. Yield: 82%.

Step 5: Synthesis of 5-(4-isocyanato-2-(2-trityl-2H-tetrazol-5-yl)phenyl)-2-isopropoxypyridine (74f): the synthesis was performed according to Step 5 of Example 1, except that 1f was replaced with 74e.

Step 6: Synthesis of 4-(tert-butyl)-N-(4-(6-isopropoxypyrid-3-yl)-3-(2-trityl-2H-tetrazol-5-yl)phenyl)piperidine-1-carboxamide (74g): the synthesis was performed according to Step 6 of Example 1, except that 1g was replaced with 74f, and 4-tert-butylcyclohexylamine was replaced with 4-tert-butylpiperidine hydrochloride. Yield through the two steps: 42%.

Step 7: Synthesis of 4-(tert-butyl)-N-(4-(6-isopropoxypyrid-3-yl)-3-(2H-tetrazol-5-yl)phenyl)piperidine-1-carboxamide (49): the synthesis was performed according to Step 7 of Example 1, except that 1h was replaced with 74g. Yield: 67%.

ESI-MS: m/z = 465 [M+H]⁺. ¹H NMR (400 MHz, DMSO-*d*₆) δ 16.33 (s, 1H), 8.56 (s, 1H), 8.46 - 8.42 (m, 2H), 8.09 (dd, *J* = 7.5, 1.5 Hz, 1H), 7.89 (d, *J* = 7.5 Hz, 1H), 7.73 (dd, *J =* 7.5, 1.5 Hz, 1H), 7.02 (d, *J =* 7.5 Hz, 1H), 4.88 - 4.79 (m, 1H), 4.27 - 4.20 (m, 2H), 2.75 - 2.67 (m, 2H), 1.71 - 1.65 (m, 2H), 1.35 (d, *J =* 6.8 Hz, 6H), 1.25 - 1.18 (m, 1H), 1.17 - 1.07 (m, 2H), 0.86 (s, 9H).

### Preparation Example 50

### Synthesis of 4-(tert-butyl)-N-(3-fluoro-4-(5-methoxypyrid-3-yl)-5-(2H-tetrazol-5-yl)phenyl)piperidine-1-carboxamide (Compound 50):

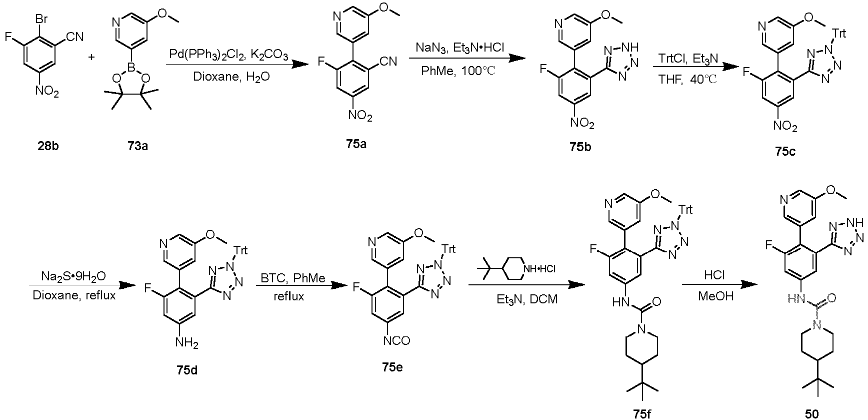

Step 1: Synthesis of 3-fluoro-2-(5-methoxypyrid-3-yl)-5-nitrobenzonitrile (75a): the synthesis was performed according to Step 1 of Example 1, except that 1a was replaced with 28a, and 1b was replaced with 73a. Yield: 74%.

Step 2: Synthesis of 3-(2-fluoro-4-nitro-6-(2H-tetrazol-5-yl)phenyl)-5-methoxypyridine (75b): the synthesis was performed according to Step 2 of Example 1, except that 1c was replaced with 75a. Yield: 87%.

Step 3: Synthesis of 3-(2-fluoro-4-nitro-6-(2-trityl-2H-tetrazol-5-yl)phenyl)-5-methoxypyridine (75c): the synthesis was performed according to Step 3 of Example 1, except that 1d was replaced with 75b. Yield: 85%.

Step 4: Synthesis of 3-fluoro-4-(5-methoxypyrid-3-yl)-5-(2-trityl-2H-tetrazol-5-yl)phenylamine (75d): the synthesis was performed according to Step 4 of Example 1, except that 1e was replaced with 75c. Yield: 81%.

Step 5: Synthesis of 3-(2-fluoro-4-isocyanato-6-(2-trityl-2H-tetrazol-5-yl)phenyl)-5-methoxypyridine (75e): the synthesis was performed according to Step 5 of Example 1, except that 1f was replaced with 75d.

Step 6: Synthesis of 4-(tert-butyl)-N-(3-fluoro-4-(5-methoxypyrid-3-yl)-5-(2-trinitro-2H-tetrazol-5-yl)phenyl)piperidine-1-carboxamide (75f): the synthesis was performed according to Step 6 of Example 1, except that 1g was replaced with 75e, and 4-tert-butylcyclohexylamine was replaced with 4-tert-butylpiperidine hydrochloride. Yield through the two steps: 41%.

Step 7: Synthesis of 4-(tert-butyl)-N-(3-fluoro-4-(5-methoxypyrid-3-yl)-5-(2H-tetrazol-5-yl)phenyl)piperidine-1-carboxamide (50): the synthesis was performed according to Step 7 of Example 1, except that 1h was replaced with 75f. Yield: 61%.

ESI-MS: m/z = 455 [M+H]⁺. ¹H NMR (400 MHz, DMSO-*d*₆) δ 16.23 (s, 1H), δ 8.84 (s, 1H), 7.85 (d, *J =* 2.7 Hz, 1H), 7.78 (dd, *J =* 8.1, 2.3 Hz, 1H), 7.43 (d, *J* = 8.5 Hz, 1H), 7.29 (dd, *J* = 8.6, 2.6 Hz, 1H), 6.70 (d, *J =* 8.6 Hz, 1H), 4.33 - 4.19 (m, 5H), 2.75 (t, *J =* 11.8 Hz, 2H), 1.65 (d, *J =* 11.5 Hz, 2H), 1.27 - 1.19 (m, 1H), 1.16 - 1.09 (m, 2H), 0.86 (s, 9H).

### Preparation Example 51

### Synthesis of 4-(tert-butyl)-N-(4-(6-methylpyrid-3-yl)-3-(2H-tetrazol-5-yl)phenyl)piperidine-1-carboxamide (Compound 51):

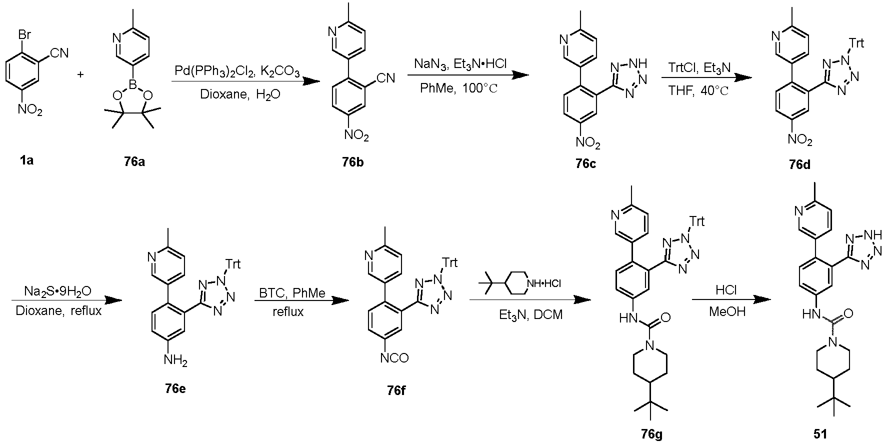

Step 1: Synthesis of 2-(6-methylpyrid-3-yl)-5-nitrobenzonitrile (76b): the synthesis was performed according to Step 1 of Example 1, except that 1b was replaced with 76a. Yield: 86%.

Step 2: Synthesis of 2-methyl-5-(4-nitro-2-(2H-tetrazol-5-yl)phenyl)pyridine (76c): the synthesis was performed according to Step 2 of Example 1, except that 1c was replaced with 76b. Yield: 88%.

Step 3: Synthesis of 2-methyl-5-(4-nitro-2-(2-trityl-2H-tetrazol-5-yl)phenyl)pyridine (76d): the synthesis was performed according to Step 3 of Example 1, except that 1d was replaced with 76c. Yield: 91%.

Step 4: Synthesis of 4-(6-methylpyrid-3-yl)-3-(2-trityl-2H-tetrazol-5-yl)phenylamine (76e): the synthesis was performed according to Step 4 of Example 1, except that 1e was replaced with 76d. Yield: 83%.

Step 5: Synthesis of 5-(4-isocyanato-2-(2-trityl-2H-tetrazol-5-yl)phenyl)-2-methylpyridine (76f): the synthesis was performed according to Step 5 of Example 1, except that 1f was replaced with 76e.

Step 6: Synthesis of 4-(tert-butyl)-N-(4-(6-methylpyrid-3-yl)-3-(2-trityl-2H-tetrazol-5-yl)phenyl)piperidine-1-carboxamide (76g): the synthesis was performed according to Step 6 of Example 1, except that 1g was replaced with 76f, and 4-tert-butylcyclohexylamine was replaced with 4-tert-butylpiperidine hydrochloride. Yield through the two steps: 48%.

Step 7: Synthesis of 4-(tert-butyl)-N-(4-(6-methylpyrid-3-yl)-3-(2H-tetrazol-5-yl)phenyl)piperidine-1-carboxamide (51): the synthesis was performed according to Step 7 of Example 1, except that 1h was replaced with 76g. Yield: 58%.

ESI-MS: m/z = 421 [M+H]⁺. ¹H NMR (400 MHz, DMSO-*d*₆) δ 16.33 (s, 1H), δ 8.84 (s, 1H), 7.89 (d, *J* = 2.6 Hz, 1H), 7.86 (d, *J =* 2.3 Hz, 1H), 7.77 (dd, *J =* 8.5, 2.3 Hz, 1H), 7.43 (d, *J* = 8.5 Hz, 1H), 7.29 (dd, *J* = 8.6, 2.6 Hz, 1H), 6.70 (d, *J =* 8.6 Hz, 1H), 4.33 - 4.19 (m, 2H), 2.71 (t, *J =* 11.8 Hz, 2H), 2.44 (s, 1H), 1.69 (d*, J =* 11.5 Hz, 2H), 1.19 - 1.15 (m, 1H), 1.14 - 1.08 (m, 2H), 0.86 (s, 9H).

### Preparation Example 52

### Synthesis of 4-(tert-butyl)-N-(4-(2-methylpyridin-4-yl)-3-(2H-tetrazol-5-yl)phenyl)piperidine-1-carboxamide (Compound 52):

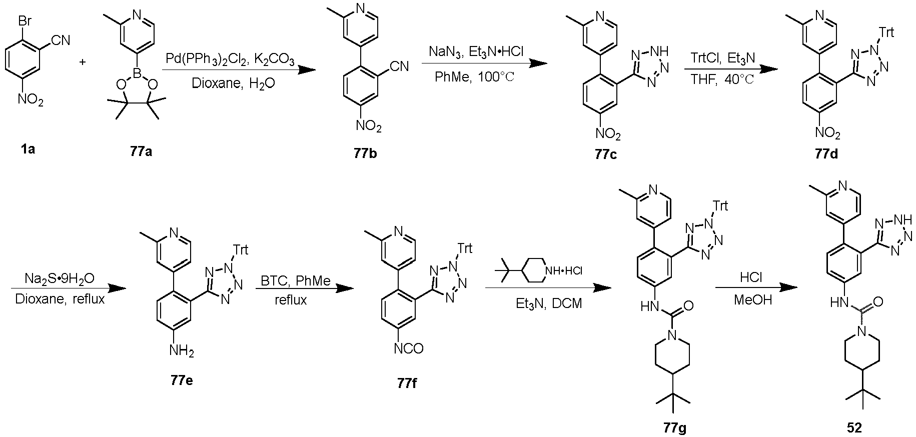

Step 1: Synthesis of 2-(2-methylpyridin-4-yl)-5-nitrobenzonitrile (77b): the synthesis was performed according to Step 1 of Example 1, except that 1b was replaced with 77a. Yield: 86%.

Step 2: Synthesis of 2-methyl-4-(4-nitro-2-(2H-tetrazol-5-yl)phenyl)pyridine (77c): the synthesis was performed according to Step 2 of Example 1, except that 1c was replaced with 77b. Yield: 90%.

Step 3: Synthesis of 2-methyl-4-(4-nitro-2-(2-trityl-2H-tetrazol-5-yl)phenyl)pyridine (77d): the synthesis was performed according to Step 3 of Example 1, except that 1d was replaced with 77c. Yield: 89%.

Step 4: Synthesis of 4-(2-methylpyridin-4-yl)-3-(2-trityl-2H-tetrazol-5-yl)phenylamine (77e): the synthesis was performed according to Step 4 of Example 1, except that 1e was replaced with 77d. Yield: 83%.

Step 5: Synthesis of 4-(4-isocyanato-2-(2-trityl-2H-tetrazol-5-yl)phenyl)-2-methylpyridine (77f): the synthesis was performed according to Step 5 of Example 1, except that 1f was replaced with 77e.

Step 6: Synthesis of 4-(tert-butyl)-N-(4-(2-methylpyridin-4-yl)-3-(2-trityl-2H-tetrazol-5-yl)phenyl)piperidine-1-carboxamide (77g): the synthesis was performed according to Step 6 of Example 1, except that 1g was replaced with 77f, and 4-tert-butylcyclohexylamine was replaced with 4-tert-butylpiperidine hydrochloride. Yield through the two steps: 48%.

Step 7: Synthesis of 4-(tert-butyl)-N-(4-(2-methylpyridin-4-yl)-3-(2H-tetrazol-5-yl)phenyl)piperidine-1-carboxamide (52): the synthesis was performed according to Step 7 of Example 1, except that 1h was replaced with 77g. Yield: 71%.

ESI-MS: m/z = 421 [M+H]⁺. ¹H NMR (400 MHz, DMSO-*d*₆) δ 16.23 (s, 1H), δ 8.89 (s, 1H), 7.99 (d, *J =* 2.6 Hz, 1H), 7.83 (d, *J =* 2.5 Hz, 1H), 7.79 (dd, *J = 8.5, 2.3* Hz, 1H), 7.43 (d*, J =* 8.5 Hz, 1H), 7.29 (dd, *J* = 8.6, 2.6 Hz, 1H), 6.70 (d, *J =* 8.6 Hz, 1H), 4.35 - 4.21 (m, 2H), 2.74 (t, *J =* 11.8 Hz, 2H), 2.51 (s, 1H), 1.65 (d, *J =* 11.5 Hz, 2H), 1.24 - 1.17 (m, 1H), 1.15 - 1.09 (m, 2H), 0.86 (s, 9H).

### Preparation Example 53

### Synthesis of 4-(tert-butyl)-N-(4-(cyclopentylaminoformyl)-3-fluoro-5-(2H-tetrazol-5-yl)phenyl)piperidine-1-carboxamide (Compound 53):

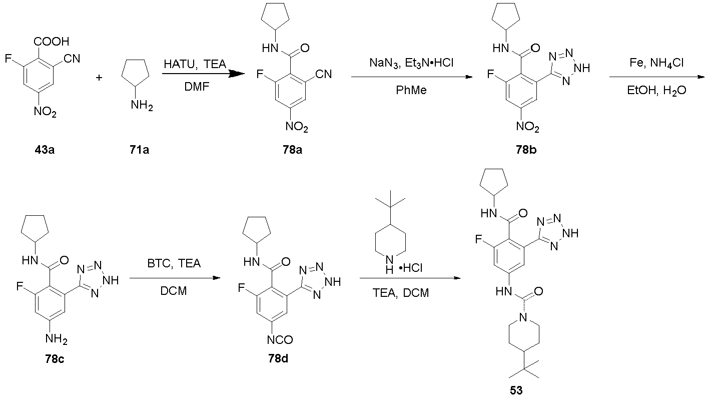

Step 1: Synthesis of 2-cyano-N-cyclopentyl-6-fluoro-4-nitrobenzamide (78a): the synthesis was performed according to Step 1 of Example 46, except that 68a was replaced with 43a. Yield: 73%.

Step 2: Synthesis of N-cyclopentyl-2-fluoro-4-nitro-6-(2H-tetrazol-5-yl)benzamide (78b): the synthesis was performed according to Step 2 of Example 1, except that 1c was replaced with 78a. Yield: 93%.

Step 3: Synthesis of 4-amino-N-cyclopentyl-2-fluoro-6-(2H-tetrazol-5-yl)benzamide (78c): the synthesis was performed according to Step 2 of Example 16, except that 40b was replaced with 78b. Yield: 82%.

Step 4: Synthesis of N-cyclopentyl-2-fluoro-4-isocyanato-6- (2H-tetrazol-5-yl)benzamide (78d): the synthesis was performed according to Step 3 of Example 16, except that 40c was replaced with 78c.

Step 5: Synthesis of 4-(tert-butyl)-N-(4-(cyclopentylaminoformyl)-3-fluoro-5-(2H-tetrazol-5-yl)phenyl)piperidine-1-carboxamide (53): the synthesis was performed according to Step 6 of Example 1, except that 1g was replaced with 78d, and 4-tert-butylcyclohexylamine was replaced with 4-tert-butylpiperidine hydrochloride. Yield through the two steps: 50%.

ESI-MS: m/z = 459 [M+H]⁺.¹H NMR (500 MHz, DMSO-*d*₆) δ 16.21 (s, 1H), 8.65 (s, 1H), 8.37 (d, *J =* 1.5 Hz, 1H), 8.14 (d, *J =* 7.5 Hz, 1H), 7.74 (dd, *J* = 7.5, 1.7 Hz, 1H), 4.76 - 4.65 (m, 1H), 4.22 (d, *J =* 13.1 Hz, 2H), 2.75 (t, *J =* 12.5 Hz, 2H), 1.69 (d, *J =* 12.2 Hz, 4H), 1.63 - 1.52 (m, 2H), 1.48 - 1.39 (m, 1H), 1.34 - 1.25 (m, 2H), 1.23 - 1.09 (m, 4H), 0.85 (s, 9H).

### Preparation Example 54

### Synthesis of 4-(tert-butyl)-N-(3-fluoro-4-((5-methylpyridin-2-yl)aminoformyl)-5-(2H-tetrazol-5-yl)phenyl)piperidine-1-carboxamide (Compound 54):

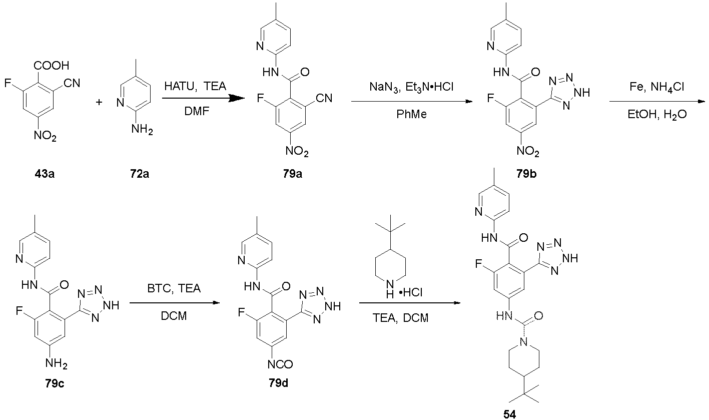

Step 1: Synthesis of 2-cyano-6-fluoro-N-(5-methylpyridin-2-yl)-4-nitrobenzamide (79a): the synthesis was performed according to Step 1 of Example 46, except that 68a was replaced with 43a, and 71a was replaced with 72a. Yield: 69%.

Step 2: Synthesis of 2-fluoro-N-(5-methylpyridin-2-yl)-4-nitro-6-(2H-tetrazol-5-yl)benzamide (79b): the synthesis was performed according to Step 2 of Example 16, except that 1c was replaced with 79a. Yield: 90%.

Step 3: Synthesis of 4-amino-2-fluoro-N-(5-methylpyridin-2-yl)-6-(2H-tetrazol-5-yl)benzamide (79c): the synthesis was performed according to Step 2 of Example 16, except that 40b was replaced with 79b. Yield: 83%.

Step 4: Synthesis of 2-fluoro-4-isocyanato-N-(5-methylpyridin-2-yl)-6-(2H-tetrazol-5-yl)benzamide (79d): the synthesis was performed according to Step 3 of Example 16, except that 40c was replaced with 79c.

Step 5: Synthesis of 4-(tert-butyl)-N-(3-fluoro-4-((5-methylpyridin-2-yl)aminoformyl)-5-(2H-tetrazol-5-yl)phenyl)piperidine-1-carboxamide (54): the synthesis was performed according to Step 6 of Example 1, except that 1g was replaced with 79d, and 4-tert-butylcyclohexylamine was replaced with 4-tert-butylpiperidine hydrochloride. Yield through the two steps: 47%.

ESI-MS: m/z = 441 [M+H]⁺.¹H NMR (500 MHz, DMSO-*d*₆) δ 16.23 (s, 1H), 8.69 (s, 1H), 8.34 (d, *J =* 1.5 Hz, 1H), 8.01 (dd, *J =* 1.5, 0.7 Hz, 1H), 7.78 - 7.69 (m, 2H), 7.64 - 7.55 (m, 1H), 4.27 - 4.20 (m, 2H), 2.79 - 2.68 (m, 2H), 2.56 (s, 1H), 1.71 - 1.65 (m, 2H), 1.25 - 1.18 (m, 1H), 1.18 - 1.07 (m, 2H), 0.86 (s, 9H).

### Preparation Example 55

### Synthesis of 4-(tert-butyl)-N-(3-fluoro-4-(6-methylpyrid-3-yl)-5-(2H-tetrazol-5-yl)phenyl)piperidine-1-carboxamide (Compound 55):

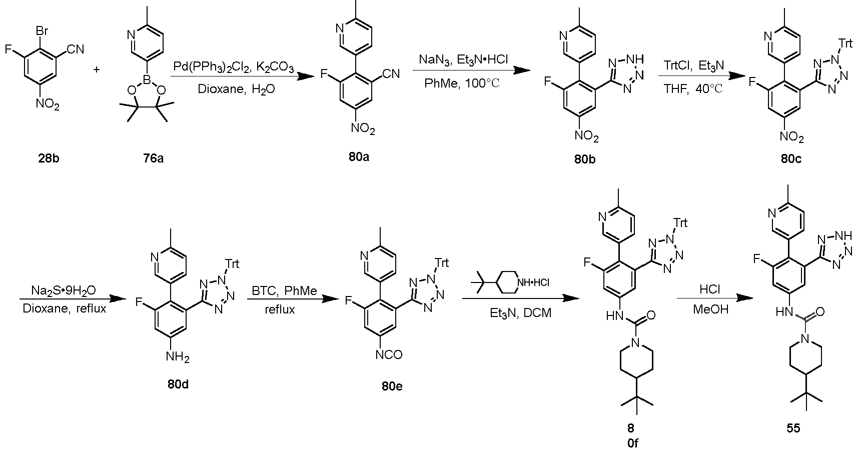

Step 1: Synthesis of 3-fluoro-2-(6-methylpyrid-3-yl)-5-nitrobenzonitrile (80a): the synthesis was performed according to Step 1 of Example 1, except that 1a was replaced with 28b, 1b was replaced with 76a. Yield: 87%.

Step 2: Synthesis of 5-(2-fluoro-4-nitro-6-(2H-tetrazol-5-yl)phenyl)-2-methylpyridine (80b): the synthesis was performed according to Step 2 of Example 1, except that 1c was replaced with 80a. Yield: 91%.

Step 3: Synthesis of 5-(2-fluoro-4-nitro-6-(2-trityl-2H-tetrazol-5-yl)phenyl)-2-methylpyridine (80c): the synthesis was performed according to Step 3 of Example 1, except that 1d was replaced with 80b. Yield: 93%.

Step 4: Synthesis of 3-fluoro-4-(6-methylpyrid-3-yl)-5-(2-trityl-2H-tetrazol-5-yl)phenylamine (80d): the synthesis was performed according to Step 4 of Example 1, except that 1e was replaced with 80c. Yield: 48%.

Step 5: Synthesis of 5-(2-fluoro-4-isocyanato-6-(2-trityl-2H-tetrazol-5-yl)phenyl)-2-methylpyridine (80e): the synthesis was performed according to Step 5 of Example 1, except that 1f was replaced with 80d.

Step 6: Synthesis of 4-(tert-butyl)-N-(3-fluoro-4-(6-methylpyrid-3-yl)-5-(2-trityl-2H-tetrazol-5-yl)phenyl)piperidine-1-carboxamide (80f): the synthesis was performed according to Step 6 of Example 1, except that 1g was replaced with 80e, and 4-tert-butylcyclohexylamine was replaced with 4-tert-butylpiperidine hydrochloride. Yield through the two steps: 46%.

Step 7: Synthesis of 4-(tert-butyl)-N-(3-fluoro-4-(6-methylpyrid-3-yl)-5-(2H-tetrazol-5-yl)phenyl)piperidine-1-carboxamide (55): the synthesis was performed according to Step 7 of Example 1, except that 1h was replaced with 80f. Yield: 61%.

ESI-MS: m/z = 439 [M+H]⁺. ¹H NMR (400 MHz, DMSO-*d*₆) δ 16.31 (s, 1H), δ 8.81 (s, 1H), 7.89 (d, *J =* 2.3 Hz, 1H), 7.67 (dd, *J =* 8.5, 2.3 Hz, 1H), 7.41 (d, *J =* 8.5 Hz, 1H), 7.28 (dd, *J =* 8.6, 2.6 Hz, 1H), 6.71 (d, *J =* 8.6 Hz, 1H), 4.33 - 4.19 (m, 2H), 2.71 (t, *J =* 11.8 Hz, 2H), 2.44 (s, 1H), 1.69 (d, *J =* 11.5 Hz, 2H), 1.25 - 1.19 (m, 1H), 1.14 - 1.11 (m, 2H), 0.86 (s, 9H).

### Preparation Example 56

### Synthesis of 4-(tert-butyl)-N-(3-fluoro-4-(6-isopropoxypyrid-3-yl)-5-(2H-tetrazol-5-yl)phenyl)piperidine-1-carboxamide (Compound 56):

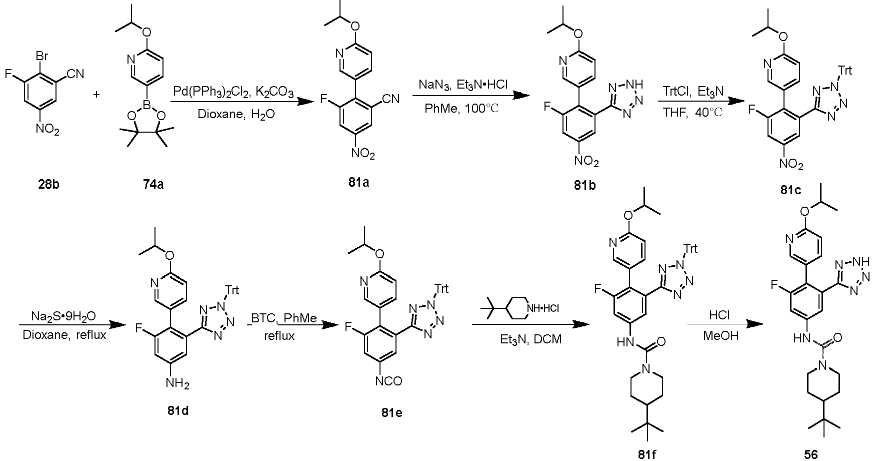

Step 1: Synthesis of 3-fluoro-2-(6-isopropoxypyrid-3-yl)-5-nitrobenzonitrile (81a): the synthesis was performed according to Step 1 of Example 1, except that 1a was replaced with 28b, and 1b was replaced with 74a. Yield: 82%.

Step 2: Synthesis of 5-(2-fluoro-4-nitro-6-(2H-tetrazol-5-yl)phenyl)-2-isopropoxypyridine (81b): the synthesis was performed according to Step 2 of Example 1, except that 1c was replaced with 81a. Yield: 91%.

Step 3: Synthesis of 5-(2-fluoro-4-nitro-6-(2-trityl-2H-tetrazol-5-yl)phenyl)-2-isopropoxypyridine (81c): the synthesis was performed according to Step 3 of Example 1, except that 1d was replaced with 81b. Yield: 86%.

Step 4: Synthesis of 3-fluoro-4-(6-isopropoxypyrid-3-yl)-5-(2-trinitro-2H-tetrazol-5-yl)phenylamine (81d): the synthesis was performed according to Step 4 of Example 1, except that 1e was replaced with 81c. Yield: 81%.

Step 5: Synthesis of 5-(2-fluoro-4-isocyanato-6-(2-trityl-2H-tetrazol-5-yl)phenyl)-2-isopropoxypyridine (81e): the synthesis was performed according to Step 5 of Example 1, except that 1g was replaced with 81d.

Step 6: Synthesis of 4-(tert-butyl)-N-(3-fluoro-4-(6-isopropoxypyrid-3-yl)-5-(2-trityl-2H-tetrazol-5-yl)phenyl)piperidine-1-carboxamide (81f): the synthesis was performed according to Step 6 of Example 1, except that 1g was replaced with 81e, and 4-tert-butylcyclohexylamine was replaced with 4-tert-butylpiperidine hydrochloride. Yield through the two steps: 41%.

Step 7: Synthesis of 4-(tert-butyl)-N-(3-fluoro-4-(6-isopropoxypyrid-3-yl)-5-(2H-tetrazol-5-yl)phenyl)piperidine-1-carboxamide (56): the synthesis was performed according to Step 7 of Example 1, except that 1h was replaced with 81f. Yield: 65%.

ESI-MS: m/z = 483 [M+H]⁺. ¹H NMR (400 MHz, DMSO-*d*₆) δ 16.33 (s, 1H), 8.56 (s, 1H), 8.46 - 8.42 (m, 2H), 7.89 (d, *J =* 7.5 Hz, 1H), 7.73 (dd, *J* = 7.5, 1.5 Hz, 1H), 7.02 (d, *J =* 7.5 Hz, 1H), 4.88 - 4.79 (m, 1H), 4.27 - 4.20 (m, 2H), 2.73 - 2.67 (m, 2H), 1.78 - 1.66 (m, 2H), 1.38 (d, *J =* 6.8 Hz, 6H), 1.29 - 1.19 (m, 1H), 1.16 - 1.07 (m, 2H), 0.86 (s, 9H).

### Preparation Example 57

### Synthesis of 4-(tert-butyl)-N-(4-(4-(tert-butyl)-1H-imidazol-1-yl)-3-fluoro-5-(2H-tetrazol-5-yl)phenyl)piperidine-1-carboxamide (Compound 57):

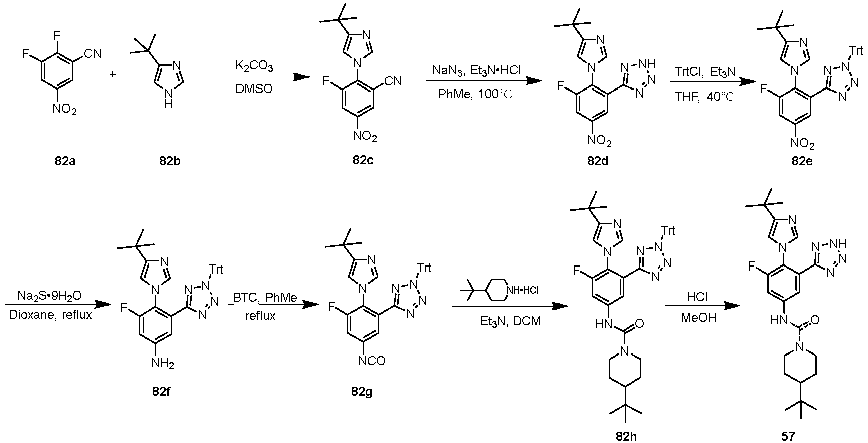

Step 1: Synthesis of 2-(4-(tert-butyl)-1H-imidazol-1-yl)-3-fluoro-5-nitrobenzonitrile (82c): 1.00 g (5.44 mmol) of 2,3-difluoro-5-nitrobenzonitrile, 0.56 g (4.53 mmol) of 4-tert-butylimidazole and 1.88 g (13.58 mmol) of potassium carbonate were added to a reaction flask. Then, 20 mL of dimethyl sulfoxide was added. The mixture was warmed to 90 °C, and the reaction was carried out for 5 h. After the reaction was completed, the mixture was cooled to the room temperature, diluted with water, extracted with ethyl acetate, washed with saturated brine, dried over anhydrous sodium sulfate, filtered, concentrated under reduced pressure, and the residue was purified by silica gel column chromatography (PE:EA= 2:1, v/v) to obtain 0.87 g of 82c. Yield: 67%.

Step 2: Synthesis of 5-(2-(4-(tert-butyl)-1H-imidazol-1-yl)-3-fluoro-5-nitrophenyl)-2H-tetrazole (82d): the synthesis was performed according to Step 2 of Example 1, except that 1c was replaced with 82c. Yield: 81%.

Step 3: Synthesis of 5-(2-(4-(tert-butyl)-1H-imidazol-1-yl)-3-fluoro-5-nitrophenyl)-2-trityl-2H-tetrazole (82e): the synthesis was performed according to Step 3 of Example 1, except that 1c was replaced with 82c. Yield: 91%.

Step 4: Synthesis of 4-(4-(tert-butyl)-1H-imidazol-1-yl)-3-fluoro-5-(2-trityl-2H-tetrazol-5-yl)phenylamine (82f): the synthesis was performed according to Step 4 of Example 1, except that 1e was replaced with 81e. Yield: 81%.

Step 5: Synthesis of 5-(2-(4-(tert-butyl)-1H-imidazol-1-yl)-3-fluoro-5-isocyanatophenyl)-2-trityl-2H-tetrazole (82g): the synthesis was performed according to Step 5 of Example 1, except that 1f was replaced with 81f.

Step 6: Synthesis of 4-(tert-butyl)-N-(4-(4-(tert-butyl)-1H-imidazol-1-yl)-3-fluoro-5-(2-trityl-2H-tetrazol-5-yl)phenyl)piperidine-1-carboxamide (82h): the synthesis was performed according to Step 6 of Example 1, except that 1g was replaced with 82g, and 4-tert-butylcyclohexylamine was replaced with 4-tert-butylpiperidine hydrochloride. Yield through the two steps: 41%.

Step 7: Synthesis of 4-(tert-butyl)-N-(4-(4-(tert-butyl)-1H-imidazol-1-yl)-3-fluoro-5-(2H-tetrazol-5-yl)phenyl)piperidine-1-carboxamide (57): the synthesis was performed according to Step 7 of Example 1, except that 1h was replaced with 82h. Yield: 61%.

ESI-MS: m/z = 470 [M+H]⁺. ¹H NMR (600 MHz, DMSO-*d*₆) δ 16.02 (s, 1H), δ 8.57 (s, 1H), 8.20 (d, *J =* 1.5 Hz, 1H), 8.00 (s, 1H), 7.96 (s, 1H), 7.71 (dd, *J* = 8.0, 1.5 Hz, 1H), 4.28 - 4.22 (m, 2H), 2.76 - 2.68 (m, 2H), 1.72 - 1.66 (m, 2H), 1.33 (s, 9H), 1.25 - 1.19 (m, 1H), 1.17 - 1.08 (m, 2H), 0.86 (s, 9H).

### Preparation Example 58

### Synthesis of 4-(tert-butyl)-N-(4-(4-(tert-butyl)-1H-imidazol-1-yl)-3-(2H-tetrazol-5-yl)phenyl)piperidine-1-carboxamide (Compound 58):

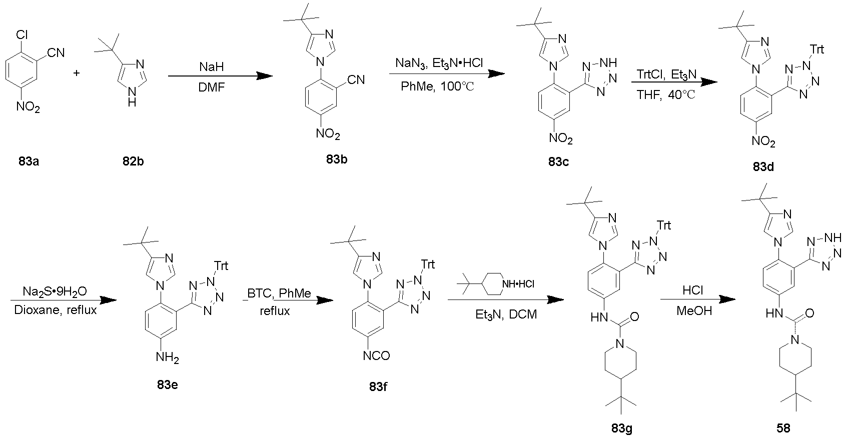

Step 1: Synthesis of 2-(4-(tert-butyl)-1H-imidazol-1-yl)-5-nitrobenzonitrile (83b): 1.00 g (8.05 mmol) of 4-tert-butylimidazole was dissolved in 20 mL of anhydrous DMF. The solution was cooled to 0°C, and 0.39 g (9.66 mmol) of sodium hydride (60%) was added in batches. After the addition was completed, the mixture was warmed to the room temperature, and the reaction was carried out for 0.5 h. Then, 2.20 g (12.08 mmol) of 2-chloro-5-nitrobenzonitrile was added. The reaction was further carried out for 1 h. After the reaction was completed, the mixture was diluted with water, extracted with ethyl acetate, washed with saturated brine, dried over anhydrous sodium sulfate, filtered, concentrated under reduced pressure, and the residue was purified by silica gel column chromatography (PE: EA = 2:1, v/v) to obtain 1.50 g of 83b. Yield: 69%.

Step 2: Synthesis of 5-(2-(4-(tert-butyl)-1H-imidazol-1-yl)-5-nitrophenyl)-2H-tetrazole (83c): the synthesis was performed according to Step 2 of Example 1, except that 1c was replaced with 83b. Yield: 87%.

Step 3: Synthesis of 5-(2-(4-(tert-butyl)-1H-imidazol-1-yl)-5-nitrophenyl)-2-trityl-2H-tetrazole (83d): the synthesis was performed according to Step 3 of Example 1, except that 1d was replaced with 83c. Yield: 91%.

Step 4: Synthesis of 4-(4-(tert-butyl)-1H-imidazol-1-yl)-3-(2-trityl-2H-tetrazol-5-yl)phenylamine (83e): the synthesis was performed according to Step 4 of Example 1, except that 1e was replaced with 83d. Yield: 81%.

Step 5: Synthesis of 5-(2-(4-(tert-butyl)-1H-imidazol-1-yl)-5-isocyanatophenyl)-2-trityl-2H-tetrazole (83f): the synthesis was performed according to Step 5 of Example 1, except that 1f was replaced with 83e.

Step 6: Synthesis of 4-(tert-butyl)-N-(4-(4-(tert-butyl)-1H-imidazol-1-yl)-3-(2-trityl-2H-tetrazol-5-yl)phenyl)piperidine-1-carboxamide (83g): the synthesis was performed according to Step 6 of Example 1, except that 1g was replaced with 83f, and 4-tert-butylcyclohexylamine was replaced with 4-tert-butylpiperidine hydrochloride. Yield through the two steps: 43%.

Step 7: Synthesis of 4-(tert-butyl)-N-(4-(4-(tert-butyl)-1H-imidazol-1-yl)-3-(2H-tetrazol-5-yl)phenyl)piperidine-1-carboxamide (58): the synthesis was performed according to Step 7 of Example 1, except that 1h was replaced with 83g. Yield: 65%.

ESI-MS: m/z = 452 [M+H]⁺. ¹H NMR (400 MHz, DMSO-*d*₆) δ 16.08 (s, 1H), δ 8.75 (s, 1H), 7.76 - 7.65 (m, 2H), 7.59 (s, 1H), 7.51 (d*, J =* 8.5 Hz, 1H), 6.98 (s, 1H), 4.28 - 4.15 (m, 2H), 2.75 - 2.64 (m, 2H), 1.70 - 1.63 (m, 2H),1.39 (s, 9H) 1.20 - 1.16 (m, 1H), 1.13 - 1.04 (m, 2H), 0.85 (s, 9H).

### Preparation Example 59

### Synthesis of 1-(4-(tert-butyl)cyclohexyl)-3-(3',4'-dimethoxy-2-(2H-tetrazol-5-yl)-[1,1'-biphenyl]-4-yl)urea (Compound 59):

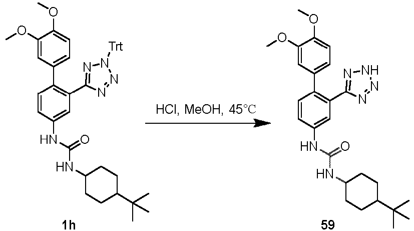

0.12 g (0.16 mmol) of 1h and 12 mL of methanol were added to a reaction flask. Concentrated hydrochloric acid was added dropwise until the pH was 1-2. The reaction was carried out at 45°C for 1 h. After the reaction was completed, the mixture was diluted with water, extracted with ethyl acetate, dried over anhydrous sodium sulfate, filtered, and concentrated under reduced pressure. The residue was recrystallized from ethyl acetate to obtain 0.03 g of 59. Yield: 40%.

ESI-MS: m/z = 479 [M+H]⁺.

### Preparation Example 60

### Synthesis of 1-(3',4'-dimethoxy-2-(2H-tetrazol-5-yl)-[1,1'-biphenyl]-4-yl)-3-(4-(trifluoromethyl)phenyl)urea (Compound 60):

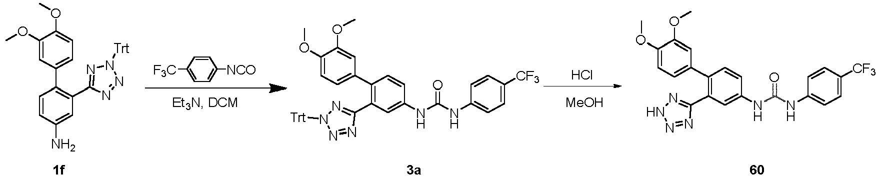

Step 1: Synthesis of 1-(3',4'-dimethoxy-2-(2-trityl-2H-tetrazol-5-yl)-[1,1'-biphenyl]-4-yl)-3-(4-(trifluoromethyl)phenyl)urea (3a): 0.50 g (0.92 mmol) of 1f, 20 mL of DCM and 0.09 g (0.92 mmol) of triethylamine were added to a reaction flask. The mixture was cooled to 0°C. A solution of 0.17 g (0.92 mmol) of 4-trifluoromethylphenylisocyanate in 5 mL of DCM was added dropwise. After the addition was complete, the mixture was warmed to the room temperature. The reaction was carried out for 1 h. After the reaction was completed, the mixture was concentrated under reduced pressure and the residue was purified by silica gel column chromatography (PE: EA = 2: 1, v/v) to obtain 0.28 g of 3a. Yield: 42%.

Step 2: Synthesis of 1-(3',4'-dimethoxy-2-(2H-tetrazol-5-yl)-[1,1'-biphenyl]-4-yl)-3-(4-(trifluoromethyl)phenyl)urea (Compound 60): the synthesis was performed according to Step 7 of Example 1, except that 1h was replaced with 3a. Yield: 71%.

ESI-MS: m/z = 485 [M+H]⁺.¹H NMR (500 MHz, Chloroform-d) δ 16.21 (s, 1H), δ 9.23 (s, 1H), 9.13 (s, 1H), 7.80 (s, 1H), 7.75 - 7.59 (m, 5H), 7.53 (d, *J =* 8.5 Hz, 1H), 6.88 (d, *J =* 8.2 Hz, 1H), 6.65 - 6.52 (m, 2H), 3.74 (s, 3H), 3.59 (s, 3H).

### Preparation Example 61

### Synthesis of 1-(3',4'-dimethoxy-2-(2H-tetrazol-5-yl)-[1,1'-biphenyl]-4-yl)-3-(4-methylcyclohexyl)urea (Compound 61):

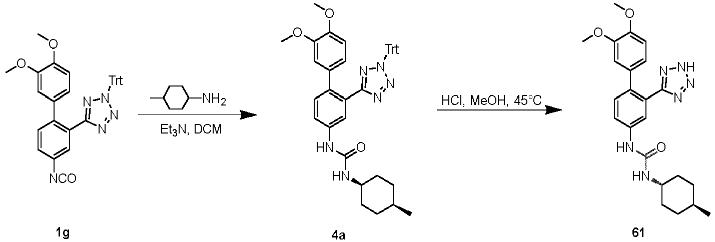

Step 1: Synthesis of 1-(3',4'-dimethoxy-2-(2-trityl-2H-tetrazol-5-yl)-[1,1'-biphenyl]-4-yl)-3-(4-methylcyclohexyl)urea (4a): the synthesis was performed according to Step 6 of Example 1, except that 4-tert-butylcyclohexylamine was replaced with 4-methylcyclohexylamine. Yield: 39%.

Step 2: Synthesis of 1-(3',4'-dimethoxy-2-(2H-tetrazol-5-yl)-[1,1'-biphenyl]-4-yl)-3-(4-methylcyclohexyl)urea (61): the synthesis was performed according to Step 7 of Example 1, except that 1h was replaced with 4a. Yield: 53%.

ESI-MS: m/z = 437[M+H]⁺.

### Preparation Example 62

### Synthesis of 1-(3',4'-dimethoxy-2-(2H-tetrazol-5-yl)-[1,1'-biphenyl]-4-yl)-3-((trans)-4-methylcyclohexyl)urea (Compound 62):

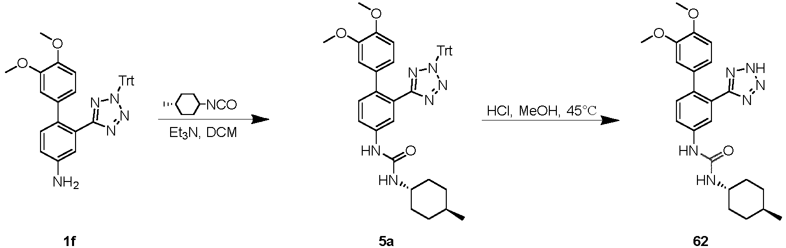

Step 1: Synthesis of 1-(3',4'-dimethoxy-2-(2-trityl-2H-tetrazol-5-yl)-[1,1'-biphenyl]-4-yl)-3-(4-methylcyclohexyl)urea (5a): the synthesis was performed according to Step 1 of Example 60, except that 4-trifluoromethylphenyl isocyanate was replaced with trans-4-methylcyclohexyl isocyanate. Yield: 42%.

Step 2: Synthesis of 1-(3',4'-dimethoxy-2-(2H-tetrazol-5-yl)-[1,1'-biphenyl]-4-yl)-3-(4-methylcyclohexyl)urea (62): the synthesis was performed according to Step 7 of Example 1, except that 1h was replaced with 5a. Yield: 61%.

ESI-MS: m/z = 437[M+H]⁺. ¹H NMR (500 MHz, DMSO-*d*₆) δ 16.26 (s, 1H), 8.50 (s, 1H), 8.16 (d, *J =* 2.7 Hz, 1H), 7.48 (dd, *J =* 9.0, 2.8 Hz, 1H), 6.94 (d, *J =* 8.8 Hz, 1H), 6.86 (d, *J =* 9.0 Hz, 1H), 6.80 (d, *J =* 2.8 Hz, 1H), 6.52 (d, *J* = 8.5 Hz, 1H), 5.99 (d, *J* = 7.8 Hz, 1H), 3.74 (s, 3H), 3.73 (s, 3H), 3.41 - 3.35 (m, 1H), 1.94 - 1.80 (m, 2H), 1.75 - 1.62 (m, 2H), 1.44 - 1.23 (m, 1H), 1.20 - 1.10 (m, 2H), 1.04 - 0.93 (m, 2H), 0.88 (d, *J =* 6.5 Hz, 3H).

### Preparation Example 63

### Synthesis of 1-(4-(3,4-dimethoxyphenoxy)-3-(2H-tetrazol-5-yl)phenyl)-3-((cis)-4-methylcyclohexyl)urea (Compound 63):

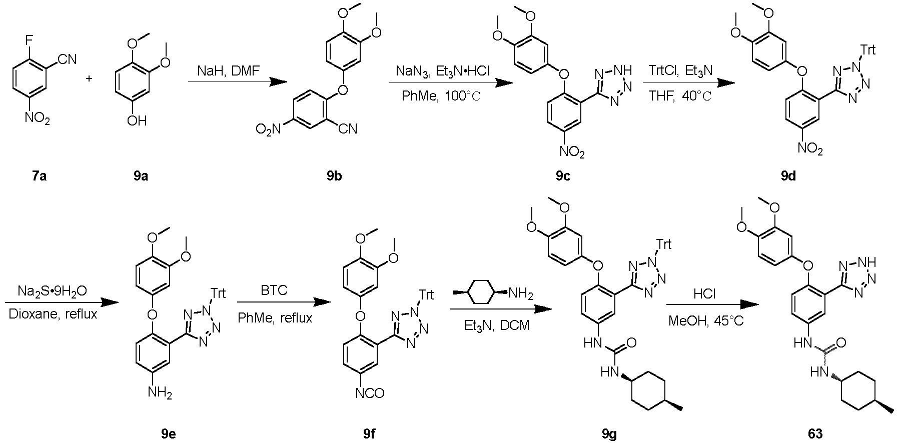

Step 1: Synthesis of 2-(3,4-dimethoxyphenoxy)-5-nitrobenzonitrile (9b): the synthesis was performed according to Step 1 of Example 1, except that 7b was replaced with 9a, and the residue was recrystallized from ethyl acetate. Yield: 62%.

Step 2: Synthesis of 5-(2-(3,4-dimethoxyphenoxy)-5-nitrophenyl)-2H-tetrazole (9c): the synthesis was performed according to Step 2 of Example 1, except that 1c was replaced with 9b. Yield: 68%.

Step 3: Synthesis of 5-(2-(3,4-dimethoxyphenoxy)-5-nitrophenyl)-2-trityl-2H-tetrazole (9d): the synthesis was performed according to Step 3 of Example 1, except that 1d was replaced with 9c. Yield: 78%.

Step 4: Synthesis of 4-(3,4-dimethoxyphenoxy)-3-(2-trityl-2H-tetrazol-5-yl)phenylamine (9e): the synthesis was performed according to Step 4 of Example 1, except that 1e was replaced with 9d. Yield: 73%.

Step 5: Synthesis of 5-(2-(3,4-dimethoxyphenoxy)-5-isocyanatophenyl)-2-trityl-2H-tetrazole (9f): the synthesis was performed according to Step 5 of Example 1, except that 1f was replaced with 9e, and the residue was directly used for the next step.

Step 6: Synthesis of 1-(4-(3,4-dimethoxyphenoxy)-3-(2-trityl-2H-tetrazol-5-yl)phenyl)-3-(4-methylcyclohexyl)urea (9g): the synthesis was performed according to Step 6 of Example 1, except that 4-tert-butylcyclohexylamine was replaced with 4-methylcyclohexylamine. Yield through the two steps: 33%.

Step 7: Synthesis of 1-(4-(3,4-dimethoxyphenoxy)-3-(2H-tetrazol-5-yl)phenyl)-3-((cis)-4-methylcyclohexyl)urea (63): the synthesis was performed according to Step 7 of Example 1, except that 1h was replaced with 9g. Yield: 42%.

ESI-MS: m/z =453[M+H]⁺.

### Preparation Example 64

### Synthesis of 1-(4-(3,4-dimethoxyphenoxy)-3-(2H-tetrazol-5-yl)phenyl)-3-((trans)-4-methylcyclohexyl)urea (Compound 64):

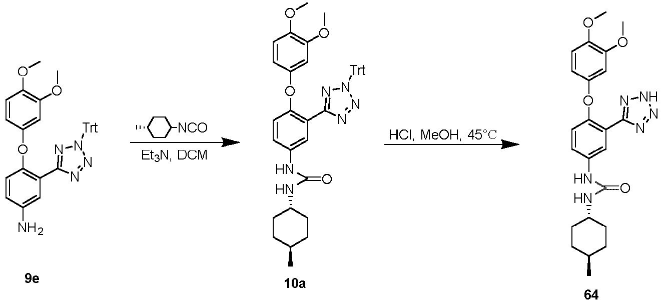

Step 1: Synthesis of 1-(4-(3,4-dimethoxyphenoxy)-3-(2-trityl-2H-tetrazol-5-yl)phenyl)-3-(4-methylcyclohexyl)urea (10a): the synthesis was performed according to Step 1 of Example 60, except that 1f was replaced with 9e, and 4-trifluoromethylphenyl isocyanate was replaced with trans-4-methylcyclohexyl isocyanate. Yield: 36%.

Step 2: Synthesis of 1-(4-(3,4-dimethoxyphenoxy)-3-(2H-tetrazol-5-yl)phenyl)-3-((trans)-4-methylcyclohexyl)urea (64): the synthesis was performed according to Step 7 of Example 1, except that 1h was replaced with 10a Yield: 59%.

ESI-MS: m/z =453[M+H]⁺.¹H NMR (500 MHz, DMSO-*d*₆) δ 16.26 (s, 1H), 8.50 (s, 1H), 8.16 (d, *J =* 2.7 Hz, 1H), 7.48 (dd, *J* = 9.0, 2.8 Hz, 1H), 6.94 (d, *J =* 8.8 Hz, 1H), 6.86 (d, *J =* 9.0 Hz, 1H), 6.80 (d, *J =* 2.8 Hz, 1H), 6.52 (d, *J =* 8.5 Hz, 1H), 5.99 (d, *J* = 7.8 Hz, 1H), 3.74 (s, 3H), 3.73 (s, 3H), 3.42 - 3.35 (m, 1H), 1.93 - 1.79 (m, 2H), 1.75 - 1.56 (m, 2H), 1.38 - 1.25 (m, 1H), 1.24 - 1.10 (m, 2H), 1.04 - 0.92 (m, 2H), 0.88 (d, *J =* 6.5 Hz, 3H).

### Preparation Example 65

### Synthesis of 1-((trans)-4-(tert-butyl)cyclohexyl)-3-(3',4'-dimethoxy-2-(2H-tetrazol-5-yl)-[1,1'-biphenyl]-4-yl)-1-methylurea (Compound 65):

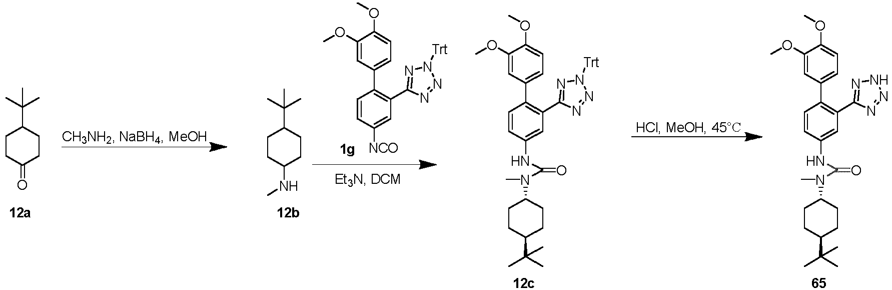

Step 1: Synthesis of 4-(tert-butyl)-N-methylcyclohexyl-1-amine (12b): 0.30 g (1.94 mmol) of 12a and 10 mL of methanol were added to a reaction flask, and then a 30% solution of 0.7 g (5.82 mmol) of methylamine in methanol was added. The reaction was carried out at the room temperature for 1 h. Then, the reaction solution was cooled to -78°C, and 0.08 g (2.13 mmol) of sodium borohydride was added. The reaction was carried out for 1 h while keeping the mixture at this temperature. Then, the mixture was slowly warmed to the room temperature, and the reaction was carried out for 6 h. After the reaction was completed, the reaction solution was concentrated under reduced pressure, diluted with water, extracted with ethyl acetate, dried over anhydrous sodium sulfate, filtered, and concentrated under reduced pressure. The residue was directly used for the next step.

Step 2: Synthesis of 3-(3',4'-dimethoxy-2-(2-trityl-2H-tetrazol-5-yl)-[1,1'-biphenyl]-4-yl)-1-methyl-1-(4-methylcyclohexyl)urea (12c): the synthesis was performed according to Step 6 of Example 1, except that 4-tert-butylcyclohexylamine was replaced with 12b. Yield through the two steps: 29%.

Step 3: Synthesis of 1-((trans)-4-(tert-butyl)cyclohexyl)-3-(3',4'-dimethoxy-2-(2H-tetrazol-5-yl)-[1,1'-biphenyl]-4-yl)-1-methylurea (65): the synthesis was performed according to Step 7 of Example 1, except that 1h was replaced with 12c. Yield: 54%.

ESI-MS: m/z=493[M+H]⁺.¹H NMR (500 MHz, DMSO-*d*₆) δ 16.14 (s, 1H), 8.51 (s, 1H), 7.83 - 7.74 (m, 2H), 7.46 (d, *J =* 8.4 Hz, 1H), 6.87 (d, *J =* 8.1 Hz, 1H), 6.61 - 6.54 (m, 2H), 4.01 - 3.92 (m, 1H), 3.74 (s, 3H), 3.59 (s, 3H), 2.82 (s, 3H), 1.81 (d, *J =* 12.5 Hz, 2H), 1.72 - 1.61 (m, 2H), 1.54 - 1.41 (m, 2H), 1.16 - 1.07 (m, 2H), 1.04 - 0.95 (m, 1H), 0.86 (s, 9H).

### Preparation Example 66.

### Synthesis of 1-(4-(3,6-dihydro-2H-pyran-4-yl)-3-(2H-tetrazol-5-yl)phenyl)-3-((trans)-4-methylcyclohexyl)urea (Compound 66):

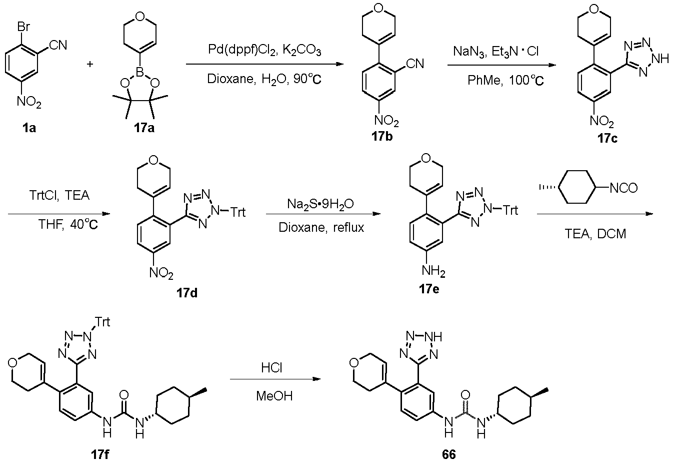

Step 1: Synthesis of 2-(3,6-dihydro-2H-pyran-4-yl)-5-nitrobenzonitrile (17b): 1.00 g (4.40 mmol) of 1a, 1.23 g (4.62 mmol) of 17b, 1.21 g (8.81 mmol) of potassium carbonate, 0.16 mg (0.22 mmol) of Pd(dppf)Cl₂, 30 mL of 1,4-dioxane and 7 mL of water were added to a reaction flask. The reaction was carried out at 90°C for 2 h under nitrogen protection. After the reaction was completed, the reaction solution was cooled to the room temperature. Then, the resultant mixture was diluted with water, extracted with ethyl acetate, washed with saturated brine, dried over anhydrous sodium sulfate, filtered, and concentrated under reduced pressure. The residue was purified by silica gel column chromatography (PE: EA = 3: 1, v/v) to obtain 0.93 g of 17b. Yield: 92%.

Step 2: Synthesis of 5-(2-(3,6-dihydro-2H-pyran-4-yl)-5-nitrophenyl)-2H-tetrazole (17c): the synthesis was performed according to Step 2 of Example 1, except that 1c was replaced with 17b. Yield: 85%.

Step 3: Synthesis of 5-(2-(3,6-dihydro-2H-pyran-4-yl)-5-nitrophenyl)-2-trityl-2H-tetrazole (17d): the synthesis was performed according to Step 3 of Example 1, except that 1d was replaced with 17c. Yield: 67%.

Step 4: Synthesis of 4-(3,6-dihydro-2H-pyran-4-yl)-3-(2-trityl-2H-tetrazol-5-yl)phenylamine (17e): the synthesis was performed according to Step 4 of Example 1, except that 1e was replaced with 17d. Yield: 74%.

Step 5: Synthesis of 1-(4-(3,6-dihydro-2H-pyran-4-yl)-3-(2-trityl-2H-tetrazol-5-yl)phenyl)-3-(4-methylcyclohexyl)urea (17f): the synthesis was performed according to Step 1 of Example 60, except that 1f was replaced with 17e, 4-trifluoromethylphenyl isocyanate was replaced with 4-methylcyclohexyl isocyanate. Yield through the two steps: 43%.

Step 6: 1-(4-(3,6-dihydro-2H-pyran-4-yl)-3-(2H-tetrazol-5-yl)phenyl)-3-((trans)-4-methylcyclohexyl)urea (66): the synthesis was performed according to Step 7 of Example 1, except that 1h was replaced with 17f. Yield: 46%.

ESI-MS: m/z =383[M+H]⁺.¹H NMR (500 MHz, Chlorofonn-*d*) δ 16.41 (s, 1H), 8.55 (s, 1H), 7.68 (s, 1H), 7.48 (dd, *J* = 8.5, 2.4 Hz, 1H), 7.28 (d, *J* = 8.4 Hz, 1H), 6.10 (d, *J =* 7.8 Hz, 1H), 5.36 (s, 1H), 4.00 (d, *J =* 2.3 Hz, 2H), 3.65 (t, *J =* 5.3 Hz, 2H), 3.36 - 3.34 (m, 1H), 2.04 - 1.99 (m, 2H), 1.90 - 1.82 (m, 2H), 1.70 - 1.61 (m, 2H), 1.36 - 1.25 (m, 1H), 1.16 - 1.07 (m, 2H), 1.03 - 0.92 (m, 2H), 0.86 (d, *J =* 6.5 Hz, 3H).

### Preparation Example 67

### Synthesis of 1-((cis)-4-methylcyclohexyl)-3-(4-(tetrahydro-2H-pyran-4-yl)-3-(2H-tetrazol-5-yl)phenyl)urea (Compound 67):

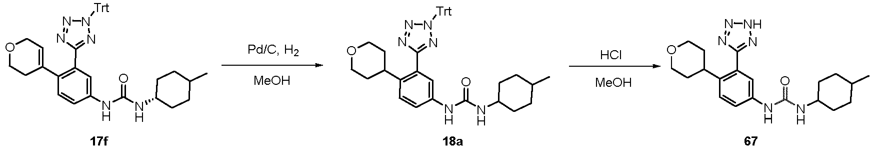

Step 1: Synthesis of 1-(4-methylcyclohexyl)-3-(4-(tetrahydro-2H-pyran-4-yl)-3-(2-trityl-2H-tetrazol-5-yl)phenyl)urea (18a): 0.10 g (0.19 mmol) of 17f was dissolved in 3 mL of methanol. The reaction was carried out at the room temperature for 5 h under 1 MPa of hydrogen pressure. After the reaction was completed, Pd/C was removed by filtration. The filtrate was concentrated under reduced pressure to obtain 18a, and the residue was purified by silica gel column chromatography to obtain 18a. Yield: 88%.

Step 2: Synthesis of 1-((cis)-4-methylcyclohexyl)-3-(4-(tetrahydro-2H-pyran-4-yl)-3-(2H-tetrazol-5-yl)phenyl)urea (67): the synthesis method of 67 was similar to that of 1, except that 1h was replaced with 18a. Yield: 65%.

ESI-MS: m/z =385[M+H]⁺

### Preparation Example 68

### Synthesis of 1-((trans)-4-methylcyclohexyl)-3-(4-(tetrahydro-2H-pyran-4-yl)-3-(2H-tetrazol-5-yl)phenyl)urea (Compound 68):

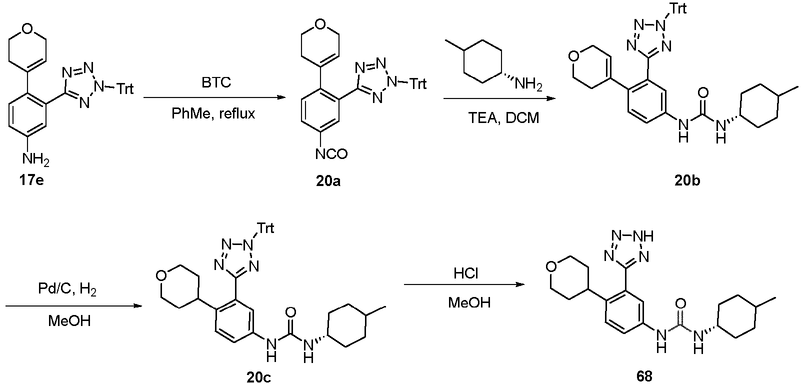

Step 1: Synthesis of 5-(2-(3,6-dihydro-2H-pyran-4-yl)-5-isocyanatophenyl)-2-trityl-2H-tetrazole (20a): 100 mg (0.20 mmol) of 17e, 31 mg (0.10 mmol) of triphosgene and 10 mL of anhydrous toluene were added to a reaction flask. The reaction was carried out under reflux for 4 h. After the reaction was completed, the mixture was concentrated under reduced pressure to obtain 20a, which was directly used for the next step.

Step 2: Synthesis of 1-(4-(3,6-dihydro-2H-pyran-4-yl)-3-(2-trityl-2H-tetrazol-5-yl)phenyl)-3-(4-methylcyclohexyl)urea (20b): 23 mg (0.20 mmol) of trans-p-methylcyclohexylamine, 5 mL of DCM and 21 mg (0.20 mmol) of triethylamine were added to the reaction flask. The mixture was cooled to 0°C. Then, a solution of 20a in DCM (1 mL) was added dropwise. After the addition was completed, the mixture was warmed to the room temperature, and the reaction was carried out for 1 h. After the reaction was completed, the mixture was concentrated under reduced pressure, and the residue was purified by silica gel column chromatography (PE: EA= 2: 1, v/v) to obtain 65 mg of 20b. Yield through the two steps: 41%.

Step 3: Synthesis of 1-(4-methylcyclohexyl)-3-(4-(tetrahydro-2H-pyran-4-yl)-3-(2-trityl-2H-tetrazol-5-yl)phenyl)urea (20c): the synthesis method of 20c was similar to that of 18a, except that 17f was replaced with 20b. Yield: 83%.

Step 4: Synthesis of 1-((trans)-4-methylcyclohexyl)-3-(4-(tetrahydro-2H-pyran-4-yl)-3-(2H-tetrazol-5-yl)phenyl)urea (68): the synthesis method of 68 was similar to that of 1, except that 1h was replaced with 20c. Yield: 63%.

ESI-MS: m/z =385 [M+H]⁺.¹H NMR (600 MHz, DMSO-*d*₆) δ 16.51 (s, 1H), δ 8.45 (s, 1H), 7.64 (s, 1H), 7.47 (dd, *J =* 8.6, 2.4 Hz, 1H), 7.41 (d, *J =* 8.6 Hz, 1H), 6.07 (d, *J =* 7.8 Hz, 1H), 3.94 - 3.85 (m, 2H), 3.40 - 3.34 (m, 1H), 3.31 - 3.25 (m, 2H), 3.10 - 2.94 (m, 1H), 1.90 - 1.81 (m, 2H), 1.71 - 1.62 (m, 4H), 1.61 - 1.53 (m, 2H), 1.37 - 1.28 (m, 1H), 1.20 - 1.09 (m, 2H), 1.05 - 0.93 (m, 2H), 0.87 (d, *J =* 6.5 Hz, 3H).

### Preparation Example 69

### Synthesis of 1-(4-(benzo[d][1,3]dioxolan-5-yl)-3-(2H-tetrazol-5-yl)phenyl)-3-((trans)-4-methylcyclohexyl)urea (Compound 69):

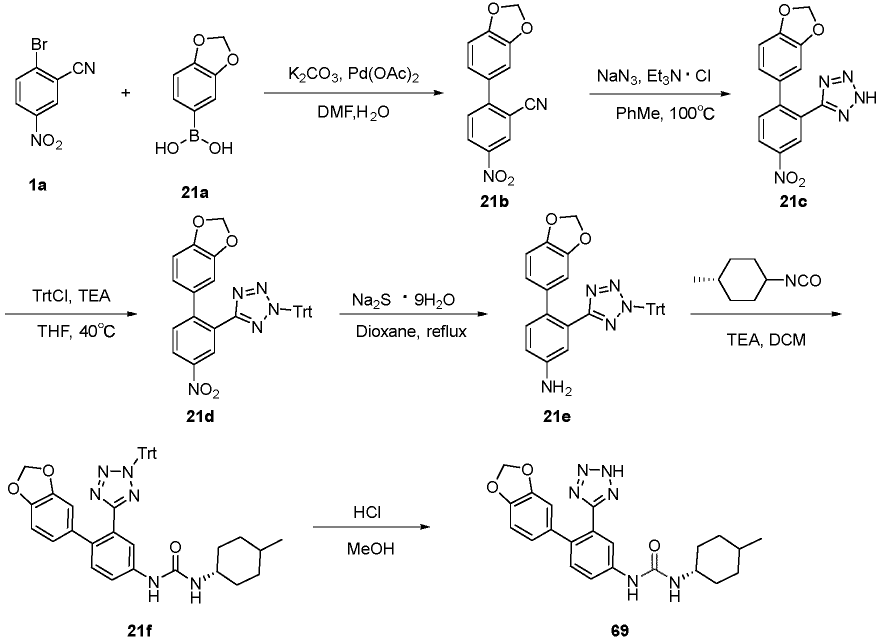

Step 1: Synthesis of 2-(benzo[d][1,3]dioxolan-5-yl)-5-nitrobenzonitrile (21b): 1.00 g (4.41 mmol) of 1a, 0.88 g (5.29 mmol) of 21a, 0.05 g (0.22 mmol) of palladium acetate, 30 mL of DMF and an 6 mL of aqueous solution of 1.21 g (8.81 mmol) of potassium carbonate were added to a reaction flask. The reaction was carried out at the room temperature for 3 h under nitrogen protection. After the reaction was completed, the mixture was diluted with water, extracted with ethyl acetate, washed with saturated brine, dried over anhydrous sodium sulfate, and concentrated under reduced pressure. The residue was recrystallized from ethyl acetate to obtain 1.04 g of 21b. Yield: 88%.

Step 2: Synthesis of 5-(2-(benzo[d][1,3]dioxolan-5-yl)-5-nitrophenyl)-2H-tetrazole (21c): the synthesis was performed according to Step 2 of Example 1, except that 17b was replaced with 21b. Yield: 72%.

Step 3: Synthesis of 5-(2-(benzo[d][1,3]dioxolan-5-yl)-5-nitrophenyl)-2-trityl-2H-tetrazole (21d): the synthesis was performed according to Step 3 of Example 1, except that 17c was replaced with 21c. Yield: 79%.

Step 4: Synthesis of 4-(benzo[d][1,3]dioxolan-5-yl)-3-(2-trityl-2H-tetrazol-5-yl)phenylamine (21e): the synthesis was performed according to Step 4 of Example 1, except that 17d was replaced with 21d. Yield: 85%.

Step 5: Synthesis of 1-(4-(benzo[d][1,3]dioxolan-5-yl)-3-(2-trityl-2H-tetrazol-5-yl)phenyl)-3-(4-methylcyclohexyl)urea (21f): the synthesis was performed according to Step 1 of Example 60, except that 17e was replaced with 21e. Yield through the two steps: 42%.

Step 6: Synthesis of 1-(4-(benzo[d][1,3]dioxolan-5-yl)-3-(2H-tetrazol-5-yl)phenyl)-3-((trans)-4-methylcyclohexyl)urea (69): the synthesis was performed according to Step 7 of Example 1, except that 1h was replaced with 21f. Yield: 61%.

ESI-MS: m/z =421[M+H]⁺¹H NMR (500 MHz, Chloroform-*d*) δ 16.23 (s, 1H), 8.62 (s, 1H), 7.72 (d, *J =* 2.4 Hz, 1H), 7.56 (dd*, J =* 8.4, 2.3 Hz, 1H), 7.37 (d, *J =* 8.5 Hz, 1H), 6.80 (d, *J =* 8.0 Hz, 1H), 6.62 (d, *J =* 1.7 Hz, 1H), 6.48 - 6.37 (m, 1H), 6.14 (d, *J =* 7.8 Hz, 1H), 6.01 (s, 2H), 3.47 - 3.36 (m, 1H), 1.94 - 1.78 (m, 2H), 1.73 - 1.60 (m, 2H), 1.40 - 1.24 (m, 1H), 1.23 - 1.08 (m, 2H), 1.06 - 0.92 (m, 2H), 0.87 (d, *J =* 6.5 Hz, 3H).

### Preparation Example 70

### Synthesis of 1-(4-(benzo[d][1,3]dioxolan-5-yl)-3-(2H-tetrazol-5-yl)phenyl)-3-((cis)-4-(tert-butyl)cyclohexyl)urea (Compound 70):

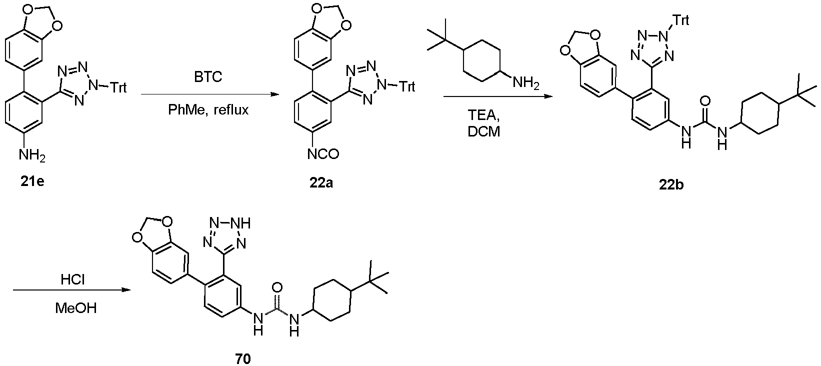

Step 1: Synthesis of 5-(2-(benzo[d][1,3]dioxolan-5-yl)-5-isocyanatophenyl)-2-trityl-2H-tetrazole (22a): the synthesis was performed according to Step 5 of Example 1, except that 17e was replaced with 21e, and the residue was directly used for the next step.

Step 2: Synthesis of 1-(4-(benzo[d][1,3]dioxolan-5-yl)-3-(2-trityl-2H-tetrazol-5-yl)phenyl)-3-(4-(tert-butyl)cyclohexyl)urea (22b): the synthesis was performed according to Step 6 of Example 1, except that 20a was replaced with 22a, and trans-4-methylcyclohexylamine was replaced with 4-tert-butylcyclohexylamine. Yield through the two steps: 52%.

Step 3: Synthesis of 1-(4-(benzo[d][1,3]dioxolan-5-yl)-3-(2H-tetrazol-5-yl)phenyl)-3-((cis)-4-(tert-butyl)cyclohexyl)urea (70): the synthesis was performed according to Step 7 of Example 1, except that 1h was replaced with 22b. Yield: 75%.

ESI-MS: m/z =463 [M+H]⁺

### Preparation Example 71

### Synthesis of 1-(4-(6-ethoxypyrid-3-yl)-3-(2H-tetrazol-5-yl)phenyl)-3-((trans)-4-methylcyclohexyl)urea (Compound 71):

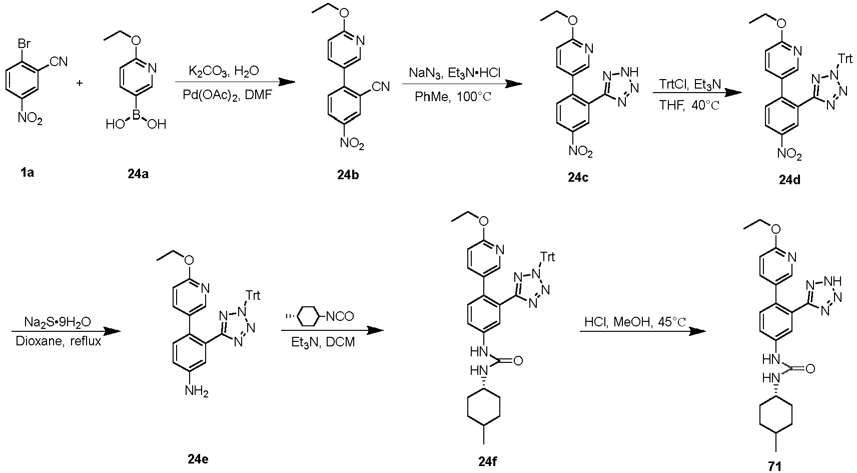

Step 1: Synthesis of 2-(6-ethoxypyrid-3-yl)-5-nitrobenzonitrile (24b): the synthesis was performed according to Step 1 of Example 1, except that 1b was replaced with 24a. Yield: 81%.

Step 2: Synthesis of 2-ethoxy-5-(4-nitro-2-(2H-tetrazol-5-yl)phenyl)pyridine (24c): the synthesis was performed according to Step 2 of Example 1, except that 1c was replaced with 24b. Yield: 58%.

Step 3: Synthesis of 2-ethoxy-5-(4-nitro-2-(2-trityl-2H-tetrazol-5-yl)phenyl)pyridine (24d): the synthesis was performed according to Step 3 of Example 1, except that 1d was replaced with 24c. Yield: 75%.

Step 4: Synthesis of 4-(6-ethoxypyrid-3-yl)-3-(2-trityl-2H-tetrazol-5-yl)phenylamine (24e): the synthesis was performed according to Step 4 of Example 1, except that 1e was replaced with 24d. Yield: 87%.

Step 5: Synthesis of 1-(4-(6-ethoxypyrid-3-yl)-3-(2-trityl-2H-tetrazol-5-yl)phenyl)-3-(4-methylcyclohexyl)urea (24f): the synthesis was performed according to Step 1 of Example 60, except that 1f was replaced with 24e. Yield: 46%.

Step 6: Synthesis of 1-(4-(6-ethoxypyrid-3-yl)-3-(2H-tetrazol-5-yl)phenyl)-3-((trans)-4-methylcyclohexyl)urea (71): the synthesis was performed according to Step 7 of Example 1, except that 1h was replaced with 24f. Yield: 47%.

ESI-MS: m/z =422[M+H]⁺.¹H NMR (500 MHz, DMSO-*d*₆) δ 16.41 (s, 1H), 8.66 (s, 1H), 7.88 (d, *J =* 2.5 Hz, 1H), 7.79 (d, *J =* 2.3 Hz, 1H), 7.60 (dd, *J* = 8.5, 2.4 Hz, 1H), 7.41 (d, *J =* 8.5 Hz, 1H), 7.28 (dd, *J =* 8.5, 2.6 Hz, 1H), 6.69 (d, *J =* 8.5 Hz, 1H), 6.16 (d, *J =* 7.7 Hz, 1H), 4.28 (q, *J =* 7.0 Hz, 2H), 3.45 - 3.36 (m, 1H), 1.93 - 1.83 (m, 2H), 1.71 - 1.64 (m, 2H), 1.31 (t, *J =* 7.0 Hz, 3H), 1.22 - 1.09 (m, 2H), 1.07 - 0.93 (m, 2H), 0.88 (d, *J =* 6.5 Hz, 3H).

### Preparation Example 72

### Synthesis of 1-(cis)-4-methylcyclohexyl-3-(4-(tetrahydropyran-4-yl)oxy)-3-(2H-tetrazol-5-yl)phenyl)urea (Compound 72):

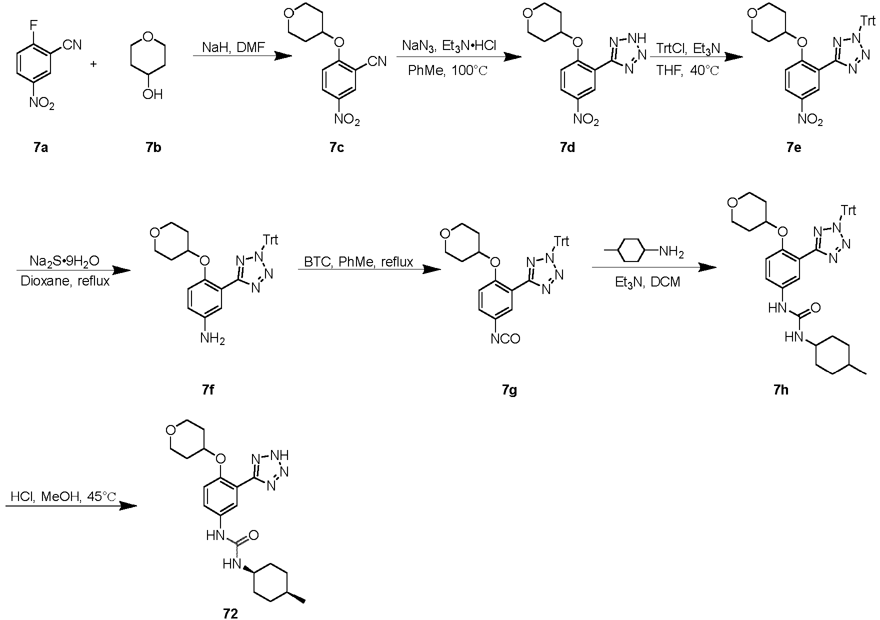

Step 1: Synthesis of 5-nitro-2-((tetrahydro-2H-pyran-4-yl)oxy)benzonitrile (7c): 1.00 g (9.79 mmol) of tetrahydro-2H-pyran-4-ol and 20 mL of anhydrous DMF were added to a reaction flask. The mixture was cooled to 0°C, and then 0.47 g (11.75 mmol) of 60% sodium hydride was added. The mixture was kept at this temperature, and the reaction was carried out for 0.5 h. Then, 1.79 g (10.77 mmol) of 2-fluoro-5-nitrobenzonitrile was added. After the addition was completed, the mixture was warmed to the room temperature. The reaction was carried out for 1 h. After the reaction was completed, the mixture was diluted with water, extracted with ethyl acetate, washed with saturated brine, dried over anhydrous sodium sulfate, filtered, and concentrated under reduced pressure. The residue was purified by silica gel column chromatography (PE:EA=2: 1, v/v) to obtain 1.92 g of 7c. Yield: 79%.

Step 2: Synthesis of 5-(5-nitro-2-(((tetrahydro-2H-pyran-4-yl)oxy)phenyl)-2H-tetrazole (7d): 0.50 g (2.01 mmol) of 7c, 0.39 g (6.03 mmol) of sodium azide, 0.83 g (6.03 mmol) of triethylamine hydrochloride and 15 mL of anhydrous toluene were added to a reaction flask. The reaction was carried out at 100°C for 12 h. After the reaction was completed, the mixture was diluted with water, acidified with dilute hydrochloric acid, extracted with ethyl acetate, dried over anhydrous sodium sulfate, filtered, and concentrated under reduced pressure. The residue was recrystallized from ethyl acetate to obtain 0.46 g of 7d. Yield: 79%.

Step 3: Synthesis of 5-(5-nitro-2-((tetrahydro-2H-pyran-4-yl)oxy)phenyl)-2-trityl-2H-tetrazole (7e): the synthesis was performed according to Step 3 of Example 1, except that 1d was replaced with 7d. Yield: 65%.

Step 4: Synthesis of 4-((tetrahydro-2H-pyran-4-yl)oxy)-3-(2-trityl-2H-tetrazol-5-yl)phenylamine (7f): the synthesis was performed according to Step 4 of Example 1, except that 1e was replaced with 7e. Yield: 95%.

Step 5: Synthesis of 5-(5-isocyanato-2-((tetrahydro-2H-pyran-4-yl)oxy)phenyl)-2-trityl-2H-tetrazole (7g): the synthesis was performed according to Step 5 of Example 1, except that 1f was replaced with 7f, and the residue was directly used for the next step.

Step 6: Synthesis of 1-(4-methylcyclohexyl)-3-(4-((tetrahydro-2H-pyran-4-yl)oxy)-3-(2-trityl-2H-tetrazol-5-yl)phenyl)urea (7h): the synthesis was performed according to Step 6 of Example 1, except that 1g was replaced with 7g. Yield through the two steps: 36%.

Step 7: Synthesis of 1-(cis)-4-methylcyclohexyl-3-(4-(tetrahydropyran-4-yl)oxy)-3-(2H-tetrazol-5-yl)phenyl)urea (72): the synthesis was performed according to Step 7 of Example 1, except that 1h was replaced with 7h. Yield: 50%.

ESI-MS: m/z = 401 [M+H]⁺.

### Preparation Example 73

### Synthesis of 1-(trans)-4-methylcyclohexyl-3-(4-tetrahydropyran-4-yl)oxy)-3-(2H-tetrazol-5-yl)phenyl)urea (Compound 73):

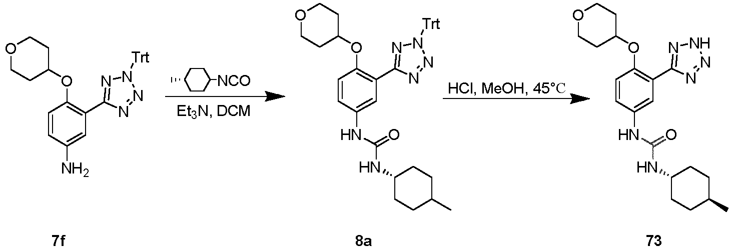

Step 1: Synthesis of 1-(4-methylcyclohexyl)-3-(4-((tetrahydro-2H-pyran-4-yl)oxy)-3-(2-trityl-2H-tetrazol-5-yl)phenyl)urea (8a): the synthesis was performed according to Step 1 of Example 60, except that 4-trifluoromethylphenyl isocyanate was replaced with trans-4-methylcyclohexyl isocyanate, and 1f was replaced with 7f. Yield: 44%.

Step 2: Synthesis of 1-(trans)-4-methylcyclohexyl-3-(4-tetrahydropyran-4-yl)oxy)-3-(2H-tetrazol-5-yl)phenyl)urea (73): the synthesis was performed according to Step 7 of Example 1, except that 1h was replaced with 8a. Yield: 58%.

ESI-MS: m/z = 401 [M+H]⁺. ¹H NMR (500 MHz, Chloroform-*d*) δ 15.87 (s, 1H), 8.36 (s, 1H), 7.93 (d, *J =* 2.7 Hz, 1H), 7.52 (dd, *J =* 9.0, 2.8 Hz, 1H), 7.23 (d, *J* = 9.1 Hz, 1H), 5.94 (d, *J* = 7.8 Hz, 1H), 4.70 - 4.43 (m, 1H), 3.87 - 3.59 (m, 2H), 3.44 - 3.37 (m, 2H), 3.37 - 3.34 (m, 1H), 2.03 - 1.80 (m, 4H), 1.79 - 1.58 (m, 4H), 1.43 - 1.24 (m, 1H), 1.20 - 1.07 (m, 2H), 1.03 - 0.91 (m, 2H), 0.87 (d, *J =* 6.5 Hz, 3H).

### Biological evaluations

### Test Example 1. FLIPR assay for inhibitory activity against human B1R and B2R

The inhibitory activity of the compounds against human B1R and B2R channels was determined by calcium ion flux assay using fluorometric imaging plate reader (FLIPR method). See literatures of Lee, Chen et al. for the method. [Lee et al. 2008] [Chen et al. 2011]
1. Cell culture: HEK293 recombinant cell lines (WuXi AppTec, Shanghai, China) stably expressing human B1R and B2R were cultured in DMEM (Invitrogen, Cat#11965) medium containing 10% calf serum (Excellbio, Cat#FSP500), 5 µg/ml Geneticin (Invitrogen, Cat#10131) and Penicillin/Streptomycin (Invitrogen, Cat#15140). The incubator conditions were 37°C and ambient humidity containing 5% CO₂.
2. Preparation of the cell assay plate: B1R and B2R cells were seeded in a 384-well assay plate (Greiner, Cat#781090) at a density of 20,000 cells per well (20 µl/well), and cultured overnight at 37°C in an incubator with 5% CO₂. The next day, Fluo-4 DirectTM Loading Buffer (containing 5 mM Probenicid) containing 2x (8 µM) Fluo-4 Direct (Invitrogen, Cat#F10471) was added to each of the wells. The cells were cultured at 37°C for 50 min, and then culture for at the room temperature 10 min.
3. Preparation of the compound assay plate: The compound was dissolved in 100% DMSO to make a 30 mM stock solution and stored at -20°C. On the day of use, the stock solution of the compound was thawed at the room temperature. Each of the compounds was serially diluted at 1:5 in DMSO culture medium with a starting concentration of 30 µM for 9 concentration points on a 384-well plate. Each of compounds was diluted in duplicate for testing. [Des-Arg10]-HOE140 was used as a positive control compound, and diluted in the same manner with a starting concentration of 3 µM.
4. FLIPR assay: The cell assay plate was placed in a FLIPR instrument (Molecular Probes). The compounds in the compound assay plate were added to the corresponding wells on the cell assay plate (10 µL/well) using an automatic program. The calcium ion fluorescence signal was recorded to determine whether the compound may have agonist activity. After 10 minutes, a B1R agonist [Lys-des-Arg9]-Bradykinin2 (10 µL/well) with a final concentration of 4.6 nM (EC80) was added to each of the wells to stimulate the generation of intracellular calcium ion flux signals. The Ca⁺⁺-dependent fluorescence signal was continuously monitored at a wavelength of 538 nm to analyze the inhibitory activity of the compound.
5. Data analysis: The data was collected and analysized by a FLIPR program. The inhibitory (or agonist) activity of the compound at each concentration was evaluated by the fluorescence peak. The IC₅₀ values of the compounds were calculated using EXCEL and PRISM programs.
6. Assay results: The inhibitory activity of the compounds of Examples 1-73 of the present invention against B1R (FLIPR method) was shown in Table 1.

**Table 1. Inhibitory activity of Compound against B1R (FLIPR method)**

| Compound | B1R (IC₅₀, nM) | Compound | B1R (IC₅₀, nM) | Compound | B1R (IC₅₀, nM) |
|---|---|---|---|---|---|
| 1 | 40 | 26 | 54.4 | 51 | 17.8 |
| 2 | 148 | 27 | 25.7 | 52 | 34.9 |
| 3 | 1330 | 28 | 24.5 | 53 | 48.1 |
| 4 | 5320 | 29 | 34.6 | 54 | 67.9 |
| 5 | 162 | 30 | 35.4 | 55 | 8.9 |
| 6 | 23.6 | 31 | 19.2 | 56 | 7.6 |
| 7 | 57.8 | 32 | 14.2 | 57 | 8.1 |
| 8 | 14.9 | 33 | 14.6 | 58 | 8.6 |
| 9 | 45.9 | 34 | 21.4 | 59 | 35.4 |
| 10 | 18.3 | 35 | 92.9 | 60 | 247 |
| 11 | 14.6 | 36 | 48.3 | 61 | 92.2 |
| 12 | 148 | 37 | 72.7 | 62 | 60.2 |
| 13 | 26.2 | 38 | 83.7 | 63 | 7708 |
| 14 | 23.5 | 39 | 82.4 | 64 | 9904 |
| 15 | 39.8 | 40 | 2039 | 65 | 43.5 |
| 16 | 28.2 | 41 | 448 | 66 | 1648 |
| 17 | 29.6 | 42 | 109 | 67 | 13319 |
| 18 | 9.9 | 43 | 95.2 | 68 | 7495 |
| 19 | 1140 | 44 | 106 | 69 | 4913 |
| 20 | 32.3 | 45 | 103 | 70 | 303 |
| 21 | 21.1 | 46 | 74.6 | 71 | 66.1 |
| 22 | 30.2 | 47 | 93.9 | 72 | 12005 |
| 23 | 44.6 | 48 | 372 | 73 | 13720 |
| 24 | 35.7 | 49 | 9.6 | | |
| 25 | 25.2 | 50 | 54.8 | | |

From the data in the above table, it can be seen that the compounds of Examples 1-73 (Table 1) have inhibitory activity against B1R. In contrast, those compounds have low inhibitory activity against B2R, with IC₅₀>30000nM (FLIPR method). Therefore, it can be shown that all of those compounds have good selectivity for B1R.

In addition, it has been established in the present invention for the first time that the stereochemistry configuration of cyclohexylurea covered by Formula Ib also has a certain effect on the B1 antagonist activity. For example, for Compound 61 and Compound 62, the activity of Compound 62 in trans configuration is higher than that of Compound 61 in cis configuration.

### Test Example 2. Pharmacokinetics in viro in rats and liver microsome metabolism in vitro

1. Administration to animals: SD rats were used, and the compound was administered by intravenous injection (3 mg/kg) or by intragasric administration (30 mg/kg). At different time points after administration, blood samples were collected into EDTA blood sample collection tubes. Plasma was separated by centrifugation and stored at -20°C for the analysis afterwards.
2. Handling of blood samples and LC-MS/MS analysis: the standard curve and formulation and processing of quality control sample: The compound stock solution was diluted with 50% methanol in water to form a standard curve working solution containing each compound at a concentration of 40-20000 ng/mL and a quality control working solution of 120, 1200, and 16000 ng/mL. 47.5 µL of blank rat plasma were respectively taken, and added into 2.50 µL of standard curve working solution or the quality control working solution, to formulate the standard curve samples containing the compound at a concentration of 2.00-1000.00 ng/mL and the quality control samples having a concentration of 6.00, 600.00 and 800.00 ng/mL. 400 µL of acetonitrile (containing 5 ng/mL of verapamil as an internal standard) was added respectively. The samples were vortexed for 3 min, centrifuged at 20000 rcf, 4°C for 15 min. The supernatant was taken for LC-MS/MS analysis.
3. Treatment of the compound blood samples: 5 µL of plasma sample was taken, to which 95 µL of blank rat plasma was added, and 800 µL of acetonitrile (containing 5 ng/mL of verapamil as an internal standard) was added. The samples were vortexed for 3min, centrifuged at 15000 rcf, 4°C for 10 min. The supernatant was taken for LC-MS/MS analysis (chromatographic column: ACQUITY UPLC^{®} BEH C18 2.1x50 mm 1.7 µm; mobile phase A: 0.1% formic acid in water; mobile phase B: acetonitrile; flow rate: 0.35 mL/min).
4. Analysis of the results: pharmacokinetic parameters were fit and calculated by WinNonlin software using statistical matrix method, mainly including kinetic parameters Tmax, t_{1/2}, Cmax, AUC0₋ₜ, AUC0-∞, Cl, Vd, F, etc. The pharmacokinetic data were calculated and processed using Microsoft Excel, such as mean and standard deviation. The results were shown in Table 2A.

In addition, the LC-MS/MS method was used to determine the substrate metabolites of 5 major subtypes of human liver microsomal CYP450 enzymes (including CYP1A2, CYP2D6, CYP2C8, CYP2C9, and CYP3A4). The substrate method was used to determine the activity of human liver microsomal CYP450 enzyme. The established incubation system was verified using known inhibitors to investigate the inhibitory effect of the compound on CYP450 enzyme. For the enzyme induction test, a primary cultured human hepatocyte incubation system was used. 3-methylcholanthrene, phenobarbital, and rifampicin were used as positive control inducers of CYP1A2, CYP2B6, and CYP3A4, and phenacetin, bupropion, and testosterone were used as the substrate for CYP1A2, CYP2B6, and CYP3A4. The CYP450 enzyme activity was calculated by measuring the generation rate of 3 specific substrate metabolites in the incubation solution. Meanwhile, the total RNA was extracted from the human hepatocytes after incubation, and the real-time fluorescence quantitative PCR method was used to determine the mRNA expression level of CYP1A2, CYP2B6, and CYP3A4. The enzyme activity and mRNA transcription level were used as endpoints to investigate the inductive effect of the compound on human liver P450 isoenzymes CYP1A2, CYP2B6, and CYP3A4. The liver microsomal metabolism parameters *in vitro* are shown in Table 2B taking Compound 8 as an example.

**Table 2A. Pharmacokinetic parameters of Compound in vivo in rats**

| Administration route | Pharmacokinetic parameters | Compound 71 | Compound 8 |
|---|---|---|---|
| 3 mg/kg intravenous injection in rats | Clearance (L/h/kg) | 0.145 | 0.592 |
| | Half-life (h) | 0.93 | 0.56 |
| | Apparent volume of distribution (Vss) (L/kg) | 0.156 | 0.328 |
| | Area under the curve of plasma concentration-time (AUC) (h*ng/mL) | 20679 | 5089 |
| | Peak concentration (Cmax) (ng/mL) | 37523 | 16424 |
| 3 mg/kg oral administration in rats | Half-life (h) | 3.63 | 3.37 |
| | Peak time (h) | 2.72 | 0.78 |
| | Peak concentration (Cmax) (ng/mL) | 914 | 1389 |
| | Area under the curve of plasma concentration-time (AUC) (h*ng/mL) | 7480 | 3126 |
| | F% | 3.6 | 6.1 |

**Table 2B. Liver microsomal metabolism parameters in vitro of Compound 8**

| | |
|---|---|
| Human LM clearance (L/h/kg) | 0.51 |
| Rat LM clearance (L/h/kg) | 0.59 |
| CYP inhibition IC₅₀ [µM] | 1A2 (50), 2C8 (50), 2C9 (50), 2D6 (50), 3A4 (50) |
| CYP induction IC₅₀ [µM] | CYP1A2, CYP2B6, CYP3A4: no significant inductive effect |

### Test Example 3. Effect of the compound in rat model of acute lung injury

1. Experimental aminals: SPF-grade male Wistar rats, weighed 180-220 g, 6-8 weeks old.
2. Drugs and reagents: LPS (0000120154, SIGMA); dexamethasone (WXBD5583V, SIGMA); Compound 71; Compound 8; Evans Blue: (C2130098, Aladdin).
3. Establishment of animal model: The rats were adaptively fed for one week. After the animals were anesthetized, 3 mg/kg LPS (B8) was injected intratracheally to prepare the rat model of acute lung injury.
4. Animal grouping and administration: The animals were randomly divided into 6 groups (12 rats per group) of negative control group (LPS was replaced by normal saline), model control group, positive control group (dexamethasone sodium phosphate injection, 5 mg/ml), Compound 71 group and Compound 8 group. At 2 hours before modeling (before tracheal injection of LPS), the test drug (30 mg/kg, the vehicle was 0.3% DMSO in saline) was intraperitoneally injected. At 1 hour after modeling, the test drug was administered once again (at the same dose). At 12 hours after modeling, the animals were sacrificed, and the samples were collected for the tests of various indexes.
5. Experimental methods
5.1 Test of pulmonary vascular permeability: 5 animals were randomly assigned to each of the groups. At 30 mininutes before sacrificing the animals, Evens Blue (20 mg/kg) was injected via the tail vein. At 30 minutes after the injection, the animals were immediately sacrificed. 10 mL of cold PBS was perfused into the heart to flush the blood in the pulmonary vessels. The superior lobe of the left lung was weighed. The lung tissue was cut into pieces, and added to formamide at 3 mL/100 mg of lung tissue, and incubated at 37°C for 24 hours. The absorbance was measured at 620 nm with a microplate reader. Meanwhile, solutions of Evens Blue in formamide at various concentrations were prepared to make a standard curve. The content of Evens Blue in the lung tissue was calculated.
5.2 ELISA method: 5 rats per group. The serum, alveolar lavage fluid, and tissue homogenate of one lung of each of the rats were subjected to ELISA assay to determine the TNF-α and IL-6 levels.

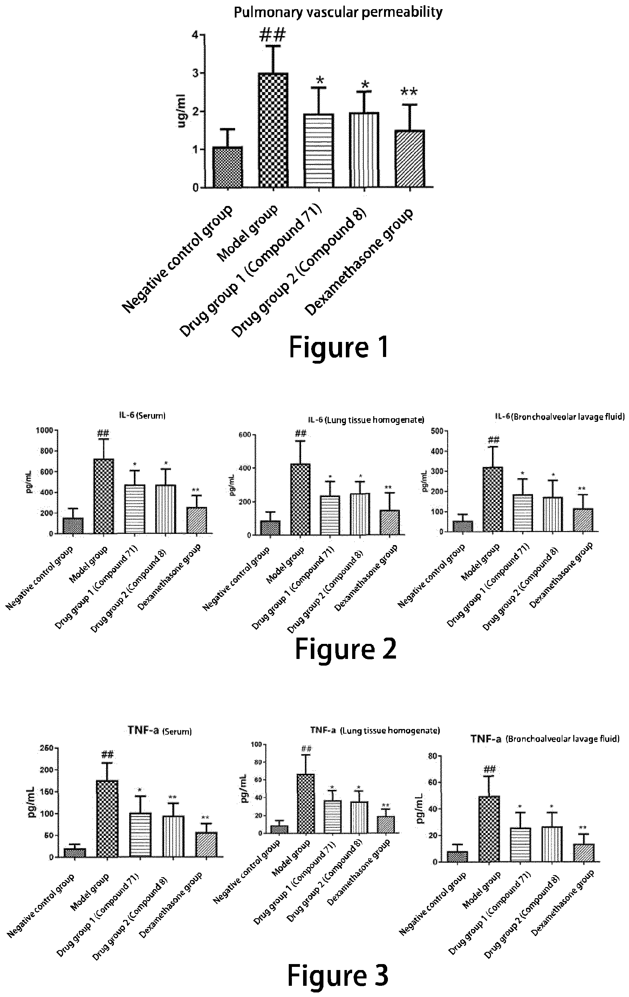

6. Experimental results
6.1 Vascular permeability in the rat model: it can be see from the result for the test of vascular permeability that the vascular permeability is sharply increased in the model group. Taking the model group as the control, both the test drug group and the positive control group of dexamethasone have significantly reduced the vascular permeability, as shown in Table 3 and Figure 1.

**Table 3. Vascular permeability of each group in the rat model**

| Groups | | EB (µg/mL), (n=5) |
|---|---|---|
| Negative control group | | 1.08 ± 0.46 |
| Model group | | 3.00 ± 0.70## |
| Drug group 1 (Compound 71) | | 1.94 ± 0.66* |
| Drug group 2 (Compound 8) | | 1.97± 0.53* |
| Dexamethasone group | | 1.50 ± 0.65** |
| Note: ## indicates P<0.01 as compared with the negative control group; * indicates P<0.05 as compared with the model group; ** indicates P<0.01 as compared with the model group. | | |

6.2 Expression levels of IL-6 and TNF-α in the rat model: TNF-α and IL-6 as representative inflammatory factors were determined by ELISA to observe the degree of systemic inflammatory response (in serum) and the degree of local inflammatory response in the lung tissue. It can be seen from the results that Compounds 8 and 71 could effectively reduce the expression levels of IL-6 and TNF-α, thus proving their efficacy in controlling inflammatory response, as shown in Table 4, Figure 2 and Table 6, Figure 3.

**Table 4. Expression levels of IL-6 in serum, lung tissue, and bronchoalveolar lavage fluid samples of the rats**

| Groups | IL-6 (serum) | IL-6 (homogenate of lung tissue) | IL-6 (bronchoalveolar lavage fluid) |
|---|---|---|---|
| Negative control group | 156.50±87.78 | 88.81±50.32 | 55.26±30.65 |
| Model group | 726.00±188.41^{##} | 428.42±132.83^{##} | 320.81±99.27^{##} |
| Drug group 1 (Compound 71) | 473.14±134.88* | 236.26±84.91* | 184.29±75.57* |
| Drug group 2 (Compound 8) | 470.76±151.60* | 250.10±70.26* | 171.55±81.93* |
| Dexamethasone group | 255.58±109.87** | 150.17±100.46** | 115.59±67.57** |

| | | | |
|---|---|---|---|
| Note: ## indicates P < 0.01 as compared with the negative control group; *: indicates P < 0.05 compared with the model group; **: indicates P<0.01 compared with the model group. | | | |

**Table 5. Expression levels of TNF-α in serum, lung tissue, and bronchoalveolar lavage fluid samples of the rats(ELISA)**

| Groups | TNF-α (serum) | TNF-α (homogenate of lung tissue) | TNF-α (bronchoalveolar lavage fluid) |
|---|---|---|---|
| Negative control group | 20.59±9.21 | 8.95±5.22 | 8.33±5.10 |
| Model group | 176.06±39.67^{##} | 66.86±21.46^{##} | 49.83±14.94^{##} |
| Drug group 1 (Compound 71) | 101.63±37.40* | 37.03±10.88* | 25.73±11.35* |
| Drug group 2 (Compound 8) | 94.89±28.07** | 3 5.74±11.82* | 26.72±10.35* |
| Dexamethasone group | 57.26±19.27** | 19.17±7.55** | 13.62±7.33** |

| | | | |
|---|---|---|---|
| Note: ## indicates P<0.01 as compared with the negative control group; *: indicates P<0.05 as compared with the model group; **: indicates P<00.01 as compared with the model group | | | |

The above data demonstrate that the test compounds can significantly improve the LPS-induced increase in vascular permeability and reduce the levels of IL-6 and TNF-α in blood, alveolar fluid and lung tissue in rats, confirming the therapeutic effect of B1R inhibitors in acute pulmonary edema.

### Test Example 4. Effect of the compound in rat model of diabetic retinopathy

1. Analysis of drug concentration in rat retinas after intraocular injection of the compound
   1.1 Experimental aminals: SPF-grade male Wistar rats weighed 180-220 g.
   1.2 Drugs and reagents: Compound 71; sodium chloride injection (Shandong Qidu Pharmaceutical Co., Ltd., Batch No.: 3B151014124); acetonitrile (chromatographically pure, TEDIA, USA); methanol (chromatographically pure, SIGMA-ALDRICH product); triethylamine (analytically pure, Hangzhou Gaojing Fine Chemical Co., Ltd.).
   1.3 Experimental grouping and administration: according to the sampling time, 33 male Wistar rats were divided into 11 groups (three rats per group) of: (1) control group; (2) 0 h; (3) 2 h; (4) 6 h; (5) 24 h; (6) 48 h; (7) 72 h; (8) 5 d; (9) 7 d; (10) 10 d; (11) 14 d. Three rats in the control group were injected with solvent (sterile normal saline) intraocularly, and the remaining 30 rats were injected with Compound 71 intraocularly at a concentration of 0.5% by using a 5 µL microinjector, and 5 µL of drug was injected per bulbus oculi.
   1.4 Sampling and detection index: The retina of the rat in the control group was taken on Day 14 (14 d), and the retina of the remaining 30 rats was taken at the corresponding time points. The retina were frozen in liquid nitrogen, and then subjected to the subsequent HPLC detection.
   1.5 HPLC detection method:
      1.5.1 Chromatographic conditions: The mobile phase was chromatographic grade acetonitrile-water = 25-75 (v/v). The flow rate was 0.5 mL/min. The column temperature was 25°C. The UV detection wavelength was 254 nm. The injection volume was 20 µL.
      1.5.2 Sample processing and preparation: the sample of the rat retina was taken out from the ultra-low temperature (-80°C) refrigerator, accurately weighed and placed in a glass homogenizer, and ethyl acetate as an organic solvent pre-cooled in a 4°C refrigerator was added at a ratio of 100 mL/g. The homogenizer was placed in an ice bath for homogenization. After homogenization, all the homogenate was poured into a 5 mL stoppered centrifuge tube. The centrifugation was perforemed at a low temperature of 4°C for 5 min (5000 r/min). All the organic phase as the upper layer was accurately taken out and placed in a glass tube with conical bottom. The remaining residue of retina was extracted with 2 mL ethyl acetate repeatedly once. After centrifugation, the two organic phases were combined. Then, the centrifuge tube with conical bottom was blowed for dryness in a nitrogen flow in a 45°C water bath. The residue was redissolved with 0.1 mL of chromatographic methanol, vortex mixed for 1 min, and centrifuged for 5 min at a high speed of 18000 r/min. 20µL of the supernatant was taken, and analyzed in a high-performance liquid chromatography system.
      1.5.3 Sample determination: After intraocular injection of 5 µL of 0.5% Compound 71, the retina was taken at different time points to analyze the drug content of the test compound in the retina.
      1.5.4 Experimental results and conclusions: According to the retinal drug concentration-time curve, it is in conformity to the non-absorption twocompartment model. According to the pharmacokinetic non-absorption twocompartment model theory, the pharmacokinetic parameters of the drug were calculated. The results were shown in Table 6.

**Table 6. Pharmacokinetic parameters for intraocular injection of Compound 71**

| | |
|---|---|
| Distribution half-life | 6.97h |
| Elimination half-life | 407.65h |
| Area under the time curve AUC | 901.40 mg·h/L |
| Apparent distribution volume | 0.001676 L |
| Total clearance CLT | 5.55×10⁻⁵ L/h |

After intraocular injection of 0.5% Compound 71 (5 µL per eye) in rats, on Day 7, the retinal drug concentration of Compound 2 was 1.54 µM on day 7, which was 23 times higher than its IC₅₀ value. On Day 14, the retinal concentration was 1.24 µM, which was 19 times higher than its IC₅₀ value. Therefore, the pharmacokinetic parameters for intraocular Compound 71 can fulfil the preliminary requirement of long-term efficacy for administration by intraocular injection.

### 2. Analysis of intraocular and retinal drug concentration in rats after administration of the compound by eye drop

2.1 Experimental aminals: SPF grade male SD rats, weighed 180-200 g, were used and adaptively fed for one week.

2.2 Drugs and reagents: Compound 71 and Compound 8; sodium chloride injection (Guangxi Yuyuan Pharmaceutical Co., Ltd.); sterile filter having 0.22 µm pore size (Millex).

2.3 Experimental animal grouping: the animals were randomly divided into 3 groups (6 rats per group) of a negative control group (normal saline), a Compound 71 group and a Compound 8 group.

2.4 Administration method and sampling: 2% Compound (10 µl) dissolved in normal saline (after filtration) was dropped into the surface of each rat's eye with a pipette for 10 s. The eye drop was applied twice a day for a total of 7 days. Samples were taken at 1 and 16 hours after the second administration on Day 7 of administration to determine the ocular distribution concentration of the drug.

2.5 Detection of secretion of porphyrin: 16 hours after the second administration on Day 7 of administration to rat, before sacrificing the rat, the eyes were rinsed with normal saline by pipette. The rinsing solution was frozen at -80°C for subsequent detection of irritant inflammatory response on the surface of the bulbus oculi.

### 2.6 Measurement of the drug content in the eye tissue by HPLC:

The first batch of samples: 1 hour after the second eye drop on Day 7: 3 rats (2 males and 1 female) were taken from each group. The bulbus oculi were removed, and were thoroughly rinsed for 3 times with normal saline using a pipette. The washing solution was quickly dried with a qualitative filter paper. The cornea, vitreous body and retina were separated under a stereomicroscope, and frozen at -80°C for subsequent measurement.

The second batch of samples: On the morning of Day 8, i.e., at 16 hours after the second eye drop on Day 7: the remaining 3 rats (1 male and 2 females) were taken from each group. The bulbus oculi were removed, and were thoroughly rinsed for 3 times with normal saline using a pipette. The washing solution was quickly dried with a qualitative filter paper. The cornea, vitreous body and retina were separated under a stereomicroscope, and frozen at -80°C for subsequent detection.

### 2.7 Experimental results and conclusions

2.7.1 Ophthalmic observation of the animals: After eye drops, the eyes of the rats in the negative control group and the test compound group showed no abnormalities, no redness, swelling, congestion, etc.

2.7.2 Result of measurement of secretion of porphyrin: it was further determined whether inflammation or bleeding in the eyes occurred after eye drops by ELISA essay of free protoporphyrin (FEP) in erythrocytes. As indicated by the reults, the level of FEP in the sample of eye rinsing solution was at a low concentration level for the rats in both the test drug group and the negative control group. There was no significant difference (P>0.05). The results are shown in Table 7.

**Table 7. FEP expression levels in the sample of eye rinsing solution for the rat**

| | | Left eye (ng/mL) | Right eye (ng/mL) |
|---|---|---|---|
| 1h | Negative control group | 1.54±0.04 | 1.51±0.06 |
| | Test group of Compound 71 | 1.50±0.02 | 1.54±0.08 |
| | Test group fo Compound 8 | 1.53±0.03 | 1.45±0.02 |
| 16h | Negative control group | 1.51±0.05 | 1.52±0.06 |
| | Test group of Compound 71 | 1.61±0.06 | 1.55±0.09 |
| | Test group fo Compound 8 | 1.57±0.08 | 1.57±0.06 |

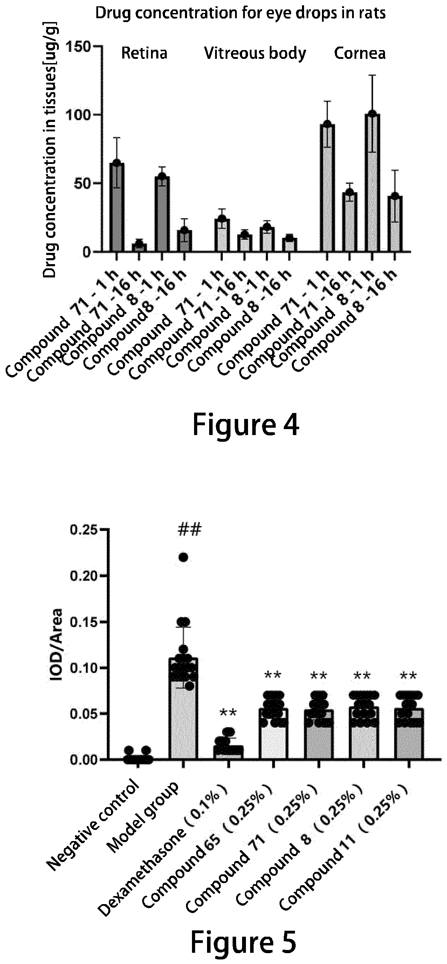

2.7.3 Results of HPLC measurement of drug content in eye tissue: Under the selected chromatographic conditions for analysis, drug impurities were well separated. The chromatograms for a sample of standard solution, a sample of blank homogenate, a smple of blank homogenate added with Compound 71 or Compound 8, and a sample of retinal homogenate samples were compared. The comparison results showed that no interference peaks occured at the corresponding retention time of the drug. Under the selected HPLC measurement conditions, the average recovery rates for two compounds under test were both above 90%. The intra-day and inter-day coefficients of variation were both less than 10%, which indicates that the test drugs such as Compound 71 and Compound 8 under test had a good recovery rate in the retinal homogenate. The precision can meet the measurement standard. As shown by the accuracy results, the accuracy for all of the high, medium and low concentration retinal spiked quality control samples (QC) within the drug curve range was higher than 95%. After sampling, the drug content in eye tissue was measured by HPLC, and the results were shown in Table 8 and Figure 4.

**Table 8. Content of test compounds in the sample of eye tissue**

| Eye tissue | test grouping | Compound 71 (µg/g) | Compound 8 (µg/g) |
|---|---|---|---|
| Retina | Normal saline group | / | / |
| | Group of 1 h after administration | 64.85±18.32 | 55.03±6.91 |
| | Group of 16 h after administration | 6.07±3.06 | 15.81±8.41 |
| Vitreous body | Normal saline group | / | / |
| | Group of 1h after administration | 24.23±7.07 | 18.18±4.65 |
| | Group of 16 hours after administration | 12.74±3.49 | 10.13±2.67 |
| Cornea | Normal saline group | / | / |
| | Group of 1 h after administration | 93.09±16.92 | 100.84±28.21 |
| | Group of 16 h after administration | 43.48±6.59 | 40.69±18.84 |

As shown by the results, the content of the test compound was highest in the cornea, followed by the retina, and the lowest in the vitreous body. Meanwhile, the contents of Compound 71 and Compound 8 in those three tissues 16 hours after administration were significantly lower than 1 hour after administration.

2.7.4 Conclusion: The test compound exhibited good safety when administered via eye drops without manifest ophthalmic irritation. Meanwhile, the test results of this experiment prove that the compound of the present invention can enter the eye via eye drops, and arrive at the expected action site of retina, and the level of the compound measured after sustained for 16 hours is much higher than its effective concentration (IC50). The test results confirmed the possibility of the compound in exerting a therapeutic effect when administered via eye drops.

### 3. Effects of the compound in a rat model of diabetic retinopathy

3.1 Laboratary animals: SPF-grade male Wistar rats, weighed 180-220 g.

3.2 Drugs and reagents: The test compounds include Compounds 65, 71, 8 and 11; STZ (MACKLIN, Catalog No.: S817944); dexamethasone (MACKLIN, Catalog No.: D829854); Evans blue: aladdin (Catalog No. E104208); Concanavalin A/FITC (SIGMA, Catalog No.: C7642); Albumin (SIGMA, Catalog No.: A6414); Hematoxylin (Sigma, Catalog No.: H9627); Eosin (Sigma, Catalog No.: E6003); RNA rapid extraction kit (Generay, Catalog No.: GK3016); reverse transcription kit HiScript-II Q RT SuperMix for qPCR (Vazyme, Catalog No.: R222-01); qPCR reagent ChamQ SYBR Color qPCR Master Mix (Vazyme, catalog No.: Q411-02); experimental primers were synthesized and purified by Shanghai Sunny Biotechnology Co., Ltd.; the remaining reagents were domestic analytical reagents.

3.3 Animal modeling and grouping: After one week of adaptive feeding, 134 male Wistar rats (weighed 220-260 g) were administered 65 mg/kg of STZ by one-time intraperitoneal injection to prepare a rat model of type I diabetic. The rats were fasting but water was allowable overnight for 15 h. The formulated STZ solution was intraperitoneally injected according to body weight. After 1 h, the diet was resumed and blood glucose was measured at 72 h. The successfully modeled diabetic rats (120 rats) were selected for continued feeding, and randomly divided into the following 6 groups according to blood glucose (20 rats per group), plus a group of normal rats with no STZ injected as a negative control group. See Table 9.

**Table 9. Effects of Compound on Rat Diabetic Retinopathy Model - experimental grouping**

| Experimental group | Drugs administered | Number |
|---|---|---|
| Negative control group | Normal rats | 20 |
| Model group | Normal rats + STZ | 20 |
| Positive drug control group | Normal rats + STZ + Dexamethasone | 20 |
| Drug group 1 | Normal rats + STZ + Compound 65 (0.25%) | 20 |
| Drug group 2 | Normal rats + STZ + Compound 71 (0.25%) | 20 |
| Drug group 3 | Normal rats + STZ + Compound 8 (0.25%) | 20 |
| Drug group 4 | Normal rats + STZ + Compound 11 (0.25%) | 20 |

3.4 Administration: The rats continued to be fed until blood glucose was measured once before administration. After blood glucose reached the standard, drugs were intravitreally injected, and indexs were subsequently tested after 14 days. Blood glucose was measured once before sampling.

3.5 Evans blue staining method (7 groups, 6 rats per group): Evans blue was intravenously injected. 2 hours later, normal saline was perfused into the left ventricle. The retina was taken to compare between the stained area and the unstained area to detect retinal vascular permeability.

3.6 Retinal leukocyte stasis detection method (7 groups, 6 rats per group): PBS was perfused into the left ventricle for 1 min to remove red blood cells and other substances. Concanavalin A (FITC (Sigma, C7642) was perfused (20 µg/ml in PBS, 5 mg/kg) to mark adherent leukocytes. 4% PFA was perfused for 4 min for fixation. 1% Albumin (Sigma, A6414) (in PBS) was perfused for 1 min, and then PBS was perfused for 2 min. The retina was taken for retinal wholemount. Thereafter, the stasis of leukocytes on the vascular endothelium was observed under a fluorescence microscope.

3.7 QPCR method (7 groups, 6 rats per group): The retina of the left eye was separated. Total RNA was extracted, and reverse transcribed into cDNA. QPCR was used to detect the level of B1R, COX-2, VEGF-A, VEGF receptor type 2, IL-1b, and iNOS.

### 3.8 Experimental results and conclusions

3.8.1 Observation of the animals and measuremt of the blood glucose: After STZ administration, the blood glucose of all experimental animals was increased significantly, and remained at a stable high level until the retina was removed for sampling. As shown in Tables 10 and 11, it was proved that a rat model of diabetes was successfully established.

**Table 10 Blood glucose level of rats before administration (x±sd)**

| Groups | Blood glucose concentration of the rats in Batch 1(mmol/L) | Blood glucose concentration of the rats in Batch 2 (mmol/L) |
|---|---|---|
| Negative control group | 3.52±1.65 | / |
| Model group | 19.62±2.66** | 18.69±1.43** |
| Dexamethasone (0.1%) | 19.65±2.23** | 18.24±1.17** |
| Compound 65 (0.25%) | 20.51±2.36** | 18.01±1.29** |
| Compound 71 (0.25%) | 19.05±0.77** | 18.49±1.39** |
| Compound 8 (0.25%) | 20.89±2.43** | 18.70±1.24** |
| Compound 11 (0.25%) | 20.48±2.16** | 18.64±1.91** |

| | | |
|---|---|---|
| Note: * indicates p < 0.05, ** indicates p < 0.01, as compared with the negative control group. | | |

**Table 11 Blood glucose level of rats before sampling (x±sd)**

| Groups | Blood glucose concentration of the rats in Batch 1 (mmol/L) | Blood glucose concentration of the rats in Batch 2 (mmol/L) |
|---|---|---|
| Negative control group | 2.70±0.95 | / |
| Model group | 20.49±1.64** | 18.90±2.06** |
| Dexamethasone (0.1%) | 19.19±1.26** | 18.93±0.58** |
| Compound 65 (0.25%) | 19.15±1.25** | 18.27±1.98** |
| Compound 71 (0.25%) | 19.52±1.07** | 18.53±1.93** |
| Compound 8 (0.25%) | 19.03±1.51** | 18.56±1.40** |
| Compound 11 (0.25%) | 19.02±1.50** | 18.57±1.41** |

| | | |
|---|---|---|
| Note: * indicates p < 0.05, ** indicates p < 0.01, as compared with the negative control group. | | |

Blood glucose was tested on D3 after STZ injection. The blood glucose value was significantly higher than that of animals not injected with STZ. After confirming that the diabetes model was successfully established, the animals continued to be fed. After 8 weeks of feeding, the rats in the normal group had conditions such as transparent lenses, clear fundus, and no bleeding or leakage. In the model group, conditions such as posterior subcapsular opacity and anterior subcapsular opacity of the lens were occasionally observed in the model group. Most of the rats did not exhibit a manifest external pathological change. The blood glucose test results after administration and before sampling showed that the blood glucose concentration in each of the administration groups and the model group was significantly higher than that in the control group, and there was no significant difference as compared with before administration.

3.8.2 Evan blue staining: as shown in Table 12 and Figure 5, the negative control group has almost no permeability; the retinal permeability of the rats in the model group was increased significantly as compared with the negative control group. On this basis, injection of dexamethasone as a positive drug or the drug under test can reduce the permeability in model rats, and the positive drug dexamethasone has the most significant effect.

**Table 12 Test results of retinal vascular permeability in the rats**

| Groups | IOD/Area |
|---|---|
| Negative control group | 0.00±0.00 |
| Model group | 0.11±0.03^{##} |
| Dexamethasone (0.1%) | 0.02±0.01** |
| Compound 65 (0.25%) | 0.06±0.01** |
| Compound 71 (0.25%) | 0.05±0.01** |
| Compound 8 (0.25%) | 0.06±0.01** |
| Compound 11 (0.25%) | 0.06±0.01** |

| | |
|---|---|
| Note: *^{##}P<*0.01*, ^{#}P<*0.05*,* as compared with the negative control group; ***P*<0.01, **P*<0.05, as compared with the positive control group. | |

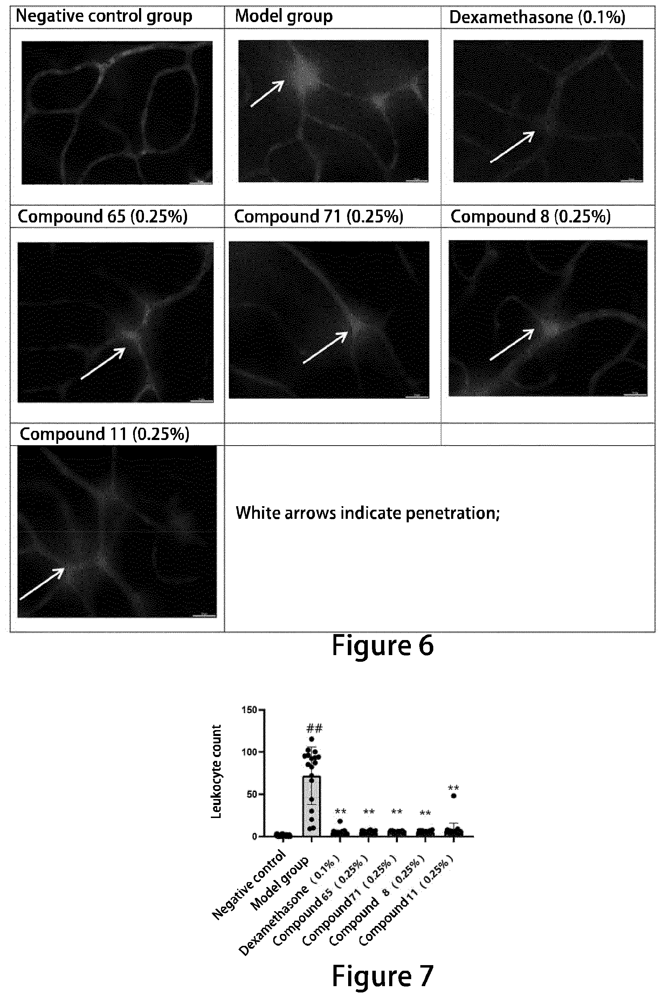

In addition, as shown in the fluorescent staining results in Figure 6, almost no vascular permeability was observed in the retina of the rats in the negative control group. As compared with the negative control group, the retinal vascular permeability in the rats in the model group was significantly increased. On this basis, intraocular injection of dexamethasone or Compound 65, Compound 71, Compound 8 or Compound 11 significantly reduced the retinal vascular penetration in rats.

3.8.3 Retinal leukocyte stasis detection: as shown in Table 13 and Figure 7, the number of retinal leukocytes of the rats in the negative control group was very low. As compared with the negative control group, the number of retinal leukocytes of the rats in the model group was significantly increased. On this basis, the number of retinal leukocytes of the model rats was significantly reduced after intraocular injection of dexamethasone, or Compound 65, Compound 71, Compound 8 or Compound 11 under test.

**Table 13. Detection Results of retinal leukocyte stasis in the rats**

| Groups | Leukocyte count |
|---|---|
| Negative control group | 1.13±0.92 |
| Model group | 71.78±32.96^{##} |
| Dexamethasone (0.1%) | 4.17±3.21** |
| Compound 65 (0.25%) | 5.14±1.20** |
| Compound 71 (0.25%) | 5.22±1.13** |
| Compound 8 (0.25%) | 5.32±1.38** |
| Compound 11 (0.25%) | 7.20±8.44** |

| | |
|---|---|
| Note: *^{##}P<*0.01, *^{#}P<*0.05, as compared with the negative control group; ***P*<0.01, **P*<0.05, as compared with the model group. | |

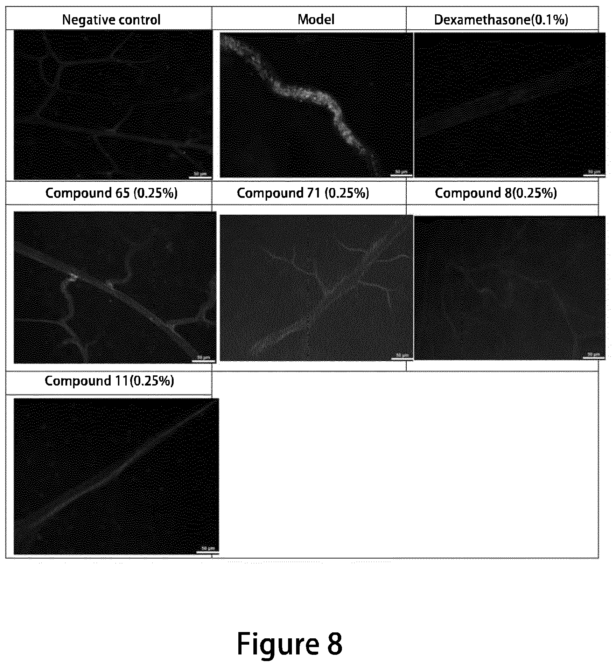

In addition, as shown in the fluorescent staining results in Figure 8, almost no stasis of fluorescently stained leukocytes was observed in the retina of the rats in the negative control group. As compared with the negative control group, stasis of leukocyte in the retina of rats in the model group was significantly increased. On this basis, the number of leukocyte stasis in the retina of rats was significantly reduced after intraocular injection of dexamethasone, or Compound 65, Compound 71, Compound 8 or Compound 11.

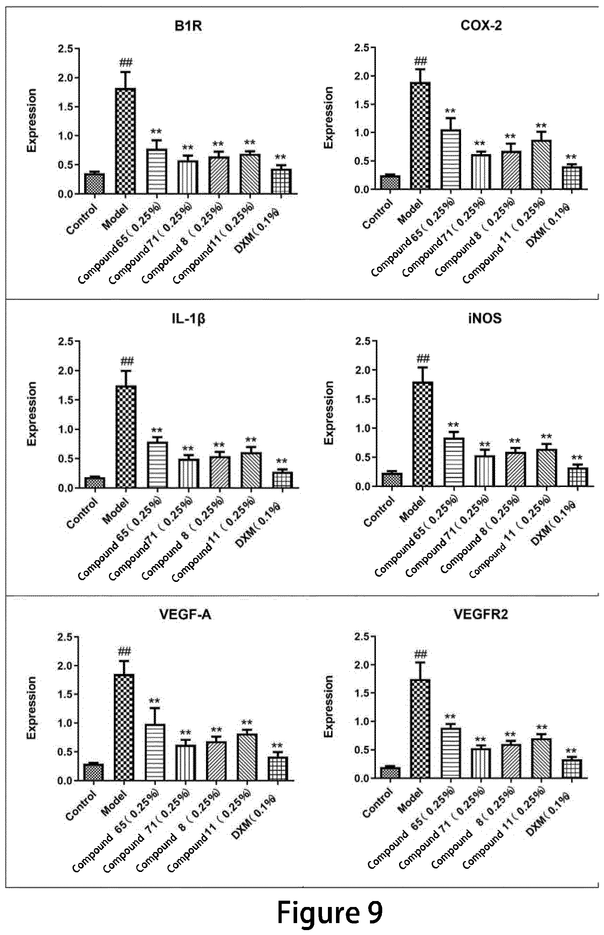

3.8.4 Expression levels of inflammation-related factors: as shown in Figure 9, the mRNA expression level for each of the retinal inflammation-related factors B1R, COX2, IL-1β, iNOS, VEGF-A, and VEGFR2 in the rats in the model group was recovered to different degrees as compared with the control group. However, the degree of recovery was relatively lower than that in the dexamethasone group.

3.8.5 Conclusions: as indicated by the results, each of the retinal vascular permeability, the number of retinal leukocyte stasis, and the levels of important related factors that cause inflammation in the rats in diabetic model group was increased significantly as compared with the negative control group, and was relived after treatment with dexamethasone, or Compound 65, 71, 8 or 11 under test as the drug.

In summary, the above compounds and dexamethasone can improve the retinal inflammatory response caused by diabetes, as well as reduce retinal vascular permeability, penetration and retinal leukocyte stasis number.

### Test Example 5. Study of efficacy of the compound in vivo in a mouse model of ragweed pollen-induced allergic conjunctivitis

### 1. Experimental method:

SPF-grade Balb/c mice (half male and half female, 6-8 weeks old) were used as experimental animals. On Day 0 and Day 6, ragweed pollen footpad injection was injected subcutaneously into the footpads of mice for sensitization (65 µl per mouse). On Days 10-14, in the model group, eye drop of ragweed pollen was applied to both eyes of the mice (10 µl per mouse) once a day. The eye drops were applied for 5 consecutive days to trigger local allergic conjunctivitis (Wu B, et al., Frontiers in Immunology, 2021, 12:4754). Eye drops of Compound 8 at concentrations of 0.5 and 2% and the eye drop of dexamethasone as a positive control drug at 0.1% were administered for 14 consecutive days, twice a day. On Day 14, within 30 minutes after the final challenge, the ocular clinical symptoms of the mice were observed under a microscope and scored according to the scoring criteria reported by Magone et al. (MerayoLloves J, et al., J Allergy Clin Immunol (1996) 97(5):1129-40; Magone MT, et al., Clin Immunol Immunopathol (1998) 87(1):75-84), the allergic response of the eyes were observed, including 4 indexes: conjunctival edema, conjunctival congestion, eyelid congestion and edema, and tearing. The scores were recorded as 0-3 points according to the severity (including none, mild, moderate and severe). The detailed scoring criteria were shown in Table 14. The left eye of the mouse was taken with the eyelid (keeping the conjunctiva intact), fixed with polyformaldehyde, sectioned in paraffin, Hematoxylin-Eosin stained (HE stained), and toluidine blue stained (for detection of mast cells). Photographs were taken to analyze the inflammatory cell infiltration of the conjunctival tissue, and observe whether the mast cells in the conjunctiva aggregated and whether a phenomenon of degranulation occurred. The expression of allergic indicators IL-4 and IgE in the conjunctival tissue was detected by immunohistochemistry.

**Table 14. Scoring criteria for observation of ocular clinical symptoms**

| Evaluation indicators | Grade score | Criteria |
|---|---|---|
| Conjunctival edema | 0 | No changes caused by disease |
| | 1 | Mild localized conjunctival edema |
| | 2 | Diffuse edema, involving the fornices |
| | 3 | Conjunctival edema causing shallow and narrow conjunctival sac |
| Conjunctival congestion | 0 | No changes caused by disease |
| | 1 | Mild diffuse vascular congestion |
| | 2 | Diffuse congestion, more pronounced near the fornices |
| | 3 | Conjunctival congestion with subconjunctival hemorrhage |
| Eyelid congestion and edema | 0 | No changes caused by disease |
| | 1 | Mild diffuse eyelid congestion and edema |
| | 2 | Diffuse eyelid congestion and edema with narrowed palpebral fissures |
| | 3 | Severe eyelid congestion and edema with difficulty in opening the eyelids |
| Tearing | 0 | No changes caused by disease |
| | 1 | Lacrimal gland accumulation at the caruncle |
| | 2 | epiphora beyond palpebral margin |
| | 3 | persistent epiphora |

### 2. Experimental results:

### 2.1 Observation of ocular clinical symptoms

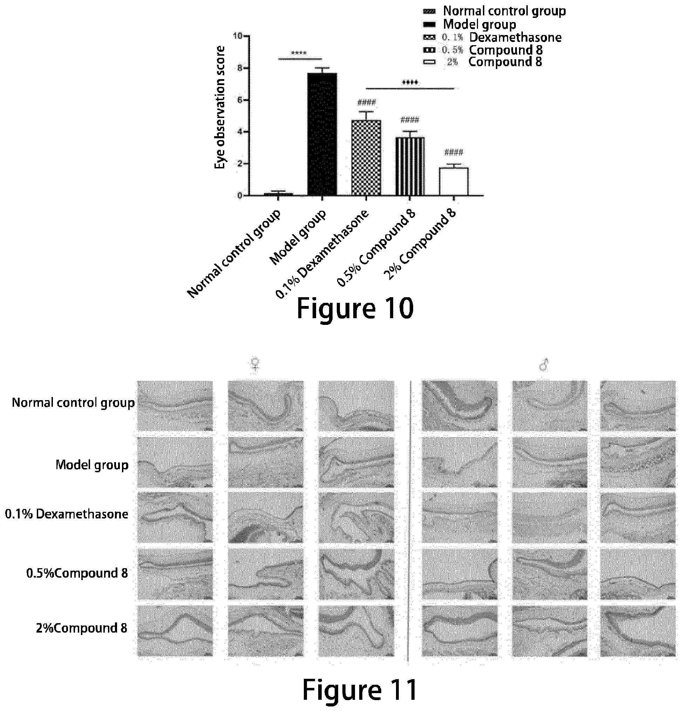

Within 30 minutes after the final challenge, the ocular clinical symptoms were observed and scored under a microscope. It can be seen that as compared with the mice of the normal control group, the eyes of the mice in the model group had more severe conjunctival edema and congestion with epiphora beyond palpebral margin and severe localized eyelid congestion and edema with a statistically significant difference. After treatment with 0.1% dexamethasone as a positive control or 0.5% Compound 8, the conjunctival edema and congestion was improved to mild-to-moderate, with diffuse mild eyelid congestion and edema with lacrimal gland accumulation at the caruncle, which was significantly different from the model group. After treatment with 2% Compound 8, part of the rats had mild localized conjunctival edema or lacrimal gland accumulation at the caruncle, while the symptoms of conjunctival congestion and eyelid congestion and edema disappeared and recovered to the normal level, and the symptom improvement was significantly superior over that of the positive control dexamethasone group (P<0.0001). As indicated by the results, Compound 8 improves ocular clinical symptoms in a concentrationdependent manner (see Table 15 and Figure 10).

**Table 15. Clinical symptom score results**

| Groups | Scores |
|---|---|
| Normal control group | 0.17±0.11 |
| Model group | 7.67±0.33* |
| Positive control group | 4.75±0.51^{#} |
| Compound 8 (0.5%) | 3.67±0.36^{#} |
| Compound 8 (2%) | 1.75±0.22^{◆} |

| | |
|---|---|
| Note: ***P*<0.0001, as compared with the normal control group; *^{##}P<*0.0001, as compared with the model group; *^{◆}P<*0.0001*,* as compared with the positive control group. | |

### 2.2 Hematoxylin-eosin staining and toluidine blue staining

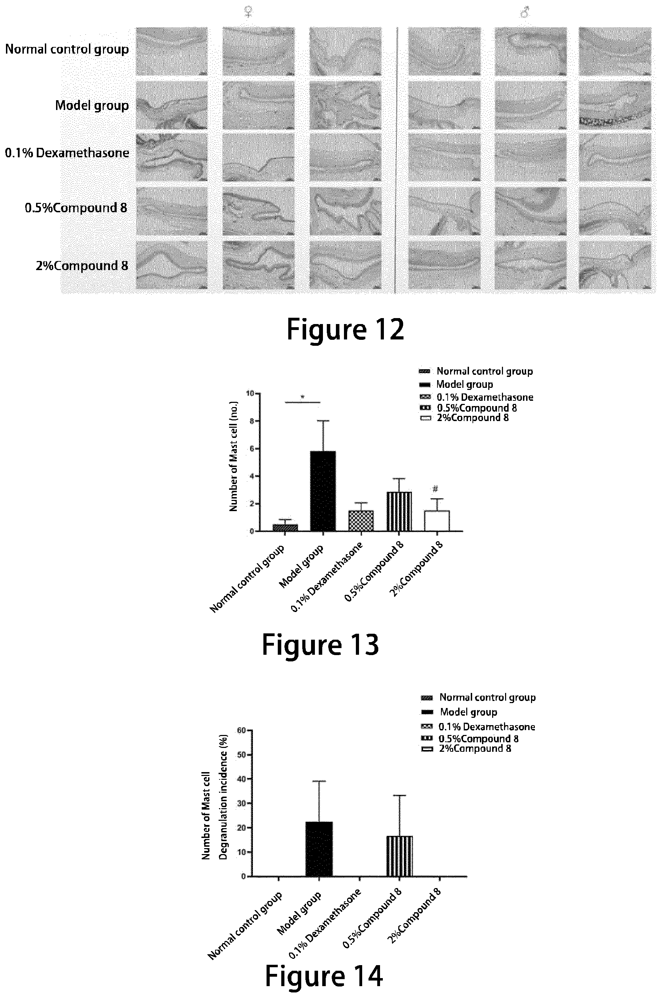

In the normal control group, the conjunctival tissue structure was intact, with no evidence of mast cell aggregation and degranulation, no significant lesions, and some samples showed hemorrhagic phenomena. A few samples had thinning of the conjunctival epithelium and visible inflammatory cell infiltration. In the model group, most samples showed inflammatory cell infiltration and loose tissue structure in the substantia propria of conjunctiva, accompanied by manifest mast cell degranulation and hemorrhage, with some samples showing thinning of the epithelial cells. In the positive control group treated with dexamethasone, most samples still exhibited loose conjunctival tissue, but there was a significant improvement in overall inflammatory cell infiltration, no manifest mast cell degranulation, and only a few samples showed hemorrhage and epithelial thinning. In the 0.5% Compound 8 group, inflammatory cell infiltration was still visible, some samples had epithelial thinning, and a few samples showed manifest mast cell degranulation. In the 2% Compound 8 group, some samples showed dilated capillaries, congestion, and loose tissue structure, but there was an improvement in inflammatory cell infiltration, and no mast cell degranulation was observed. It indicates that Compound 8 improves inflammatory cell infiltration and conjunctival tissue in a dose-dependent manner, and 2% Compound 8 significantly reduces mast cell degranulation in an allergic response (P<0.05). The results of HE staining and toluidine blue staining were shown in Figures 11 and 12, and the results of inflammatory cell count were shown in Figures 13 and 14.

### 2.3 Measurement of the expression of allergy indicators IL-4 and IgE in conjunctival tissue by immunohistochemistry

The mouse eye, including the eyelids (with intact conjunctiva), was sectioned after deparaffinization, and subjected to antigen retrieval and serum blocking. Diluted IL-4 or IgE antibodies were added and incubated overnight. After incubation with secondary antibodies, DAB staining was performed, followed by counterstaining with hematoxylin. The slides were then mounted and observed under an electron microscope, with photographs taken. Semi-quantitative analysis was conducted using Image-J software. Based on the cumulative optical density and regional area values of each image, the average optical density was calculated to detect the positive expression of IL-4 or IgE.

As indicated by the expression of IL-4 in ocular tissues, as compared with the normal control group (0.2±0.04), the expression of IL-4 in the ocular tissue of the model group was higher (0.38±0.08, about 1.8 times). After treatment with dexamethasone as a positive control or 2% Compound 8, the IL-4 level was reduced to a level similar to that of the normal control group (0.23±0.09 and 0.24±0.06, respectively). In addition, Compound 8 exhibits a concentrationdependent effect on the expression of IL-4 in the tissue. Although there was no significant difference in overall statistical analysis, there was a noticeable trend in the degree."

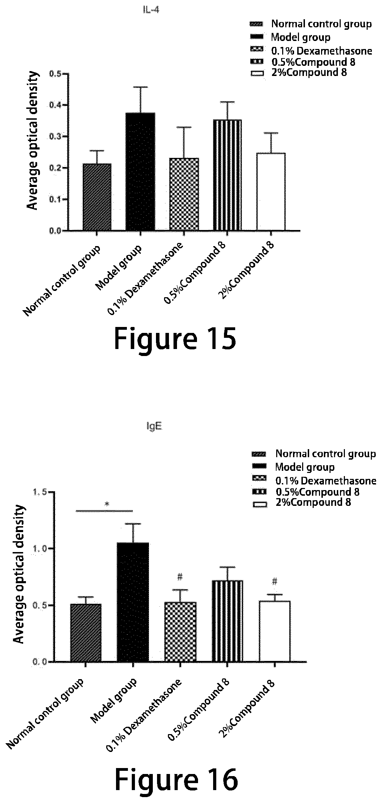

As indicated by the expression of IgE in ocular tissues, as compared with the normal control group (0.52±0.06), the IgE expression in the ocular tissue of the model group was higher (1.05±0.17, about 2 times) with a statistically significant difference (P< 0.05). both dexamethasone as a positive control or 2% Compound 8 significantly reduced the level of IgE in the tissue (0.53±0.11 and 0.54±0.06, respectively) to a level similar to that of IL-4 in the normal control group (P<0.05). 0.5 % Compound 8 could also reduce the expression of IgE to a certain extent (0.72±0.12). The efficacy of Compound 8 was dose-dependent on the concentration of IgE in the tissue. The measured results of IL-4 and IgE by immunohistochemistry are shown in Figures 15 and 16, respectively.

### 3. Experimental Conclusions

In summary, dexamethasone as a positive drug had a therapeutic effect on allergic conjunctivitis, and Compound 8 had a therapeutic effect on allergic conjunctivitis in a dose-dependent manner. The efficacy of 2% Compound 8 was comparable to that of dexamethasone.

The raw materials and equipment used in the present invention, unless otherwise specified, are all commonly used raw materials and equipment in the field. The methods used in the present invention, unless otherwise specified, are all conventional methods in the field.

The above description is merely a preferred embodiment of the present invention and does not limit the scope of the invention. Any simple modifications, viariation, and equivalent alteration made to the above embodiment according to the technical concept of the invention still fall within the protection scope of the claimed technical solution.

### Industrial Applicability

The present invention provides an N-tetrazolyl aryl urea-based derivative, which can be used as a bradykinin B1 receptor antagonist, and is useful in the prevention or treatment of diseases mediated by bradykinin B1 receptor. Therefore, it can be formulated into a corresponding medicament suitable for industrial application.

Although the present invention is described in detail herein, the present invention is not limited thereto, and technicians in the field of the present invention can make modifications based on the principles of the present invention. Therefore, all modifications made according to the principles of the present invention should be understood to fall within the protection scope of the present invention.

## Claims

1. An N-tetrazolyl aryl urea-based derivative, **characterized in that**: which is a compound represented by Formula I, or a cis-trans isomer thereof, or a pharmaceutically acceptable salt or solvate thereof; 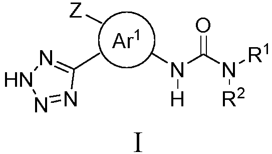 wherein:
R¹ and R² together with the N atom attached thereto form a 3- to 8-membered heterocyclic group containing at least 1 nitrogen atom, wherein the 3-to 8-membered heterocyclic group is optionally substituted by 1 to 3 of identical or different functional groups A, and the functional group A is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, alkylamino, dialkylamino, acetylamino and cycloamino; or
R¹ and R² together with the N atom attached thereto form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂, wherein the 8- to 12-membered bicyclic heterocyclic group comprises a spiro heterocyclic group, a bridged bicyclic heterocyclic group and a fused bicyclic heterocyclic group, and the 8- to 12-membered bicyclic heterocyclic group is optionally substituted by 1 to 3 of identical or different functional groups B, and the functional group B is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino; or
each of R¹ and R² is independently selected from the group consisting of the following groups:
(i) H, C₁-C₁₀ alkyl, C₂-C₁₀ alkenyl and C₂-C₁₀ alkynyl, wherein C₁-C₁₀ alkyl, C₂-C₁₀ alkenyl, or C₂-C₁₀ alkynyl is optionally substituted by 1 to 3 of identical or different functional groups C, and the functional group C is selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino;
(ii) phenyl, 5- to 6-membered heteroaryl, C₅-C₇ cycloalkyl and 5- to 7-membered heterocyclic group, wherein the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, 5- to 6-membered heteroaryl, C₅-C₇ cycloalkyl or 5- to 7-membered heterocyclic group is optionally substituted by 1 to 3 of identical or different functional groups D, and the functional group D is selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino;
Ar¹ is selected from the group consisting of phenyl, 5- to 6-membered heteroaryl, C₅-C₇ cycloalkyl and C₉-C₁₀ dicycloalkyl, wherein the 5- to 6-membered heteroaryl contains 1 or 2 heteroatoms or heteroatom-containing groups independently selected from the group consisting of NH, N, O, S, SO and SO₂, and the phenyl, 5- to 6-membered heteroaryl, C₅-C₇ cycloalkyl or C₉-C₁₀ dicycloalkyl is optionally substituted by 1 to 3 of identical or different functional groups E, and the functional group E is selected from the group consisting of hydroxyl, halogen, amino, cyano, nitro, C₁-C₆ alkyl, C₁-C₆- alkoxy, halogenated C₁-C₆ alkyl, halogenated C₁-C₆ alkoxy, alkylamino, dialkylamino, acetylamino, N-methyl-N-acetylamino and cycloamino;
Z is selected from the group consisting of phenyl, 5- to 6-membered heteroaryl, C₅-C₇ cycloalkyl, 5- to 7-membered heterocyclic group, C₉-C₁₀ dicycloalkyl, 9- to 10-membered fused bicyclic heterocyclic group, 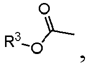 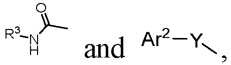
wherein the 5- to 6-membered heteroaryl, 5- to 7-membered heterocyclic group or 9- to 10-membered fused bicyclic heterocyclic group contains 1, 2 or 3 heteroatoms or heteroatom-containing groups independently selected from the group consisting of NH, N, O, S, SO and SO₂, and the phenyl, 5- to 6-membered heteroaryl, C₅-C₇ cycloalkyl, 5- to 7-membered heterocyclic group, C₉-C₁₀ dicycloalkyl or 9- to 10-membered fused bicyclic heterocyclic group is optionally substituted by 1 to 3 of identical or different functional groups F, and the functional group F is selected from the group consisting of hydroxyl, halogen, amino, cyano, nitro, C₁-C₆ alkyl, C₃-C₇ cycloalkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino, or alternatively, the functional group F forms a 5- to 8-membered heterocyclic ring fused to a phenyl, a 5- to 6-membered heteroaryl, a C₅-C₇ cycloalkyl, a 5- to 7-membered heterocyclic group, a C₉-C₁₀ dicycloalkyl, or a 9- to 10-membered fused bicyclic heterocyclic group,
R³ is selected from the group consisting of C₁-C₆ alkyl, C₃-C₇ cycloalkyl, C₃-C₇ cycloalkenyl, 5- to 7-membered heterocyclic group, phenyl and 5- to 6-membered heteroaryl, and the C₁-C₆ alkyl, C₃-C₇ cycloalkyl, C₃-C₇ cycloalkenyl, 5- to 7-membered heterocyclic group, phenyl or 5- to 6-membered heteroaryl is optionally substituted by 1 to 3 of identical or different functional groups G, and the functional group G is selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino,
Y is selected from the group consisting of O or NH, and Ar² is selected from the group consisting of phenyl, 5- to 6-membered heteroaryl, C₁₀-C₂₀ fused cyclic group, C₁-C₆ alkyl, 5- to 7-membered cycloalkyl, 5- to 7-membered heterocyclic group and C₁-C₆ alkoxy, the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, 5- to 6-membered heteroaryl, C₁₀-C₂₀ fused cyclic group, C₁-C₆ alkyl, 5- to 7-membered cycloalkyl, 5- to 7-membered heterocyclic group, or C₁-C₆ alkoxy is optionally substituted by 1 to 3 of identical or different functional groups H, and the functional group H is selected from the group consisting of hydroxyl, halogen, amino, cyano, nitro, C₁-C₆ alkyl, C₁-C₆ alkoxy, halogenated C₁-C₆ alkyl, halogenated C₁-C₆ alkoxy, alkylamino, dialkylamino, acetylamino, N-methyl-N-acetylamino and cycloamino.

2. An N-tetrazolyl aryl urea-based derivative, **characterized in that**: which is a compound represented by Formula I, or a cis-trans isomer thereof, or a pharmaceutically acceptable salt or solvate thereof; 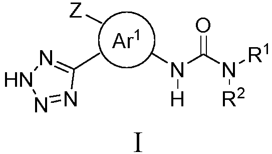 wherein:
R¹ and R² together with the N atom attached thereto form a 3- to 8-membered heterocyclic group containing at least 1 nitrogen atom, wherein the 3-to 8-membered heterocyclic group is substituted by 1 to 3 of identical or different functional groups A, and the functional group A is selected from the group consisting of hydroxyl, halogen, amino, cyano, nitro, C₁-C₃ alkyl, C₁-C₃ alkenyl, C₁-C₃ alkynyl, C₁-C₃ alkoxy, halogenated C₁-C₃ alkyl, C₁-C₃ fluoroalkoxy, alkylamino, dialkylamino, acetylamino or cycloamino; or
R¹ and R² together with the N atom attached thereto form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂, wherein the 8- to 12-membered bicyclic heterocyclic group comprises a spiro cycloalkyl, a bridged bicycloalkyl and a fused bicycloalkyl, and the 8- to 12-membered bicyclic heterocyclic group is substituted by 1 to 3 of identical or different functional groups B, and the functional group B is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino; or
each of R¹ and R² is independently selected from the group consisting of the following groups:
(i) H, C₁-C₁₀ alkyl, C₂-C₁₀ alkenyl, C₂-C₁₀ alkynyl, wherein the C₁-C₁₀ alkyl, C₂-C₁₀ alkenyl, or C₂-C₁₀ alkynyl is substituted by 1 to 3 of identical or different functional groups C, and the functional group C is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino; or
(ii) phenyl, 5- to 6-membered heteroaryl, C₅-C₇ cycloalkyl, 5- to 7-membered heterocyclic group, wherein the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, heteroatom or heteroatom-containing group is substituted by 1 to 3 of identical or different functional groups D, and the functional group D is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino or cycloamino;
Ar¹ is selected from the group consisting of:
phenyl, 5- to 6-membered heteroaryl, C₅-C₇ cycloalkyl, C₉-C₁₀ fused bicyclic group, wherein the 5- to 6-membered heteroaryl contains 1 or 2 heteroatoms or heteroatom-containing groups independently selected from the group consisting of NH, N, O, S, SO and SO₂, and the phenyl, heteroatom or heteroatom-containing group is substituted by 1 to 3 of identical or different functional groups E, and the functional group E is selected from the group consisting of hydroxyl, halogen, amino, cyano, nitro, C₁-C₆ alkyl, C₁-C₆- alkoxy, halogenated C₁-C₆ alkyl, C₁-C₆ fluoroalkoxy, alkylamino, dialkylamino, acetylamino, N-methyl-N-acetylamino or cycloamino;
Z is selected from the group consisting of the following groups:
(i) phenyl, 5- to 6-membered heteroaryl, C₅-C₇ cycloalkyl, 5- to 7-membered heterocyclic group, C₉-C₁₀ fused bicyclic group, 9- to 10-membered fused heterobicyclic group, wherein the 5- to 6-membered heteroaryl, 5- to 7-membered heterocyclic group or 9- to 10-membered fused heterobicyclic group contains 1, 2 or 3 heteroatoms or heteroatom-containing groups independently selected from the group consisting of NH, N, O, S, SO and SO₂, and the phenyl, heteroatom or heteroatom-containing group is substituted by 1 to 3 of identical or different functional groups F, and the functional group F is selected from the group consisting of hydroxyl, halogen, amino, cyano, nitro, C₁-C₆ alkyl, C₁-C₆ alkoxy, halogenated C₁-C₆ alkyl, C₁-C₆ fluoroalkoxy, alkylamino, dialkylamino, acetylamino, N-methyl-N-acetylamino and cycloamino, or alternatively, the functional groups F form a 5- to 8-membered heterocyclic ring; or
(ii) 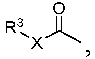 wherein X is selected from the group consisting of O or NH, and R³ is selected from the group consisting of C₁-C₆ alkyl, C₃-C₇ cycloalkyl, C₃-C₇ cycloalkenyl, 5- to 7-membered heterocyclic group, phenyl, 5- to 6-membered heteroaryl, and the C₁-C₆ alkyl, C₃-C₇ cycloalkyl, C₃-C₇ cycloalkenyl, 5- to 7-membered heterocyclic group, phenyl, or 5- to 6-membered heteroaryl is substituted by 1 to 3 of identical or different functional groups G, and the functional group G is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
(iii) 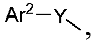 wherein Y is selected from the group consisting of O or NH, and Ar² is selected from the group consisting of phenyl, 5- to 6-membered heteroaryl, C₁₀-C₂₀ fused cyclic group, C₁-C₆ alkyl, 5- to 7-membered cycloalkyl, 5- to 7-membered heterocyclic group, C₁-C₆ alkoxy, wherein the phenyl, 5- to 6-membered heteroaryl, C₁₀-C₂₀ fused cyclic group, C₁-C₆ alkyl, 5- to 7-membered cycloalkyl, 5- to 7-membered heterocyclic group, or C₁-C₆ alkoxy is substituted by 1 to 3 of identical or different functional groups H, and the functional group H is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂.

3. The N-tetrazolyl aryl urea-based derivative according to claim 1 or 2, **characterized in that**: Ar¹ is selected from the group consisting of the following phenyl, pyridyl, pyrazinyl groups: 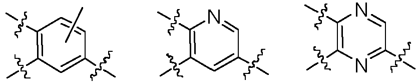 wherein, the group is substituted by 1 to 3 of identical or different functional groups I, and the functional group I is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino.

4. The N-tetrazolyl aryl urea-based derivative according to claim 1 or 2, **characterized in that**: which is a compound represented by Formula Ia or Ib, or a cis-trans isomer thereof, or a pharmaceutically acceptable salt thereof; 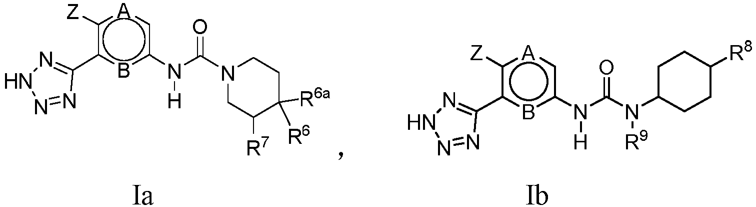 in the compound represented by Formula Ia:
each of A and B is independently a carbon atom or a nitrogen atom;
Z is selected from the group consisting of the following groups:
(i) phenyl, pyridyl, pyrazolyl, imidazolyl, indazolyl, wherein the group is substituted by 1 to 3 of identical or different functional groups J, and the functional group J is selected from the group consisting of hydroxyl, halogen, amino, cyano, nitro, C₁-C₆ alkyl, C₁-C₆ cycloalkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino, or alternatively, the functional groups J form a 5-membered heterocyclic ring; or
(ii) 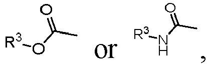 wherein R³ is selected from the group consisting of C₁-C₆ alkyl, C₁-C₆ cycloalkyl, C₃-C₇ cycloalkyl, C₃-C₇ aryl, 5- to 7-membered heterocyclic group, phenyl, 5- to 6-membered heteroaryl, wherein the C₁-C₆ alkyl, C₁-C₆ cycloalkyl, C₃-C₇ cycloalkyl, C₃-C₇ aryl, 5- to 7-membered heterocyclic group, phenyl, or 5- to 6-membered heteroaryl is substituted by 1 to 3 of identical or different functional groups K, and the functional group K is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
(iii) phenoxy, wherein the phenoxy is substituted by 1 to 3 of identical or different functional groups L, and the functional group L is selected from the group consisting of hydroxyl, halogen, amino, cyano, nitro, C₁-C₃ alkyl, C₁-C₃ alkoxy, halogenated C₁-C₃ alkyl, C₁-C₃ fluoroalkoxy, alkylamino, dialkylamino, acetylamino, N-methyl-N-acetylamino or cycloamino; or
(iv) 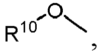 wherein R¹⁰ is selected from the group consisting of C₁-C₆ alkyl, 5- to 7-membered cycloalkyl, 5- to 6-membered heteroaryl, 5- to 7-membered heterocyclic group, wherein the C₁-C₆ alkyl, 5- to 7-membered cycloalkyl, 5- to 6-membered heteroaryl, or 5- to 7-membered heterocyclic group is substituted by 1 to 3 of identical or different functional groups M, and the functional group M is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂;
R^{6a} is a hydrogen atom, and R⁶ and R⁷ are independently selected from the group consisting of C₁-C₆ alkyl, C₁-C₆ cycloalkyl, C₃-C₁₀ cycloalkyl, wherein the C₁-C₆ alkyl, C₁-C₆ cycloalkyl, or C₃-C₁₀ cycloalkyl is substituted by 1 to 3 of identical or different functional groups N, and the functional group N is selected from the group consisting of hydroxyl, halogen, amino, cyano, nitro; or
R^{6a} is a hydrogen atom, and R⁶ and R⁷ together with the two carbon atoms attached thereto form a 4- to 8-membered cyclic group, or a 4- to 8-membered bicyclic heterocyclic group containing 1 heteroatom or heteroatom-containing group independently selected from the group consisting of NH, N, O, S, SO and SO₂, wherein the 4- to 8-membered bicyclic heterocyclic group is substituted by 1 to 3 of identical or different functional groups O, and the functional group O is selected from the group consisting of hydroxyl, halogen, amino, cyano, nitro; or
R⁷ is a hydrogen atom, and R⁶ and R^{6a} together with the two carbon atoms attached thereto form a 4- to 8-membered cyclic group, or a 4- to 8-membered heterocyclic group containing 1 heteroatom or heteroatom-containing group independently selected from the group consisting of NH, N, O, S, SO and SO₂, wherein the 4- to 8-membered cyclic group or the 4- to 8-membered heterocyclic group is substituted by 1 to 3 of identical or different functional groups P, and the functional group P is selected from the group consisting of hydroxyl, halogen, amino, cyano, nitro;
in the compound represented by Formula Ib:
each of A and B is independently a carbon atom or a nitrogen atom;
Z is selected from the group consisting of the following groups:
(i) phenyl, pyridyl, pyrazolyl, imidazolyl, indazolyl, wherein the phenyl, pyridyl, pyrazolyl, imidazolyl, or indazolyl is substituted by 1 to 3 of identical or different functional groups Q, and the functional group Q is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino; or
(ii) 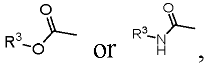 wherein R³ is selected from the group consisting of C₁-C₆ alkyl, C₁-C₆ cycloalkyl, C₃-C₁₀ cycloalkyl, C₃-C₁₀ aryl, wherein the C₁-C₆ alkyl, C₁-C₆ cycloalkyl, C₃-C₁₀ cycloalkyl, or C₃-C₁₀ aryl is substituted by 1 to 3 of identical or different functional groups R, and the functional group R is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino; or
(iii) phenoxy, wherein the phenoxy is substituted by 1 to 3 of identical or different functional groups S, and the functional group S is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino; or
(iv) 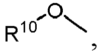 wherein R¹⁰ is selected from the group consisting of C₁-C₆ alkyl, 5- to 7-membered cycloalkyl, 5- to 6-membered heteroaryl, 5- to 7-membered heterocyclic group, wherein the C₁-C₆ alkyl, 5- to 7-membered cycloalkyl, 5- to 6-membered heteroaryl, or 5- to 7-membered heterocyclic group is substituted by 1 to 3 of identical or different functional groups T, and the functional group T is selected from the group consisting of hydroxyl, halogen, amino, cyano, nitro, C₁-C₆ alkyl, C₂-C₆ alkenyl, C₂-C₆ alkynyl, C₁-C₆ alkoxy, halogenated C₁-C₄ alkyl, C₁-C₄ fluoroalkoxy, alkylamino, dialkylamino, acetylamino, N-methyl-N-acetylamino or cycloamino, and the 5- to 6-membered heteroaryl or 5- to 7-membered heterocyclic group contains 1, 2 or 3 heteroatoms or heteroatom-containing groups independently selected from the group consisting of NH, N, O, S, SO and SO₂;
R⁸ is selected from the group consisting of C₁-C₆ alkyl, C₁-C₆ cycloalkyl, C₃-C₁₀ aryl, wherein the C₁-C₆ alkyl, C₁-C₆ cycloalkyl, or C₃-C₁₀ aryl is substituted by 1 to 3 of identical or different functional groups U, and the functional group U is selected from the group consisting of hydroxyl, halogen, amino, cyano, nitro;
R⁹ is selected from the group consisting of C₁-C₆ alkyl, C₁-C₆ cycloalkyl, C₃-C₁₀ aryl, wherein the C₁-C₆ alkyl, C₁-C₆ cycloalkyl, or C₃-C₁₀ aryl is substituted by 1 to 3 of identical or different functional groups V, and the functional group V is selected from the group consisting of hydroxyl, halogen, amino, cyano, and nitro.

5. The N-tetrazolyl aryl urea-based derivative according to claim 1 or 2, **characterized in that**:
R¹ and R² together with the N atom attached thereto form a 3- to 8-membered heterocyclic group containing at least 1 nitrogen atom, wherein the 3-to 8-membered heterocyclic group is optionally substituted by 1 to 3 of identical or different functional groups A, and the functional group A is selected from the group consisting of C₁-C₆ alkyl, C₁-C₆ alkoxy, halogenated C₁-C₆ alkyl and halogenated C₁-C₆ alkoxy; or
R¹ and R² together with the N atom attached thereto form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂, wherein the 8- to 12-membered bicyclic heterocyclic group comprises spiro cycloalkyl, bridged bicycloalkyl and fused bicycloalkyl, and the 8- to 12-membered bicyclic heterocyclic group is optionally substituted by 1 to 3 of identical or different functional groups B, and the functional group B is selected from the group consisting of C₁-C₆ alkyl, C₁-C₆ alkoxy, halogenated C₁-C₆ alkyl and halogenated C₁-C₆ alkoxy; or
each of R¹ and R² is independently selected from the group consisting of the following groups:
(i) H and C₁-C₆ alkyl, wherein C₁-C₆ alkyl is optionally substituted by 1 to 3 of identical or different functional groups C, and the functional group C is selected from the group consisting of C₁-C₆ alkoxy and halogenated C₁-C₆ alkoxy;
(ii) phenyl and C₅-C₇ cycloalkyl, wherein the phenyl or C₅-C₇ cycloalkyl is optionally substituted by 1 to 3 of identical or different functional groups D, and the functional group D is selected from the group consisting of C₁-C₆ alkyl, C₁-C₆ alkoxy, halogenated C₁-C₆ alkyl and halogenated C₁-C₆ alkoxy;
Ar¹ is selected from the group consisting of phenyl and 5- to 6-membered heteroaryl, wherein the 5- to 6-membered heteroaryl contains 1 or 2 heteroatoms or heteroatom-containing groups independently selected from the group consisting of NH, N, O, S, SO and SO₂, and the phenyl or 5- to 6-membered heteroaryl is optionally substituted by 1 to 3 of identical or different functional groups E, and the functional group E is selected from the group consisting of halogen, C₁-C₆ alkyl, C₁-C₆ alkoxy, halogenated C₁-C₆ alkyl and halogenated C₁-C₆ alkoxy;
Z is selected from the group consisting of phenyl, 5- to 6-membered heteroaryl, 5- to 7-membered heterocyclic group, 9- to 10-membered fused bicyclic heterocyclic groups, 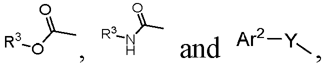
wherein the 5- to 6-membered heteroaryl, 5- to 7-membered heterocyclic group, or 9- to 10-membered fused bicyclic heterocyclic groups contains 1, 2 or 3 heteroatoms or heteroatom-containing groups independently selected from the group consisting of NH, N, O, S, SO and SO₂, and the phenyl, 5- to 6-membered heteroaryl, 5- to 7-membered heterocyclic group or 9- to 10-membered fused bicyclic heterocyclic group is optionally substituted by 1 to 3 of identical or different functional groups F, and the functional group F is selected from the group consisting of halogen, C₁-C₆ alkyl, C₃-C₇ cycloalkyl, C₁-C₆ alkoxy, halogenated C₁-C₆ alkyl and halogenated C₁-C₆ alkoxy, or the functional group F forms a 5- to 8-membered heterocyclic ring fused to a phenyl;
R³ is selected from the group consisting of C₁-C₆ alkyl, C₃-C₇ cycloalkyl, 5-to 7-membered heterocyclic group and 5- to 6-membered heteroaryl, and the C₁-C₆ alkyl, C₃-C₇ cycloalkyl or 5- to 6-membered heteroaryl is optionally substituted by 1 to 3 of identical or different functional groups G, and the functional group G is selected from the group consisting of C₁-C₆ alkyl, C₁-C₆ alkoxy, halogenated C₁-C₆ alkyl and halogenated C₁-C₆ alkoxy,
Y is O, and Ar² is selected from the group consisting of phenyl, 5- to 6-membered heteroaryl and 5- to 7-membered heterocyclic group, the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, 5- to 6-membered heteroaryl or 5- to 7-membered heterocyclic group is optionally substituted by 1 to 3 of identical or different functional groups H, and the functional group H is selected from the group consisting of C₁-C₆ alkyl, C₁-C₆ alkoxy, halogenated C₁-C₆ alkyl and halogenated C₁-C₆ alkoxy.

6. The N-tetrazolyl aryl urea-based derivative according to claim 5, **characterized in that**:
R¹ and R² together with the N atom attached thereto form a 3- to 8-membered heterocyclic group containing at least 1 nitrogen atom, wherein the 3-to 8-membered heterocyclic group is optionally substituted by 1 to 3 of identical or different functional groups A, and the functional group A is selected from the group consisting of C₁-C₆ alkyl and halogenated C₁-C₆ alkyl; or
R¹ and R² together with the N atom attached thereto form an 8- to 12-membered bicyclic heterocyclic group containing at least 1 nitrogen atom and additionally containing 0, 1 or 2 heteroatoms or heteroatom-containing groups independently selected from the group consisting of NH, N, O, S, SO and SO₂, wherein the 8- to 12-membered bicyclic heterocyclic group comprises spiro cycloalkyl and fused bicycloalkyl; or
R¹ is selected from the group consisting of H and C₁-C₆ alkyl; and R² is selected from the group consisting of phenyl and C₅-C₇ cycloalkyl, wherein the phenyl or C₅-C₇ cycloalkyl is optionally substituted by 1 to 3 of identical or different functional groups D, and the functional group D is selected from the group consisting of C₁-C₆ alkyl and halogenated C₁-C₆ alkyl;
Ar¹ is selected from the group consisting of phenyl and 5- to 6-membered heteroaryl, wherein the 5- to 6-membered heteroaryl contains 1 or 2 heteroatoms or heteroatom-containing groups independently selected from the group consisting of NH, N, O, S, SO and SO₂, and the phenyl is optionally substituted by 1 to 3 of identical or different halogens;
Z is selected from the group consisting of phenyl, 5- to 6-membered heteroaryl, 5- to 7-membered heterocyclic group, 9- to 10-membered fused bicyclic heterocyclic group, 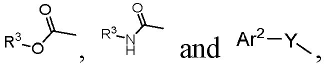
wherein the 5- to 6-membered heteroaryl, 5- to 7-membered heterocyclic group, or 9- to 10-membered fused bicyclic heterocyclic group contains 1, 2 or 3 heteroatoms or heteroatom-containing groups independently selected from the group consisting of NH, N, O, S, SO and SO₂, and the phenyl, 5- to 6-membered heteroaryl, 5- to 7-membered heterocyclic group or 9- to 10-membered fused bicyclic heterocyclic group is optionally substituted by 1 to 3 of identical or different functional groups F, and the functional group F is selected from the group consisting of halogen, C₁-C₆ alkyl, C₃-C₇ cycloalkyl, C₁-C₆ alkoxy and halogenated C₁-C₆ alkyl, or the functional group F forms a 5- to 8-membered heterocyclic ring fused to a phenyl;
R³ is selected from the group consisting of C₁-C₆ alkyl, C₃-C₇ cycloalkyl, 5-to 7-membered heterocycloalkyl and 5- to 6-membered heteroaryl, and the C₃-C₇ cycloalkyl, 5- to 7-membered heterocycloalkyl or 5- to 6-membered heteroaryl is optionally substituted by 1 to 3 of identical or different functional groups G, and the functional group G is selected from the group consisting of C₁-C₆ alkyl;
Ar² is selected from the group consisting of phenyl, 5- to 6-membered heteroaryl and 5- to 7-membered heterocyclic group, the 5- to 6-membered heteroaryl or 5- to 7-membered heterocyclic group contains 1, 2 or 3 heteroatoms or heteroatom-containing groups independently selected from the group consisting of NH, N, O, S, SO and SO₂, and the phenyl, 5- to 6-membered heteroaryl or 5- to 7-membered heterocyclic group is optionally substituted by 1 to 3 of identical or different substituents selected from the group consisting of C₁-C₆ alkyl and C₁-C₆ alkoxy.

7. The N-tetrazolyl aryl urea-based derivative according to claim 5, **characterized in that**:
R¹ and R² together with the N atom attached thereto form a piperidyl, and the piperidyl is optionally substituted by substituent(s) selected from the group consisting of methyl, tert-butoxy and trifluoromethyl; or
R¹ and R² together with the N atom attached thereto form an azaoxaspirodecyl or an octahydrofuranopyridyl;
R¹ is selected from the group consisting of H and methyl; and R² is selected from the group consisting of phenyl and cyclohexyl, the phenyl is optionally substituted by trifluoromethyl, and the cyclohexyl is optionally substituted by methyl or tert-butyl;
Ar¹ is selected from the group consisting of phenyl and pyridyl, and the phenyl is optionally substituted by a fluorine atom;
Z is selected from the group consisting of phenyl, phenoxy, pyridyl, indazolyl, imidazolyl, tetrahydropyranyl, tetrahydropyranyloxy, dihydropyranyl, 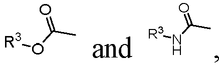 the phenyl, pyridyl, indazolyl, imidazolyl, tetrahydropyranyl or dihydropyranyl is optionally substituted by 1 to 3 of identical or different functional groups F, and the functional group F is selected from the group consisting of methyl, tert-butyl, methoxy, ethoxy, isopropoxy, cyclobutyl, -Cl, difluoromethyl and trifluoromethyl, or the functional group F forms a dioxolane ring fused to a phenyl, and the phenoxy is optionally substituted by 1 to 2 methoxy groups,
R³ is selected from the group consisting of ethyl, cyclopentyl, cyclohexyl, tetrahydrofuranyl, and pyridyl optionally substituted by methyl.

8. The N-tetrazolyl aryl urea-based derivative according to claim 1 or 2, **characterized in that**: which is a compound represented by Formula Ia, Ib or Ic, or a cis-trans isomer thereof, or a pharmaceutically acceptable salt thereof; 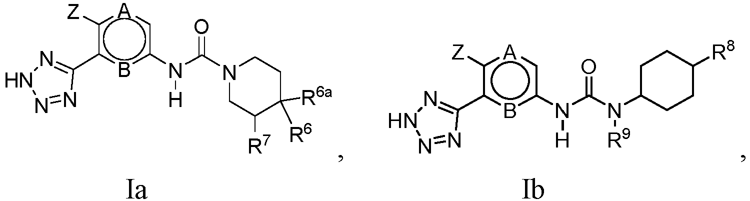 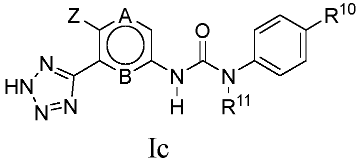 in the compound represented by Formula Ia:
each of A and B is independently a carbon atom or a nitrogen atom;
Z is selected from the group consisting of phenyl, pyridyl, indazolyl, imidazolyl, tetrahydropyranyl, dihydropyranyl, 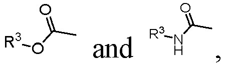 the phenyl, pyridyl, indazolyl, imidazolyl, tetrahydropyranyl or dihydropyranyl is optionally substituted by 1 to 3 of identical or different functional groups F, and the functional group F is selected from the group consisting of methyl, tert-butyl, methoxy, ethoxy, isopropoxy, cyclobutyl, -Cl, difluoromethyl and trifluoromethyl, or the functional group F forms a dioxolane ring fused to a phenyl, R³ is selected from the group consisting of ethyl, cyclopentyl, cyclohexyl, tetrahydrofuranyl, and pyridyl optionally substituted by methyl;
each of R⁷, R^{6a} and R⁶ is independently selected from the group consisting of H, methyl, tert-butoxy and trifluoromethyl; or
R⁷ is a hydrogen atom, R⁶ and R^{6a} together with the carbon atoms attached thereto form an oxolane ring; or
R^{6a} is a hydrogen atom, R⁶ and R⁷ together with the carbon atoms attached thereto form an oxolane ring;
in the compound represented by Formula Ib:
each of A and B is independently a carbon atom or a nitrogen atom;
Z is selected from the group consisting of phenyl, phenoxy, pyridyl, dihydropyranyl, tetrahydropyranyl and tetrahydropyranyloxy, wherein the phenyl or phenoxy is optionally substituted by 1 to 2 methoxy groups, or optionally fused to a dioxolane ring, the pyridyl is optionally substituted by an ethoxy;
R⁸ is tert-butyl or methyl;
R⁹ is H or methyl;
in the compound represented by Formula Ic:
each of A and B is independently a carbon atom or a nitrogen atom;
Z is selected from the group consisting of phenyl, phenoxy, pyridyl, dihydropyranyl, tetrahydropyranyl and tetrahydropyranyloxy, wherein the phenyl or phenoxy is optionally substituted by 1 to 2 methoxy groups, or optionally fused to a dioxolane ring, the pyridyl is optionally substituted by an ethoxy;
R¹⁰ is trifluoromethyl;
R¹¹ is H.

9. The N-tetrazolyl aryl urea-based derivative according to claim 1 or 2, **characterized in that**: which is selected from the group consisting of the following compounds, or a cis-trans isomer thereof, or a pharmaceutically acceptable salt or solvate thereof;
4-(tert-butyl)-N-(3',4'-dimethoxy-2-(2H-tetrazol-5-yl)-[1,1'-biphenyl]-4-yl)piperidine-1-carboxamide,
N-(3',4'-dimethoxy-2-(2H-tetrazol-5-yl)-[1,1'-biphenyl]-4-yl)-4-(trifluoromethyl)piperidine-1-carboxamide,
N-(3',4'-dimethoxy-2-(2H-tetrazol-5-yl)-[1,1'-biphenyl]-4-yl)-1-oxy-8-azaspiro[4.5]decane-1-carboxamide,
N-(3',4'-dimethoxy-2-(2H-tetrazol-5-yl)-[1,1'-biphenyl]-4-yl)hexahydrofuro[2,3-c]pyridine-6(2H)-carboxamide,
N-(4-(6-ethoxypyrid-3-yl)-3-(2H-tetrazol-5-yl)phenyl)-4-methylpiperidine-1-carboxamide,
4-(tert-butyl)-N-(4-(6-ethoxypyrid-3-yl)-3-(2H-tetrazol-5-yl)phenyl)piperidine-1-carboxamide,
4-(tert-butyl)-N-(6'-ethoxy-3-(2H-tetrazol-5-yl)-[2,3'-bipyridyl]-5-yl)piperidine-1-carboxamide,
4-(tert-butyl)-N-(4-(6-ethoxypyrid-3-yl)-3-fluoro-5-(2H-tetrazol-5-yl)phenyl)piperidine-1-carboxamide,
4-(tert-butyl)-N-(4-(1-cyclobutyl-1H-pyrazol-4-yl)-3-(2H-tetrazol-5-yl)phenyl)piperidine-1-carboxamide,
4-(tert-butyl)-N-(4-(1-methyl-1H-indazol-5-yl)-3-(2H-tetrazol-5-yl)phenyl)piperidine-1-carboxamide,
4-(tert-butyl)-N-(2-fluoro-3',4'-dimethoxy-6-(2H-tetrazol-5-yl)-[1,1'-biphenyl]-4-yl)piperidine-1-carboxamide,
N-(3',4'-dimethoxy-2-(2H-tetrazol-5-yl)-[1,1'-biphenyl]-4-yl)-4,4-dimethylpiperidine-1-carboxamide,
4-(tert-butyl)-N-(4'-methoxy-3'-methyl-2-(2H-tetrazol-5-yl)-[1,1'-biphenyl]-4-yl)piperidine-1-carboxamide,
4-(tert-butyl)-N-(3'-chloro-4'-methoxy-2-(2H-tetrazol-5-yl)-[1,1'-biphenyl]-4-yl)piperidine-1-carboxamide,
cyclohexyl 4-(4-(tert-butyl)piperidine-1-formamido)-2-(2H-tetrazol-5-yl)benzoate,
cyclohexyl 4-(4-(tert-butyl)piperidine-1-formamido)-2-fluoro-6-(2H-tetrazol-5-yl)benzoate,
ethyl 4-(4-(tert-butyl)piperidine-1-formamido)-2-fluoro-6-(2H-tetrazol-5-yl)benzoate,
4-(tert-butyl)-N-(3-fluoro-5-(2H-tetrazol-5-yl)-4-(6-(trifluoromethyl)pyrid-3-yl)phenyl)piperidine-1-carboxamide,
4-(tert-butyl)-N-(3-fluoro-5-(2H-tetrazol-5-yl)-4-(6-(trifluoromethyl)pyrid-3-yl)phenyl)piperidine-1-carboxamide,
N-(2-fluoro-3',4'-methoxy-6-(2H-tetrazol-5-yl)-[1,1'-biphenyl]-4-yl)-4-methylpiperidine-1-carboxamide,
4-methyl-N-(4-(1-methyl-1H-indazol-5-yl)-3-(2H-tetrazol-5-yl)phenyl)piperidine-1-carboxamide,
4-methyl-N-(4-(1-methyl-1H-indazol-5-yl)-3-(2H-tetrazol-5-yl)phenyl)piperidine-1-carboxamide,
cyclohexyl 2-fluoro-4-(4-methylpiperidine-1-formamido)-6-(2H-tetrazol-5-yl)benzoate,
ethyl 2-fluoro-4-(4-methylpiperidine-1-formamido)-6-(2H-tetrazol-5-yl)benzoate,
N-(4-(6-ethoxypyrid-3-yl)-3-fluoro-5-(2H-tetrazol-5-yl)phenyl)-4-(trifluoromethyl)piperidine-1-carboxamide,
N-(2-fluoro-3',4'-methoxy-6-(2H-tetrazol-5-yl)-[1,1'-biphenyl]-4-yl)-4-(trifluoromethyl)piperidine-1-carboxamide,
N-(4-(1-methyl-1H-indazol-5-yl)-3-(2H-tetrazol-5-yl)phenyl)-4-(trifluoromethyl)piperidine-1-carboxamide,
N-(3'-chloro-4'-methoxy-2-(2H-tetrazol-5-yl)-[1,1'-biphenyl]-4-yl)-4-(trifluoromethyl)piperidine-1-carboxamide,
cyclohexyl 2-fluoro-6-(2H-tetrazol-5-yl)-4-(4-(trifluoromethyl)piperidine-1-formamido)benzoate,
ethyl 2-fluoro-6-(2H-tetrazol-5-yl)-4-(4-(trifluoromethyl)piperidine-1-formamido)benzoate,
N-(4-(6-ethoxypyrid-3-yl)-3-fluoro-5-(2H-tetrazol-5-yl)phenyl)-4-methylpiperidine-1-carboxamide,
4-(tert-butyl)-N-(3-fluoro-4-(1-methyl-1H-indazol-5-yl)-5-(2H-tetrazol-5-yl)phenyl)piperidine-1-carboxamide,
4-(tert-butyl)-N-(3'-chloro-2-fluoro-4'-methoxy-6-(2H-tetrazol-5-yl)-[1,1'-biphenyl]-4-yl)piperidine-1-carboxamide,
N-(3-fluoro-4-(1-methyl-1H-indazol-5-yl)-5-(2H-tetrazol-5-yl)phenyl)-4-(trifluoromethyl)piperidine-1-carboxamide,
4-(tert-butyl)-N-(6-(3,4-dimethoxyphenyl)-5-(2H-tetrazol-5-yl)pyrid-3-yl)piperidine-1-carboxamide,
4-(tert-butyl)-N-(6-(1-methyl-1H-indazol-5-yl)-5-(2H-tetrazol-5-yl)pyrid-3-yl)piperidine-1-carboxamide,
ethyl 5-(4-(tert-butyl)piperidine-1-formamido)-3-(2H-tetrazol-5-yl)pyridylcarboxylate,
cyclohexyl 5-(4-(tert-butyl)piperidine-1-formamido)-3-(2H-tetrazol-5-yl)pyridylcarboxylate,
N-(4-(benzo[d][1,3]dioxolan-5-yl)-3-(2H-tetrazol-5-yl)phenyl)-4-(tert-butyl)piperidine-1-carboxamide,
4-(tert-butyl)-N-(4-(tetrahydro-2H-pyran-4-yl)-3-(2H-tetrazol-5-yl)phenyl)piperidine-1-carboxamide,
4-(tert-butyl)-N-(4-(3,4-dihydro-2H-pyran-4-yl)-3-(2H-tetrazol-5-yl)phenyl)piperidine-1-carboxamide,
4-(tert-butyl)-N-(4-(6-(difluoromethyl)pyrid-3-yl)-3-(2H-tetrazol-5-yl)phenyl)piperidine-1-carboxamide,
cyclopentyl 4-(4-(tert-butyl)piperidine-1-formamido)-2-(2H-tetrazol-5-yl)benzoate,
tetrahydrofuran-3-yl 4-(4-(tert-butyl)piperidine-1-formamido)-2-(2H-tetrazol-5-yl)benzoate,
ethyl 4-(4-(tert-butyl)piperidine-1-formamido)-2-(2H-tetrazol-5-yl)benzoate,
4-(tert-butyl)-N-(4-(cyclopentylaminoformyl)-3-(2H-tetrazol-5-yl)phenyl)piperidine-1-carboxamide,
4-(tert-butyl)-N-(4-((5-methylpyridin-2-yl)aminoformyl)-3-(2H-tetrazol-5-yl)phenyl)piperidine-1-carboxamide,
4-(tert-butyl)-N-(4-(5-methoxypyrid-3-yl)-3-(2H-tetrazol-5-yl)phenyl)piperidine-1-carboxamide,
4-(tert-butyl)-N-(4-(6-isopropoxypyrid-3-yl)-3-(2H-tetrazol-5-yl)phenyl)piperidine-1-carboxamide,
4-(tert-butyl)-N-(4-(5-methoxypyrid-3-yl)-3-(2H-tetrazol-5-yl)phenyl)piperidine-1-carboxamide,
4-(tert-butyl)-N-(4-(6-methylpyrid-3-yl)-3-(2H-tetrazol-5-yl)phenyl)piperidine-1-carboxamide,
4-(tert-butyl)-N-(4-(2-methylpyridin-4-yl)-3-(2H-tetrazol-5-yl)phenyl)piperidine-1-carboxamide,
4-(tert-butyl)-N-(4-(cyclopentylaminoformyl)-3-fluoro-5-(2H-tetrazol-5-yl)phenyl)piperidine-1-carboxamide,
4-(tert-butyl)-N-(3-fluoro-4-((5-methylpyridin-2-yl)aminoformyl)-5-(2H-tetrazol-5-yl)phenyl)piperidine-1-carboxamide,
4-(tert-butyl)-N-(3-fluoro-4-(6-methylpyrid-3-yl)-5-(2H-tetrazol-5-yl)phenyl)piperidine-1-carboxamide,
4-(tert-butyl)-N-(3-fluoro-4-(6-isopropoxypyrid-3-yl)-5-(2H-tetrazol-5-yl)phenyl)piperidine-1-carboxamide,
4-(tert-butyl)-N-(4-(4-(tert-butyl)-1H-imidazol-1-yl)-3-fluoro-5-(2H-tetrazol-5-yl)phenyl)piperidine-1-carboxamide,
4-(tert-butyl)-N-(4-(4-(tert-butyl)-1H-imidazol-1-yl)-3-(2H-tetrazol-5-yl)phenyl)piperidine-1-carboxamide,
1-(4-(tert-butyl)cyclohexyl)-3-(3',4'-dimethoxy-2-(2H-tetrazol-5-yl)-[1,1'-biphenyl]-4-yl)urea,
1-(3',4'-dimethoxy-2-(2H-tetrazol-5-yl)-[1,1'-biphenyl]-4-yl)-3-(4-(trifluoromethyl)phenyl)urea,
1-(3',4'-dimethoxy-2-(2H-tetrazol-5-yl)-[1,1'-biphenyl]-4-yl)-3-((cis)-4-methylcyclohexyl)urea,
1-(3',4'-dimethoxy-2-(2H-tetrazol-5-yl)-[1,1'-biphenyl]-4-yl)-3-((trans)-4-methylcyclohexyl)urea,
1-(4-(3,4-dimethoxyphenoxy)-3-(2H-tetrazol-5-yl)phenyl)-3-((cis)-4-methylcyclohexyl)urea,
1-(4-(3,4-dimethoxyphenoxy)-3-(2H-tetrazol-5-yl)phenyl)-3-((trans)-4-methylcyclohexyl)urea,
1-((trans)-4-(tert-butyl)cyclohexyl)-3-(3',4'-dimethoxy-2-(2H-tetrazol-5-yl)-[1,1'-biphenyl]-4-yl)-1-methylurea,
1-(4-(3,6-dihydro-2H-pyran-4-yl)-3-(2H-tetrazol-5-yl)phenyl)-3-((trans)-4-methylcyclohexyl)urea,
1-((cis)-4-methylcyclohexyl)-3-(4-(tetrahydro-2H-pyran-4-yl)-3-(2H-tetrazol-5-yl)phenyl)urea,
1-((trans)-4-methylcyclohexyl)-3-(4-(tetrahydro-2H-pyran-4-yl)-3-(2H-tetrazol-5-yl)phenyl)urea,
1-(4-(benzo[d][1,3]dioxolan-5-yl)-3-(2H-tetrazol-5-yl)phenyl)-3-((trans)-4-methylcyclohexyl)urea,
1-(4-(benzo[d][1,3]dioxolan-5-yl)-3-(2H-tetrazol-5-yl)phenyl)-3-((cis)-4-(tert-butyl)cyclohexyl)urea,
1-(4-(6-ethoxypyrid-3-yl)-3-(2H-tetrazol-5-yl)phenyl)-3-((trans)-4-methylcyclohexyl)urea,
1-(cis)-4-methylcyclohexyl-3-(4-(tetrahydropyran-4-yl)oxy)-3-(2H-tetrazol-5-yl)phenyl)urea,
1-(trans)-4-methylcyclohexyl-3-(4-tetrahydropyran-4-yl)oxy)-3-(2H-tetrazol-5-yl)phenyl)urea.

10. A method for preparing the N-tetrazolyl aryl urea-based derivative according to claim 1 or 2, **characterized in that**:
for the compound represented by Formula I, wherein R¹ and R² are non-hydrogen groups, or R¹ and R² together with the N atom attached thereto form a heterocyclic group containing at least 1 nitrogen atom, the method for preparing comprises the steps of:
Step 1: coupling 2-halogenated-5-nitroaromatic carbonitrile represented by Formula a with Z-B(OH)₂ boric acid represented by Formula b under an alkaline condition to obtain a compound represented by Formula c;
Step 2: subjecting the cyano group in the compound represented by Formula c to addition with sodium azide to obtain a tetrazolyl intermediate represented by Formula d;
Step 3: performing Trt-protection to obtain an intermediate represented by Formula e;
Step 4: reducing the nitro group to obtain an amino compound represented by Formula f;
Step 5: converting the amino compound to an isocyanate compound represented by Formula g;
Step 6: reacting the isocyanate compound with an amine to produce a urea compound represented by Formula h;
Step 7: subjecting Trt-deprotection to the urea represented by Formula h to obtain the target compound; 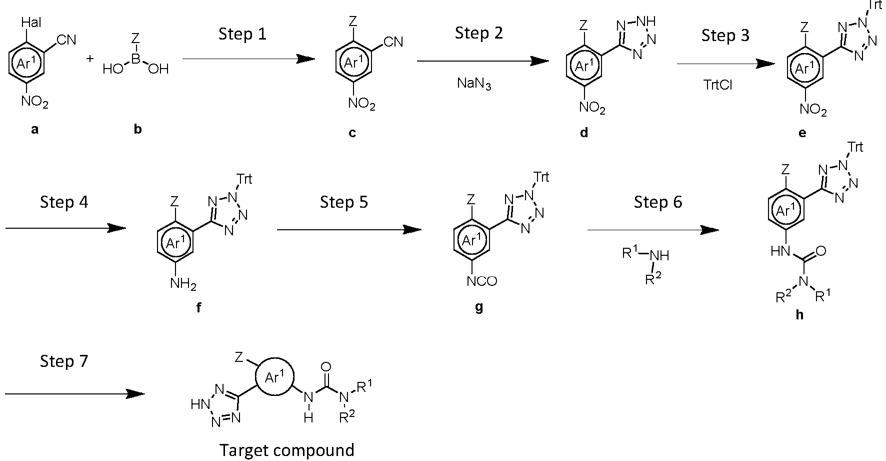 or
for the compound represented by Formula I, wherein Z is substituted or unsubstituted phenoxy, the method for preparing comprises the steps of:
subjecting the 2-halogenated-5-nitroaromatic carbonitrile represented by Formula a to a nucleophilic substitution reaction with a substituted phenol represented by Formula b2 under an alkaline condition to obtain a phenol ether-based intermediate represented by Formula c2;
and performing Steps 2 to 7 as described above to finally obtain the corresponding target compound; 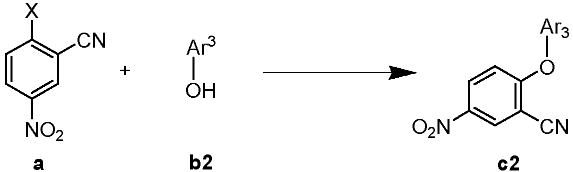 or
for the compound represented by Formula I, wherein Z is 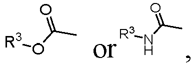 the method for preparing comprises the steps of:
performing Steps 2 to 7 as described above by replacing the compound represented by Formula c obtained in Step 1 as described above with 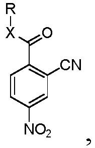 to finally obtain the target product;
or
for the compound represented by Formula I, wherein one of R¹ or R² is a hydrogen atom, the method for preparing comprises the steps of:
performing Steps 1 to 4 as described above to obtain the amino intermediate represented by Formula f;
reacting the amino intermediate directly with an isocyanate compound to produce a urea compound represented by Formula hl;
subjecting Trt-deprotection to the urea represented by Formula h1 to obtain the target compound;

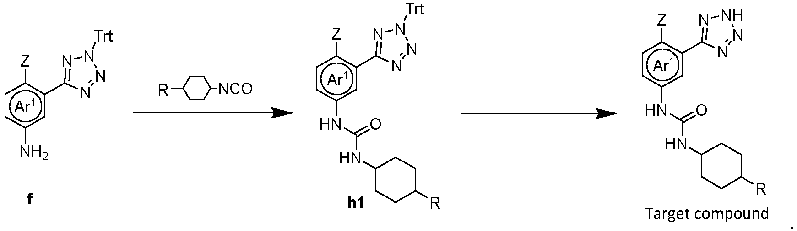

11. A pharmaceutical composition comprising the N-tetrazolyl aryl urea-based derivative according to any one of claims 1 to 9, a pharmaceutically acceptable carrier or excipient, and optionally other therapeutic agents.

12. The pharmaceutical composition according to claim 11, wherein the pharmaceutical composition is an injection, an oral preparation, or an eye drop.

13. Use of the N-tetrazolyl aryl urea-based derivative according to any one of claims 1 to 9 in the preparation of a bradykinin B1 receptor antagonist.

14. Use of the N-tetrazolyl aryl urea-based derivative according to any one of claims 1 to 9 alone or in combination with other drugs in the preparation of a medicament for preventing or treating a disease mediated by the bradykinin B1 receptor.

15. The use according to claim 14, **characterized in that**: the disease or a syndrome, condition or symptom thereof is related to an inflammatory response caused by an infectious disease, including pneumonia, pulmonary edema and acute respiratory distress syndrome caused by COVID-19 infection, pneumonia and type I hypersensitivity caused by respiratory syncytial virus, bacterial sepsis and shock caused by sepsis; or intestinal inflammation, including colitis; or complications caused by diabetes, including retinal edema and lesions, macular degeneration, neuralgia, diabetic hand and foot ulcers caused by diabetes; or ophthalmic inflammatory diseases.

16. The use according to claim 15, **characterized in that**: the disease is selected from the group consisting of acute pneumonia, pulmonary edema and acute respiratory distress syndrome caused by COVID-19, diabetic retinopathy, age-related macular degeneration, diabetic neuralgia, allergic conjunctivitis, chronic conjunctivitis and uveitis.

17. The use according to claim 13 or 14, **characterized in that**: the bradykinin B1 receptor antagonist or the medicament comprises at least one active ingredient and one or more pharmaceutically acceptable carriers or excipients; the active ingredient is at least one of the compound represented by Formula I, a cis-trans isomer thereof, and a pharmaceutically acceptable salt or solvate thereof.

18. The use according to claim 17, **characterized in that**: the pharmaceutically acceptable salt is an alkali metal salt, an alkaline earth metal salt or an ammonium salt.

19. The use according to claim 18, **characterized in that**: the alkali metal salt is a sodium salt, a potassium salt or a lithium salt; the alkaline earth metal salt is a calcium salt or a magnesium salt.

20. The use according to claim 19, **characterized in that**: the pharmaceutically acceptable salt is a sodium salt or a potassium salt.

21. The use according to claim 17, **characterized in that**: the carrier or excipient comprises one or more of conventional diluents, fillers, adhesives, wetting agents, disintegrants, absorption promoters, surfactants, adsorption carriers, lubricants, flavoring agents and sweeteners in the pharmaceutical field.

22. The use according to claim 17, **characterized in that**: the bradykinin B1 receptor antagonist or the medicament is in the form of tablets, capsules, patches, emulsions, suspensions, gels, powders, granules, oral solutions, eye drops or injections.
